(19) Europäisches Patentamt
European Patent Office
Office européen des brevets

(11) EP 4 365 172 A1

(12) EUROPEAN PATENT APPLICATION
published in accordance with Art. 153(4) EPC

(43) Date of publication:
**08.05.2024 Bulletin 2024/19**

(21) Application number: **22833161.7**

(22) Date of filing: **28.06.2022**

(51) International Patent Classification (IPC):
***C07D 413/04*** (2006.01) ***C07D 413/14*** (2006.01)
***A01P 7/02*** (2006.01) ***A01P 7/04*** (2006.01)
***A01N 43/824*** (2006.01)

(52) Cooperative Patent Classification (CPC):
**A01N 43/82; A01P 7/02; A01P 7/04; C07D 413/04; C07D 413/14**

(86) International application number:
**PCT/JP2022/025756**

(87) International publication number:
**WO 2023/277014 (05.01.2023 Gazette 2023/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.06.2021 JP 2021107327**

(71) Applicant: **Sumitomo Chemical Company, Limited**
**Tokyo 103-6020 (JP)**

(72) Inventors:
- **TASHIRO, Masayuki**
**Takarazuka-shi, Hyogo 665-8555 (JP)**
- **MURAKAMI, Shinichiro**
**Tokyo 103-6020 (JP)**
- **SHIODA, Takayuki**
**Takarazuka-shi, Hyogo 665-8555 (JP)**
- **MINEGISHI, Hidemitsu**
**Takarazuka-shi, Hyogo 665-8555 (JP)**
- **TANABE, Takamasa**
**Tokyo 103-6020 (JP)**

(74) Representative: **Vossius & Partner**
**Patentanwälte Rechtsanwälte mbB**
**Siebertstrasse 3**
**81675 München (DE)**

(54) **HETEROCYCLIC COMPOUND AND HARMFUL-ARTHROPOD-CONTROLLING COMPOSITION CONTAINING SAME**

(57) The present invention provides a compound that has an excellent control effect on harmful arthropods, and is represented by formula (I) [wherein $Z^1$ and $Z^2$ represent an oxygen atom, etc., $G^1$ represents $CR^{1a}$, etc., $G^2$ represents $CR^{1C}$, etc., $G^3$ represents $CR^{1d}$, etc., Q represents a C6-C10 aryl group, etc., $R^{1b}$ represents a C1-C6 chain hydrocarbon group, etc., and $R^{1a}$, $R^{1c}$ and $R^{1d}$ represent a C1-C6 chain hydrocarbon group, etc.] or its N-oxide, or salts thereof.

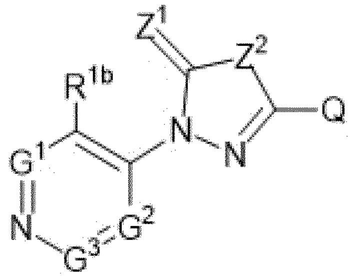

(I)

EP 4 365 172 A1

## Description

TECHNICAL FIELD

This application claims priority to and the benefit of Japanese Patent Application No. 2021-107327 filed June 29, 2021, the entire contents of which are incorporated herein by reference.

The present invention relates to a heterocyclic compound and a composition for controlling harmful arthropods comprising the same.

BACKGROUND ART

To date, in order to control harmful arthropods, some compounds have been studied. For example, a certain class of compound has been known to have an effect on controlling pests (see Patent Document 1).

CITATION LIST

PATENT DOCUMENT

Patent Document 1: NO 2006/044552

SUMMARY OF THE INVENTION

(PROBLEMS TO BE SOLVED BY INVENTION)

An object of the present invention is to provide a compound having an excellent efficacy for controlling harmful arthropods.

(MEANS TO SOLVE PROBLEMS)

The present inventors have intensively studied to find compounds having an excellent efficacy for controlling harmful arthropods, and as a result, found that a compound represented by the below-mentioned formula (I) has an excellent efficacy for controlling harmful arthropods.

That is, the present invention includes the followings.

[1] A compound represented by formula (I):

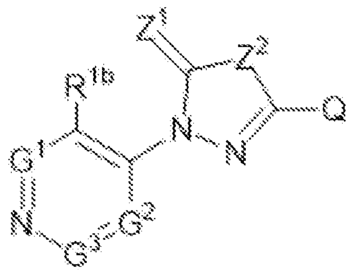

(I)

wherein

$Z^1$ and $Z^2$ are identical or different from each other and each represents an oxygen atom or a sulfur atom,
a combination of $G^1$, $G^2$, and $G^3$ represents
a Combination wherein $G^1$ represents a nitrogen atom, $G^2$ represents $CR^{1c}$, and $G^3$ represents $CR^{1d}$;
a combination wherein $G^1$ represents $CR^{1a}$, $G^2$ represents a nitrogen atom or $CR^1$, $G^3$ represents $CR^{1d}$**;** or
a combination wherein $G^1$ represents $CR^1$, $G^2$ represents $CR^1$, and $G^3$ represents a nitrogen atom,
$R^{1b}$ represents a C1-C6 chain hydrocarbon group, a C1-C6 alkoxy group (the C1-C6 chain hydrocarbon group and the C1-C6 alkoxy group may be optionally substituted with one or more substituents selected from Group A), a C3-C7 cycloalkyl group, a C1-C6 alkylsulfanyl group, a C1-C6 alkylsulfinyl group, a C1-C6 alkylsulfonyl group (the C3-C7 cycloalkyl group, the C1-C6 alkylsulfanyl group, the C1-C6 alkylsulfinyl group and the C1-C6 alkylsulfonyl group may be optionally substituted with one or more substituents selected from Group B), a C3-C7 cycloalkenyl groups which may be optionally substituted with one or more substituents selected from Group C, a phenyl group, a three- to seven-membered nonaromatic heterocyclic group {the phenyl group and the three- to seven- membered nonaromatic heterocyclic group may be optionally substituted with one or more substituents selected from Group D, a nitro group, $NR^2R^3$ , or a halogen atom,

$R^{1c}$ represents a C1-C6 chain hydrocarbon group, a C1-C6 alkoxy group (the C1-C6 chain hydrocarbon group and the C1-C6 alkoxy group may be optionally substituted with one or more substituents selected from Group A), a C3-C7 cycloalkyl group, a C1-C6 alkylsulfanyl group, a C1-C6 alkylsulfinyl group, a C1-C6 alkylsulfonyl group (the C3-C7 cycloalkyl group, the C1-C6 alkylsulfanyl group, the C1-C6 alkylsulfinyl group and the C1-C6 alkylsulfonyl group may be optionally substituted with one or more substituents selected from Group B), a C3-C7 cycloalkenyl group which may be optionally substituted with one or more substituents selected from Group C, a phenyl group, a three- to seven-membered nonaromatic heterocyclic group (the phenyl group and the three- to seven- membered nonaromatic heterocyclic group may be optionally substituted with one or more substituents selected from Group D}, a nitro group, $NR^2R^3$, a halogen atom, or a hydrogen atom,

$R^{1a}$ and $R^{1d}$ are identical or different from each other and each represents a C1-C6 chain hydrocarbon group which may be optionally substituted with one or more halogen atoms, a halogen atom, or a hydrogen atom,

Q represents a C6-C10 aryl group which may be optionally substituted with one or more substituents selected from Group H, or a five- to ten- membered aromatic heterocyclic group which may be optionally substituted with one or more substituents selected from Group 1,

$R^2$, $R^{2a}$ and R are identical or different from each other and each represents a C1-C6 chain hydrocarbon group which may be optionally substituted with one or more halogen atoms, or a hydrogen atom,

$R^3$ and $R^{3a}$ are identical or different from each other and each represents a C1-C6 chain hydrocarbon group which may be optionally substituted with one or more substituents selected from Group E, a C3-C7 cycloalkyl group, a C3-C7 cycloalkenyl group (the C3-C7 cycloalkyl group and the C3-C7 cycloalkenyl group may be optionally substituted with one or more substituents selected from Group C), a phenyl group, a five- or six-membered aromatic heterocyclic group {the phenyl group and the five- or six- membered aromatic heterocyclic group may be optionally substituted with one or more substituents selected from Group F}, $S(O)_2R^{13}$, or a hydrogen atom,

$R^{13}$ represents a C1-C6 chain hydrocarbon group which may be optionally substituted with one ot more halogen atoms, or a phenyl group which may be optionally substituted with one or more substituents selected from Group F.

Group A is a group consisting of a C1-C6 alkoxy group, a C3-C6 alkenyloxy group, a C3-C6 alkynyloxy group, a C1-C6 alkylsulfanyl group, a C1-C6 alkylsulfinyl group, a C1-C6 alkylsulfonyl group, a C3-C6 cycloalkyl group {the C1-C6 alkoxy group, the C3-C6 alkenyloxy group, the C3-C6 alkynyloxy group, the C1-C6 alkylsulfanyl group, the C1-C6 alkylsulfinyl group, the C1-C6 alkylsulfouyl group and the C3-C6 cycloalkyl group may be optionally substituted with one or more halogen atoms}, a hydroxy group, a cyano group and a halogen atom.

Group B is a group consisting of a C1-C6 chain hydrocarbon group, a C1-C6 alkoxy group, a C3-C6 alkenyloxy group, a C3-C6 alkynyloxy group (the C1-C6 chain hydrocarbon group, the C1-C6 alkoxy group, the C3-C6 alkenyloxy group and the C3-C6 alkynyloxy group may be optionally substituted with one or more halogen atoms}, a hydroxy group, a cyano group, a nitro group and a halogen atom.

Group C is a group consisting of a C1-C6 alkyl group which may be optionally substituted with one or more halogen atoms, a cyano group and a halogen atom.

Group D is a group consisting of a C1-C6 chain hydrocarbon group which may be optionally substituted with one or more halogen atoms, a five- or six- membered aromatic heterocyclic group which may be optionally substituted with one or more substituents selected from Group F, $OR^{17}$, $NR^{16}R^{17}$, $C(O)R^{17}$, $C(O)NR^{10}R^{17}$, OC(O)R, OC(O)OR, $NR^{17}C(O)R^{16a}$, $NR^{17}C(O)OR$, $C(O)OR^{17}$, a cyano group, a nitro group and a halogen atom.

$R^{16a}$ represents a C1-C6 alkyl group, or a C3-C6 cycloalkyl group (the C1-C6 alkyl group and the C3-C6 cycloalkyl group may be optionally substituted with one or more halogen atoms),

$R^{16}$ and $R^{17}$ are identical or different from each other and each represents a C1-C6 alkyl group, a C3-C6 cycloalkyl group (the C1-C6 alkyl group and the C3-C6 cycloalkyl group may be optionally substituted with one or more halogen atoms}, or a hydrogen atom.

Group E is a group consisting of a C1-C6 alkoxy group, a C3-C7 cycloalkyl group (the C1-C6 alkoxy group and the C3-C7 cycloalkyl group may be optionally substituted with one or more halogen atoms), a phenyl group, a five- or six-membered aromatic heterocyclic group (the phenyl group and the five- or six- membered aromatic heterocyclic group may be optionally substituted with one or more substituents selected from Group F}, a three- to seven- membered nonaromatic heterocyclic group which may be optionally substituted with one or more substituents selected from Group G, $NHR^{14}$, $NR^{14}R^{15}$, an amino group, a cyano group and a halogen atom.

$R^{14}$ and $R^{15}$ are identical or different from each other and each represents a C1-C6 alkyl group which may be optionally substituted with one or more halogen atoms.

Group E is a group consisting of a C1-C6 chain hydrocarbon group, a C1-C6 alkoxy group, a C3-C6 alkenyloxy group, a C3-C6 alkynyloxy group, a C1-C6 alkylsulfanyl group, a C1-C6 alkylsulfinyl group, a C1-C6 alkylsulfonyl group. (the C1-C6 chain hydrocarbon group, the C1-C6 alkoxy group, the C3-C6 alkenyloxy group, the C3-C6 alkynyloxy group, the C1-C6 alkylsulfanyl group, the C1-C6 alkylsulfinyl group and the C1-C6 alkylsulfonyl group may be optionally substituted with one or more halogen atoms}, $NHR^{14}$, $NR^{14}R^{15}$, $C(O)R^{14}$, $OC(O)]R^{14}$, $C(O)OR^{14}$, a hydroxy group, a sulfanyl group, an amino group, a cyano group, a nitro group and a halogen atom.

Group G is a group consisting of a C1-C6 chain hydrocarbon group, a C1-C6 alkoxy group, a C3-C6 alkenyloxy group, a C3-C6 alkynyloxy group {the C1-C6 chain hydrocarbon group, the C1-C6 alkoxy group, the C3-C6 alkenyloxy group and the C3-C6 alkynyloxy group may be optionally substituted with one or more halogen atoms}, and a halogen atom.

Group H is a group consisting of a C1-C6 chain hydrocarbon group which may be optionally substituted with one or more substituents selected from Group A, a C3-C7 cycloalkyl group which may be optionally substituted with one or more substituents selected from Group B, a C3-C7 cycloalkenyl group which may be optionally substituted with one or more substituents selected from Group C, a phenyl group which may be optionally substituted with one or more substituents selected from Group D, a five- or six- membered aromatic heterocyclic group which may be optionally substituted with one or more substituents selected from Group D, $OR^{3a}$, $NR^{2a}R^{3a}$, $NR^{2b}R^{3b}$, $NR^5NR^{2a}R^{3a}$, $NR^5OR^{2a}$, $NR^2C(O)R^{6a}$, $NR^5NR^2C(O)R^{6a}$, $NR^5NR^{2a}C(O)OR^7$, $NR^2C(O)NR^8R^9$, $NR^5NR^{2a}C(O)NR^{10}R^{11}$, $N{=}CHNR^{10}R^{11}$, $N{=}S(O)_pR^8R^9$, $C(O)R^{12}$, $C(O)NR^{10}R^{11}$, $C(O)NR^{2a}S(O)_2R^{13}$, $CR^5{=}NOR^{12}$, $NR^{2a}CR{=}NOR^{12}$, $S(O)R^{13}$, a cyano group, a nitro group and a halogen atom.

p is 0 or 1,

to is 0, 1 or 2,

$R^{2b}$ and $R^{3b}$ combine together with a nitrogen atom to which they are bounded to form a three- to seven-membered nonaromatic heterocyclic group which may be optionally substituted with one or more substituents selected from Group B,

$R^6$ represents a C1-C6 chain hydrocarbon group, a C3-C7 cycloalkyl group, a (C3-C6 cycloalkyl)C1-C3 alkyl group (the C1-C6 chain hydrocarbon group, the C3-C7 cycloalkyl group and the (C3-C6 cycloalkyl)C1-C3 alkyl group may be optionally substituted with one or more halogen atoms}, a phenyl group, a five- or six- membered aromatic heterocyclic group (the phenyl group and the five- or six- membered aromatic heterocyclic group may be optionally substituted with one or more substituents selected from Group F), or a hydrogen atom.

$R^{6a}$ represents a C1-C6 chain hydrocarbon group, a C3-C7 cycloalkyl group, a (C3-C6 cycloalkyl) C1-C3 alkyl group (the C1-C6 chain hydrocarbon group, the C3-C7 cycloalkyl group and the (C3-C6 cycloalkyl)C1-C3 alkyl group may be optionally substituted with one or more halogen atoms), a phenyl group, or a five- or six- membered aromatic heterocyclic group {the phenyl group and the five- or six- membered aromatic heterocyclic group may be optionally substituted with one or more substituents selected, from Group F},

$R^7$ represents a C1-C6 chain hydrocarbon group, a C3-C7 cycloalkyl group, a (C3-C6 cycloalkyl)C1-C3 alkyl group (the C1-C6 chain hydrocarbon group, the C3-C7 cycloalkyl group and the (C3-C6 cycloalkyl)C1-C3 alkyl group may be optionally substituted with one or more halogen atoms), or a phenyl C1-C3 alkyl group (the phenyl moiety in the phenyl C1-C3 alkyl group may be optionally substituted with one or more substituents selected from Group F),

$R^8$ and $R^9$ are identical or different from each other and each represents a C1-C6 alkyl group which may be optionally substituted with one or more halogen atoms,

$R^{10}$ represents a C1-C6 alkyl group which may be optionally substituted with one or more halogen atoms, or a hydrogen atom,

$R^{11}$ represents a C1-C6 chain hydrocarbon group which may be optionally substituted with one or more substituents selected from Group B, or a C3-C7 cycloalkyl group which may be optionally substituted with one or more substituents selected from Group G, $S(O)_2R^{6a}$, or a hydrogen atom,

$R^{12}$ represents a C1-C6 chain hydrocarbon group which may be optionally substituted with one or more halogen atoms, a phenyl group which may be optionally substituted with one or more substituents selected from Group F, or a hydrogen atom.

Group I is a group consisting of a C1-C6 chain hydrocarbon group which may be optionally substituted with one or more substituents selected from Group A, a C3-C7 cycloalkyl group which may be optionally substituted with one or more substituents selected from Group B, a C3-C7 cycloalkenyl group which may be optionally substituted with one or more substituents selected from Group C, $OR^{3a}$, $NR^{2a}R^{3a}$, $NR^{2b}R^{3b}$, $NR^5NR^{2a}R^{3a}$, $NR^5OR^2$, $NR^{2a}C(O)R$, $NR^5NR^2C(O)R^{6a}$, $NR^5NR^{2a}C(O)OR^7$, $NR^{2a}C(O)NR^8R$, $NR^5NR^{2a}C(O)NR^{10}R^{11}$, $N{=}CHNR^{10}R^{11}$, $N{=}S(O)_pR^8R^9$, $C(O)R^{12}$, $C(O)NR^{10}R^{11}$, $C(O)NR^{2a}S(O)_2R^{13}$, $CR{=}NOR^{12}$, $NR^{2a}CR^5{=}NOR^{12}$, $S(O)R$, a cyano group, a nitro group and a halogen atom.]

(hereinafter, the compound represented by formula (I) is referred to as "Present compound N"), or its N-oxide or a salt thereof (hereinafter, the compound represented by formula (I), or its N-oxide or a salt thereof are

referred to as "Present compound").

[2] The compound according to [1] wherein

$Z^2$ represents an oxygen atom,
Q represents a phenyl group, a naphthyl group {the phenyl group and the naphthyl group may be optionally substituted with one or more substituents selected from Group J}, a furanyl group, a thienyl group, a pyrazolyl group, an imidazolyl group, an oxazolyl group, a thiazolyl group, an isoxazolyl group, a pyridyl group, a pyridazinyl group, a pyrimidinyl group, a pyrazihyl group, a quinolyl group, an imidazopyridyl group, a benzofuranyl group, or a benzothiophenyl group {the furanyl group, the thienyl group, the pyrazolyl group, the imidazolyl group, the oxazolyl group, the thiazolyl group, the isoxazolyl group, the pyridyl group, the pyridazinyl group, the pyrimidinyl group, the pyrazinyl group, the quinolyl group, the imidazopyridyl group, the benzofuranyl group and the benzothiophenyl group may be optionally substituted with one or more substituents, selected from Group K},
Group J is a group consisting of a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a phenyl group which may be optionally substituted with one or more halogen atoms, a five- or six-membered aromatic heterocyclic group which may be optionally substituted with one or more halogen atoms, $OR^{3a}$, $NR^{2a}R^{3a}$, $NR^{2b}R^{3b}$, $NR^5NR^{2a}R^{3a}$, $NR^5OR^{2a}$, $NR^{2a}C(O)R$, $NR^5NR^{2a}C(O)R^{6a}$, $NRNR^{2a}C(O)OR^7$, $NR^{2a}C(O)NR^8R^9$, $NR^5NR^{2a}C(O)NR^{10}R^{11}$, $N{=}CHNR^{10}R^{11}$, $N{=}S(O)_pR^8R$, $C(O)R^{12}$, $C(O)NR^{10}R^{11}$, $C(O)NR^{2a}S(O)_2R^{13}$, $CR{=}NOR^{12}$, $NR^{2a}CR{=}NOR^{12}$, $S(O)R^{13}$, a cyano group, a nitro group and a halogen atom.
Group K is a group consisting of a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, $OR^{3a}$, $NR^{2a}R$, $NRR^{3b}$, $NRNR^{2a}R^{3a}$, $NR^5OR$, $NR^{2a}C(O)R$, $NRNR^{2a}C(O)R^{6a}$, $NRNR^{2a}C(O)OR^7$, $NRC(O)NR^8R^9$, $NRNR^{2a}C(O)NR^{10}R^{11}$, $N{=}CHNR^{10}R^{11}$, $N{=}S(O)_pR^8R^9$, $C(O)R^{12}$, $C(O)NR^{10}R^{11}$, $C(O)NR^{2a}S(O)_2R^{13}$, $CR^5{=}NOR^{12}$, $NR^{2a}CR^5{=}NOR^{12}$, $S(O)R^{13}$, a cyano group, a nitro group and a halogen atom,
or its N-oxide or a salt thereof.

[3] The compound according to [1] or [2] wherein $G^1$ represents $CR^{1a}$, $G^2$ represents a nitrogen atom or $CR^1$, and $G^3$ represents $CR^{1d}$, or its N-oxide or a salt thereof.
[4] The compound according to any one of [1] to [3] wherein
$R^{1b}$ represents a C1-C6 chain hydrocarbon group which may be optionally substituted with one or more halogen atoms, a C3-C7 cycloalkyl group which may be optionally substituted with one or more halogen atoms, or a halogen atom, and $R^1$ represents a C1-C6 chain hydrocarbon group which may be optionally substituted with one or more halogen atoms, a C3-C7 cycloalkyl group which may be optionally substituted with one or more halogen atoms, a halogen atom, or a hydrogen atom,
or its N-oxide or a salt thereof.
[5] The compound according to any one of [1] to [4] wherein

Q represents a phenyl group, a naphthyl group {the phenyl group and the naphthyl group may be optionally substituted with one or more substituents selected from Group L}, a pyridyl group, or a quinolyl group {the pyridyl group and the quinolyl group may be optionally substituted with one or more substituents selected from Group M},
Group L is a group consisting of a C1-C6 chain hydrocarbon group which may be optionally substituted with one or more halogen atoms, a C3-C7 cycloalkyl group which may be optionally substituted with one or more halogen atoms, a. phenyl group which may be optionally substituted with one or more halogen atoms, $OR^{19}$, $S(O)R^{19}$, and a halogen atom,
Group M is a group consisting of a C1-C6 chain hydrocarbon group which may be optionally substituted with one or more halogen atoms, a C3-C7 cycloalkyl group which may be optionally substituted with one or more halogen atoms, $OR^{19}$, $S(O)R^{19}$, and a halogen atom,
$R^{19}$ represents a C1-C3 chain hydrocarbon group which may be optionally substituted with one or more halogen atoms, or its N-oxide or a salt thereof.

[6] The compound according to any one of [1] to [5] wherein Q represents a group represented by 01:

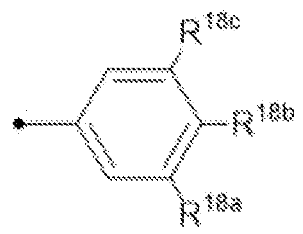

Q1

[wherein,

$R^{18a}$, $R^{18b}$, and $R^{18c}$ are identical or different from each other and each represents a C1-C6 chain hydrocarbon group which may be optionally substituted with one or more halogen atoms, a C3-C7 cycloalkyl group which may be optionally substituted with one or more halogen atoms, a phenyl group which may be optionally substituted with one or more halogen atoms, $OR^{19}$, $S(O)R^{19}$, a halogen atom, or a hydrogen atom, and
$R^{19}$ represents a C1-C3 chain hydrocarbon group which may be optionally substituted with one or more halogen atoms] , its N-oxide or a salt thereof.

[7] The compound according to any one of [1] to [6] wherein $Z^1$ and $Z^2$ represent an oxygen atom, or its N-oxide or a salt thereof.
[8] A composition for controlling harmful arthropod comprising the compound according to any one of [1] to [7], or its N-oxide or a salt thereof.
[9] A composition which comprises one or more ingredients selected from the group consisting of Group (a), Group (b), Group (c) and Group (d), as well as the compound according to any one of [1] to [7], or its N-oxide or a salt thereof:

Group (a): a group consisting of an insecticidal ingredient, a miticidal ingredient, and a nematicidal ingredient;
Group (b): a fungicidal ingredient;
Group (c): a plant growth modulating ingredient; and
Group (d): a repellent ingredient.

[10] A method for controlling a harmful arthropod which comprises applying an effective amount of the compound according to any one of [1] to [7], or its N-oxide or a salt thereof, or an effective amount of the composition according to [9] to a harmful arthropod or a habitat where a harmful arthropod lives.
[11] A seed or vegetative reproductive organ carrying an effective amount of the compound according to any one of [1] to [7], or its N-oxide or a salt thereof, or an effective amount of the composition according to [9].
[12] A compound represented by formula (II):

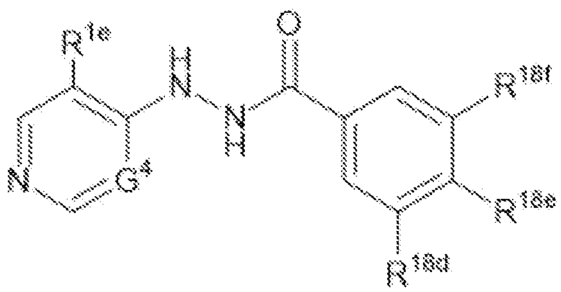

(II)

[wherein,

$G^4$ represents $CR^1$ or a nitrogen atom,
$R^{1e}$ represents a C1-C6 chain hydrocarbon group which may be optionally substituted with one or more halogen atoms, a C3-C7 cycloalkyl group which may be optionally substituted with one or more halogen atoms, or a halogen atom,
$R^{1f}$ represents a C1-C6 chain hydrocarbon group which may be optionally substituted with one or more halogen atoms, a C3-C7 cycloalkyl group which may be optionally substituted with one or more halogen atoms, a halogen

atom, or a hydrogen atom,

$R^{18d}$ and $R^{18e}$ are identical or different from each other and each represents a C1-C6 chain hydrocarbon group which may be optionally substituted with one or more halogen atoms, a C3-C7 cycloalkyl group which may be optionally substituted with one or more halogen atoms, a phenyl group which may be optionally substituted with one or more halogen atoms, $OR^{19}$, $S(O)_nR^{19}$, a halogen atom, or a hydrogen atom,

$R^{18f}$ represents a C1-C6 chain hydrocarbon group which may be optionally substituted with one or more halogen atoms, a C3-C7 cycloalkyl group which may be optionally substituted with one or more halogen atoms, a phenyl group which may be optionally substituted with one or more halogen atoms, $OR^{19}$, $S(O)R^{19a}$, or a halogen atom,

$R^{19a}$ represents a C1-C3 chain hydrocarbon group which may be optionally substituted with one or more halogen atoms, and

n is 0, 1 or 2

(hereinafter, referred to as "Intermediate compound A").

[EFFECT OF INVENTION]

The present invention can control harmful arthropods.

MODE FOR CARRYING OUT THE INVENTION

The substituents as used herein are explained as follows.

The term "halogen atom" represents a fluorine atom, a chlorine atom, a bromine atom, or an iodine atom.

When a substituent has two or more halogen atoms or substituents, the halogen atoms or the substituents may be identical to or different from each other.

The expression "CX-CY" as used herein represents that the number of carbon atoms is from X to Y. For example, the expression "C1-C6" represents that the number of carbon atoms is from 1 to 6.

The term "chain hydrocarbon group" represents an alkyl group, an alkenyl group, or an alkynyl group.

Examples of the "alkyl group" include methyl group, ethyl group, propyl group, isopropyl group, 1,1-dimethylpropyl group, 1,2-dimethylpropyl group, 1-ethylpropyl group, butyl group, sec-butyl group, tert-butyl group, pentyl group, and hexyl group.

Examples of the "alkenyl group" include vinyl group, 1-propenyl group, 2-propenyl group. 1-methyl-1-propenyl group, 1-methyl-2-propenyl group, 1,2-dimethy-l,1-propenyl group, 1-ethyl-2-propenyl group, 3-butenyl group, 4-pentenyl group, and 5-hexenyl group.

Examples of the "alkynyl group" include ethynyl group, 1-propynyl group, 2-propynyl group, 1-methyl-2-propynyl group, 1,1-dimethyl-2-propynyl group, 1-ethyl-2-propynyl group, 2-butynyl group, 4-pentynyl group, and 5-hexynyl group.

Examples of the "alkoxy group" include methoxy group, ethoxy group, propoxy group, isopropoxy group, butoxy group, tert-butoxy group, pentyloxy group, and hexyloxy group.

Examples of the "alkenyloxy group" include 2-propenyloxy group, 2-butenyloxy group, and 5-hexenyloxy group.

Examples of the "alkynyloxy group" include 2-propynyloxy group, 2-butynyloxy group, and 5-hexynyloxy group.

Examples of the "alkylsulfanyl group" include methylsulfanyl group, ethylsulfanyl group, isopropylsulfanyl group, and hexylsulfanyl group.

Examples of the "alkylsulfinyl group" include methylsulfinyl group, ethylsulfinyl group, isopropylsulfinyl group, and hexylsulfinyl group.

Examples of the "alkylsulfonyl group" include methylsulfonyl group, ethylsulfonyl group, isopropylsulfonyl group, and hexylsulfonyl group.

Examples of the "cycloalkyl group" include cyclopropyl group, cyclobutyl group, cyclopentyl group, cyclohexyl group, cycloheptyl group.

Examples of the "cycloalkenyl group" include cyclopropenyl group, cyclobufenyl group, cyclopentenyl group, cyclohexenyl group, and cycloheptenyl group.

Examples of the "C6-C10 aryl group" include phenyl group, indenyl group, indanyl group, naphthyl group, and tetrahydronaphthyl group.

Examples of the "three- to seven- membered nonaromatic heterocyclic group" include aziridinyl group, oxiranyl group, thiranyl group, azetidinyl group, oxetanyl group, thietanyl group, pyrrolidinyl group, tetrahydrofuranyl group, tetrahydrothienyl group, piperidyl group, pyranyl group, dihydropyranyl group, tetrahydropyranyl group, tetrahydrothiopyranyl group, azepanyl group, oxepanyl group, thiepanyl group, pyrazolynyl group, pyrazolidinyl group, imidazolinyl group, imidazolidinyl group, oxazolinyl group, thiazolinyl group, oxazolidinyl group, thiazolidinyl group, isoxazolinyl group, isoxazolidinyl group, isothiazolynyl group, isothiazolidinyl group, dioxolyl group, dioxalanyl group, dioxanyl group, morpholinyl group, thiomorpholinyl group, and piperazinyl group.

Examples of five- to ten- membered aromatic heterocyclic group include a five-membered aromatic heterocyclic

group, a six-membered aromatic heterocyclic group, a nine-membered aromatic heterocyclic group, and a ten-membered aromatic heterocyclic group. Examples of the five-membered aromatic heterocyclic group include pyrrolyl group, furyl group, thienyl group, pyrazolyl group, imidazolyl group, triazolyl group, tetrazolyl group, oxazolyl group, isoxazolyl group, thiazolyl group, isothiazolyl group, oxadiazolyl group, and thiadiazolyl group. Examples of the six-membered aromatic heterocyclic group include pyridyl group, pyridazinyl group, pyrimidinyl group, pyrazinyl group, triazinyl group and tetrazinyl group. Examples of the nine-membered aromatic heterocyclic group include indolyl group, indazolyl group, benzoimidazolyl group, imidazopyridyl group, benzothiophenyl group, and benzofuranyl group. Examples of the ten-membered aromatic heterocyclic group include quinolyl group, isoquinolyl group, quinazolinyl group, and quinoxalinyl group.

Examples of the phenyl moiety in "phenyl C1-C3 alkyl group {the phenyl moiety in the phenyl C1-C3 alkyl group may be optionally substituted with one or more substituents selected from Group F} include benzyl group, 2-fluorobenzyl group, 4-chlorobenzyl group, 4-(trifluoromethyl)benzyl group, and 2-[4-(trifluoromethyl)phenyl]ethyl group.

Examples of the N-oxide of the compound represented by formula (I) include the compound represented by the following formula.

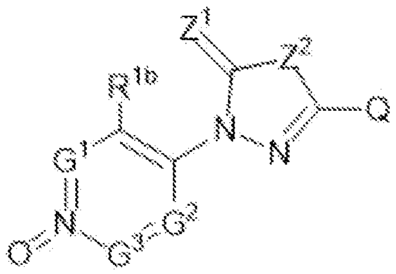

[wherein the symbols are the same as defined above]

The present compound and the intermediate compound A may be existed as one or more stereoisomers. Examples of the stereoisomer include enantiomer, diastereoisomer, and geometric isomer. Each stereoisomer, and stereoisomer mixture(s) in an arbitrary ratio thereof are included in the present compound.

The present compound or its N-oxide, and the intermediate compound A may form acid addition salts. Examples of the acid to form the acid addition salt include inorganic acids such as hydrogen chloride, phosphoric acid, and sulfuric acid; and organic acids such as acetic acid, trifluoroacetic acid, benzoic acid, and p-toluenesulfonic acid. The acid addition salt may be obtained by mixing the present compound or its H-oxide, and the intermediate compound A with an acid.

Embodiments of the compound N of the present invention include the following compounds.

[Embodiment 1] A compound N of the present invention wherein $R^{1b}$ represents a C1-C6 chain hydrocarbon group which may be optionally substituted with one or more substituents selected from Group A, a C3-C7 cycloalkyl group which may be optionally substituted with one or more substituents selected from Group B, a C3-C7 cycloalkenyl group which may be optionally substituted with one or more substituents selected from Group C, a phenyl group which may be optionally substituted with one or more substituents selected from Group D, a nitro group, or a halogen atom, and $R^{1c}$ represents a C1-C6 chain hydrocarbon group which may be optionally substituted with one or more substituents selected from Group A, a C3-C7 cycloalkyl group which may be optionally substituted with one or more substituents selected from Group B, a C3-C7 cycloalkenyl group which may be optionally substituted with one or more substituents selected from Group C, a phenyl group, a four- to eight- membered heterocyclic group (the phenyl group and the four- to eight- membered heterocyclic group may be optionally substituted with one or more substituents selected from Group D), a nitro group, a halogen atom, or a hydrogen atom.

[Embodiment 2] The compound N of the present invention wherein $R^{1b}$ represents a C1-C6 chain hydrocarbon group which may be optionally substituted with one or more halogen atoms, a C3-C7 cycloalkyl group which may be optionally substituted with one or more halogen atoms, or a halogen atom, and $R^{1c}$ represents a C1-C6 chain hydrocarbon group which may be optionally substituted with one or more halogen atoms, a C3-C7 cycloalkyl group which may be optionally substituted with one or more halogen atoms, a halogen atom, or a hydrogen atom.

[Embodiment 3] The compound N of the present invention wherein $R^{1b}$ represents a C1-C6 chain hydrocarbon group which may be optionally substituted with one or more halogen atoms, or a halogen atom, and $R^{1c}$ represents a C1-C6 chain hydrocarbon group which may be optionally substituted with one or more halogen atoms, a halogen atom, or a hydrogen atom.

[Embodiment 4] The compound N of the present invention wherein $R^{1b}$ and $R^{1c}$ are identical to or different from each other and each represents a halogen atom.

[Embodiment 5] The compound N of the present invention wherein $R^{1b}$ and $R^{1c}$ represent a chlorine atom.

[Embodiment 6] The compound N of the present invention wherein $G^1$ represents $CR^{1a}$, $G^2$ represents a nitrogen atom or $CR^{1c}$, G represents $CR^{1d}$, and $R^1$ and $R^{1d}$ represent a hydrogen atom.

[Embodiment 7] The compound N of the present invention wherein $G^1$ represents $CR^{1a}$, $G^2$ represents $CR^1$, $G^3$ represents $CR^{1d}$, and $R^1$ and $R^{1d}$ represent a hydrogen atom.

[Embodiment 8] The compound according to Embodiment 1 wherein $G^1$ represents $CR^{1a}$, G represents a nitrogen atom or $CR^{1c}$, G represents $CR^{1d}$, and $R^{1a}$ and $R^{1d}$ represents a hydrogen atom.

[Embodiment 9] The compound according to Embodiment 2 wherein $G^1$ represents $CR^{1a}$, $G^2$ represents a nitrogen atom or $CR^{1c}$, $G^3$ represents $CR^1$, and $R^{1a}$ and $R^{1d}$ represents a hydrogen atom.

[Embodiment 10] The compound according to Embodiment 3 wherein $G^1$ represents $CR^{1a}$, $G^2$ represents a nitrogen atom or $CR^{1c}$, $G^3$ represents $CR^{1d}$, and $R^1$ and $R^{1d}$ represents a hydrogen atom.

[Embodiment 11] The compound according to Embodiment 4 wherein $G^1$ represents $CR^{1a}$, $G^2$ represents a nitrogen atom or $CR^{1c}$, $G^3$ represents $CR^{1d}$, and $R^{1a}$ and $R^{1d}$ represents a hydrogen atom.

[Embodiment 12] The compound according to Embodiment 5 wherein $G^1$ represents $CR^{1a}$, $G^2$ represents a nitrogen atom or $CR^{1c}$, $G^3$ represents $CR^{1d}$, and $R^{1a}$ and $R^{1d}$ represent a hydrogen atom.

[Embodiment 13] The compound according to Embodiment 1 wherein $G^1$ represents $CR^{1a}$, $G^2$ represents $CR^{1c}$, $G^3$ represents, $CR^{1d}$, and $R^1$ and $R^{1d}$ represent a hydrogen atom.

[Embodiment 14] The compound according to Embodiment 2 wherein $G^1$ represents $CR^{1a}$, $G^2$ represents $CR^1$, G represents $CR^{1d}$, and $R^{1a}$ and $R^{1d}$ represent a hydrogen atom.

[Embodiment 15] The compound according to Embodiment 3 wherein $G^1$ represents $CR^{1a}$, $G^2$ represents $CR^{1c}$, $G^3$ represents $CR^{1d}$, and $R^{1a}$ and $R^{1d}$ represent a hydrogen atom.

[Embodiment 16] The compound according to Embodiment 4 wherein $G^1$ represents $CR^1$, $G^2$ represents $CR^{1c}$, G represents $CR^{1d}$, and $R^1$ and $R^{1d}$ represent a hydrogen atom.

[Embodiment 17] The compound according to Embodiment 5 wherein $G^1$ represents $CR^{1a}$, $G^2$ represents $CR^{1c}$, $G^3$ represents $CR^{1d}$, and $R^{1a}$ and $R^{1d}$ represent a hydrogen atom.

[Embodiment 18] The compound N of the present invention wherein $Z^2$ represents an oxygen atom.

[Embodiment 19] The compound N of the present invention wherein $Z^1$ and $Z^2$ represent an oxygen atom.

[Embodiment 20] The compound according to Embodiment 1 wherein $Z^2$ represents an oxygen atom.

[Embodiment 21] The compound according to Embodiment 2 wherein $Z^2$ represents an oxygen atom.

[Embodiment 22] The compound according to Embodiment 3 wherein $Z^2$ represents an oxygen atom.

[Embodiment 23] The compound according to Embodiment 4 wherein $Z^2$ represents an oxygen atom.

[Embodiment 24] The compound according to Embodiment 5 wherein $Z^2$ represents an oxygen atom.

[Embodiment 25] The compound according to Embodiment 6 wherein $Z^2$ represents an oxygen atom.

[Embodiment 26] The compound according to Embodiment 7 wherein $Z^2$ represents an oxygen atom.

[Embodiment 27] The compound according to Embodiment 8 wherein $Z^2$ represents an oxygen atom.

[Embodiment 28] The compound according to Embodiment 9 wherein $Z^2$ represents an oxygen atom.

[Embodiment 29] The compound according to Embodiment 10 wherein $Z^2$ represents an oxygen atom.

[Embodiment 30] The compound according to Embodiment 11 wherein $Z^2$ represents an oxygen atom.

[Embodiment 31] The compound according to Embodiment 12 wherein $Z^2$ represents an oxygen atom.

[Embodiment 32] The compound according to Embodiment 13 wherein $Z^2$ represents an oxygen atom.

[Embodiment 33] The compound according to Embodiment 14 wherein $Z^2$ represents an oxygen atom.

[Embodiment 34] The compound according to Embodiment 15 wherein $Z^2$ represents an oxygen atom.

[Embodiment 35] The compound according to Embodiment 16 wherein $Z^2$ represents an oxygen atom.

[Embodiment 36] The compound according to Embodiment 17 wherein $Z^2$ represents an oxygen atom.

[Embodiment 37] The compound according to Embodiment 1 wherein $Z^1$ and $Z^2$ represent an oxygen atom.

[Embodiment 38] The compound according to Embodiment 2 wherein $Z^1$ and $Z^2$ represent an oxygen atom.

[Embodiment 39] The compound according to Embodiment 3 wherein $Z^1$ and $Z^2$ represent an oxygen atom.

[Embodiment 40] The compound according to Embodiment 4 wherein $Z^1$ and $Z^2$ represent an oxygen atom.

[Embodiment 41] The compound according to Embodiment 5 wherein $Z^1$ and $Z^2$ represent an oxygen atom.

[Embodiment 42] The compound according to Embodiment 6 wherein $Z^1$ and $Z^2$ represent an oxygen atom.

[Embodiment 43] The compound according to Embodiment 7 wherein $Z^1$ and $Z^2$ represent an oxygen atom.

[Embodiment 44] The compound according to Embodiment 8 wherein $Z^1$ and $Z^2$ represent an oxygen atom.

[Embodiment 45] The compound according to Embodiment 9 wherein $Z^1$ and $Z^2$ represent an oxygen atom.

[Embodiment 46] The compound according to Embodiment 10 wherein $Z^1$ and $Z^2$ represent an oxygen atom.

[Embodiment 47] The compound according to Embodiment 11 wherein $Z^1$ and $Z^2$ represent an oxygen atom.

[Embodiment 48] The compound according to Embodiment 12 wherein $Z^1$ and $Z^2$ represent an oxygen atom.

[Embodiment 49] The compound according to Embodiment 13 wherein $Z^1$ and $Z^2$ represent an oxygen atom.

[Embodiment 50] The compound according to Embodiment 14 wherein $Z^1$ and $Z^2$ represent an oxygen atom.

[Embodiment 51] The compound according to Embodiment 15 wherein $Z^1$ and $Z^2$ represent an oxygen atom.
[Embodiment 52] The compound according to Embodiment 16 wherein $Z^1$ and $Z^2$ represent an oxygen atom.
[Embodiment 53] The compound according to Embodiment 17 wherein $Z^1$ and $Z^2$ represent an oxygen atom.
[Embodiment 54] The compound according to any one of Embodiment 1 to Embodiment 53 or the compound N of the present invention wherein Q represents a phenyl group, a naphthyl group (the phenyl group and the naphthyl group may be optionally substituted with one or more substituents selected from Group H}, a furanyl group, a thienyl group, a pyrazolyl group, an imidazolyl group, an oxazolyl group, a thiazolyl group, an isoxazolyl group, a pyridyl group, a pyridazinyl group, a pyrimidinyl group, a pyrazinyl group, a quinolyl group, an imidazopyridyl group, a benzofuranyl group, or a benzothiophenyl group (the furanyl group, the thienyl group, the pyrazolyl group, the imidazolyl group, the oxazolyl group, the thiazolyl group, the isoxazolyl group, the pyridyl group, the pyridazinyl group, the pyrimidinyl group, the pyrazinyl group, the quinolyl group, the imidazopyridyl group, the benzofuranyl group and the benzothiophenyl group may be optionally substituted with one or more substituents selected from Group I).
[Embodiment 55] The compound according to any one of Embodiment 1 to Embodiment 53 or the compound N of the present invention wherein Q represents a phenyl group, a naphthyl group (the phenyl group and the naphthyl group may be optionally substituted with one or more substituents selected from Group H), a furanyl group, a thienyl group, an oxazolyl group, a pyridyl group, a quinolyl group, a benzofuranyl group, or a benzothiophenyl group {the furanyl group, the thienyl group, the oxazolyl group, the pyridyl group, the quinolyl group, the benzofuranyl group and the benzothiophenyl group may be optionally substituted with one or more substituents selected from Group I}.
[Embodiment 56] The compound according to any one of Embodiment 1 to Embodiment 53 or the compound N of the present invention wherein Q represents a phenyl group, a naphthyl group {the phenyl group and the naphthyl group may be optionally substituted with one or more substituents selected from Group H}, a pyridyl group, or a quinolyl group (the pyridyl group and the quinolyl group may be optionally substituted with one or more substituents selected from Group I).
[Embodiment 57] The compound according to any one of Embodiment 1 to Embodiment 53 or the compound N of the present invention wherein Q represents a C6-C10 aryl group which may be optionally substituted with one or more substituents selected from Group N or a five- to ten-membered aromatic heterocyclic group which may be optionally substituted with one or more substituents selected from Group O.

Group N is a group consisting of a C1-C6 chain hydrocarbon group, a C3-C7 cycloalkyl group, a phenyl group, a five- or six- membered aromatic heterocyclic group {the C1-C6 chain hydrocarbon group, the C3-C7 cycloalkyl group, the phenyl group, or the five- or six- membered aromatic heterocyclic group which may be optionally substituted with one or more halogen atoms}, $OR^3$, $NR^2R^3$, $NRC(O)R^6$, $S(O)_mR^{13}$, a cyano group, a nitro group and a halogen atom.
Group Q is a group consisting of a C1-C6 chain hydrocarbon group, a C3-C7 cycloalkyl group {the C1-C6 chain hydrocarbon group and the C3-C7 cycloalkyl group may be optionally substituted with one or more halogen atoms}, $OR^3$, $NR^2R^3$ , $NR^2C(O)R^6$, $S(O)R^{13}$, a cyano group, a nitro group and a halogen atom.

[Embodiment 58] The compound according to any one of Embodiment 1 to Embodiment 53 or the compound N of the present invention wherein Q represents a phenyl group, a naphthyl group {the phenyl group and the naphthyl group may be optionally substituted with one or more substituents selected from Group N}, a furanyl group, a thienyl group, a pyrazolyl group, an imidazolyl group, an oxazolyl group, a thiazolyl group, an isoxazolyl group, a pyridyl group, a pyridazinyl group, a pyrimidinyl group, a pyrazinyl group, a quinolyl group, an imidazopyridyl group, a benzofuranyl group, or a benzothiophenyl group {the furanyl group, the thienyl group, the pyrazolyl group, the imidazolyl group, the oxazolyl group, the thiazolyl group, the isoxazolyl group, the pyridyl group, the pyridazinyl group, the pyrimidinyl group, the pyrazinyl group, the quinolyl group, the imidazopyridyl group, the benzofuranyl group and the benzothiophenyl group may be optionally substituted with one or more substituents selected from Group O}.
[Embodiment 59] The compound according to any one of Embodiment 1 to Embodiment 53 or the compound N of the present invention wherein Q represents a phenyl group, a naphthyl group (the phenyl group and the naphthyl group may be optionally substituted with one or more substituents selected from Group N), a furanyl group, a thienyl group, an oxazolyl group, a pyridyl group, a quinolyl group, a benzofuranyl group, or a benzothiophenyl group (the furanyl group, the thienyl group, the oxazolyl group, the pyridyl group, the quinolyl group, the benzofuranyl group and the benzothiophenyl group may be optionally substituted with one or more substituents selected from Group O) .
[Embodiment 60] The compound according to any one of Embodiment 1 to Embodiment 53 or the compound N of the present invention wherein Q represents a phenyl group, a naphthyl group {the phenyl group and the naphthyl group may be optionally substituted with one or more substituents selected from Group N}, a pyridyl group, or a quinolyl group [the pyridyl group and the quinolyl group may be optionally substituted with one or more substituents

selected from Group O}.
[Embodiment 61] The compound according to any one of Embodiment 1 to Embodiment 53 or the compound N of the present invention wherein Q represents a C6-C10 aryl group which may be optionally substituted with one or more substituents selected from Group P or a five- to ten-membered aromatic heterocyclic group may be optionally substituted with one or more substituents selected from Group Q.

Group P is a group consisting of a C1-C6 chain hydrocarbon group, a C3-C7 cycloalkyl group, a phenyl group (the C1-C6 chain hydrocarbon group, the C3-C7 cycloalkyl group, or the phenyl group may be optionally substituted with one or more halogen atoms}, $OR^3$, $S(O)R^1$, and a halogen atom.
Group Q is a group consisting of a C1-C6 chain hydrocarbon group, a C3-C7 cycloalkyl group (the C1-C6 chain hydrocarbon group and the C3-C7 cycloalkyl group may be optionally substituted with one or more halogen atoms), $OR^3$, $S(O)R^1$, and a halogen atom.

[Embodiment 62] The compound according to any one of Embodiment 1 to Embodiment 53 or the compound N of the present invention wherein Q represents a phenyl group, a naphthyl group (the phenyl group and the naphthyl group may be optionally substituted with one or more substituents selected from Group P), a furanyl group, a thienyl group, a pyrazolyl group, an imidazolyl group, an oxazolyl group, a thiazolyl group, an isoxazolyl group, a pyridyl group, a pyridazinyl group, a pyrimidinyl group, a pyrazinyl group, a quinolyl group, an imidazopyridyl group, a benzofuranyl group, or a benzothiophenyl group {the furanyl group, the thienyl group, the pyrazolyl group, the imidazolyl group, the oxazolyl group, the thiazolyl group, the isoxazolyl group, the pyridyl group, the pyridazinyl group, the pyrimidinyl group, the pyrazinyl group, the quinolyl group, the imidazopyridyl group, the benzofuranyl group and the benzothiophenyl group may be optionally substituted with one or more substituents selected from Group Q}.
[Embodiment 63] The compound according to any one of Embodiment 1 to Embodiment 53 or the compound N of the present invention wherein Q represents a phenyl group, a naphthyl group (the phenyl group and the naphthyl group may be optionally substituted with one or more substituents selected from Group P), a furanyl group, a thienyl group, an oxazolyl group, a pyridyl group, a quinolyl group, a benzofuranyl group, or a benzothiophenyl group {the furanyl group, the thienyl group, the oxazolyl group, the pyridyl group, the quinolyl group, the benzofuranyl group and the benzothiophenyl group may be optionally substituted with one or more substituents selected from Group Q}.
[Embodiment 64] The compound according to any one of Embodiment 1 to Embodiment 53 or the compound N of the present invention wherein Q represents a phenyl group, a naphthyl group (the phenyl group and the naphthyl group may be optionally substituted with one or more substituents selected from Group P), a pyridyl group, or a quinolyl group {the pyridyl group and the quinolyl group may be optionally substituted with one or more substituents selected from Group Q}.
[Embodiment 65] The compound according to any one of Embodiment 1 to Embodiment 53 or the compound N of the present invention wherein Q represents a group represented by Q1, $R^{18a}$ and $R^{18c}$ are identical to or different from each other and each represents a C1-C3 chain hydrocarbon group which may be optionally substituted with one or more halogen atoms, a C3-C6 cycloalkyl group which may be optionally substituted with one or more halogen atoms, $OR^{19}$, $S(O)R^{19}$, a halogen atom, or a hydrogen atom, and $R^{18b}$ represents a C1-C3 chain hydrocarbon group which may be optionally substituted with one or more halogen atoms, $OR^{19}$, a halogen atom, or a hydrogen atom.
[Embodiment 66] The compound according to any one of Embodiment 1 to Embodiment 53 or the compound N of the present invention wherein Q represents a group represented by Q1, $R^{18a}$ and $R^{18}$ are identical to or different from each other and each represents a C1-C3 chain hydrocarbon group which may be optionally substituted with one or more halogen atoms, $OR^{19}$, $S(O)R^{19}$, a fluorine atom, a chlorine atom, or a hydrogen atom, and $R^{18b}$ represents a fluorine atom, a chlorine atom, or a hydrogen atom.
[Embodiment 67] The compound according to any one of Embodiment 1 to Embodiment 53 or the compound N of the present invention wherein Q represents a group represented by Q1, $R^{18a}$, $R^{18b}$, and $R^{18c}$ are identical to or different from each other and each represents a hydrogen atom or a chlorine atom (with the proviso that all of $R^{18a}$, $R^{18b}$, and $R^{18c}$ do not represent a hydrogen atom).
[Embodiment 68] The compound N of the present invention wherein $R^{1b}$ represents a C1-C6 chain hydrocarbon group which may be optionally substituted with one or more halogen atoms, a C1-C6 alkoxy group which may be optionally substituted with one or more halogen atoms, a C3-C7 cycloalkyl group which may be optionally substituted with one or more halogen atoms, a C1-C6 alkylsulfanyl group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, or a halogen atom, and $R^{1c}$ represents a C1-C6 chain hydrocarbon group which may be optionally substituted with one or more halogen atoms, a C1-C6 alkoxy group which may be optionally substituted with one or more halogen atoms, a C3-C7 cycloalkyl group which may be optionally substituted with one or more halogen atoms, a C1-C6 alkylsulfanyl

group which may be optionally substituted with one or more halogen atoms, a C1-C6 alkylsulfinyl group which may be optionally substituted with one or more halogen atoms, a C1-C6 alkylsulfonyl group which may be optionally substituted with one or more halogen atoms, a halogen atom, or a hydrogen atom.

[Embodiment 69] The compound N of the present invention wherein $R^{1b}$ represents a C1-C6 chain hydrocarbon group which may be optionally substituted with one or more halogen atoms, a C1-C6 alkoxy group which may be optionally substituted with one or more halogen atoms, a C3-C7 cycloalkyl group which may be optionally substituted with one or more halogen atoms, or a halogen atom, and $R^{1c}$ represents a C1-C6 chain hydrocarbon group which may be optionally substituted with one or more halogen atoms, a C1-C6 alkoxy group which may be optionally substituted with one or more halogen atoms, a C3-C7 cycloalkyl group which may be optionally substituted with one or more halogen atoms, a halogen atom, or a hydrogen atom.

[Embodiment 70] The compound according to Embodiment 68 wherein $G^1$ represents $CR^{1a}$, $G^2$ represents a nitrogen atom or $CR^{1c}$, $G^3$ represents $CR^{1d}$, and $R^{1a}$ and $R^{1d}$ represent a hydrogen atom.

[Embodiment 71] The compound according to Embodiment 69 wherein $G^1$ represents $CR^{1a}$, $G^2$ represents a nitrogen atom or $CR^{1c}$, $G^3$ represents $CR^{1d}$, and $R^{1a}$ and $R^{1d}$ represent a hydrogen atom.

[Embodiment 72] The compound according to Embodiment 68 wherein $G^1$ represents $CR^{1a}$, $G^2$ represents $CR^{1c}$, $G^3$ represents $CR^{1d}$, and $R^{1a}$ and $R^{1d}$ represent a hydrogen atom.

[Embodiment 731 The compound according to Embodiment 69 wherein $G^1$ represents $CR^{1a}$, $G^2$ represents $CR^{1c}$, G represents $CR^{1d}$, and $R^{1a}$ and $R^{1d}$ represent a hydrogen atom.

[Embodiment 74] The compound according to Embodiment 68 wherein $Z^2$ represents an oxygen atom.

[Embodiment 75] The compound according to Embodiment 69 wherein $Z^2$ represents an oxygen atom.

[Embodiment 76] The compound according to Embodiment 70 wherein $Z^2$ represents an oxygen atom.

[Embodiment 77] The compound according to Embodiment 71 wherein $Z^2$ represents an oxygen atom.

[Embodiment 78] The compound according to Embodiment 72 wherein $Z^2$ represents an oxygen atom.

[Embodiment 79] The compound according to Embodiment 73 wherein $Z^2$ represents an oxygen atom.

[Embodiment 8Q] The compound according to Embodiment 68 wherein $Z^1$ and $Z^2$ represents an oxygen atom.

[Embodiment 81] The compound according to Embodiment 69 wherein $Z^1$ and $Z^2$ represents an oxygen atom.

[Embodiment 82] The compound according to Embodiment 70 wherein $Z^1$ and $Z^2$ represents an oxygen atom.

[Embodiment 83] The compound according to Embodiment 71 wherein $Z^1$ and $Z^2$ represents an oxygen atom.

[Embodiment 84] The compound according to Embodiment 72 wherein $X^1$ and $X^2$ represents an oxygen atom.

[Embodiment 851 The compound according to Embodiment 73 wherein $Z^1$ and $Z^2$ represents an oxygen atom.

[Embodiment 86] The compound according to any one of Embodiment 68 to Embodiment 85 wherein Q represents a phenyl group, a naphthyl group (the phenyl group and the naphthyl group may be optionally substituted with one or more substituents selected from Group P), a pyridyl, group, or a quinolyl group (the pyridyl group and the quinolyl group may be optionally substituted with one or more substituents selected from Group Q).

[Embodiment 87] The compound according to any one of Embodiment 68 to Embodiment 85 wherein Q represents a group represented by 01, $R^{18a}$ and $R^{18c}$ are identical to or different from each other and each represents a C1-C3 chain hydrocarbon group which may be optionally substituted with one or more halogen atoms, a C3-C6 cycloalkyl group which may be optionally substituted with one or more halogen atoms, $OR^{19}$, $S(O)_mR^1$, a halogen atom, or a hydrogen atom, and R represents a C1-C3 chain hydrocarbon group which may be optionally substituted with one or more halogen atoms, $OR^{19}$, a halogen atom, or a hydrogen atom.

(Embodiment 88) The compound according to any one of Embodiment 68 to Embodiment 85 wherein Q represents a group represented by Q1, $R^{18a}$ and $R^{18}$ are identical to or different from each other and each represents a C1-C3 chain hydrocarbon group which may be optionally substituted with one or more halogen atoms, $OR^{19}$, $S(O)R^{19}$, a fluorine atom, a chlorine atom, or a hydrogen atom, and $R^{18b}$ represents a fluorine atom, a chlorine atom, or a hydrogen atom.

[Embodiment 89] The compound according to any one of Embodiment 68 to Embodiment 85 wherein Q represents a group represented by Q1, and $R^{18a}$, $R^{18b}$, and $R^{18c}$ are identical to or different from each other and each represents a hydrogen atom or a chlorine atom (with the proviso that all of $R^{18a}$, $R^{18b}$, and $R^{18c}$ do not represent a hydrogen atom).

[Embodiment 90] The compound according to any one of Embodiment 1 to Embodiment 53, Embodiment 68 to Embodiment 85 or the compound N of the present invention wherein Q represents a phenyl group which is substituted with one or more s substituents selected from Group R.

Group R is a group consisting of a C1-C4 chain hydrocarbon group which may be optionally substituted with one or more halogen atoms, a cyclopropyl group which may be optionally substituted with one or more halogen atoms, a phenyl group which may be optionally substituted with one or more halogen atoms, $OR^{20}$, $S(O)R^{21}$, a cyano group, a nitro group and a halogen atom.

$R^{20}$ represents a C1-C3 chain hydrocarbon group which may be optionally substituted with one or more halogen atoms, a phenyl group {the phenyl group may be optionally substituted with one or more halogen atoms} or a

hydrogen atom,
$R^{21}$ represents a C1-C3 chain hydrocarbon group which may be optionally substituted with one or more halogen atoms, or a phenyl group {the phenyl group may be optionally substituted with one or more halogen atoms}.

[Embodiment 91] The compound according to any one of Embodiment 1 to Embodiment 53, Embodiment 68 to Embodiment 85, or the compound N of the present invention wherein Q represents a phenyl group which is substituted with two or more substituents selected from Group R.
[Embodiment 92] The compound according to any one of Embodiment 1 to Embodiment 53, Embodiment 68 to Embodiment 85, or the compound N of the present invention wherein Q represents a phenyl group which is substituted with one or more substituents selected from Group S.
Group S is a group consisting of a C1-C4 chain hydrocarbon group which may be optionally substituted with one or more halogen atoms, a phenyl group which may be optionally substituted with one or more halogen atoms, $OR^{20}$, $S(O)R^{21}$, a cyano group, a nitro group and a halogen atom.
[Embodiment 93] The compound according to any one of Embodiment 1 to Embodiment 53, Embodiment 68 to Embodiment 85, or the compound N of the present invention wherein Q represents a phenyl group which is substituted with two or more substituents selected from Group S.
[Embodiment 94] The compound according to any one of Embodiment 1 to Embodiment 53, or the compound N of the present invention wherein Q represents a group represented by $Q_1$, $R^{18a}$, $R^{18b}$ and $R^{28c}$ are identical to or different from each other and each represents a C1-C4 chain hydrocarbon group which may be optionally substituted with one or more halogen atoms, a cyclopropyl group which may be optionally substituted with one or more halogen atoms, $OR^{20}$, $S(O)R^{21}$, a cyano group, a nitro group, a halogen atom or a hydrogen atom.
[Embodiment 95] The compound according to any one of Embodiment 1 to Embodiment 53, Embodiment 68 to Embodiment 85, or the compound N of the present invention wherein Q represents a group represented by Q1, $R^{18a}$ and $R^{18c}$ are identical to or different from each other and each represents a C1-C4 chain hydrocarbon group which may be optionally substituted with one or more halogen atoms, a cyclopropyl group which may be optionally substituted with one or more halogen atoms, $OR^{20}$, $S(O)R^{21}$, a cyano group, a nitro group, a halogen atom, or a hydrogen atom, $R^{18b}$ represents a C1-C4 chain hydrocarbon group which may be optionally substituted with one or more halogen atoms, $OR^{20}$, $S(O)R^{21}$, a cyano group, a nitro group, a halogen atom, or a hydrogen atom.
[Embodiment 96] The compound according to any one of Embodiment 1 to Embodiment 53, Embodiment 68 to Embodiment 85, or the compound N of the present invention wherein wherein Q represents a group represented by Q1, R represents a C1-C4 chain hydrocarbon group which may be optionally substituted with one or more halogen atoms, a cyclopropyl group which may be optionally substituted with one or more halogen atoms, $OR^{20}$, $S(O)R^{21}$, a cyano group, a nitro group, a halogen atom, or a hydrogen atom, and $R^{18b}$ represents a C1-C4 chain hydrocarbon group which may be optionally substituted with one or more halogen atoms, $OR^{20}$, $S(O)R^{21}$, a cyano group, a nitro group, a halogen atom, or a hydrogen atom, and $R^{18c}$ represents a hydrogen atom.
[Embodiment 97] The compound according to any one of Embodiment 1 to Embodiment 53, Embodiment 68 to Embodiment 85, or the compound N of the present invention wherein wherein Q represents a group represented by Q1, $R^{18a}$ represents a C1-C4 chain hydrocarbon group which may be optionally substituted with one or more halogen atoms, a cyclopropyl group which may be optionally substituted with one or more halogen atoms, $OR^{20}$, $S(O)_mR^{21}$, a cyano group, a nitro group, or a halogen atom, and $R^{18b}$ represents a C1-C4 chain hydrocarbon group which may be optionally substituted with one or more halogen atoms, $OR^{20}$, $S(O)R^{21}$, a cyano group, a nitro group, a halogen atom, or a hydrogen atom, and $R^{18c}$ represents a hydrogen atom.
[Embodiment 98] The compound according to any one of Embodiment 1 to Embodiment 53, Embodiment 68 to Embodiment 85, or the compound N of the present invention wherein Q represents a group represented by Q1, $R^{18a}$ represents a C1-C4 chain hydrocarbon group which may be optionally substituted with one or more halogen atoms, a cyclopropyl group which may be optionally substituted with one or more halogen atoms, $OR^{20}$, $S(O)R^{21}$, a cyano group, a nitro group, a halogen atom, or a hydrogen atom, $R^{18b}$ represents a C1-C4 chain hydrocarbon group which may be optionally substituted with one or more halogen atoms, $PR^{20}$, $S(O)_mR^{21}$, a cyano group, a nitro group, or a halogen atom, and $R^{18c}$ represents a hydrogen atom.
[Embodiment 99] The compound according to any one of Embodiment 1 to Embodiment 53, Embodiment 68 to Embodiment 85, or the compound N of the present invention wherein Q represents a group represented by Q1, $R^{18a}$ represents a C1-C4 chain hydrocarbon group which may be optionally substituted with one or more halogen atoms, a cyclopropyl group which may be optionally substituted with one or more halogen atoms, $OR^{20}$, $S (O)R^{21}$, a cyano group, a nitro group, or a halogen atom, $R^{18b}$ represents a C1-C4 chain hydrocarbon group which may be optionally substituted with one or more halogen atoms, $OR^{20}$, $S(O)R^{21}$, a cyano group, a nitro group, or a halogen atom, and $R^{18c}$ represents a hydrogen atom.
[Embodiment 1001 The compound according to any one of Embodiment 1 to Embodiment 53, Embodiment 68 to Embodiment 85, or the compound N of the present invention wherein Q represents a group represented by Q1,

$R^{18a}$ and $R^{18c}$ represent a hydrogen atom, $R^{18b}$ represents a C1-C4 chain hydrocarbon group which may be optionally substituted with one or more halogen atoms, $OR^{20}$, $S(O)R^{21}$, a cyano group, a nitro group, or a halogen atom,
[Embodiment 101] The compound according to any one of Embodiment 1 to Embodiment. 53, Embodiment 68 to Embodiment 85, or the compound N of the present invention wherein a represents a group represented by Q1, $R^{18a}$ represents a C1-C4 chain hydrocarbon group which may be optionally substituted with one or more halogen atoms, a cyclopropyl group which may be optionally substituted with one or more halogen atoms, $OR^{20}$, $S(O)R^{21}$, a cyano group, a nitro group, or a halogen atom, and $R^{18b}$ and $R^{18c}$ represent a hydrogen atom.

Next, a process for preparing the present compound is described.

Process 1

A compound represented by formula (I-2) (hereinafter, referred to as "compound (1-2)") can be prepared by reacting a compound represented by formula (I-1) (hereinafter, referred to as "compound (I-1)") with a sulfurizing agent.

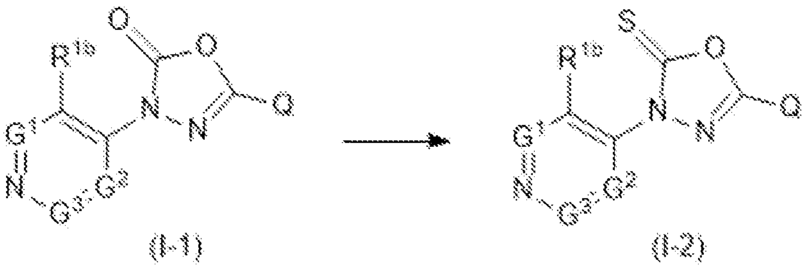

[wherein the symbols are the same as defined above]

The reaction is usually carried out in a solvent or in the absence of a solvent. Examples of the solvents include ethers (such as tetrahydrofuran (hereinafter, referred to as "THF"), 1,4-dioxane, 1,2-dimethoxyethane (hereinafter, referred to as "DME"),methyl tert-butyl ether (hereinafter, referred to as "MTBE"), diethyl ether) (hereinafter, collectively referred to as "ethers"); halogenated hydrocarbons (such as dichloromethane, chloroform) (hereinafter, collectively referred to as "halogenated hydrocarbons"); hydrocarbons (such as hexane, toluene, xylene) (hereinafter, collectively referred to as "hydrocarbons"); nitriles (such as acetonitrile) (hereinafter, collectively referred to as "nitriles"); organic bases (such as triethyl amine, diisopropyl ethyl amine, pyridine, 4-(dimethylamino)pyridine) (hereinafter, collectively referred to as "organic bases"); and mixtures of these solvents.

Examples of the sulfurizing agent include phosphorus pentasulfide, 2,4-bis(4-methoxyphenyl)-1,3-dithia-2,4-diphosphetan-2,4-disulfide (hereinafter, referred to as "Lawesson's reagent").

In the reaction, the sulfurizing agent is usually used within a range of 1 to 3 molar ratio(s) as opposed to 1 mole of the compound (1-1).

The reaction temperature in the reaction is usually within a range of 0 to 200°C. The reaction period in the reaction is usually within a range of 1 to 24 hour(s),

When the reaction is completed, water is added to a reaction mixture, and the reaction mixture is extracted with organic solveht(s), and the organic layer is worked up (for example, drying and concentration) to obtain the compound (I-2).

Process 2

A compound represented by formula (1-3) (hereinafter, referred to as "compound (I-3)" can be prepared by reacting a compound represented by formula (M-1) (hereinafter, referred to as "compound (M-1)" with a carbonylating agent.

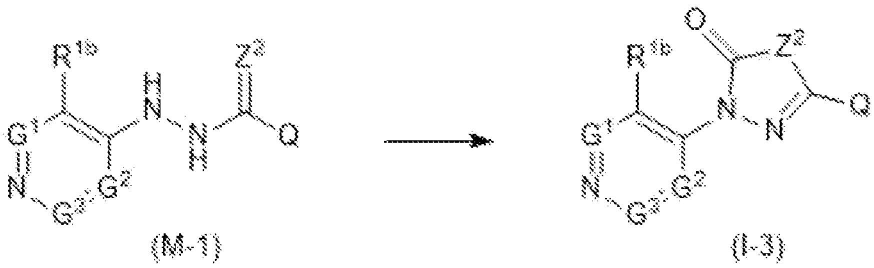

(M-1) (I-3)

[wherein the symbols are the same as defined above]

The reaction is usually conducted in a solvent. Examples of the solvents include ethers, halogenated hydrocarbons, hydrocarbons, and mixtures of these solvents.

Examples of the carbonylating agent include phosgene, triphosgene, dimethyl carbonate, and carbonyl diimidazole (hereinafter, referred to as "CDI").

A base may be used in the reaction a needed. Examples of the base include organic bases; alkali metal carbonates (such as sodium carbonate, potassium carbonate) (hereinafter, referred to as "alkali metal carbonates"); and alkali metal hydrides (such as sodium hydride) (hereinafter, referred to as "alkali metal hydrides").

In the reaction, the carbonylating agent is usually used within a range of 1 to 2 molar ratio(s), as opposed to 1 mole of the compound (M-1).

When a base is used in the reaction, the base is usually used within a range of 2 to 4 molar ratios, as opposed to 1 mole of the compound (M-1).

The reaction temperature in the reaction is usually within a range of 0 to 120°C. The reaction period in the reaction is usually within a range of 0.5 to 24 hours,

When the reaction is completed, water is added to a reaction mixture, and the reaction mixture is extracted with organic solveht(s), and the organic layer is worked up (for example, drying and concentration) to obtain the compound (I-3) .

Process 3

A compound represented by formula (1-4) (hereinafter, referred to as "compound (I-4)") can be prepared by reacting the compound (M-1) with a thiocarbonylating agent.

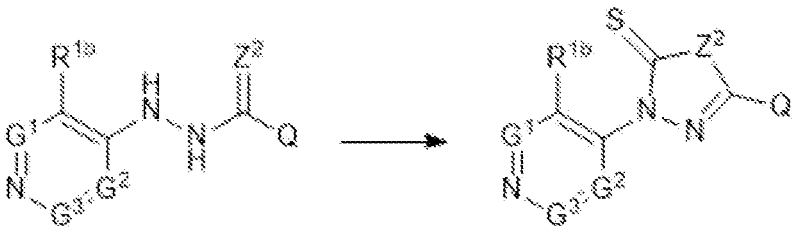

(M-1) (I-4)

[wherein the symbols are the same as defined above]

Examples of the thiocarbonylatihg agent include thiophoagene.

The reaction can be conducted by using the thiocarbonylating agent in place of the carbonylating agent according to the process 2.

Process. 4

A compound represented by formula (1-5) (hereinafter, referred to as "compound (1-5)") can be prepared by reacting a compound represented by formula (M-2) (hereinafter, referred to as "compound (M-2)" with a compound represented by formula (M-3) (hereinafter, referred to as "compound (M-3)") in the presence of a base.

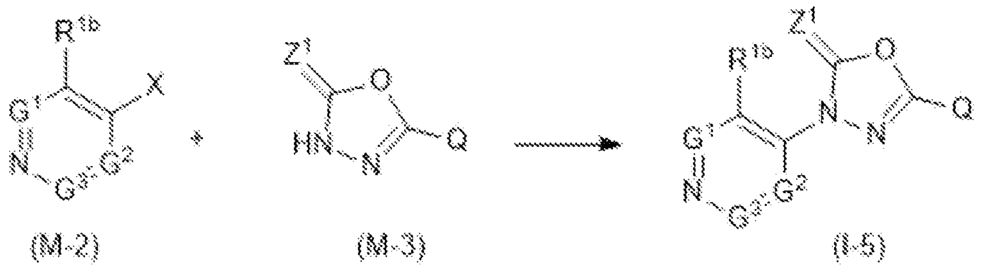

(M-2) (M-3) (I-5)

[wherein X represents a halogen atom, and the other symbols are the same as defined above]

The reaction is usually conducted in a solvent. Examples of the solvent include ethers; hydrocarbons; nitriles; apromatic polar solvents (such as dimethylformamide (hereinafter, referred to as "DMF"), N-methylpyrrolidone (hereinafter, referred to as "NMP"), dimethylsulfoxide (hereinafter, referred to as "DMSO")) (hereinafter, collectively referred to as "apromatic polar solvents"); and mixtures of these solvents.

Examples of the bases include organic bases, alkali metal carbonates, and alkali metal hydrides.

In the reaction, the compound (M-3) is usually used within a range of 0.8 to 1.2 molar ratios, and the base is usually used within a range of 1 to 3 molar ratio(s), as, opposed to 1 mole of the compound (M-2).

A metallic catalyst may be used in the reaction as needed. Examples of the metallic catalyst include copper catalysts (such as copper (I) iodide, copper (I) bromide, copper (I) chloride, copper (I) oxide, copper (I) trifluoromethane sulfonate benzene complex, tetrakis (acetonitrile)copper (I) hexafluorophosphate, and copper (I) 2-thiophenecarboxylate); nickel catalysts (such as bis(cyclooctadiene)nickel (0), nickel (II) chloride); palladium catalysts (such as palladium (II) acetate, tetrakis(triphenylphosphine) palladium (o), II), tris(dibenzylideneacetone)dipalladium (II)).

When the metallic catalyst is used in the reaction, the metallic catalyst is usually used within a range of 0.01 to 0.5 molar ratios as opposed to 1 mole of the compound (M-2).

A ligand may be used in the reaction as needed. Examples of the ligand include triphenylphosphine, 4, 5-bis(diphenylphosphino)-9,9-dimethyl xanthene (hereinafter, referred to as "Xantphos"), 2,2'-bis(diphenylphosphino)-1,1'-binaphthyl, 1,1'-bis(diphenylphosphino)ferrocene, 2-dicylohexylphoshiho-2',4',6'-triisopropylbiphenyl, 2-dicyclohexylphoshino-2',6'-dimethoxybiphenyl, 1,2-bis(diphenylphoshino)ethane, 2,2'-bipyridine, 2-aminoethanol, 8-hydroxyquinoline, 1,10-phenanthroline, trans-1,2-cyclohexanediamine, trans-N,N'-dimethylcyclohexane-1,2-diamine, and N,N'-dimethylenediamine.

When a ligand is used in the reaction, the ligand is usually used within a range of 0.01 to 0.5 molar ratios, as opposed to 1 mole of the compound (K-2),

The reaction temperature of the reaction is usually within a range of -20 to 150°C. The reaction period is usually within a range of 0.5 to 24 hours.

When the reaction is completed, water is added to the reaction mixtures, and the reaction mixtures are extracted with organic solvent(s), and the organic layers are worked up (for example, drying and concentration) to obtain the compound (1-5).

The Compound (M-2) is a commercially available compound, or can be prepared according to a known method.

Process 5

An N-oxide compound of the compound represented by formula (I) can be prepared by reacting the compound represented by formula (I) with an oxidizing agent. The reaction can be conducted according to the method described in U.S. 2018/0009778 A1 or WO 2016/121970 A1.

Reference Process 1

A compound represented by formula (M-10) (hereinafter, referred to as "compound (M-10")) and a compound represented by formula (M-1S) (hereinafter, referred to as "compound (M-1S)") can be prepared according to the Scheme below.

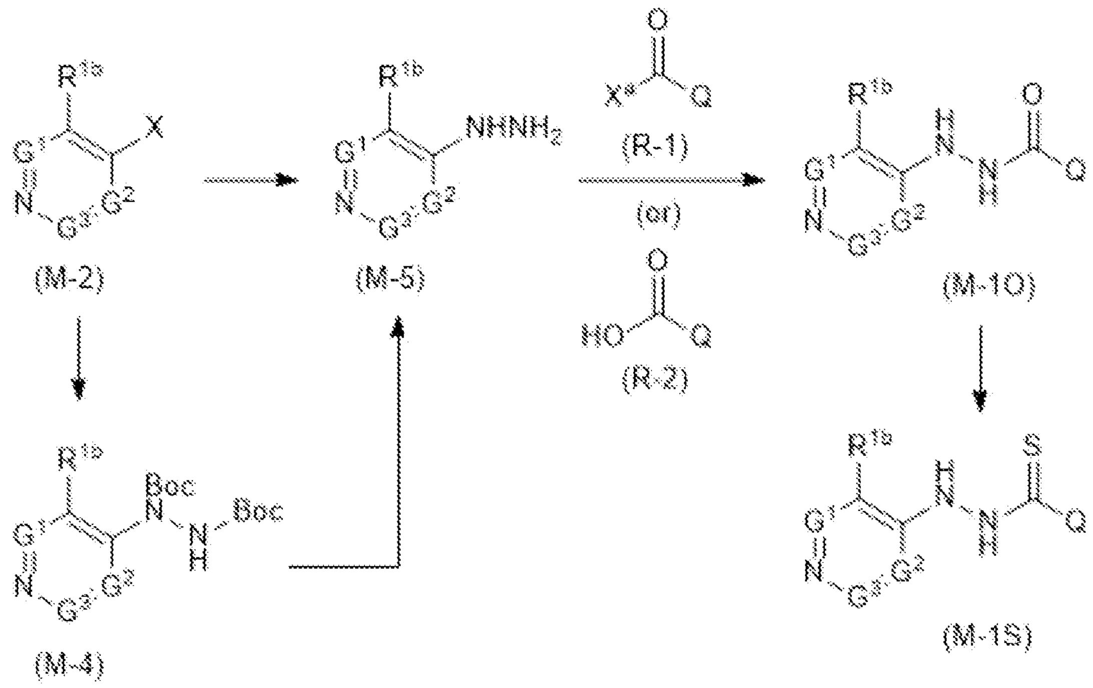


[wherein $X^a$ represents a fluorine atom or a chlorine atom, Boc represents a t-butoacycarbonyl group, and the other symbols are the same as defined above.]

A compound represented by formula (M-4) (hereinafter, referred to as "compound (M-4)") can be prepared by using di-tert-butyl hydrazodicarboxylate in place of the compound. (M-3) according to the process 4.

A compound represented by formula (M-5) (hereinafter, referred to as "compound (M-5)") can be prepared by reacting the compound (M-4) with an acid.

The reaction is usually conducted in a solvent. Examples of the solvent include halogenated hydrocarbons, water, and mixture of these solvents.

Examples of the acid include mineral acids such as hydrochloric acid and sulfuric acid.

In the reaction, the acid is usually used within a range of 1 to 100 molar ratios as opposed to 1 mole of the compound (M-4).

The reaction temperature is usually within a range of 0 to 150°C. The reaction period is usually within a range of 0.1 to 24 hours,

When the reaction is completed, water is added to the reaction mixtures, and the reaction mixtures are extracted with organic solvent(s), and the organic layers are worked up (for example, drying and concentration) to obtain the compound (M-5).

Also the compound (M-5) can be also prepared by reacting the compound (M-2) with hydrazine monohydrate.

The reaction is conducted in a solvent or in the absence of a solvent. Examples of the solvents include ethers; halogenated hydrocarbons; hydrocarbons; nitriles; nitrogen-containing aromatic compounds (such as pyridine) (hereinafter, referred to as "nitrogen-containing aromatic compounds"); aromatic polar solvents; and mixtures of these solvents.

In the reaction, the hydrazine monohydrate is usually used within a range of 1 to 10 molar ratios as opposed to a mole of the compound (M-2).

A base may be used in the reaction as needed. Examples of the base include organic bases, alkali metal carbonates, and alkali metal hydrides. When a base is used in the reaction, the base is usually used within a range of 1 to 10 molar ratios as opposed to 1 mole of the compound (M-2).

The reaction temperature is usually within a range of -20 to 150°C. The reaction period is usually within a range of 0.5 to 24 hours.

When the reaction is completed, water is added to the reaction mixtures, and the reaction mixtures are extracted with organic solvent(s), and the organic layers are worked up (for example, drying and concentration) to obtain the compound (M-5).

The compound (M-10) can be prepared by reacting the compound (M-5) with a compound (R-1) (hereinafter, referred to as "compound (R-1)" in the presence of a base.

The reaction is conducted in a solvent or in the absence of a solvent. Examples of the solvents include ethers, halogenated hydrocarbons, hydrocarbons, nitriles, nitrogen-containing aromatic compounds, aromatic polar solvents, and mixtures of these solvents.

Examples of the bases include organic bases, alkali metal carbonates, and alkali metal hydrides.
In the reaction, the compound (R-1) is usually used within a range of 0.5 to 1.5 molar ratios, and the base is usually used within a range of 1 to 3 molar ratio(s), as opposed to 1 mole of the compound (M-5).
The reaction temperature is usually within a range of -20 to 150°C. The reaction period is usually within a range of 0.5 to 24 hours.
When the reaction is completed, water is added to the reaction mixtures, and the reaction mixtures are extracted with organic solvent(s), and the organic layers are worked up (for example, drying and concentration) to obtain the compound (M-5).
The compound (R-1) is a commercially available compound or can be prepared according to known method.
Also the compound (M-10) can be prepared by reacting the compound (M-5) with a compound represented by formula (R-2) (hereinafter, referred to as "compound (R-2)") in the presence of a condensing agent and a base.
The reaction is conducted in a solvent or in the absence of a solvent. Examples of the solvent include ethers, halogenated hydrocarbons, hydrocarbons, nitriles, nitrogen-containing aromatic compounds, aromatic polar solvents, and mixtures of these solvents.
Examples of the condensing agent include 1H-benzotriazol-1-yloxy tris(dimethylamino)phosphonium hexafluorophosphate (hereinafter, referred to as "BOP"), N,N'-dicyclohexylcarbodiimide, and 1-ethyl-3-(3-dimethylaminopropyl)carbodiimide hydrochloride.
Examples of the base include organic bases, alkali metal carbonates, and alkali metal hydrides.
In the reaction, the compound (R-2) is usually used within a range of 0,5 to 1.5 molar ratios, and the condensing agent is usually used within a range of 1 to 1.5 molar ratio(s), and the base is usually used within a range of 1 to 3 molar ratio (s), as opposed to 1 mole of the compound (M-5).
An additive agent may be used in the reaction as needed. Examples of the additive agent include ethyl 2-cyano-2-(hydroxyamino)acetate, 1-hydroxybenzotriazole, and 1-hydroxy-T-aza-benzotrialzale. When an additive agent is used in the reaction, the additive agent is usually used within a range of 0.1 to 0.5 molar ratios as opposed to 1 mole of the compound (M-5).
The reaction temperature is usually within a range of -20 to 150°C. The reaction period is usually within a range of 0.5 to 24 hours.
When the reaction is completed, water is added to the reaction mixtures, and the reaction mixtures are extracted with organic solvent(s), and the organic layers are worked up (for example, drying and concentration) to obtain the compound (M-10).
The compound (R-2) is a commercially available compound, or can be prepared according to a known method.
The compound (M-1S) can be prepared by reacting the compound (M-10) with a sulfurizing agent. The reaction can be conducted by using the compound (M-10) in place of the compound (I-1) according to the process 1.

Reference Process 2

The compound (M-10) can be prepared by reacting the compound (M-2) with a compound represented by formula (R-3) (hereinafter, referred to as "compound (R-3)") in the presence of a base.

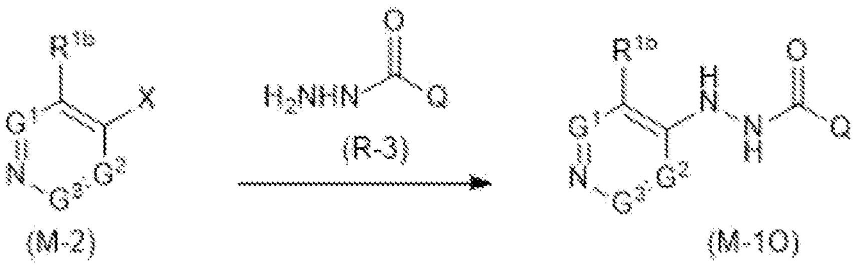

[wherein the symbols are the same as defined above]
The reaction can be conducted by using the compound (R-3) in place of the compound (M-3) according to the process 4.
The compound (R-3) is a commercially available compound or can be prepared according to the known method.

Reference Process 3

A compound represented by formula (M-30) (hereinafter, referred to as "compound (M-30) ") and a compound represented by formula (M-3S) (hereinafter, referred to as "compound (M-3S)") can be prepared according to the following

scheme.

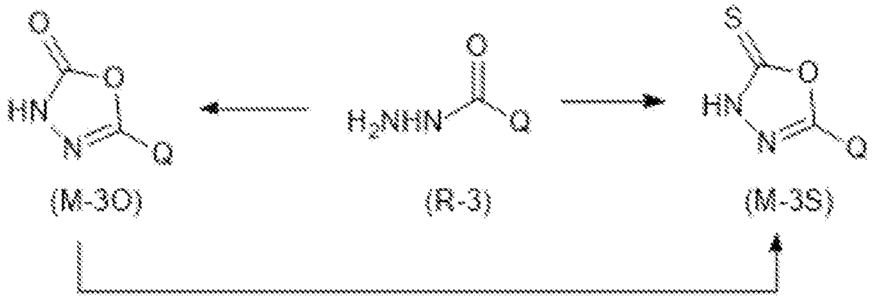

[wherein the symbols are the same as defined above]

The compound (M-30) can be prepared by reacting a compound represented by formula (R-3) (hereinafter, referred to as "compound (R-3)") with a carbonylating agent.

The reaction can be conducted by using the compound (R-3) in place of the compound (M-1) according to the Process 2.

A compound (M-3S) can be prepared by reacting the compound (R-3) with a thiocarbonylating agent.

The reaction can be conducted using the compound (R-3) in place of the compound (M-1) according to the Process 3,

The compound (M-3S) can be also prepared by reacting the compound (M-30) with a sulfurizing agent.

The reaction can be conducted by using the compound (M-3O) in place of the compound (1-1) according to the Process 1.

The present compound can be mixed or combined with one or more ingredients selected from the group consisting of the following Group (a), Group (b), Group (c), and Group (d) (hereinafter, referred to as "present ingredient").

The mixing or combining represents that the present compound and the present ingredient are used concurrently, separately, or at an interval.

When the present compound and the present ingredient are concurrently used, the present compound and the present ingredient may be incorporated as a separate formulation or one formulation.

One aspect of the present invention relates to a composition comprising one or more ingredients selected from the group consisting of the Group (a), the Group (b), the Group (c), and the Group (d) (that is, the present ingredient), and the present compound (hereinafter, referred to as "Composition A").

The Group (a) is a group consisting of acetylcholinesterase inhibitors (for example, carbamate insecticides and organophosphate insecticides), GABA-gated chloride ion channel blockers, (for example, phenylpyrazole insecticides), sodium channel modulators (for example, pyrethroid, insecticides), nicotinic acetylcholine receptor antagonist modulators (for example, neonicotinoid insecticides), nicotinic acetylcholine receptor allosteric modulators, glutamate-gated chloride ion channel allosteric modulators (for example, macrolide insecticides), juvenile hormone mimics, multisite inhibitors, chordotonal organ TRPV channel modulators, mites growth regulators, mitochondrial ATP synthase inhibitors, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, inhibitors of mitochondrial electron transport chain complex I, II, III, and IV, voltage-dependent sodium channel blockers, Inhibitors of acetyl CoA carboxylase, ryanodine receptor modulators (for example, diamide insecticides), chordotonal organ modulators, microbial fungicides, and other insecticidal ingredients, other miticidal ingredients and other nematicidal ingredients. These agents are described in the classification based on the IRAC mode of action.

The Group (b) is a group consisting of nucleic acid synthesis inhibitors (for example, phenylamide fungicides and acylamino acid fungicides), cytostatic and cytoskeletal inhibitors (for example, MBC fungicides), respiration inhibitors (for example, QoI fungicides and QiI fungicides), amino-acid synthesis and protein synthesis inhibitors (for example, anilinopyridine fungicides), signal-transduction inhibitors, lipid synthesis and membrane synthesis inhibitors, sterol biosynthesis inhibitors (for example, DMI fungicides such as triazoles), cell wall synthesis inhibitors, melanin synthesis inhibitors, plant defense inducer, multisite fungicides, microbial fungicides, and other fungicidal ingredients. These agents are described in the classification based on the FRAC mode of action.

The Group (c) represents a group of plant growth modulating ingredients (including mycorrhizal fungus and rhizobia).

The Group (d) represents a group of repellent ingredients.

Examples of combinations of the present ingredient and the present compound are recited as follows. For example, the "alanycarb + SX" indicates a combination of alanycarb and SX.

The abbreviation "SX" means to any one of the present compounds selected from the compound classes SX1

to SX931. Also any of the present ingredients as described below are a known ingredient, and can be obtained from a commercially available drug or can be prepared according to a known method. When the present ingredient represents a microorganism, the present ingredient can be obtained from a microorganism depositary authority. The number in parentheses represents CAS RN (registered trademark).

A combination of the present ingredient in the above-mentioned Group (a) and the present compound: abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, Celastrus angulatus (bark of Celastrus angulatus) + SX, bendiocarb + SX, benfluthrin + SX, benfuracarb + SX, bensultap + SX, benzoximate + SX, benzpyrimosran + SX, beta-cyfluthrin + SX, beta-cypermethrin + SX, bifenazate +. SX, bifenthrin + SX, bioallethrin + SX, bioresmethrin + SX, bistrifluron + SX, borax + SX, boric acid + SX, btoflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat. + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cyoloniliprole + SX, cyclobutrifluram) + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphsnothr.in + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC (2-methyl-4,6-dinitrophenol) + SX, doramectin + SX, dried leaves of Dryopteris filix-maa + SX, eraamectin-benzoate + SX, empenthrin + SX, endosulfan + SX, EPN (O-fethyl O-(4-nitrophenyl)phenylphosphonothioate) + SX, epsilon-metofluthrin + SX, epsilon-momfluorQthrin + SX, esfenvalerate + SX, ethiofencarb + SX, ethion + SX, ethiprole + SX, ethoprophos + SX, etofenprox + SX , etoxazole + SX, extract of Artemisia absinthium + SX, extract of Azadirachta indica + SX, extract of Cassia nigricans + SX, extract of clitoria ternatea + SX, extract of Symphytum officinale + SX, extracts or simulated blend of Chenopodium ambrosioldes + SX, extract of Tanacetum vulgare + SX, extract of Urtica dioica + SX, extract of Viscum album + SX, famphut + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuzon + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, fluralaner + SX, fluvalinate + SX, fluxametainide + SX, formetanate + SX, fosthiazate + SX, ftxrameth=in + SX, furathiocarb + SX, gamma-cyhalothrin + SX, GS-omega/kappa HXTX-Hvla peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoprocarb + SX, isopropyl-O-(methoxyaminothiophosphoryl)salicylate + SX, isoxathion + SX, ivermectin + SX, kadethrin + SX, kappa-tefluthrin + SX, kappa-bifenthrin + SX, kinoprene + SX, lambda-cyhalothrin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, meyinphos + SX, milbemectin +. SX, milbemycin oxime + SX, momfluorothrin + SX, monocrotophos + SX, moxidectin + SX, naled + SX, nicofluprole + SX, nicotine. + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phoaphamidon + SX, phosphine + SX, phoxim + SX, pirimicarb + SX, pirimiphoa-methyl + SX, potassium cyanide + SX, prallethrin + SX, profenofos + SX, profluthrin + SX, propargite + SX, propetampbos + SX, propoxur + SX, propylene glycol alginate + SX, prothiofoa + SX, pyflubumide + SX, pymetrozine + SX, pyraclofos + SX, pyrethrins + SX, pyridaben + SX, pyridalyl + SX, pyridaphenthion. + SX, pyrifluquinazone + SX, .pyrimidifen + SX, pyriminostrobin + SX, pyriprole + SX, pyriproxyfen + SX, quinelphos + SX, resmethrin + SX, rotenone + SX, ryanodine + SX, aarolaner + SX, selamectin + SX, sigma-cypermethrin + SX, ailafluofen + SX, sodium borate + SX, sodium metaborate + SX, spidoxamat + SX, spinetoram + SX, spinosad + SX, apirobudifen + SX, spirodiclofen + SX, spiromeslfen + SX, spiropidion + SX, spirotetramat + SX, sulflutamid + SX, sulfotep + SX, sulfoxaflor + SX, sulfur + SX, sulfuryl fluoride + SX, tartar emetic + SX, tau-fluvalinimte + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioidea near ambrosioides + SX, tetrachlorantraniliprole + SX, tetrachlorvinphos + SX, tetradifon + SX, tetramethrin + SX,

tetramethylfluthrin + SX, tetraniliprole + SX, thetacypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tiorantraniliprole + SX, tioxazafen + SX, tolfenpyrad + SX, tralomethrin + SX, transfluthrin + SX, triazamate + SX, triazophoa + SX, trichlorfon + SX, trifluenfuronate + SX, triflumazopyrim + SX, triflumurob + SX, trimethacarb + SX, tyclopyrazoflor + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5,-dihydro-1,2-oxazol-3-yl}indan-1-yl)propanamide (1118626-57-5) + SX, 2-({2-fluoro-4-methyl-5-[(2,2,2-trifluoroethyl)sulfinyl]phenyl}imino)-3-(2,2,2-trifluoroethyl)-1,3-thiazolidin-4-one (1445683-71-5) + SX, (2Z)-2-({2-fluoro-4-methyl-5-[(R)-(2,2,2-trifluoroethyl)sulfinyl]phenyl}imino)-3-(2,2,2-trifluoroethyl)-1,3-thiazolidin-4-one (2377084-09-6) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, 1,4-dimethyl-2-[2-(pyridin-3-yl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl}-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido(2,3-b][1,4]oxazepin-10-one (2607927-97-7) + SX,

BT drop protein Cry1Ab + SX, BT crop protein CrylAc + SX, BT crop protein Cry1Fa + SX, BT crop protein Cry1A.105 + SX, BT crop protein Cry2Ab + SX, BT crop protein Vip3A + SX, BT crop protein mCry3A + SX, BT crop protein Cry3Ab + SX, BT crop protein Cry3Bb + SX, BT crop protein Cry34Ab1/Cry35Ab1 + SX, Adoxophyea orana granulosis virus. BV-0001 strain + SX, Anticarsia gemmatalis mNPV + SX, Autographs californica mNPV + SX, Cydia pomonella GV strain V15 + SX, Cydia pomonella GV strain V22 + SX, Cryptophlebia leucotreta GV + SX, Dendrolimus punctatus cypovirus + SX, Helicoverpa armigera NPV strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Hosema locustae + SX, Orgyia pseudotsugata NPV + SX, Pieris rapae GV + SX, Plodia interpunctella GV + SX, Spodoptera exigua mNPV + SX, Spodoptera littoralis mNPV + SX, Spodoptera litura NPV + SX, Arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype HSa5b + SX, Bacillus sphaericus strain ABTS1743 + SX, Bacillus thuringiensis strain AQ52 + SX, Bacillus thuringiensis strain BD#32 + SX, Bacillus thuringiensis strain CR3 71 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus, thuringiensis subsp. Kurstaki strain BMP123 + SX, Bacillus thuringiensis subsp. Kurstaki strain CCT1306) + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp.. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus, thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis vaR7 216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtaugae strain PRAA4-1T + SX, Dactyllela ellipsoapora + SX, Dectylaria thaumasia + SX, Hirsutella minnesotensis + SX, Hirsutella rhosailiensia + SX, Hirsutella thompsonii + SX, Lagenidium giganteum + SX, Lecanicillium lecanii strain KV01 + SX, Lecanicillium lecanii conidia of strain DAOM19B499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae. var. anisopliae BIPESCO S/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis + SX.

A combination of the present ingredient in the above-mentioned Group (b) and the present compound: acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + 8X, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper(II) acetate + SX, copper(II) hydroxide + SX, copper oxychloride + SX, copper(II) sulfate + SX, coumxystrobin + SX, cyazbfamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper (II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Melaleuca alternifolia + SX, extract of Reynoutria sachalinensis + SX, extract of the cotyledons of lupine plantlets ("BLAD") + SX, extract of Allium sativum + SX, extract of Equisetum arvense + SX, extract of Tropaeolum majus + SX, famoxadone + SX, fenamidone + SX, fenaminstrobin + SX, fenarimol + SX, fenbuconazole + SX, fenfutam. + SX, fenhexamid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin +SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet +. SX, fosetyl + SX, fosetyl-aluminium + SX, fuberidazole + SX, furalaxyl + SX, furametpyr + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoximmethyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbut-razox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametqstrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol. + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, Zinab + SX, ziram + SX, zoxamide +. SX, N'-[4-((3-[(4-chlorophenyl)methyl]-1,2,4-thiadiazol-5-yl)oxy)-2,5-dimethylphenyl]-N-ethyl-N-methylmethanimidamide (1202781-91-6) + SX, N'-{4-[(4,5-dichloroth.iazol-2-yl) oxy] -2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethinimidamide (2055589-28-9) + SX, N'-[2-chlaro-4-(2-fluorophenoxy)-5-methylphenyl)-N-ethyl-N-methylmethanimidamide (2055756-21-1) + SX, N*-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817829-69-5) + SX, 4-(2-bromo-4-fluorophenyl)-N-(2-chloro-6-fluorophenyl)-1,3-dimethyl-1H-pyrazol-5-amine (1362471-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenyl acrylate (3949179-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R,2S,5S)-5-(4-chlorobenzyl)-2-(chloromethyl)-2-methyl-1-(1H-1,2,4-triazol-1-ylmethyl)cyclopentan-1-ol (1801930-06-2) + SX, (1S,2R,5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-11-6) + SX, (1R,23,5S)-2-(chloromethyl)-5-(4-fluorobenzyl)-2-methyl-1-(1H-1,2,4-triazol-1-ylmethyl)cyclopentan-1-ol (1801930-08-4) + SX, (1S,2,R5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1, 2, 4-tris.zol.-.1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl]ethanol (2019215-86-0) + SX, 1-(2,4-dlfluorophenyl)-2-(1H-1,2,4-triazol-1-yl)-1-[1-(4-chloro-2,6-difluorophenoxy)cyclopropyl]ethanol (201.9215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ([2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,32)-5-([1-(4-chlorophenyl)-1H-pyrazol-3-yl]oxy)-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboxamide (2132414-04-9) + SX, N-(1-benzyl-3,3,3-trifluoro-1-methylpropyl)-8-fluoroquinoline-3-carboxamide (2132414-00-5) + SX, 4,4-dimethyl-2-((4-[5-(trifluoromethyl)-1,2,4-oxadiazol-3-yl]phenyl)methyl)isoxazolidin-3-one (2098918-25-1) + SX, 5,5-dimethyl-2-((4-[5-(trifluoromethyl)-1,2,4-oxadiazol-3-yl]phenyl)methyl)isoxazolidin-3-one (2098918-26-2) + SX, N-ethyl-2-methyl-N-(4-[5-(trifluoromethyl)-1,2,4-oxadiazol-3-yl)phenyl)methyl)propanamide + SX, N, 2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl) cyclopropanecarboxamide + SX, N-methoxy-N'-methyl-N-((4-[5-(trifluoromethyl)-1,2,4-oxadiazol-3-yl]phenyl)methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2, 4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluormethyl)benzamide (2043675-28-9) + SX, 3-(4-bromo-7-fluoroindol-1-yl)butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl]-L-alaninate + SX, (1S)-1-[1-(naphthalen-1-yl)cyclopropyl]ethyl N-[(3-hydroxy-4-methoxypyridin-2-yl)carbonyl]-L-alaninate + SX, (1S)-1-[1-(naphthalen-1-yl)cyclopropyl]ethyl N-[(3-acetoxy-4-methoxypyridin-2-yl) carbonyl] -L-alaninate + SX, (1S)-1- [1-(naphthalen-1-yl)cyclopropyl]ethylN-{[-(acetoxymethoxy)-4-methoxypyridin-2-yl]carbonyl}-L-alaninate + SX, N-({4-[5-(trifluoromethyl)-1,2,4-oxadiazol-3-yl]phenyl}methyl) cyclopropanecarboxamide + SX, N-allyl-H-([4-[5-(trifluoromethyl)-1,2,4-oxadiazol-3-yl]phenyl}methyl)acetamide + SX, N-allyl-N-((4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({2-fluorp-4- [5- (trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-((2,3-difluoro-4-[5-(trifluoromethyl)-1,2,4-oxadiazol-3-yl] phenyl}methyl)butanamide + SX, N-methoxy-N-methyl-N'-({4-[5- (trifluoromethyl) -1,2,4-oxadiazol-3-yl]phenyl)methyl)urea + SX, N,N-diethyl-N'-({4-[5-(trifluoromethyl)-1,2,4-oxadiazol-3-yl]phenyl}methyl)urea + SX, N-methyl-N'-({4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl)methyl)piperidin-2-one + SX, 4-({4-[5-(trifluoromethyl}-1,2,4-oxadiazol-3-yl]phenyl}methyl)morpholin-3-one + SX, 2-({4-[5-(trifluoromethyl)-1,2,4-oxadiazol-3-yl]phenyl]methyl)isoxazolidin-3-one + SX, 3,3-dimethyl-1-({4-(5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-((3-fluoro-4-E5-(trifluoromethyl)-1,2,4-oxadiazol-3-yl]phenyl)methyl)azepan-2~one + SX, 4,4-dimethyl-1-((4-[5-(trifluoromethyl)-1,2,4-oxadiazol-3-yl]phenyl}methyl)pyrrolidin-2-one + SX, 5-methyl-1-([4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-((4-[5-(trifluoromethyl)-1,2,4-oxadiazol-3-yl]phenyl]methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-((4-[5-(trifluoromethyl)-1,2,4-oxadiazol-3-yl]phenyl)methyl)-1H-pyrazple-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,-2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl) -2, 2-difluoroethyl] -5-methylpy.ridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl)(2,6-difluoropyridin-4-yl)amino]-N-(2,2-dimethylcyclobutyl)-5-methylthiazole-4-carboxamide + SX, 2-[(2-methylpropanoyl)(2,6-difluoropyridin-4-yl)amino]-N-(2,2-dimethylcyclobutyl)-5-methylthiazole-4-carboxamide + SX, 5-[5-(difluoromethyl)-1,3,4-oxadiaxol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl] -5-[(2-chloro-4-methylphenyl)methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, Agrobacterium radiobactor strain K1026 + SX, Agrobacterium radiobactor strain K84 + SX, Bacillus amyloliquefaciens strain PTA-4838 (Aveo (Trademark)) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subap. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerahs strain E325 + SX, Phlebiopsis gigantea strain VRA1992 + SX, Pseudomonas aurectfaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G4 1 + SX, Trichoderma virens strain GL-21 + SX, Trichoderma viride + SX, Variovorax paradoxus strain CGP4526 + SX, Harpin protein + SX.

A combination of the present ingredient in the above-mentioned Group (c) and the present compound:
1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop. + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlor.fenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufen-ethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butyric acid + SX, methyl 5-(trifluoromethyl)benzo[b:thiophene-2-carboxylate + SX, 3-[(6-chloro-4-phenylquinazolin-2-yl)amino]propane-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus ititraradices RTI-801 + SX, Rhizophagus irregularis DAOH 197198 + SX, Azorhizobium caulinodans + SX, Azospirilium amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMTA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Movaik Virus weak strain + SX.

A combination of the present ingredient in the above-mentioned Group (d) and the present compound:
anthraquinone + SX, deet + SX, icaridin + SX.

Examples of the ratio of the present compound to the present ingredient include, but are not particularly limited to 1000 : 1 to 1 : 1000, 500 : 1 to 1 : 500, 100 : 1 to 1 : 100, 50 : 1, 20 : 1, 10 : 1, 9 : 1, 8 : 1, 7 : 1, 6 : 1, 5 : 1, 4 : 1, 3 : 1, 2 : 1, 1 : 1, 1 : 2, 1 : 3, 1 : 4, 1 : 5, 1 : 6, 1 : 7, 1 : 8, 1 : 9, 1 : 10, 1 : 20, and 1 : 50 in the weight ratio (the present compound X: the present ingredient).

The present compound has control effect on harmful arthropods such as harmful insects and harmful mites, harmful nematodes, and harmful mollusks. Examples of the harmful arthropods, harmful nematodes, and harmful mollusks include the followings.

Hemiptera:

from the family Delphacidae, for example, small brown planthopper (Laodelphax striatellus), brown planthopper (Nilaparvate lugens), white-backed planthopper (Sogatella furcifera), corn planthopper (Peregrinus maidis), cereal leafhopper (Javesella pellucida), sugarcane leafhopper (Perkinsiella saccharicida), Tagosodes orizicolus, and the like;

from the family Cicadellidae, for example, green rice leafhopper (Nephotettix cincticeps), green paddy leafhopper

(Nephotettix virescens), rice leafhopper (Nephotettix nigropictus), zigzag-striped leafhopper (Recilia dorsalis), tea green leafhopper (Empoasca onukii), potato leafhopper (Empoasca fabae), corn leafhopper (Dalbulus maidia), rice leafhopper (Cofana spectra), Amrasca biguttula biguttula, and the like;

from the family Aphrophoridate, for example, European spittlebug (Philaenus spumerius), and the like;

from the family Cercopidae, for example, Mahanar.va posticata, Mahanarva fimbriolata, and the like;

from the family Aphididae, for example, bean aphid (Aphis fabae), soybean aphid (Aphis glycines), cotton aphid (Aphis gossypii), green apple aphid (Aphis pomi), apple aphid (Aphis spiraecola), green peach aphid (Myzus persicae), leaf-curling plum aphid (Brachycaudus helithrysi), cabbage aphid (Brevicoryne brassicae), rosy apple aphid (Dysaphia plantaginea), false cabbage aphid (Lipaphis erysimi), potato aphid (Macrosiphum euphorbiae)., foxglove aphid (Aulacorthum solani), lettuce aphid (Nasonovia ribisnigri), grain aphid (Rhopalosiphum padi), corn aphid (Rhopalosiphum maidis), brown citrus aphid (Toxoptera citricida), mealy plum aphid (Hyalopterus pruni), cane aphid (Melanaphis sacchari), black rice root aphid (Tetraneura nigriabdominalis), sugarcane cottony aphid (Ceratovacuna lanigera), apple woolly aphid (Eriosoma lanigerum), English grain aphid (Sitobion avenae), and the like;

from the family Phylloxeridae, for example, grapevine phylloxera (Daktulosphaira vitifoliae), Pecan phylloxera (Phylloxera devastatrix), Pecan leaf phylloxera (Phylloxera notabilis), Southern pecan leaf phylloxera (Phylloxera rusaelae), and the like;

from the family Adelgidae, for example, hemlock woolly aphid (Adelges tsugae), balsam woolly aphid (Adelqes piceae), Aphrastasia pectinatae, and the like;

from the family Pentatomidae, for example, black rice bug (Scotinophara lurida), black paddy bug (Scotinophara coarctata), common green stink bug (Nezara antennata), white-spotted spined bug (Eysarcoris aeneus), lewis spined bug (Eyaarcoris lewisi), white-spotted bug (Eysarcoris ventralis), Eysarcoris annamita, brown marmorated stink bug (Halyomorpha halys), green plant bug (Nezara viridula), Brown stink bug (Euschistus heros), Red banded stink bug (Piezodorus guildinii), Oebalus pugriax, Dichelops melacanthus, and the like;

from the family Cydnidae, for example, Scaptocoris castahea;

from the family Alydidae, for example, bean bug (Riptortus clavatus), corbett rice bug (Leptocorisa chinensis), rice bug (Leptocorisa acuta), and the like;

from the family Coreidae, for example, Cletus punctiger, Australian leaf-footed bug (Leptoglossus australis), and the like;

from the family Lygaeidae, for example, oriental chinch bug (Cavelerius sacchativorus), seed bug (Togo hemipterus), chinch bug (Blissus leucopterus), and the like;

from the family Miridae, for example, rice leaf bug (Trigonotylus caelestialium), sorghum plant bug (Stenotus rubrovittatus), wheat leaf bug (Stenodema calcarata), American tarnished plant bug (Lygus lineolaris), and the like;

from the family Aleyrodidae, for example, greenhouse whitefly (Trialeurodes vaporariorum), tobacco whitefly (Bemisia tabaci), citrus whitefly (Dialeurodes citri), citrus spiny whitefly (Aleurocanthus spiniferus), tea spiny whitefly (Aleurocanthus camelliae), Pealius euryae, and the like;

from the family Diaspididae, for example, Abgrallaspis cyanophylli, red scale (Aonidiella aurantii), San José scale (Diaspidiotus pemiciosus), white peach scale (Pseudaulacaspis pentagona), arrowhead scale (Unaspis yanonensis), citrus snow scale (Unaspis citri), and the like;

from the family Coccidae, for example, pink, wax scale (Ceroplastes rubens);

from the family Margaradidae, for example, fluted scale (Icerya purchasi) seychelles fluted scale (Icerya seychellarum), and the like;

from the family Pseudococcidae, for example, solanum mealybug (Phenacoccus solani), cotton mealybug (Phenacoccus solenopsis), Japanese mealybug (Planococcus kraunhiae), white peach scale (Pseudococcus comstocki), citrus mealybug (Pianococcus citri), currant mealybug (Pseudococcus calceolariae), long-tailed mealybug (Pseudococcus longispinus), tuttle mealybug (Brevennia rehi), and the like;

from the family Psyllidae, for example, citrus psylla (Diaphorina citri), two-spotted citrus psyllid (Trioza erytreae), pear sucker (Cacopsylla pyrisuga), Cacopsylla chinensis, potato psyllid (Bactericera cockerelli), Pear psylla (Cacopsylla pyricola), and the like;

from the family Tingidae, for example, sycamore lace bug (Corythucha ciliata), aster tingid (Corythucha marmorata), Japanese pear lace bug (Stephanitis nashi), azalea lace bug (Stephanitis pyrioides), and the like;

from the family Cimicidae, for example, common bed bug (Cimex lectularius), tropical bed bug (Cimex hemipterus), and the like;

from the family Cicadidae, for example, Quesada gigas, and the like;

from the family Reduviidae, for example, Triatoma infestans, large kissing bug (Triatoma rubrofasciata), Triatoma dimidiate, Rhodonius prolixus, and the like.

Lepidoptera:

from the family Crambidae, for example, rice stem borer (Chilo suppressalis), Dark-headed stem borer (Chilo polychrysus), white, stem borer (Scirpophaga innotata), yellow paddy borer (Scirpophaga incertulas), Rupela albina, rice leaf roller (Cnaphalocrocis medinalis), Marasmia patnalis, rice leaf roller (Marasmia exigua), cotton leaf roller (Notarcha derogata), corn borer (Ostrinia furnacalis), European corn borer (Ostrinia nubilalis), cabbage webworm (Hellula undalis), grape leafroller (Herpetogramma luctuosale), bluegrass webworm (Parapediasia teterrellus), rice case-worm (Nymphula depunetalis), Sugarcane borer (Diatraea saccharalis), eggplant fruit borer (Leucinodes orbonalis), and the like;
from the family Pyralidae, for example, lesser cornstalk borer (Elasmopalpus lignosellus), mealworm moth (Plodia interpunctella), persimmon bark borer (Euzophera batangensis), fig moth (Cadra cautella), and the like;
from the family Noctuidae, for example, cotton worm (Spodoptera litura), beet armyworm (Spodoptera exigua), rice armyworm (Mythimna separata), cabbage moth (Mamestra brassicae), pink borer (Sesamia inferens), grass armyworm (Spodoptera mauritia), green rice caterpillar (Naranga aenescens), Spodoptera frugiperda, true armyworm (Spodoptera exempta), Spodoptera cosmioides, semitropical armyworm (Spodoptera eridania), black cutworm (Agrotis ipsilon), beet worm (Autographa nigrisigna), rice looper (Plusia testucae), soybean looper (Chrysodeixis includens), Trichoplusia spp., Heliothis spp. (such as tobacco budworm (Heliothis virescens)), Helicoverpa spp. (such as tobacco budworm (Helicoverpa armigera), corn earworm (Helicoverpa zea)), Velvet-bean caterpillar (Anticarsia gemmatalis), Cotton leafworm (Alabama argillacea), Hop vine borer (Hydraecia immanis), and the like;
from the family Pieridae, for example, common cabbage worm (Pieris rapae), and the like;
from the family Tortricidae, for example, oriental fruit moth (Grapholita molesta), Grapholita dimorpha, soybean moth (Leguminivora glycinivorella), Mataumuraeses azukivora, summer fruit tortrix (Adoxophyes orana fasciata), smaller tea tortrix (Adoxophyes honmai), Japanese tea tortrix (Homona magnanima), apple tortrix (Archips fuscocupreanus), codling moth (Cydia pomonella), sugarcane shoot borer (Tetramoera schistaceana), Bean Shoot Borer (Epinotia aporemaj, Citrus fruit borer (Citripestis sagittiferella), European Grapevine Moth (Lobesia botrana), and the like.
from the family Gracillariidae, for example, tea leaf roller (Caloptilia theivora), Asiatic apple leaf miner (Phyllonorycter ringoniella), and the like;
from the family Carposinidae, for example, peach fruit moth (Carposiha sasakii), and the like;
from the family Lyonetiidae, for example, Coffee Leaf miner (Leucoptera coffeella), peach leaf miner (Lypnetia clerkella), Lyonetia prunifoliella, and the like;
from the family Lymantriidae, for example, Lymantria spp. (such as gypsy moth (Lymantria dispar)), Euproctis spp. (such as tea lymantriid (Euproctis pseudoconspersa)), and the like;
from the family Plutellidae, for example, diamondback moth (Plutella xylostella), and the like;
from the family Gelechiidae, for example, peach worm (Anarsia lineatella), sweetpotato leaf folder (Helcystogramma triannulella), pink bollworm (Pectinophora gossypiella), potato moth (Phthorimaea operculella), Tuta absoluta, and the like;
from the family Arctiidae, for example, American white moth (Hyphantria cunea), and the like;
from the family Castniidae, for example, Giant Sugarcane borer (Telchin licus), and the like;
from the family Cossidae, for example, Cossus insularls, and the like;
from the family Geometridae, for example, Ascotis selenaria, and the like;
from the family Limacodidae, for example, blue-striped nettle grub (Parasa lepida), and the like;
from the family Stathmopodidae, for example, persimmon fruit moth (Stathmopoda masinissa), and the like;
from the family Sphingidae, for example, tobacco hornworm (Acherontia lachesis), and the like;
from the family Sesiidae, for example, Nokona feralis, cherry borer (Synanthedon hector), Synanthedon tenuis, and the like:

from the family Hesperiidae, for example, rice skipper (Parnara guttata), and the like;
from the family Tineidae, for example, casemaking clothes moth (Tinea translucens), common clothes moth (Tineola bisselliella), and the like.

Thysanoptera:

from the family Thripidae, for example, western flower thrips (Frankliniella occidentalis), oriental thrips (Thrips palmi), yellow tea thrips (Scirtothripa dorsalis), onion thrips (Thrips tabaci), eastern flower thrips (Frankliniella intense), rice thrips (Stenchaetothrips biformis), Echinothrips americanus, avocado thrips (Scirtothrips perseae), and the like;
from the family Phlaeothripidae, for example, aculeated rice thrips (Haplothrips aculeatus), and the like.

Diptera:

from the family Anthomyiidae, for example, seedcorn maggot (Delia platura), onion maggot (Delia antiqua), beet leaf miner (Pegomya cunicularia), and the like;
from the family Ulidiidae, for example, sugarbeet root maggot (Tetanops myopaeformis), and the like;
from the family Agromyzidae, for example, rice leaf miner (Agromyza oryzae), tomato leaf miner (Liriomyza sativae), chrysanthemum leaf miner (Liriomyza trifolii), pea leafminer (Chromatomyia horticola), and the like;
from the family Chloropidae, for example, rice stem maggot (Chlorops oxyzae), and the like;
from the family Tephritidae, for example, melon fly (Bactrocera cucurbitae), oriental fruit fly (Bactrocera dorsalis), Malaysian fruit fly (Bactrocera latifrons), olive fruit fly (Bactrocera oleae), Queensland fruit fly (Bactrocera tryoni), Mediterranean fruit fly (Ceratitia capitata), apple maggot (Rhagoletis pomonella), Japanese cherry fruit fly (Rhacochlaena japonica), and the like;
from the family Ephydridae, for example, smaller rice leaf miner (Hydrellia griseola), whorl maggot (Hydrellia philippina), paddy stem maggot (Hydrellia saaakii), and the like;
from the family Drosophilidae, for example, cherry drosophila (Drosophila suzukii), fruit-fly (Drosophila melanogaster), and the like;
from the family Photidae, for example, Megaselia spiracularis, and the like;
from the family Psychodidae, for example, Clogmia albipunctata, and the like;
from the family Sciaridae, for example, Bradysia difformis, and the like;
from the family Cecidomyiidae, for example, hessian fly (Mayetiola destructor), paddy gall fly (Orseolia oryzae), and the like;
from the family Diopsidate, for example, Diopsis macrophthalma, and the like;
from the family Glossinidae, for example, Glossina palpalis, Glossina morsitans, and the like;
from the family Simuliidae, for example, Simulium japonicum, Simulium damnosum, and the like;
from the subfamily Phlebotominae;
from the family Tipulidae, for example, rice crane fly (Tipula aino), Common cranefly (Tipula oleracea), European cranefly (Tipula paludosa), and the like;
from the family Culicidae, for example, southern house mosquito (Culex pipiens pallens), Culex tritaeniorhynchus, Culex pipiens f. moleatus, brown house mosquito (Culex quinquefasciatus), northern house mosquito (Culex pipiens pipienis), Culex vishnui, Asian tiger mosquito (Aedes albopictus), dengue mosquito (Aedes aegypti), Chinese malaria mosquito (Anopheles sinensis), Anopheles gambiae, Anopheles stephensi, Anopheles coluzzii, Anopheles albimanus, Anopheles sundaicus, Anopheles arabiensis, Anopheles funestus, Anopheles darlingi, Anopheles farauti, Anopheles minimus, and the like;
from the family Simulidae, for example, Prosimulium yezoensis, Simulium ornatum, and the like;
from the family Tabanidae, for example, Tabanus trigonus, and the like;
from the family Muscidae, for example, house fly (Musca domestica), false stable fly (Muscina stabulans), biting house fly (Stomoxys calcitrans), buffalo fly (Haematobia irritans), and the like;
from the family Calliphoridae;
from the family Sarcophagidae;
from the family Chironomidae, for example, Chironomus plumosus, Chironomus yoshimatsui, Glyptotendipes tokunagai, and the like;
from the family Fannidae.

Coleoptera:

from the family Chrysomelidae, for example, Diabrotica spp. (such as western corn rootworm (Diabrotica virgifera virgifera), southern corn rootworm (Diabrotica undecimpunctata howardi), northern corn rootworm (Diabrotica barberi), Mexican corn rootworm (Diabrotica, virgifera zeae)), bean leaf beetle (Cerotoma trifurcata), banded cucumber beetle (Diabrotica balteata), Cucurbit Beetle (Diabrotica speciosa), bean leaf beetle (Cerotoma trifurcata)), barley leaf beetle (Oulema melanopus), cucurbit leaf beetle (Aulacophora femoralis), striped flea beetle (Phyllotreta striolata), Cabbage flea beetle (Phyllotreta cruciferae), Western black flea beetle (Phyllotreta pusilla), Cabbage stem flea beetle (Psylliodes chrysocephala), hop flea beetle (Psylliodes punctulata), Colorado potato beetle (Leptinotarsa decemlineata), rice leaf beetle (Oulema oryzae), grape colaspis (Colaspis brunnea), corn flea beetle (Chaetocnema pullcaria), sweet-potato flea beetle (Chaetocnema confinis), potato flea beetle (Epitrix cucumer1s), rice leaf beetle (Dicladispa armigera), southern corn leaf beetle (Myochrous denticollis), Laccoptera quadrimaculata, tobacco flea beetle (Epitrix hirtipennia), and the like;
from the family Carabidae, for example, Seedcorn beetle (Stenolophus lecontei), Slender seed-corn ground beetle (Clivina impressifrons), and the like;
from the family Scarabaeidae, for example, cupreus chafer (Anomala cuprea), soybean beetle. (Anomala rufocuprea), Anomala albopilosa, Japanese beetle (Popillia japonica), yellowish elongate chafer (Beptophylla picea), European Chafer (RhizottogUs majalis), Tomarua gibbosus, Holotrichia spp., Phyllophaga spp. (such as June beetle (Phyllophaga crinita)), and Diloboderus spp. (such as Diloboderus abderus), and the like;

from the family Anthriibidae, for example, coffee bean weevil (Araecerus coffeae), and the like;

from the family Aponidae, for example, sweet potato weevil (Cylas formicarius), and the like;

from the family Bruchidae, for example, Mexican bean weevil (Zabrotes subfasciatus), and the like;

from the family Scolytidae, for example, pine beetle (Tomicus piniperda) and coffee berry borer (Hypothenemus hampei);

from the family Curculionidae, for example, West Indian sweet-potato weevil (Euscepes postfasciatus), alfalfa weevil (Hypera postica), maize wevil (Sitophilus zeamais), rice weevil (Sitophilus oryzae), grain weevil (Sitophilus granarius), rice plant weevil (Echinocnemus squameua), rice water weevil (Lissorhoptrus oryzophilus), Rhabdoscelus lineaticollis, boll weevil (Anthonomus grandis), nunting billbug (Sphenophorus venatus), southern corn billbug (Sphenophorus callosus), soybean stalk weevil (Sternechus subsignatus), sugarcane weevil (Sphenophorus levis), rusty gourd-shaped weevil (Scepticus griseus), brown gourd-shaped weevil (Scepticus uniformis), Aracanthus spp. (such as Aracanthus mourei), and cotton root borer (Eutinobothrus brasiliensis);

from the family Tenebrionidae, for example, red meal beetle (Tribolium castaneum), mason beetle (Tribolium confusum), and lesser mealworm (Alphitobius diaperinus);

from the family Coccinellidae, for example, twenty-eight-spotted ladybird (Epilachna vigintioctopunctata);

from the family Bostrychidae, for example, common powder-post beetle (Lyctus brunneus), and lesser grain borer (Rhizopertha dominica);

from the family Ptinidae;

from the family Cerambycidae, for example, citrus long-horned beetle (Anoplophora malasiaca), Migdolus fryanus, and peach borer (Aromia bungii);

from the family Elateridae, for example, Melanotus okinawensis, barley wireworm (Agriotes fuecicollis),, Melanotus legatus, Anchastus spp., Conoalerus spp., Ctenicera spp., Limonius spp., and Aeolus spp.;

from the family Staphylinidae, for example, Paederus fuscipes;

from the family Dermestidae, for example, varied carpet beetle (Anthrenus verbasci), hide beetle (Dermestes maculates), and khapra beetle (Trogoderma granarium);

from the family Anabiidae, for example, tobacco beetle (Lasioderma serricorne), and biscuit beetle (Stegobium paniceum);

from the family Laemophloeidae, for example, flat grain beetle (Cryptalestes ferruginous);

from the family Silvanidae, for example, saw-toothed, grain beetle (Oryzaephilus surinamensis);

from the family Nitidulidae, for example, blossom beetle (Brassicogethes aeneus);

and the others.

Orthoptera:

from the family Acrididae, for example, oriental migratory locust (Locusta migratoria), Moroccan locust (Dociostaurus maroccanus), Australian plaque locust (Chortoicetes terminifera), red locust (Nomadacris septemfasciata), brown locust(Locustana pardalina), tree locust (Anacridium melanorhodon), Italian locust (Calliptamus itsIleus), differential grasshopper (Melanoplus differentialis), two-striped grasshopper (Melanoplus bivittatus), migratory grasshopper (Melanoplus sanguinipes), red-legged grasshopper (Melanoplus femurrubrum), clear-winged grasshopper (Camnula pellucida), desert locust (Schistocerca gregaria), Yellow-winged locust (Gastrimargus musicus), spur-throated locust (Austracris guttulosa), Japanese grasshopper (Ozya yezoensis), rice grasshopper (Oxya japonica), and Bombay locust (Patanga succincta);

from the family Gryllotalpidae, for example, oriental mole cricket (Gryllotalpa orientalis);

from the family Gryllidae, for example, house cricket (Acheta domestica), and emma field cricket (Teleogryllus emma);

from the family Tettigoniidae, for example, mormon cricket (Anabrus simplex);

and the others.

Hymenoptera:

from the family Tenthredinidae, for example, beet sawfly (Athalia rosae) and nippon cabbage sawfly (Athalia japonica);

from the family Formicidae, for example, Solenopsis spp. (such as red imported fire ant (Solenopsis invicta), tropical fire, ant (Solenopsis geminata)), Atta spp. (such as brown leaf-cutting ant (Atta capiguara), Acromyrmex spp., Paraponera clavata, black house ant (Ochetellus glaber), little red ant (Monamorium pharaonis), Argentine ant (Linepithema humile), Formica japonica, Pristomyrmex punctutus, Pheidole noda, big-headed ant (Pheidole megacephala), Camponotus spp. (such as Camponotus japonicus, Camponotus obscuripes), Pogonomyrmex spp. (such

as western harvester ant (Pogonomyrmex occidentalis)), Wasmania spp. (such as Wasmania auropunctata), and long-legged ant (Anoplolepis gracilipes);
from the family Vespidae, for example, Asian giant hornet (Vespa mandarinia, Vespa simillima, Vespa analis, Asian hornet(Vespa velutina), and Polistes jokahamae;
from the family Siricidae, for example, pine wood wasp (Urocerus gigas);
from the family Bethylidae;
and the others,

Blattodea:

from the family Ectobiidae, for example, German cockroach. (Blattella germanica);
from the family Blattidae, for example, smoky-brown cockroach (Periplaneta fuliginosa), American cockroach (Periplaneta americana), Australian cockroach (Periplaneta australasiae), brown cockroach (Periplaneta brunnea), and black cockroach (Blatta orientalis);
from the family Termitidae, for example, Japanese termite (Reticulitermes speratus), Formosan termite (Coptotermes formosanus), western drywood termite (Incisitermes minor), Cryptotermes domesticus, Odontotermes formosanus, Neotermes koshunensis, Glyptotermes satsumensis, Glyptotermes nakajimai, Glyptotermes fuscus, Hodotermopsis sjostedti, Coptotetmes guangzhouensis, Reticulitermes amamianus, Reticulitermes miyatakei, Reticuliterraes kanmonensis, Nasutitermes takasagoensis, Pericaptitermes nitobei, Sinocapritermes mushae, and Cornitermes cumulans; and the others.

Siphonaptera:

from the family Pulicidae, for example, human flea (Pulex irritans), cat flea (Ctenocephalides felis), dog flea (Ctenocephalides canis), oriental rat flea (Xenopsylla cheopis)., and chicken flea (Echidnophaga gallinacea);
from the family Pulicidae, for example, chigoe flea (Tunga penetrans);
from the family Ceratopbyllidae, for example, European rat flea (Nosopsyllus fasciatus); and the others.

Psocodae:

from the family Pediculidae, for example, head louse (Pediculus humanus capitis);
from the family Pthiridae, for example, crab louse (Pthirus pubis);
from the family Haematopinidae, for example, short-nosed cattle louse (Haematopinus eurysternus) and pig louse (Haematopinus suis);
from the family Linognathidae, for example, blue cattle louse (Linognathus vituli), sheep face louse (Linognathus ovillus, and capillate louse (Solenopotes capillatus);
from the family Bovicoliidae, for example, cattle biting louse (Bovicola bovis), sheep biting louse (Bovicola ovis), Bovicola breviceps, Damalinia forficula, and Werneckiella spp.;
from the family Trichodectidae, for example, dog biting louse (Trichodectes canis) and cat louse (Felicola subrostratus);
from the family Mehoponidae, for example, common chicken louse (Menopon gallinae), chicken body louse (Menacanthus stramineus), and Trinoton spp.;
from the family Trimenoponidae, for example, Cummingsia spp. ;
from the family Trogiidae, for example, death watch (Trogium pulsatorium);
from the family Liposcelidae or Liposcelididae, for example, book louse (Liposcelis corrodens), Liposcelis bostrychophila, Liposcelis pearmani and Liposcelis entomophila;
and the other.

Thysanura:
from the family Lepismatidae, for example, oriental silverfish (Ctenolepisma villosa) and moth fish (Lepisma saccharina); and the others.
Acari:

from the family Tetranychidae, for example, common red spider mite (Tetranychus urticae), kanzawa spider mite (Tetranychus kanzawai), red spider mite (Tetranychus evansi), citrus red mite (Panonychus citri), fruit-tree red spider mite (Panonychus ulmi), and Oligonychus spp.;
from the family Eriophyidae, for example, Japanese citrus rust mite (Aculops pelekassi), Phyllocoptruta citri, tomato mite (Aculops lycopersici), purple mite (Calacarus carinatus), tea rust mite (Acaphylla theavagrans), Eriophyes

chibaensis, apple bud mite (Aculus schlechtendali), Aceria diospyri, Aceria tosichella, and Shevtchenkella sp.;
from the family Tarsonamidae, for example, broad mite (Polyphagotarsonemus latus);
from the family Tenuipalpidae, for example, Brevipalpus phoenicis;
from the family Tuckerellidae;
from the family Ixodidae, for example, Haemaphysalis longicornis, Haemaphysalis flava, Haemaphysalis japonica, Haemaphysalis campanulata, American dog tick (Dermacentor variabilis), Dermacentor taiwanensis, Rocky Mountain wood tick (Dermacentor andersoni), netted tick (Dermacentor reticulatus), Ixodes ovatus, Ixodes persulcatus, black-legged tick (Ixodes scapularis), Ixodes pacificus, Ixodes holocyclus, Ixodes ricinus, lone star tick (Amblyomma americanum), gulf coast tick (Amblyomma maculatum), Rhipicephalus microplus, cattle tick (Rhipicephalus annulatus), brown dog tick (Rhipicephalus sanguineus), Rhipicephalus appendiculatus, and Rhlpicephalus decoloratus;
from the family Argasidae, for example, fowl tick (Argas persicus), Ornithodoros hermsi, and Ornithodoros turicata;
from the family Acaridae, for example, cereal mite (Tyrophagus putrescentiae) and grassland mite (Tyrophagus similis);
from the family Pyroglyphidae, for example, American house dust mite (Dermatophagoides fatinae) and European house dust mite (Dermatophagoides pteronyssinus);
from the family Cheyletidae, for example, Cheyletus eruditus, Cheyletus malaccensis, Chelacaropsis moorei, and Cheyletiella yasguri;
from the family Psoroptidae, for example, sheep scab mite (Psoroptes ovis), horse psoroptic mange mite (Psoroptes equi), Knemidocoptes mutans, ear mange mite (Otodectes cynotis), and Chorioptes spp.;
from the family Sarcoptidae, for example, Notoedres cati, Notoedres muris, and itch mite (Sarcoptes scabiel);
from the family Listrophoridae, for example, Listrophorus gibbox;
from the family Dermanyssi.dae, for example, bird mite (Dermanyssus gallinae);
from the family Macronyssidae, for example, feather mite. (Ornithonyssus sylviarum) and tropical rat mite (Ornithonyssus bacoti);
from the family Varroidae, for example, Varroa jacobsoni;
from the family Demodicidae, for example, dog follicle mite (Demodex canis) and cat follicle mite (Demodex cati);
from the family Trombiculidae, for example, Leptotrombidium akamushi, Leptotrombidium pallidum, and Leptotrombidium scutellare;
and the others.

Araneae:

from the family Eutichuridae, for example, Cheiracanthium japonicum;
from the family Theridiidae, for example, red-back spider (Latrodectus hasaeltii);
and the others.

Polydesmida:
from the family Paradoxosomatidae, for example, flat-backed millipede (Oxidus gracilis) and Nedyopus tambanus; and the others.
Isopoda:
from the family Armadillidiidae, common pill bug (Armadillidium vulgare);
and the others.
Chilopoda:

from the family Scutigeridae, for example, Thereuonema hilgendorfi;
from the family Scolopendridae, for example, giant tropical centipede (Scolopendra subspinipes);
from the family Ethopolyidae, for example, Bothropolys rugosus;
and the others.

Gastropoda:

from the family Limacida.e, for example, tree slug (Limax marginatus) and garden tawny slug (Limax flavus);
from the family Philomycidae, for example, Meghimatium bilineatum;
from the family Ampullariidae, for example, golden apple snail (Pomacea canaliculata);
from the family Lymnaeidae, for example, Austropeplea ollula;
and the others.

Nematoda:

from the family Aphelenchoididae, for example, rice white-tip nematode (Aphelanchoides besseyi);
from the family Pratylenchidae, for example, root lesion nematode (Pratylenchus coffeae), Pratylenchus brachyurus, California meadow nematode (Pratylenchus neglectus), and Radopholus similis;
from the family Heteroderidae, for example, Javanese root-knot nematode (Meloidogyne javanica), southern root-knot nematode (Meloidogyne incognita), guava root-knot nematodes (Meloidogyne enterolobii), northern root-knot nematode (Meloidogyne hapla), soybean cyst nematode (Heterodera glycines), potato cyst nematode (Globodera rostochiensis), and white potato cyst nematode (Globodera pallida);
from the family Hoplolaimidae, for example, Rotylenchulus reniformis;
from the family Anguinidae, for example, strawberry bud nematode (Nothotylenchus acris), and stem nematode (Ditylenchus dipsaci);
from the family Tylenchulidae, for example, citrus, nematode (Tylenchulus semipenetrans);
from the family Longidoridae, for example, dagger nematode (Xiphinema index);
from the family Trichodoridae;
from the family Parasitaphelenchidae, for example, pine wilt disease (Bursaphelenchus xylophilus);
and the others.

The harmful arthropods such as harmful insects and harmful mites, harmful mollusks and harmful nematodes may be harmful insects and harmful mites, harmful mollusks and harmful nematodes which have a reduced susceptibility to or a developed resistance to an insecticide, a mitecide, a molluscicide or a nematicide.

The method for controlling harmful arthropods of the present invention is conducted by applying an effective amount of the Present compound or the Composition A to a harmful pest directly and/or a habitat where the harmful pest lives (for example, plant, soil, an interior of a house, and animal). Examples of a method for controlling harmful arthropods of the present invention include foliar application, soil application, root application, shower application, smoking application, water-surface application, and seed application.

The Present compound or the Composition A is usually used by mixing it with inert carrier (s) such as solid carrier(s), liquid carrier(s), and gaseous carrier(s), surfactant(s), and the like, and as needed, adding thereto auxiliary agent(s) for formulation such as binder(s), dispersant(s), and stabilizer (s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, a tablet, an aerosol formulation, a resin formulation, or the like. In addition to these formulations, the Present compound or the Composition A may be used by formulating it into a dosage form described in Manual on development and use of FAO and WHO Specifications for pesticides, FAO Plant Production and Protection Papers-271-276, prepared by the FAO/WHO Joint Meeting on Pesticide Specifications, 2Q16, ISSN:0259-2517.

These formulations usually comprise 0.0001 to 99% by weight ratio of the Present compound or the Composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the gaseous carrier include fluorocarbon, butane gas, LPG (liquefied petroleum gas), dimethyl ether, nitrogen, and carbon dioxide.

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates). The specific examples of the surfactant include Nimbus (registered trademark), Assist (registered trademark), Aureo (registered trademark), Iharol (registered trademark), Silwet L-77 (registered trademark), BreakThru (registered trademark), SundanceII (registered trademark), induce (registered trademark), Penetrator (registered trademark), AgriDex (registered trademark), Lutensol A8 (registered trademark), NP-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray M (registered trademark), and BANOLE (registered trademark).

Examples of the other auxiliary agent for formulation include binders, dispersants, colorants, and stabilizers, and the specific examples thereof include polysaccharides (for example, starch, gum arable, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

As used herein, examples of the plant include whole plant, stem and leaf, flower, ear, fruit, tree stem, branch, crown, seed, vegetative reproductive organ, and seedling.

A vegetative reproduction organ means a part of plant such as root, stem, and leaf which has a growth capability even when said part is separated from the plant body and placed into soil. Examples of the vegetative reproduction organ include tuberous root, creeping root, bulb, corm or solid bulb, tuber, rhizome, stolon, rhizophore, cane cuttings, propagule, and vine cuttihg. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous, root are also collectively referred to as "bulb". For example, cultivation of potato starts with planting a tuber into soil, and the tuber to be used is generally referred to as "seed potato".

Examples of a method for controlling harmful arthropods by applying an effective amount of the Present compound or the Composition A to soils include a method of applying an effective amount of the Present compound or the Composition A to soils before planting plants or after planting plants, a method of applying an effective amount of the Present compound or the Composition A to a root part of a crop to be protected from damage such as ingestion by harmful arthropods, and a method of controlling harmful arthropods that ingest a plant by permeating and transferring an effective amount of the Present compound or the Composition A from a root into the interior of the plant body. Specifically, examples of the application method include planting hole treatment (spraying into planting holes, soil mixing after planting hole treatment), plant foot treatment (plant foot spraying, soil mixing after plant foot treatment, irrigation at plant foot, plant foot treatment at a later seeding raising stage), planting furrow treatment (planting furrow spraying, soil mixing after planting furrow treatment), planting row treatment (planting row spraying, soil mixing after planting row treatment, planting row spraying at a growing stage), planting row treatment at the time of sowing (planting row spraying at the time of sowing, soil mixing after planting row treatment at the time of sowing), broadcast treatment (overall soil surface spraying, soil mixing after broadcast treatment), side-article treatment, treatment of water surface (application to water surface, application to water surface after flooding), other soil spraying treatment (spraying of a granular formulation on leaves at a growing stage, spraying under a canopy or around a tree stem, spraying on the soil surface, mixing with surface soil, spraying into seed holes, spraying on the ground surfaces of furrows., spraying between plants), other irrigation treatment (soil irrigation, irrigation at a seedling raising stage, chemical solution injection treatment, irrigation of a plant part just above the ground, chemical solution drip irrigation, chemigation), seedling raising box treatment (spraying into a seedling raising box, irrigation of a seedling raising box, flooding into a seedling raising box with chemical solution), seedling raising tray treatment (spraying on a seedling raising tray, irrigation of a seedling raising tray, flooding into a seedling raising tray with chemical solution), seedbed treatment (spraying on a seedbed, irrigation of a seedbed, spraying on a lowland rice nursery, immersion of seedlings), seedbed soil incorporation treatment (mixing with seedbed soil, mixing with seedbed soil before sowing, spraying at sowing before covering with soils, spraying at sowing after covering with soils, mixing with covering with soils), and other treatment (mixing with culture soil, plowing under, mixing with surface soil, mixing with soil at the place where raindrops fall from a canopy, treatment at a planting position, spraying of a granule formulation on flower clusters, mixing with a paste fertilizer).

Examples of the application to seeds (or seed treatments) include an application of the Present compound or the Composition A to seeds or vegetative reproductive organs, and specific examples thereof include spraying treatment in which a suspension of the Present compound or the Composition A is sprayed onto seed surface or the vegetative reproductive organ surface in the form of mist; smearing treatment in which the Present compound or the Composition A is coated a surface of seeds or the vegetative reproductive organ; a soaking treatment in Which the seeds are soaked into the solution of the Present compound or the Composition A for a certain time; and a method for coating the seeds or the vegetative reproductive organ with a carrier containing the Present compound or the Composition A (film coating treatment., pellet coating treatment). Examples of the above-described vegetative reproductive organ include particularly seed potato.

When the Composition A is applied to seeds or vegetative reproductive organs, the Composition A may be also applied to seeds or vegetative reproductive organs as a single formulation, or the Composition A may be applied to seeds or vegetative reproductive organs as multiple different formulations by multiple times. Examples of the method in which the Composition A is applied as multiple different formulations by multiple times include, for exmaple, a method in which the formulations comprising as an active component the Present compound only are applied, and seeds or vegetative reproductive organs are air dried, followed by applying the formulations comprising the Present ingredient: and a method in which the formulations comprising as an active component the present compound and the Present ingredients are applied, and seeds or vegetative reproductive organs are air dried, followed by applying the formulations comprising the Present ingredients other than the already-applied Present ingredients, are included.

As used herein, seeds or vegetative reproductive organs carrying the present compound or the Composition A means seeds or vegetative reproductive organs in the state where the present compound or the Composition A is adhered to a surface of the seeds or the vegetative reproductive organ. The above-described seeds or vegetative reproductive organs carrying the present compound or the Composition A may be adhered by any other materials that are different from the present compound or the Composition A before or after being adhered the present compound or the Composition A to the seeds or vegetative reproductive organs.

Also, when the Composition A is adhered in a form of layer(s) to a surface of seeds or vegetative reproductive organ, the layer(s) is/are composed of one layer or a multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs carrying the present compound or the Composition A can be obtained, for example, by applying the formulations comprising the present compound or the Composition A by the above-described application method to seeds to seeds or vegetative reproductive organs.

When the Present compound or the Composition A is applied for controlling harmful arthropods in agricultural fields, the application dose thereof is usually within a range of 1 to 10,000 g of the Present compound per 10,000 $m^2$. In the case of being applied to seeds or vegetative reproductive organs, the dose of application dose thereof is usually within a range of 0.001 to 100 g of the Present compound per 1 Kg of seeds. When the Present compound or the Composition A is formulated into an emulsifiable concentrate, a wettable powder or a flowable etc., they are usually applied by diluting them with water so as to make an effective concentration of the active ingredients 0.01 to 10,000 ppm, and the dust formulation or the granular formulation, etc., is usually applied as itself without diluting them.

Also, the resin preparation which is processed into a sheet or a string may be applied by winding a plant with a sheet or a string of the resin preparation, putting a string of the resin preparation around a crop so that the plant is surrounded by the string, or laying a sheet of the resin preparation on the soil surface near the root of a plant.

When the present compound or the Composition A is used to control harmful arthropods that live inside a house, the application dose as an amount of the Present compound is usually within a range from 0.01 to 1,000 mg per 1 $m^2$ of an area to be treated, in the case of using it on a planar area. In the case of using it spatially, the application dose as an amount of the Present compound is usually within a range from 0.01 to 500 mg per 1 m of the space to be treated. When the Present compound or the Composition A is formulated into emulsifiable concentrates, wettable powders, flowables or the others, such formulations are usually applied after diluting it with water in such a way that a concentration of the active ingredient is within a range from 0.1 to 10,000 ppm. In the case of being formulated into oil solutions, aerosols, smoking agents, poison baits and the others, such formulations are used as itself without diluting it.

When the Present compound or the composition A is used for controlling external parasites of livestock such as cows, horses, pigs, sheep, goats and chickens and small animals such as dogs, cats, rats and mice, the composition of the present invention may be applied to the animals by a known method in the veterinary field. Specifically, when systemic control is intended, the composition of the present invention is administered to the animals as a tablet, a mixture with feed or a suppository, or by injection (including intramuscular, subcutaneous, intravenous and intraperitoneal injections). On the other hand, when non-systemic control is intended, the composition of the present invention is applied to the animals by means of spraying of the oil solution or aqueous solution, pour-on or spot-on treatment, or washing of the animal with a shampoo formulation, or by putting a collar or ear tag made of the resin formulations to the animal. In the case of being administered to an animal body, the dose of the compound of the Present compound is usually within a range from 0.1 to 1,000 mg per 1 log of an animal body weight.

Also, the composition of the Present compound or the Composition A may be used as an agent for controlling pests in agricultural lands such as paddy fields, fields, turfs, and orchards. Examples of the plants include the followings.

corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous, vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example,

welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus, unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus app. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

EXAMPLES

Hereinafter, the present invention is explained in more detail by Preparation Examples, Reference Preparation Examples, Formulation Examples, and Test Examples, however, the present invention should not be limited to these Examples.

As used herein, Me represents methyl group, Et represents ethyl group, Pr represents propyl group, i-Pr represents isopropyl group, t-Bu represents tert-butyl group, c-Pr represents cyclopropyl group, c-Bu represents cyclobutyl group, c-Pen represents cyclopentyl group, c-Hex represents cyclohexyl group, Ph represents phenyl group, Py2 represents 2-pyridyl group, Py3 represents 3-pyridyl group, and Py4 represents 4-pyridyl group, and Bn represents benzyl group. When c-Pr, c-Bu, c-Pen, c-Hex, Ph, Py2, Py3, and Py4 has any substituent(s), the substituent(s) is described together with a substitution position before the symbol. For example, 1-CN-c-Pr represents 1-cyanocyclopropyl group, 3,4-$F_2$-Ph represents 3,4-difluorophenyl group, 4-$CF_3$-Py2 represents 4-(trifluoromethyl)-2-pyridyl group, and 5-$OCH_2CF_2CF$-Py2 represents 5-(2,2,3,3,3-pentafluoropropoxy)-2-pyridyl group.

Firstly, Preparation examples of the compound of the present invention are described.

When a physical property of a compound is measured by a liquid chromatography / mass spectrometry analysis (hereinafter, referred to as "LCMS"), a measured molecular ion value $[M+H]^+$ or $[M-H]^-$ and a retention time (hereinafter, referred to as "RT") is described. The measured condition for liquid chromatography (hereinafter, referred to as "LC") is described below.

[LC condition]

Column: L-column2 ODS, inner diameter 4.6 mm, length 30 mm, particle size 3 pm

(Chemicals Evaluation and Research Institute, Japan)

UV measurement wavelength: 254 nm
Mobile phase: A solution: 0.1 % aqueous formic acid solution,
B solution: 0.1 % formic acid in acetonitrile
Flow Rate: 2.0 mL/min
Pump: LC-20AD (manufacturer SHIMADZU) 2 umps (high pressure gradient)
Gradient condition: The following concentration gradient shown in [Table. LC1] is liquid-transferred.

[Table LC1]

| Time (min) | A solution (%) | B solution (%) |
|---|---|---|
| 0.01 | 90 | 10 |
| 2.00 | 0 | 100 |
| 4.00 | 0 | 100 |
| 4.01 | 90 | 10 |

[MS condition]

Detector: LCMS-2020 (manufacturer SHIMADZU)
Ionization method: DUIS

Reference Preparation Example 1

Hydrazine monohydrate 48 g was added to a mixture of 3,4,5-trichloropyridine 73 g, potassium carbonate 69 g, and THF 200 mL at 55°C over 30 minutes, and the resulting mixture was stirred for 3 hours. Water was added to the resulting mixture, and the mixture was extracted with methyl isobutyl ketone. The resulting organic layer was washed with saturated brine, and dried over anhydrous sodium sulfate, and concentrated under reduced pressure to obtain an intermediate compound 1 represented by the following formula 61 g.

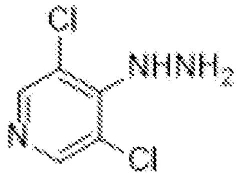

Intermediate compound 1: $^1$H-NMR ($CDCl_3$) $\delta$: 8.22 (2H, s), 6.09 (1H, s), 4.15 (2H, s) .

Reference preparation Example 1-1

The compounds which were prepared according to the Reference Preparation Example 1 and their physical property value are shown below.

A compound represented by formula (A-1):

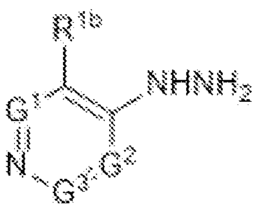

(A-1)

wherein a combination of $G^1$, $R^{1b}$, $G^2$ and $G^3$ represents any combinations described in [Table A-1]. Here, in working examples, "IM" represents an intermediate compound.

[Table A-1]

| IM | $G^1$ | $R^{1b}$ | $G^2$ | $G^3$ |
|---|---|---|---|---|
| 2 | CH | Cl | CH | CH |
| 3 | CH | Br | CH | CH |
| 4 | CH | $NO_2$ | CH | CH |
| 5 | CH | $CF_3$ | CH | CH |
| 6 | C-Cl | Cl | C-Cl | C-Cl |
| 7 | C-Cl | 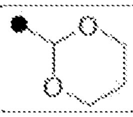 | N | CH |
| 8 | CH | Br | C-Br | CH |

Intermediate compound 2: $^1$H-NMR ($CDCl_3$) δ: 8.24-8.23 (2H, ml, 7.03 (1H, d), 6.08 (1H, s), 3.67 (2H, s).
Intermediate compound 3: $^1$H-NMR ($CDCl_3$) δ: 8.35 (1H, s), 8.24 (1H, d), 7.01 (1H, d), 6.09 (1H, s), 3.69 (2H, a).
Intermediate compound 4: $^1$H-NMR ($CDCl_3$) δ: 9.19 (18, s), 9.06 (1H, s), 8.36 (1H, dd), 7.52 (1H, d), 3.86 (2H, s).
intermediate compound 5: $^1$H-NMR ($CDCl_3$) δ: 8.46 (1H, s), 8.43 (1H, d), 7.24 (1H, d), 6.18 (1H, a), 3.70 (2H, s).
Intermediate compound 6: LCMS: 248 $[M+H]^+$, RT = 1.73 min.
Intermediate compound 7: $^1$H-NMR ($CDCl_3$) δ: 8.34 (1H, s), 7.84 (1H, s), 5.96 (1H, s), 4.28-4.24 (2H, m), 4.06-4.05 (2H, m), 4.01-3.97 (2H, m), 2.27-2.21 (1H, m), 1.53-1.50 (1H, mJ.
Intermediate compound 8: $^1$H-NMR ($CDCl_3$) δ: 8.38 (2H, s), 6.10 (1H, s), 4.12 (2H, s).

Reference Preparation Example 2

Triethylamine 0.9 mL was added to a mixture of the intermediate compound 1 0.89 g, 2-methyl-benzoic acid 0.68 mL, DMF 5 mL and BOP 2.75 g, and the mixture was stirred at room temperature for 1 hour. Aqueous saturated sodium hydrocarbonate solution was added to the resulting mixture, and the mixture was filtered. The filtered materials were washed with water and mixed solvent (ethyl acetate : hexane = 25:75) successively. The obtained solid was dried under reduced pressure to obtain an intermediate compound 9 represented by the following formula 1.10 g.

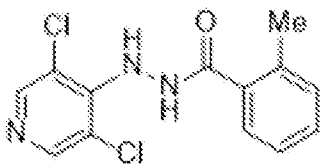

Intermediate compound 9: $^1$H-NMR ($CDCl_3$) δ: 8.36 (2H, s), 7.86 (1H, d), 7.50 (1H, d), 7.41-7.39 (1H, m), 7.28-7.28 (2H, m), 7.09 (1H, d), 2.45 (3H, s).

Reference Preparation Example 2-1

The compounds which were prepared according to the Preparation Example 2 and their physical property value are shown below.
A compound represented by formula (A-2):

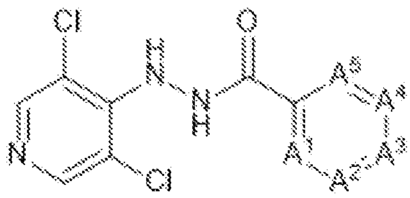 (A-2)

wherein a combination of $A^1$, $A^2$, $A^3$, $A^4$ and $A^5$ represents any combinations described in [Table A-2A], [Table A-2B],

and [Table A-2C].

[Table A-2A]

| IM | $A^1$ | $A^2$ | $A^3$ | $A^4$ | $A^5$ |
|---|---|---|---|---|---|
| 10 | C-F | CH | CH | CH | CH |
| 11 | C-Cl | CH | CH | CH | CH |
| 12 | C-Br | CH | CH | CH | CH |
| 13 | C-$CF_3$ | CH | CH | CH | CH |
| 14 | C-$OCF_3$ | CH | CH | CH | CH |
| 15 | CH | C-F | C-F | CH | CH |
| 16 | CH | C-F | CH | CH | CH |
| 17 | CH | C-Cl | CH | CH | CH |
| 18 | CH | C-Br | CH | CH | CH |
| 19 | CH | C-I | CH | CH | CH |
| 20 | CH | C-$CF_3$ | CH | CH | CH |
| 21 | CH | C-$OCF_3$ | CH | CH | CH |
| 22 | CH | C-$CH_3$ | CH | CH | CH |
| 23 | CH | CH | C-F | CH | CH |
| 24 | CH | CH | C-Cl | CH | CH |
| 25 | CH | CH | C-Br | CH | CH |
| 26 | CH | CH | C-I | CH | CH |
| 27 | CH | CH | C-$CF_3$ | CH | CH |
| 28 | CH | CH | C-$OCF_3$ | CH | CH |
| 29 | CH | CH | C-$CH_3$ | CH | CH |
| 30 | C-OMe | CH | CH | CH | CH |
| 31 | CH | C-OMe | CH | CH | CH |
| 32 | CH | CH | C-OMe | CH | CH |
| 33 | CH | CH | C-iPr | CH | CH |
| 34 | CH | CH | C-tBu | CH | CH |
| 35 | C-iPr | CH | CH | CH | CH |
| 36 | CH | C-iPr | CH | CH | CH |
| 37 | CH | C-$OCF_2CHF_2$ | CH | CH | CH |
| 38 | CH | C-CN | CH | CH | CH |
| 39 | CH | C-$CF_3$ | CH | N | CH |
| 40 | CH | C-$NO_2$ | CH | CH | CH |
| 41 | CH | CH | C-$NO_2$ | CH | CH |
| 42 | CH | 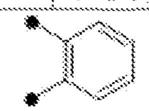 | | CH | CH |

[Table A-2B]

| IM | $A^1$ | $A^2$ | $A^3$ | $A^4$ | $A^5$ |
|---|---|---|---|---|---|
| 43 | CH | C-Br | CH | C-Cl | CH |
| 44 | CH | C-Cl | CH | CH | N |
| 45 | CH | C-Cl | N | CH | CH |
| 46 | CH | C-Cl | C-Cl | CH | CH |
| 47 | C-F | CH | C-F | CH | CH |
| 48 | C-F | CH | C-F | CH | C-F |
| 49 | CH | C-F | C-F | CH | C-F |
| 50 | CH | C-F | CH | C-F | CH |
| 51 | CH | $C-SCF_3$ | CH | CH | CH |
| 52 | CH | CH | $C-SCF_3$ | CH | CH |
| 53 | CH | CH | N | CH | N |
| 54 | CH | N | CH | CH | N |
| 55 | CH | C-Cl | CH | C-F | CH |
| 56 | CH | C-Cl | C-F | CH | CH |
| 57 | CH | $C-CF_3$ | C-F | CH | CH |
| 58 | CH | $C-OCF_3$ | C-F | CH | CH |
| 59 | CH | CH | $C-OCH_2CF_2CF_3$ | CH | N |
| 61 | N | 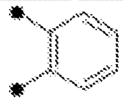 | | CH | CH |
| 62 | CH | 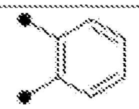 | | N | CH |
| 63 | CH | $C-CF_3$ | CH | C-F | CH |
| 64 | CH | C-F | C-F | C-F | CH |
| 65 | CH | $C-OCH_2CF_3$ | CH | CH | N |
| 66 | CH | $C-OCF_3$ | CH | C-F | CH |
| 67 | CH | $C-CF_3$ | CH | $C-CF_3$ | CH |
| 68 | CH | $C-S(O)CF_3$ | CH | CH | CH |
| 69 | CH | $C-S(O)_2CF_3$ | CH | CH | CH |
| 70 | CH | $C-CHF_2$ | CH | CH | CH |
| 61 | CH | $C-CF_2CF_3$ | CH | CH | CH |
| 71 | CH | C-OPh | CH | CH | CH |
| 72 | CH | C-Br | N | CH | CH |
| 73 | CH | $C-CF_3$ | CH | C-Cl | CH |
| 75 | CH | C-Br | CH | C-F | CH |
| 75 | CH | C-Br | C-F | CH | CH |
| 76 | CH | C-F | C-Cl | C-F | CH |
| 77 | CH | C-Br | CH | C-Br | CH |
| 78 | CH | C-OMe | CH | C-OMe | CH |
| 79 | CH | C-I | C-Me | CH | CH |
| 80 | CH | $C-OCF_3$ | CH | C-Cl | CH |

[Table A-2C]

| IM | $A^1$ | $A^2$ | $A^3$ | $A^4$ | $A^5$ |
|---|---|---|---|---|---|
| 81 | CH | C-$CF_3$ | CH | C-Br | CH |
| 82 | CH | C-F | C-Me | CH | CH |
| 83 | CH | C-Me | C-Me | CH | CH |
| 84 | CH | C-$CF_3$ | CH | C-Me | CH |
| 85 | CH | C-Br | CH | C-Me | CH |
| 86 | CH | C-F | C-Me | C-F | CH |
| 87 | C-F | C-$CF_3$ | CH | CH | CH |
| 88 | CH | C-$CF_3$ | CH | CH | C-F |
| 89 | CH | C-F | C-F | C-Cl | CH |
| 90 | CH | C-Br | C-F | CH | C-F |
| 91 | CH | CH | CH | CH | CH |
| 108 | CH | CH | C-OPh | CH | CH |
| 109 | CH | C-$CF_3$ | CH | CH | N |
| 110 | CH | C-$CF_3$ | N | CH | CH |
| 111 | N | C-$CF_3$ | CH | CH | CH |
| 112 | CH | C-$OCHF_2$ | CH | CH | CH |

Intermediate compound 10: $^1$H-NMR ($CDCl_3$) δ: 8.89 (1H, d), 8.34 (2H, s), 8.10-8.08 (1H, m), 7.58-7.53 (1H, m), 7.31-7.29 (1H, m), 7.21-7.14 (2H, m).
Intermediate compound 11: $^1$H-NMR ($CDCl_3$) δ: 8.43-8.42 (1H, m), 8.36 (2H, s), 7.75 (1H, d), 7.46-7.45 (2H, m), 1.39-1.31 (1H, m), 7.13-7.13 (1H, m).
Intermediate compound 12: $^1$H-NMR ($CDCl_3$) δ: 8.36 (2H, s), 8.15 (1H, s), 7.65 (1H, d), 7.60 (1H, d), 7.38-7.35 (2H, m), 7.11 (1H, d).
Intermediate compound 13: $^1$H-NMR ($CDCl_3$) δ: 8.38 (2H, s), 7.89 (1H, d), 7.77-7.76 (1H, m), 7.66-7.61 (3H, m), 7.07 (1H, d).
Intermediate compound 14: $^1$H-NMR ($CDCl_3$) δ: 8.74 (1H, d), 8.35 (2H, s), 1.06 (1H, dd), 7.60-7.58 (1H, m), 7.43-7.41 (1H, m), 7.37 (1H, d), 7.14 (1H, d).
Intermediate compound 15: $^1$H-NMR ($CDCl_3$) δ: 8.36 (2H, s), 8.15 (1H, d), 7.70-7.65 (1H, m), 7.59-7.57 (1H, m), 7.30-7.28 (1H, m), 7.06 (1H, d).
Intermediate compound 16: $^1$H-NMR ($CDCl_3$) δ: 8.35 (2H, a), 8.17 (1H, s), 7.58 (1H, d), 1.53-7.45 (2H, m), 7.30-7.28 (1H, m), 7.07 (1H, d).
Intermediate compound 17: $^1$H-NMR ($CDCl_3$) δ: 8.35 (2H, a), 8.18 (1H, d), 7.79-7.79 (1H, m), 7.69-7.67 (1H, m), 7.57-7.55 (1H, m), 7.44-7.42 (1H, m), 7.06 (1H, d).
Intermediate compound 18: $^1$H-NMR ($CDCl_3$) δ: 8.35 (2H, s), 8.15 (1H, d), 7.95-7.94 (1H, m), 7.74-7.70 (2H, m), 7.38-7.36 (1H, m), 7.05 (1H, d).
Intermediate compound 19: $^1$H-NMR ($CDCl_3$) δ: 8.34 (2H, s), 8.26 (1H, s), 8.15 (1H, d), 7.92-7.90 (1H, m), 7.17-7.75 (1H, m), 7.23-7.21 (1H, m), 7.04 (1H, d).
Intermediate compound 20: $^1$H-NMR ($CDCl_3$) δ: 8.36 (2H, s), 8.24 (1H, d), 8.08 (1H, s), 7.99 (1H, d), 7.85 (1H, d), 7.65 (1H, d), 7.08 (1H, d).
Intermediate compound 21: $^1$H-NMR ($CDCl_3$) δ: 8.36 (2H, a), 8.23 (1H, s), 7.72-7.7.0 (2H, m), 7.55-7.53 (1H, ml, 7.43 (1H, d), 7.08 (1H, s).
Intermediate compound 22: $^1$H-NMR ($CDCl_3$) δ: 8.34 (2H, s), 8.17 (1H, d), 7.61-7.59 (2H, m), 7.39-7.38 (2H, m), 7.08 (1H, d), 2.42 (3H, s).
Intermediate compound 23: LCMS: 300 $[M+H]^+$, RT = 1.43 min.
Intermediate compound 24: $^1$H-NMR ($CDCl_3$) δ: 8.35 (2H, s), 8.16 (1H, d), 7.75 (2H, d), 7.47 (2H, d), 7.07 (1H, d).
Intermediate compound 25: LCMS: 362 $[M+H]^+$, RT = 1.66 min.
Intermediate compound 26: LCMS: 408 $[M+H]^+$, RT = 1.70 min.
Intermediate compound 27: LCMS: 350 $[M+H]^+$, RT = 1.71 min.
Intermediate compound 28: $^1$H-NMR ($CDCl_3$) δ: 8.35 (2H, s), 8.17 (1H, d), 7.87 (2H, d), 7.33 (2H, d), 7.08 (1H, d).

Intermediate compound 29: $^1$H-NMR ($CDCl_3$) δ: 8.33 (2H, s), 8.14 (1H, d), 7.71 (2H, d), 7.29 (2H, d), 7.08 (1H, d), 2.42 (3H, s).

Intermediate compound 30: $^1$H-NMR ($CDCl_3$) δ: 10.02 (1H, d), 8.32 (2H, a), 8.18-8.16 (1H, m), 7.54-7.49 (1H, m), 7.24 (1H, d), 7.11-7.09 (1H, m), 7.04 (1H, d), 4.06 (3H, s).

Intermediate compound 31: $^1$H-NMR ($CDCl_3$) δ: 8.34 (2H, s), 8.18 (1H, d), 7.41-7.32 (3H, m), 7.12-7.07 (2H, m), 3.85 (3H, s) .

Intermediate compound 32: $^1$H-NMR ($CDCl_3$) δ: 8.33 (2H, s), 8.10-8.09 (1H, m), 7.79-7.77 (2H, m), 7.09-7.08 (1H, m), 6.97-6.96 (2H, m), 3.87 (3H, s).

Intermediate compound 33: LCMS: 324 $[M+H]^+$, RT = 1.76 min

Intermediate compound 34: $^1$H-NMR ($CDCl_3$) δ: 8,33 (2H, s), 8.16 (1B, d), 7.75 (2H, d), 7.50 (2H, d), 7.08 (1H, d), 1.34 (9H, s).

Intermediate compound 35: LCMS: 324 $[M+H]^+$, RT = 2.71 min.

Intermediate compound 36: $^1$H-NMR ($CDCl_3$) δ: 8.34 (2H, s), 8.18-8.17 (1H, m), 7.70 (1H, s), 7.59 (1H, d), 1.43-7.40 (2R, m), 7.09 (1H, s), 3.00-2.94 (1H, m), 1.27 (6H, d).

Intermediate compound 37: LCMS: 398 $[M+H]^+$, RT = 1.72 min.

Intermediate compound 38: $^1$H-NMR ($CDCl_3$) δ: 8.40 (1H, s), 8.36 (2H, s), 8.11 (1H, d), 8.05 (1H, dd), 7.87 (1H, dd), 7.65-7,63 (1H, m), 7.07 (1H, s).

Intermediate compound 39: LCMS: 351 [M+H]+, RT = 1.46 min.

Intermediate compound 40: $^1$H-NMR ($CDCl_3$) δ: 8.67-8.65 (1H, m), 8.46-0.44 (1H, m), 8.37 (2H, s), 8.33 (1H, d), 8.16 (1H, d), 7.73-7.71 (1H, m), 7.08 (1H, d).

Intermediate compound 41: $^1$H-NMR ($CDCl_3$) δ: 8.38 (2H, s), 8.35 (2H, d), 8.27 (1H, d), 7.99 (2H, d), 7.09 (1H, d).

Intermediate compound 42: $^1$H-NMR ($CDCl_3$) δ: 8.37-8.33 (4H, m), 7.96-7.94 (2H, m), 7.91 (1H, d), 7.84 (1H, d), 7.64-7.58 (2H, m), 7.16 (1H, s).

Intermediate compound 43: $^1$H-NMR ($CDCl_3$) δ: 8.36 (2H, s), 8.15 (1H, d), 7.81-7.81 (1H, m), 7.72-7.71 (2H, m), 7.02 (1H, d).

Intermediate compound 44: $^1$H-NMR ($CDCl_3$) δ: 9.81 (1H, s), 8.52 (1H, d), 8.33 (2H, s), 8.12 (1H, d), 7.51 (1H, dd), 6.97 (1H, d).

Intermediate compound 45: $^1$H-NMR ($CDCl_3$) δ: 8.57 (1H, d), 8.36 (2H, s), 8.01 (1H, s), 1.71 (1H, s), 7.58 (1H, d), 7.03 (1H, s).

Intermediate compound 46: LCMS: 352 $[M+H]^+$, RT = 1.77 min.

Intermediate compound 47: $^1$H-NMR ($CDCl_3$) 5: 8.78 (1H, d), 8.34 (2H, s), 8.12 (1H, dd), 7.11 (1H, s), 7.03 (1H, dd), 6.95 (1H, dd).

Intermediate compound 48: $^1$H-NMR ($CDCl_3$) δ: 8.36 (2H, a), 8.13 (1H, s), 7.07 (1H, d), 6.80-6.77 (28, m).

Intermediate compound 49: $^1$H-NMR ($CDCl_3$) δ: 8.80 (1H, d), 8.35 (2H, s), 7.93 (1H, d), 7.10-7.07 (2H, m).

Intermediate compound 50: $^1$H-NMR ($CDCl_3$) δ: 8.36 (2H, s), 8.18 (1H, d), 7.34-7.30 (2H, m), 7.07-7.00 (2H, m).

Intermediate compound 51: $^1$H-NMR ($CDCl_3$) δ: 8.36 (2H, s), 8.24 (1H, d), 8.10 (1H, s), 7.93 (1H, d), 7.87 (1H, d), 7.58-7.56 (1H, m), 7.08 (1H, d).

Intermediate compound 52: $^1$H-NMR ($CDCl_3$) δ: 8.36 (2H, s), 8.21 (1H, d), 7.85 (2H, d), 1.77 (2H, d), 7.08 (1H, d).

Intermediate compound 53: $^1$H-NMR ($CDCl_3$) δ: 9.86 (1H, s), 9.31 (1H, s), 9.02 (1H, d), 8.35 (2H, s), 8.05 (1H, d), 6.99 (1H, s).

Intermediate compound 54: $^1$H-NMR ($CDCl_3$) δ: 9.68 (1H, d), 9.34 (1H, d), 8.83 (1H, d), 8.60 (1H, dd), 8.34 (2H, s), 6.99 (1H, d).

Intermediate compound 55: $^1$H-NMR ($CDCl_3$) δ: 8.35 (28, s), 8.29 (1H, a), 7.58 (1H, s), 7.42 (1H, d), 7.30 (1H, d), 7.04 (1H, s).

Intermediate compound 56: $^1$H-NMR ($CDCl_3$) δ: 8.35 (2H, s), 8.13 (1H, s), 7.90-7.88 (1H, m), 7.72-7.71 (1H, m), 7.18-7.16 (1H, m), 7.05 (1H, s).

Intermediate compound 57: LCMS: 368 $[M+H]^+$, RT = 1.76 min.

Intermediate compound 58: $^1$H-NMR ($CDCl_3$) δ: 8.36 (2H, s), 8.22 (1H, d), 7.83 (1H, d), 7.77-7.74 (1H, m), 1.34-7.32 (1H, m), 7.05 (1H, d).

Intermediate compound 59: $^1$H-NMR ($CDCl_3$) δ: 9.70 (1H, s), 8.34- 8.32 (3H, m), 8.13 (1H, d), 7.37 (1H, d), 6.96 (1H, d), 4.56-4.53 (2H, m).

Intermediate compound 60: $^1$H-NMR ($CDCl_3$) δ: 10.10 (1H, d), 8.35-8.34 (3H, m), 8.21-8.17 (2H, m), 7.91 (1H, d), 7.84-7.80 (1H, m), 7.69-7.65 (1H, m), 7.03 (1H, d).

Intermediate compound 61: $^1$H-NMR ($CDCl_3$) δ: 9.30 (1H, d), 8.64 (1H, d), 8.41 (1H, d), 8.38 (2H, s), 8.18 (1H, d), 7.95 (1H, d), 7.89-7.85 (1H, m), 7.68-7.66 (1H, m), 7.15 (1H, d).

Intermediate compound 62: LCMS: 368$[M+H]^+$, RT = 1.78 min.

Intermediate compound 63: $^1$H-NMR ($CDCl_3$) δ: 8.36 (2H, s), 8.12 (1H, d), 7.47-7.46 (2H, m), 7.03 (1H, d).

Intermediate compound 64: $^1$H-NMR ($CDCl_3$) δ: 9.70 (1H, d), 8.35 (1H, d), 8.32 (2H, s), 8.12 (1H, d), 7.36 (1H, dd),

6.95 (1H, d), 4.48 (2H, q).

Intermediate compound 55: $^1$H-NMR ($CDCl_3$) δ: 8.36 (2H, s), 8.28 (1B, a), 7.48-7.45 (2H, m), 7.18 (1H, d), 7.05 (1H, s).

Intermediate compound 66: $^1$H-NMR ($CDCl_3$) δ: 8.46 (1H, d), 8.38 (2H, s), 8.27 (2H, s), 8.09 (1H, s), 7.06 (1H, d).

Intermediate compound 67: LCMS: 398 $[M+H]^+$, RT = 1.71 min.

Intermediate compound 68: LCMS: 414 $[M+H]^+$, RT = 2.21 min.

Intermediate compound 69: LCMS: 332 $[M+H]^+$, RT = 1.74 min.

Intermediate compound 70 : LCMS: 400 $[M+H]^+$, RT = 1.45 min.

Intermediate compound 71: LCMS: 372 $[M-H]^-$, RT = 1.44 min.

Intermediate compound 72: LCMS: 363 $[M+H]^+$, RT = 1.02 min.

Intermediate compound 73: LCMS: 384 $[M+H]^+$, RT = 1.44 min.

Intermediate compound 74: $^1$H-NMR ($CDCl_3$) δ: 8.36 (2.H, s), 8.23 (1H, d), 1.72 (1H, s), 7.47-7.45 (2H, m), 7.03 (1H, d).

Intermediate compound 75: LCMS: 380 $[M+H]^+$, RT = 1.28 min.

intermediate compound 76: LCMS: 352 $[M+H]^+$, RT = 1.35 min.

Intermediate compound 77: LCMS: 440 $[M+B]^+$, RT = 1.49 min.

Intermediate compound 78: $^1$H-NMR ($CDCl_3$) δ: 8.34 (2H, a), 8.13 (1H, d), 7.05 (1H, d), 6.92 (2H, s), 6.63 (1H, s), 3,83 (6H, s).

Intermediate compound 79: $^1$H-NMR ($CDCl_3$) δ: 8.34 (2H, s), 8.24 (1H, d), 8.12 (1H, d), 7.67 (1H, dd), 7.33 (1H, d), 7.04 (1H, d), 2.49 (3H, s).

Intermediate compound 80: LCMS: 400 $[M+H]^+$, RT = 1.93 min.

Intermediate compound 81: $^1$H-RMR ($CDCl_3$) δ: 8.37 (2H, s), 8.25 (1H, d), 8.11 (1H, a), 7.99-7.97 (2H, m), 7.04 (1H, d).

Intermediate compound 82: $^1$H-NMR ($CDCl_3$) δ: 8,34 (28, s), 8.13 (1H, d), 7.48-7.46 (2H, m), 7.32-7.30 (1H, m), 7,06 (1H, d), 2.34 (3H, s).

Intermediate compound 83: $^1$H-NMR ($CDCl_3$) δ: 8.33 (2H, a), 8.13 (1H, d), 7.58 (1H, s), 7.52 (1H, d), 7.22 (1H, d), 7.07 (1H, d), 2.32 (6H, s).

Intermediate compound 84: $^1$H-NMR ($CDCl_3$) δ: 8.36 (2H, a), 8.21 (1H, d), 7.85 (1H, s), 7.79 (1H, s), 7.64 (1H, a), 7.06 (1H, d), 2.49 (3H, s).

Intermediate compound 85: $^1$H-NMR ($CDCl_3$) δ: 8.35 (2H, s), 8.12 (1H, d), 7.71 (1H, s), 7.53-7.52 (2H, m), 7.03 (1H, d), 2.40 (3H, a).

Intermediate compound 86: $^1$H-NMR ($CDCl_3$) δ: 8.35 (2H, s), 8.11 (1H, s), 7.30-7.29 (2H, m), 7.04 (1H, d), 2.26 (3H, s).

Intermediate compound 87: $^1$H-NMR ($CDCl_3$) δ: 8.82 (1H, dd), 8.36 (2H, s), 8.29-8.27 (1H, m), 7.85-7.83 (1H, m), 7.44-7.42 (1H, m), 7.13 (1H, d).

Intermediate compound 88: $^1$H-NMR ($CDCl_3$) δ: 8.89 (1H, dd), 8.42 (1H, dd), 8.36 (2H, a), 7.85-7.81 (1H, m), 7.35 (1H, dd), 7.13 (1H, d).

Intermediate compound 89: $^1$H-NMR ($CDCl_3$) δ: 8.36 (2H, s), 8.16 (1H, d), 7.66-7.65 (1H, m), 7.59-7.55 (1H, m), 7.02 (1H, d) .

Intermediate compound 90: LCMS: 398 $[M+H]^+$, RT = 1.71 min.

Intermediate compound 91: $^1$H-NMR ($CDCl_3$) δ: 8.34 (2H, s), 8.28 (1H, d), 7.81 (2H, d), 7.60-7.56 (1H, ml, 7.52-7.45 (2H, m), 7.09 (1H, d).

Intermediate compound 108: $^1$H-NMR ($CDCl_3$) 5: 8.33 (2H, s), 8.11 (1H, d), 7.79-7.77 (2H, m), 7.41-7.39 (2H, m), 7.19 (1H, d), 7.06-7.03 (5H, m).

Intermediate compound 109: $^1$H-HMR ($CDCl_3$) δ: 9.85 (1H, d), 8.82 (1H, d), 8.37 (1H, s], 8.34 (2H, s), 7.74 (1H, d), 7.00 (1H, d).

Intermediate compound 110: LCMS: 351 $[M+H]^+$, RT = 1.51 min.

Intermediate compound 111: $^1$H-NHR ($CDCl_3$) δ: 9.82 (1H, d), 8.34-8.32 (3H, m), 8.12-8.09 (1H, m), 7.90 (1H, d), 6.99 (1H, as .

Intermediate compound 112: LCMS: 348 $[M+H]^+$, RT = 1.62 min.

Reference Preparation Example 2-2

The compounds which were prepared according to the Preparation Example 2 and their physical property value are shown below.

A compound represented by formula (A-3):

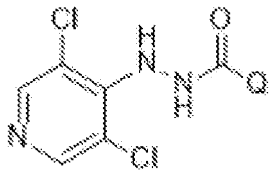 (A-3)

wherein Q represents any substituents described in [Table A-3A] and [Table A-3B].

[Table A-3A]

| IM | Q |
|---|---|
| 92 | 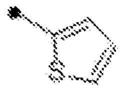 |
| 94 | 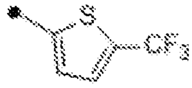 |
| 96 | 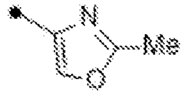 |
| 98 | 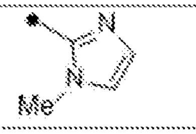 |

[Table A-3B]

| IM | Q |
|---|---|
| 93 | 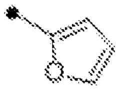 |
| 95 | 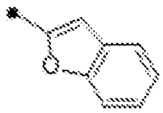 |
| 97 | 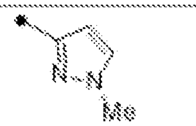 |

Intermediate compound 92: LCMS: 288 $[M+H]^+$, RT = 1.26 min.
Intermediate compound 93: LCMS: 272 $[M+H]^+$, RT = 1.06 min.
Intermediate compound 99: LCMS: 356 $[M+H]^+$, RT = 1.74 min.
Intermediate compound 95: LCMS: 322 $[M+H]^+$, RT = 1.55 min.
Intermediate compound 9.6: $^1H$-NMR ($CDCl_3$) $\delta$: 8.83 (1H, d), 8.32 (2H, s), 8.11 (1H, s), 6.90 (1H, d), 2.51 (3H, s).
Intermediate compound 97: LCMS: 286 [M+H], RT = 0.67 min.
Intermediate compound 98: LCMS: 286 $[M+H]^+$, RT = 1.04 min.

A compound represented by formula (A-4):

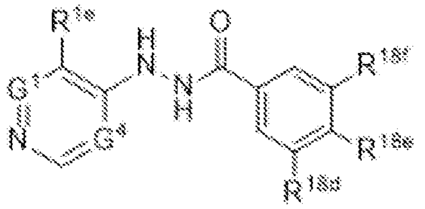

(A-4)

wherein a combination of $G^1$, $G^4$, $R^1$, $R^{18d}$, $R^{18e}$, and $R^{18f}$ represents any combinations described in [Table A-4].

[Table A-4]

| IM | $G^1$ | $G^4$ | $R^{1e}$ | $R^{18d}$ | $R^{18e}$ | $R^{18f}$ |
|---|---|---|---|---|---|---|
| 99 | C-Cl | N | 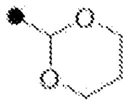 | $CF_3$ | H | H |
| 100 | C-H | C-H | Cl | $CF_3$ | H | H |
| 101 | C-H | C-H | Cl | $OCF_3$ | H | H |
| 102 | C-H | C-H | Br | $CF_3$ | H | H |
| 103 | C-H | C-H | $NO_2$ | $CF_3$ | H | H |
| 104 | C-H | C-H | $CF_3$ | $CF_3$ | H | H |
| 105 | C-H | C-Br | Br | H | H | H |
| 106 | C-H | C-Br | Br | $CF_3$ | H | H |
| 107 | C-H | C-Br | Br | $OCF_3$ | H | H |
| 114 | C-H | C-Br | Br | F | F | H |
| 115 | C-H | C-Br | Br | H | F | H |
| 116 | C-H | C-Br | Br | F | Br | H |
| 117 | C-H | C-F | Cl | $CF_3$ | H | H |
| 118 | C-H | C-F | Cl | F | F | H |

Intermediate compound 99. ; $^1H$-NMR ($CDCl_3$) δ: 9.73 (1H, s), 9.22 (18, s), 8.38 (1H, s), 8.15 (1H, s), 8.05-8.03 (1H, m), 7.84 (1H, d), 7.66-7.64 (1H, m), 6.02 (1H, s), 4.38-4.36 (2H, m), 4.06-4.03 (2H, m), 1.32-1.31 (1H, m), 1.27-1.25 (1H, m).

Intermediate compound 100: LCMS: 314 $[M-H]^-$, RT = 1.08 min.

Intermediate compound 101: LCMS: 330 $[M-H]^-$, RT = 1.16 min.

Intermediate compound 102: $^1H$-NMR ($CDCl_3$) δ: 8.51 (1H, s), 8.28 (1H, d), 8.14 (1H, s), 8.09-8.07 (2H, m), 7.88 (1H, d), 7.69-7.67 (1H, m), 6.81-6.80 (2H, m).

Intermediate compound 103: LCMS: 327 $[M+H]^+$, RT = 1.40 min.

Intermediate compound 104: LCMS: 350 [M+H], RT = 1.19 min.

Intermediate compound 105: LCMS: 370 $[M-H]^-$, RT = 1.40 min.

Intermediate compound 106: LCMS: 440 $[M+H]^+$, RT = 1.74 min.

Intermediate compound 107: LCMS: 454 $[M-H]^-$, RT = 1.78 min.

Intermediate compound 114: $^1H$-NMR ($CDCl_3$) δ: 8.49 (2H, s), 8.28-8.24 (1H, m), 7.73-7.66 (1H, m), 7.62-7.58 (1H, m), 7.33-7.24 (1H, m), 7.07-7.02 (1H, m).

Intermediate compound 115: $^1H$-NMR ($CDCl_3$) δ: 8.50 (2H, s), 8.21 (1H, d), 7.89-7.85 (2H, m), 7.20-7.16 (2H, ml, 7.08 (1H, d) .

Intermediate compound 116: $^1H$-NMR ($CDCl_3$) δ: 8.51 (2H, s), 8.24 (1H, d), 7.74-7.68 (1H, m), 7.62-7.59 (1H, m), 7.51-7.49 (1H, m), 7.06 (1H, d).

Intermediate Compound 117: $^1H$-NMR ($CDCl_3$) δ: 8.36 (1H, s), 8.29 (1H, s), 8.26-8.23 (1H, m), 8.07 (1H, s), 8.00 (1H, d), 7.85 (1H, d), 7.68-7.62 (1H, m), 6.89 (1H, s).

Intermediate compound 118: $^1H$-NMR ($CDCl_3$) δ: 8.29 (1H, s), 8.24 (1H, d), 8.19-8.16 (1H, m), 7.70-7.65 (1H, m), 7.60-7.55 (1H, m), 7.34-7.28 (1H, m), 6.87-6.84 (1H, m).

Preparation Example 1

Triethylamine 1.75 mL was added to a mixture of the intermediate compound 9 1.10 g, THF 25 mL and triphosgene 1.04 g, and the resulting mixture was stirred at room temperature for 1 hour. Aqueous saturated sodium hydrogencarbonate solution 20 mL was added to the resulting mixture, and the mixture was extracted with ethyl acetate. The resulting mixture was dried over anhydrous sodium sulfate, and concentrated under reduced pressure. The resulting residue was subjected to silica gel column chromatography (ethyl acetate: hexane = 30 : 70) to obtain a present compound 1 represented by the following formula 0.38 g.

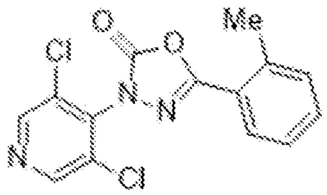

Present compound 1: $^1$H-NMR ($CDCl_3$) $\delta$: 8.71 (2H, s), 7.88 (1H, d), 7.47-7.45 (1H, m), 7.37-7.35 (2H, m), 2.63 (3H, s).

Preparation Example 1-1

The compounds which were prepared according to the Preparation Example 1 and their physical property value are shown below.

A compound represented by formula (B-1):

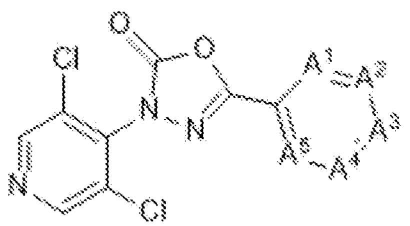

(B-1)

wherein a combination of $A^1$, $A^2$, $A^3$, $A^4$ and $A^5$ represents any combinations described in [Table B-1A], [Table B-1B] and [Table B-1C]. Here in the working examples, "Comp" represents "present compound".

[Table B-1A]

| Comp | $A^1$ | $A^2$ | $A^3$ | $A^4$ | $A^5$ |
|---|---|---|---|---|---|
| 2 | CH | CH | CH | CH | CH |
| 3 | C-Cl | CH | CH | CH | CH |
| 4 | C-Br | CH | CH | CH | CH |
| 5 | C-$CF_3$ | CH | CH | CH | CH |
| 6 | C-$OCF_3$ | CH | CH | CH | CH |
| 7 | CH | C-F | CH | CH | CH |
| 8 | CH | C-Cl | CH | CH | CH |
| 9 | CH | C-Br | CH | CH | CH |
| 10 | CH | C-I | CH | CH | CH |
| 11 | CH | C-$CF_3$ | CH | CH | CH |
| 12 | CH | C-$OCF_3$ | CH | CH | CH |
| 13 | CH | C-$CH_3$ | CH | CH | CH |
| 14 | CH | CH | C-F | CH | CH |
| 15 | CH | CH | C-Cl | CH | CH |
| 16 | CH | CH | C-Br | CH | CH |
| 17 | CH | CH | C-I | CH | CH |
| 18 | CH | CH | C-$CF_3$ | CH | CH |
| 19 | CH | CH | C-$OCF_3$ | CH | CH |
| 20 | CH | CH | C-$CH_3$ | CH | CH |
| 21 | C-OMe | CH | CH | CH | CH |
| 22 | CH | C-OMe | CH | CH | CH |
| 23 | CH | CH | C-OMe | CH | CH |
| 24 | CH | CH | C-iPr | CH | CH |
| 25 | CH | CH | C-tBu | CH | CH |
| 26 | C-iPr | CH | CH | CH | CH |
| 27 | CH | C-iPr | CH | CH | CH |
| 28 | CH | C-$OCF_2CHF_2$ | CH | CH | CH |
| 29 | C-F | CH | CH | CH | CH |
| 30 | CH | C-F | CH | C-F | CH |
| 31 | CH | C-$NO_2$ | CH | CH | CH |
| 32 | CH | CH | C-$NO_2$ | CH | CH |
| 33 | CH | 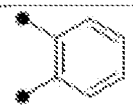 | | CH | CH |
| 34 | CH | C-Br | CH | C-Cl | CH |
| 35 | CH | C-Cl | CH | CH | N |

[Table B-1A] (Continued)

| | | | | | |
|---|---|---|---|---|---|
| 36 | CH | C-Cl | N | CH | CH |
| 37 | CH | C-Cl | C-Cl | CH | CH |
| 38 | C-F | CH | C-F | CH | CH |
| 39 | C-F | CH | C-F | CH | C-F |
| 40 | CH | C-F | C-F | CH | C-F |
| 41 | CH | C-F | C-F | CH | CH |

[Table B-1B]

| Comp | $A^1$ | $A^2$ | $A^3$ | $A^4$ | $A^5$ |
|---|---|---|---|---|---|
| 42 | CH | C-$SCF_3$ | CH | CH | CH |
| 43 | CH | CH | C-$SCF_3$ | CH | CH |
| 44 | CH | CH | N | CH | N |
| 45 | CH | N | CH | CH | N |
| 46 | CH | C-Cl | CH | C-F | CH |
| 47 | CH | C-Cl | C-F | CH | CH |
| 48 | CH | C-$CF_3$ | C-F | CH | CH |
| 49 | CH | C-$OCF_3$ | C-F | CH | CH |
| 50 | CH | CH | C-$OCH_2CF_2CF_3$ | CH | N |
| 51 | N | 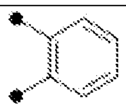 | | CH | CH |
| 52 | CH | 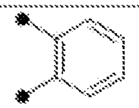 | | N | CH |
| 53 | CH | C-$CF_3$ | CH | C-F | CH |
| 54 | CH | C-F | C-F | C-F | CH |
| 55 | CH | C-$OCH_2CF_3$ | CH | CH | N |
| 56 | CH | C-$OCF_3$ | CH | C-F | CH |
| 57 | CH | C-$CF_3$ | CH | C-$CF_3$ | CH |
| 58 | CH | C-S(O)$CF_3$ | CH | CH | CH |
| 59 | CH | C-S$(O)_2CF_3$ | CH | CH | CH |
| 60 | CH | C-$CHF_2$ | CH | CH | CH |
| 61 | CH | C-$CF_2CF_3$ | CH | CH | CH |
| 62 | CH | C-OPh | CH | CH | CH |
| 63 | CH | C-Br | N | CH | CH |
| 64 | CH | C-$CF_3$ | CH | C-Cl | CH |
| 65 | CH | C-Br | CH | C-F | CH |

[Table B-1B] (Continued)

| | | | | | |
|---|---|---|---|---|---|
| 66 | CH | C-Br | C-F | CH | CH |
| 67 | CH | C-CN | CH | CH | CH |
| 68 | CH | C-Br | CH | C-Br | CH |
| 69 | CH | C-OMe | CH | C-OMe | CH |
| 70 | CH | C-I | C-Me | CH | CH |
| 71 | CH | C-OCF$_3$ | CH | C-Cl | CH |
| 72 | CH | C-CF$_3$ | CH | C-Br | CH |
| 73 | CH | C-F | C-Me | CH | CH |
| 74 | CH | C-Me | C-Me | CH | CH |
| 75 | CH | C-CF$_3$ | CH | C-Me | CH |
| 76 | CH | C-Br | CH | C-Me | CH |
| 77 | CH | C-CF$_3$ | CH | N | CH |
| 78 | C-F | C-CF$_3$ | CH | CH | CH |
| 79 | CH | C-CF$_3$ | CH | CH | C-F |
| 80 | CH | C-F | C-F | C-Cl | CH |
| 81 | CH | C-Br | C-F | CH | C-F |

[Table B-1C]

| Comp | $A^1$ | $A^2$ | $A^3$ | $A^4$ | $A^5$ |
|---|---|---|---|---|---|
| 137 | CH | CH | C-OPh | CH | CH |
| 138 | CH | C-CF$_3$ | CH | CH | N |
| 139 | CH | C-CF$_3$ | N | CH | CH |
| 140 | N | C-CF$_3$ | CH | CH | CH |
| 145 | CH | C-OCHF$_2$ | CH | CH | CH |

Present compound 2: $^1$H-NMR ($CDCl_3$) δ: 8.71 (2H, s), 7.94-7.91 (2H, m), 7.59-7.53 (3H, m).

Present compound 3: $^1$H-NMR ($CDCl_3$) δ: 8.71 (2H, s), 7.90 (1H, dd), 7.58-7.56 (1H, m), 7.53-7.49 (1H, m), 7.45-7.43 (1H, m) .

Present compound 4: $^1$H-NMR ($CDCl_3$) δ: 8.71 (2H, s), 7.86 (1H, d), 7.77 (1H, d), 7.51-7.47 (1H, m), 7.44-7.40 (1H, m).

Present compound 5: $^1$H-NMR ($CDCl_3$) δ: 8.71 (2H, s), 7.94-7.88 (2H, m), 7.75-7.74 (2H, m).

Present compound 6: $^1$H-NMR ($CDCl_3$) δ: 8.71 (2H, s), 7.96 (1H, dd), 7.65-7.62 (1H, m), 7.49-7.46 (2H, m).

Present compound 7: $^1$H-NMR ($CDCl_3$) δ: 8.72 (2H, s), 7.72 (1H, dd), 7.62 (1H, dd), 7.53-7.50 (1H, m), 7.31-7.29 (1H, m) .

Present compound 8: $^1$H-NMR ($CDCl_3$) δ: 8.72 (2H, s), 7.92 (1H, s), 7.81 (1H, d), 7.56 (1H, d), 7.48-7.46 (1H, m).

Present compound 9: $^1$H-NMR ($CDCl_3$) δ: 8.72 (2H, s), 8.08 (1H, s), 7.85 (1H, d), 7.71 (1H, d), 7.42-7.40 (1H, m).

Present compound 10: $^1$H-NMR ($CDCl_3$) δ: 8.71 (2H, s), 8.27 (1H, d), 7.91-7.88 (2H, m), 7.26-7.24 (1H, m).

Present compound 11: $^1$H-NMR ($CDCl_3$) δ: 8.72 (2H, s), 8.20 (1H, s), 8.12-8,10 (1H, m), 7.85-7.83 (1H, m), 7.71-7.67 (1H, m) .

Present compound 12: $^1$H-NMR ($CDCl_3$) δ: 8.72 (2H, s), 7.86 (1H, d), 7.77 (1H, s), 7.59-7.57 (1H, m), 7.44 (1H, d).

Present compound 13: $^1$H-NMR ($CDCl_3$) δ: 8.71 (2H, s), 7.74-7.71 (2H, m), 7.41-7.40 (2H, m), 2.44 (3H, s).

Present compound 14: $^1$H-NMR ($CDCl_3$) δ: 8.71 (2H, s), 7.95-7.91 (2H, m), 7.23-7.21 (2H, m).

Present compound 15: $^1$H-NMR ($CDCl_3$) δ: 8.71 (2H, s), 7.86 (2H, d), 7.51 (2H, d).

Present compound 16: $^1$H-NMR ($CDCl_3$) δ: 8.71 (2H, s), 7.78 (2H, d), 7.67 (2H, d).

Present compound 17: $^1$H-NMR ($CDCl_3$) δ: 8.71 (2H, s), 7.89 (2H, d), 7.63 (2H, d).

Present compound 18: $^1$H-NMR ($CDCl_3$) δ: 8.73 (2H, s), 8.05 (2H, d), 7,80 (2H, d),

Present compound 19: $^1$H-HMR ($CDCl_3$) δ: 8.72 (2H, s), 7.97 (2H, d), 7.37 (2H, d).

Present compound 20: $^1$H-HMR ($CDCl_3$) δ: 8.70 (2H, s), 7.80 (2H, d), 7.32 (2H, d), 2.44 (3H, s).

Present compound 21: $^1$H-NMR ($CDCl_3$) δ: 8.69 (2H, s), 7.82 (1H, dd), 7.55-7.53 (1H, m), 7.10-7.06 (2H, m), 3.96

(3H, s) .

Present compound 22: $^1$H-NMR ($CDCl_3$) δ: 8.71 (2H, s), 7.51 (1H, dd), 7.45-7.41 (2H, m), 7.13-7.10 (1H, m), 3.87 (3H, s).

Present compound 23: $^1$H-NMR ($CDCl_3$) δ: 8.70 (2H, s), 7.85 (2H, d), 7.01 (2H, d), 3.89 (3.H, s).

Present compound 24: $^1$H-NMR ($CDCl_3$) δ: 8.70 (2H, s), 7.89 (1H, d), 7.84 (1H, d), 7.38 (1H, d), 7.16 (1H, d), 3.02-2.95 (1H, m), 1.29 (6H, d).

Present compound 25: $^1$H-NMR ($CDCl_3$) δ: 8.70 (2H, s), 7.85 (2H, d), 7.54 (2H, d), 1.36 (9H, s).

Present compound 26: $^1$H-NMR ($CDCl_3$) δ: 8.71 (2H, s), 7.81 (1H, d), 7.53-7.51 (2H, m), 7.35-7.33 (1H, m), 3.85-3.78 (1H, m), 1.29 (6H, d).

Present compound 27: $^1$H-NMR ($CDCl_3$) δ: 8.71 (2H, s), 7.77 (1H, d), 7.75-7.72 (1H, m), 7.45-7.44 (2H, m), 3.02-2.95 (1H, m), 1.29 (6H, d).

Present compound 28: $^1$H-NMR ($CDCl_3$) δ: 8.72 (2H, s), 7.85 (1H, d), 7.78 (1H, s), 7.58-7.56 (1H, m), 7.44 (1H, d), 5.95 (1H, t).

Present compound 29: $^1$H-NMR ($CDCl_3$) δ: 8.70 (2H, d), 7.90-7.88 (1H, m), 7.60-7.56 (1H, m), 7.35-7.29 (2H, m).

Present compound 30: $^1$H-NMR ($CDCl_3$) δ: 8.72 (2H, s), 7.46-7.45 (2H, m), 7.05-1.03 (1H, m).

Present compound 31: $^1$H-NMR ($CDCl_3$) δ: 8.79 (1H, s), 8.74 (2H, s), 8.44 (1H, d), 8.24 (1H, d), 7.77-7.75 (1H, m).

Present compound 32: $^1$H-NMR ($CDCl_3$) δ: 8.74 (2H, s), 8.40 (2H, d), 8.12 (2H, d).

Present compound 33: $^1$H-NMR ($CDCl_3$) δ: 8.73 (2H, s), 8.43 (1H, s), 7.97-7.94 (3H, m), 7.91 (1H, d), 7.65-7.58 (2H, m).

Present compound 34: $^1$H-NMR ($CDCl_3$) δ: 8.72 (2H, s), 7.96-7.95 (1H, m), 7.85-7.84 (1H, m), 7.72-7.71 (1H, m) .

Present compound 35; $^1$H-NMR ($CDCl_3$) δ: 8.72 (2H, s), 8.69 (1H, dd), 7.98 (1H, dd), 7.51 (1H, dd).

Present compound 36: $^1$H-NMR ($CDCl_3$) δ: 8.74 (2H, s). 8.61 (1H, d), 7.82 (1H, d), 7.70 (1H, dd).

Present compound 37: $^1$H-NMR ($CDCl_3$) δ: 8.72 (2H, s), 8.02 (1H, d), 7.75 (1H, dd), 7.62 (1H, d).

Present compound 38: $^1$H-NMR ($CDCl_3$) δ: 8.71 (2H, s), 7.91-7.88 (1H, m), 7.09-7.00 (2H, m).

Present compound 39: $^1$H-NMR ($CDCl_3$) δ: 8.71 (2H, s), 6.91-6.85 (2H, m).

Present compound 40: $^1$H-NMR ($CDCl_3$) δ: 8.71 (2H, s), 7.74-7.71 (1H, m), 7.17-7.14 (1H, m).

Present compound 41: $^1$H-NMR ($CDCl_3$) δ: 8.76 (2H, s), 7.82-7.77 (1H, m), 7.76-7.72 (1H, m), 7.41-7.34 (1H, m).

Present compound 42: $^1$H-NMR ($CDCl_3$) δ: 8.72 (2H, s), 8.22 (1H, s), 8.05-8.04 (1H, m), 7.87-7.87 (1H, m), 7.63-7.59 (1H, m) .

Present compound 43: $^1$H-NMR ($CDCl_3$) δ: 8.72 (2H, s), 7.97 (2H, d), 7.81 (2H, d).

Present compound 44: $^1$H-NMR ($CDCl_3$) δ: 9.44 (1H, d), 9.03 (1H, d), 8.73 (2H, s), 7.91 (1H, d).

Present compound 45: $^1$H-NMR ($CDCl_3$) δ: 9.23-9.22 (1H, m), 8.80-8.79 (1H, m), 8.78-8.77 (1H, m), 8.73 (2H, a).

Present compound 46: $^1$H-NMR ($CDCl_3$) δ: 8.72 (2H, s), 7.73 (1H, s), 7,54-7.52 (1H, m), 7.32-7.30 (1H, m).

Present compound 47: $^1$H-NMR ($CDCl_3$) δ: 8.72 (2H, s), 8.00 (1H, dd), 7.83-7.81 (1H, m), 7.32-7.29 (1H, m).

Present compound 48: $^1$H-NMR ($CDCl_3$) δ: 8.74 (2H, s), 8.21-8.19 (1H, m), 8.14-8.10 (1H, m), 7.41-7.38 (1H, m).

Present compound 49: $^1$H-NMR ($CDCl_3$) δ: 8.73 (2H, s), 7.89-7.88 (2H, m), 7.40-7.38 (1H, m).

Present compound 50: $^1$H-NMR ($CDCl_3$) δ: 8.71 (2H, s), 8.52-8.52 (1H, m), 7.97-7.95 (1H, m), 7.43-7.41 (1H, m), 4.59-4.56 (2H, m).

Present compound 51: $^1$H-HMR ($CDCl_3$) δ: 8.73 (2H, s), 8.36 (1H, d), 8.29 (1H, d), 8.05 (1H, d), 7.92 (1H, d), 7.85-7.83 (1H, m), 7,70-7.68 (1H, m).

Present compound 52: $^1$H-HMR ($CDCl_3$) δ: 9.40 (1H, d), 8.74 (2H, s), 8.69 (1H, d), 8.20 (1H, d), 7.97 (1H, d), 7.89-7.87 (1H, m), 7.70-7.68 (1H, m).

Present compound 53: $^1$H-NMR ($CDCl_3$) δ: 8.71 (2H, d), 8.00 (1H, s), 7.81 (1H, d), 7.55 (1H, d).

Present compound 54: $^1$H-NMR ($CDCl_3$) δ: 8.72 (2H, s), 7.59-7.56 (2H, m).

Present compound 55: $^1$H-NMR ($CDCl_3$) δ: 8.71 (2H, s), 8.53 (1H, d), 7.96-7.95 (1H, m), 7.43-7.41 (1H, m), 4.51 (2H, q).

Present compound 56: $^1$H-NMR ($CDCl_3$) δ: 8.73 (2H, d), 7.59-7.58 (2H, m), 7.18 (1H, d).

Present compound 57: $^1$H-NMR ($CDCl_3$) δ: 8.75 (2H, s), 8.37 (2H, s), 8.08 (1H, s).

Present compound 58: $^1$H-HMR ($CDCl_3$) δ: 8.73 (2H, d), 8.33 (1H, s), 8.21 (1H, d), 8.01 (1H, d), 7.83-7.81 (1H, m).

Present compound 59: $^1$H-NMR ($CDCl_3$) δ: 8.73 (2H, d), 8.58 (1H, s), 8.38-8.36 (1H, m), 8.24. (1H, d), 7.90-7.88 (1H, m).

Present compound 60: $^1$H-NMR ($CDCl_3$) δ: 8.72 (2H, s), 8.08 (1H, s), 8.04 (1H, d), 7.73 (1H, d), 7.66-7.64 (1H, m), 6.72 (1H, t).

Present compound 61: $^1$H-NMR ($CDCl_3$) δ: 8.73 (2H, s), 8.15-8.13 (2H, m), 7.82 (1H, d), 7.72-7.70 (1H, m).

Present compound 62: $^1$H-NMR ($CDCl_3$) δ: 8.70 (2H, s), 7.65-7.63 (1H, m), 7.51-7.51 (1H, m), 7.48-7.46 (1H, m), 7.40-7.38 (2H, m), 7.20-7.18 (2H, m), 7.07-7.04 (2H, m).

Present compound 63: $^1$H-HMR ($CDCl_3$) δ: 8.74 (2H, s), 8.59 (1H, d), 7.98 (1H, s), 7.73 (1H, d).

Present compound 64: $^1$H-NMR ($CDCl_3$) δ: 8.72 (2H, s), 8.09 (1H, s), 8.07 (1H, s), 7.81 (1H, s).

Present compound 65: $^1$H-NMR ($CDCl_3$) δ: 8.71 (2H, s), 7.88 (1H, s), 7.58-7.56 (1H, m), 7.47-7.45 (1H, m).
Present compound 66: $^1$H-NMR ($CDCl_3$) δ: 8.72 (2H, s), 8.16-8.15 (1H, m), 7.87-7.85 (1H, m), 7.30-7.29 (1H, m).
Present compound 67: $^1$H-NMR ($CDCl_3$) δ: 8.73 (2H, s), 8.22 (1H, s), 8.15 (1H, d), 7.87 (1H, d), 7.69 (1H, t).
Present compound 68: $^1$H-NMR ($CDCl_3$) δ: 8.72 (2B, s), 8.00 (2H, s), 7.87 (1H, s).
Present compound 69: $^1$H-NMR ($CDCl_3$) δ: 8.71 (2H, s), 7.05 (2H, d), 6.65-6.64 (1H, m), 3.85 (6H, s).
Present compound 70: $^1$H-NMR ($CDCl_3$) δ: 8.71 (2H, s), 8.35 (1H, d), 7.78 (1H, dd), 7.37 (1H, d), 2.52 (3H, s).
Present compound 71: $^1$H-NMR ($CDCl_3$) δ: 8.73 (2H, s), 7.87 (1H, s), 7.67 (1H, s), 7.44 (1H, a).
Present compound 12: $^1$H-NMR ($CDCl_3$) δ: 8.73 (2H, s), 8.25 (1H, s), 8.11 (1H, s), 7.96 (1H, s).
Present compound 73: $^1$H-NMR ($CDCl_3$) δ: 8.71 (2H, s), 7.60 (1H, dd), 7.55 (1H, dd), 7,35-7.33 (1H, m), 2.37 (3H, d).
Present compound 74: $^1$H-HMR ($CDCl_3$) δ: 8.70 (2H, s), 7.68 (1H, s), 7.64 (1H, d), 7.29-7.28 (1H, m), 2.35 (3H, s), 2.33 (3H, s).
Present compound is: $^1$H-HMR ($CDCl_3$) δ: 8.72 (2H, s), 7.99 (1H, s), 7.91 (1H, a), 7.64 (1H, s), 2.52 (3H, s)
Present compound 76: $^1$H-NMR ($CDCl_3$) δ: 8.71 (2H, s), 7.87 (1H, s), 7.66 (1H, s), 7.54 (1H, s), 2.42 (3H, s).
Present compound 77: $^1$H-NMR ($CDCl_3$) δ: 9.34 (1H, s), 9.08 (1H, s), 8.74 (2H, s), 8.43 (1H, s).
Present compound 78: $^1$H-NMR ($CDCl_3$) δ: 8.72 (2H, s), 8.11-8.09 (1H, m), 7.86-7.84 (1H, m), 7.47-7.45 (1H, m).
Present compound 79: $^1$H-NMR ($CDCl_3$) δ: 8.72 (2H, s), 8.19 (1H, dd), 7.87-7.83 (1H, m), 7.42-7.40 (1H, m).
Present compound 80: $^1$H-NMR ($CDCl_3$) δ: 8.72 (2H, s), 7.80-7.78 (1H, m), 7.69-7.64 (1H, m).
Present compound 81: $^1$H-NMR ($CDCl_3$) δ: 8.71 (2H, s), 8.12-8.11 (1H, m), 7.12-7.09 (1H, m).
Present compound 137: $^1$H-NMR ($CDCl_3$) δ: 8.71 (2H, s), 7.87 (2H, d), 7.42 (2H, d), 7.22 (1H, t), 1.11-7.06 (4H, m).
Present compound 138: $^1$H-NMR ($CDCl_3$ ) δ: 9.00 (1H, d), 8.73 (2H, d), 8.19 (1H, s), 7.73 (1H, d).
Present compound 139: $^1$H-NMR ($CDCl_3$) δ: 8.97 (1H, d), 8.75 (2H, s), 8.17 (1H, s), 7.97 (1H, d).
Present compound 140: $^1$H-NMR ($CDCl_3$) δ: 8.72 (2H, s), 8.18-8.09 (2H, m), 7.89 (1H, d).
Present compound 145: $^1$H-NMR ($CDCl_3$) δ: 8.73 (2H, d), 7.79 (1H, d), 7.69 (1H, s), 7.58-7.52 (1H, m), 7.36 (1H, d), 6.59 (1H, t).

Preparation Example 1-2

The compounds which were prepared according to the Preparation Example 1 and their physical property value are shown below.

A compound represented by formula (B-2):

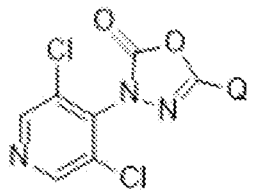

(B-2)

wherein Q represents any substituents described in [Table B-2A] and [Table B-2B].

[Table B-2A]

| Comp | Q |
|---|---|
| 82 | 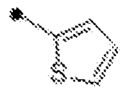 |
| 84 | 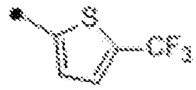 |
| 86 | 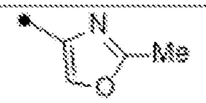 |
| 88 | 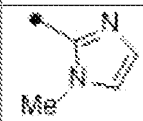 |

[Table B-2B]

| Comp | Q |
|---|---|
| 83 | 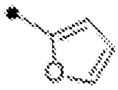 |
| 85 | 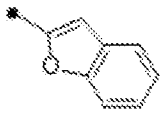 |
| 87 | 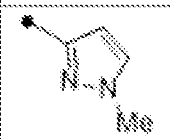 |

Present compound 82: $^1$H-HMR ($CDCl_3$) δ: 8.70 (2H, s), 7.72 (1H, dd), 7.60 (1H, dd), 7.19 (1H, dd) .
Present compound 83: $^1$H-NMR ($CDCl_3$) δ: 8.70 (2H, s), 7.66 (1H, dd), 7.13 (1H, dd), 6.62 (1H, dd).
Present compound 84: $^1$H-NMR ($CDCl_3$) δ: 8.72 (2H, s), 7.67-7.66 (1H, m), 7.52-7.50 (1H, m).
Present compound 85: $^1$H-NMR ($CDCl_3$) δ: 8.72 (2H, s), 7.72 (1H, d), 7.60 (1H, d), 7.49-7.47 (2H, m), 7.38-7.34 (1H, m) .
Present compound 86: $^1$H-NMR ($CDCl_3$) δ: 8.69 (2H, a), 8.13 (1H, s), 2.57 (3H, a).
Present compound 87: $^1$H-NMR ($CDCl_3$) δ: 8.69 (2H, s), 7.49 (1H, d), 6.78 (1H, d), 4.03 (3H, s).
Present compound 88: $^1$H-NMR ($CDCl_3$) δ: 8.71 (2H, s), 7.28 (1H, s), 7.12 (1H, s), 3.98 (3H, s).

A compound represented by formula (B-3):

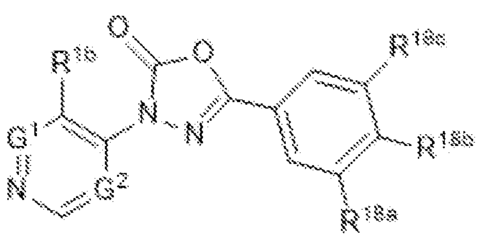

(B-3)

wherein a combination of $G^1$, $G^2$, $R^{1b}$, $R^{18a}$, $R^{18b}$, and $R^{18c}$ represents any combinations described in [Table B-3A] .

[Table B-3A]

| Comp | $G^1$ | $G^2$ | $R^{1b}$ | $R^{18a}$ | $R^{18b}$ | $R^{18c}$ |
|---|---|---|---|---|---|---|
| 89 | C-Cl | N | 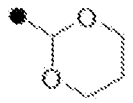 | $CF_3$ | H | H |
| 90 | C-H | C-H | Cl | $CF_3$ | H | H |
| 91 | C-H | C-H | Cl | $OCF_3$ | H | H |
| 92 | C-H | C-H | Br | $CF_3$ | H | H |
| 93 | C-H | C-H | $NO_2$ | $CF_3$ | H | H |
| 94 | C-H | C-H | $CF_3$ | $CF_3$ | H | H |
| 95 | C-H | C-Br | Br | H | H | H |
| 96 | C-H | C-Br | Br | $CF_3$ | H | H |
| 97 | C-H | C-Br | Br | $OCF_3$ | H | H |
| 143 | C-H | C-Br | Br | F | F | H |
| 146 | C-H | C-Br | Br | H | F | H |
| 147 | C-H | C-Br | Br | F | Br | H |
| 148 | C-H | C-F | Cl | $CF_3$ | H | H |
| 149 | C-H | C-F | Cl | F | F | H |

Present compound 89: $^1$H-NMR ($CDCl_3$) δ: 9.00 (1H, s), 8.24 (1H, s), 8.14 (1H, d), 7.84 (1H, d), 7.70-7.68 (1H, m), 5.92 (1H, s), 4.25-4.23 (2H, m), 3.94-3.91 (2H, m), 2.17-2.14 (1H m), 1.41 (1H, m) .

Present compound 90: $^1$H-HMR ($CDCl_3$) δ: 8.81 (1H, s), 8.66 (1H, d), 8.20 (1H, s), 8.12 (1H, d), 7.84 (1H, d), 7.70-7.68 (1H, m), 7,58 (1H, d).

Present compound 91: $^1$H-NMR ($CDCl_3$) δ: 8.80 (1H, s), 8.65 (1H, d), 7.90-7.85 (1H, m), 7.79 (1H, s), 7.62-7.56 (2H, m), 7.47-7.42 (1H, m).

Present compound 92: $^1$H-NMR ($CDCl_3$) δ: 8.94 (1H, s), 8.69 (1H, d), 8.20 (1H, s), 8.12 (1H, d), 7.84 (1H, d), 7.70-7.68 (1H, m), 7.56 (1H, d).

Present compound 93: $^1$H-NMR ($CDCl_3$) δ: 9.17 (1H, d), 8.93 (1H, dd), 8.18 (1H, s), 8.11 (1H, d), 7.92 (1H, dd), 7.88 (1H, d), 7.72-7.70 (1H, m).

Present compound 94: $^1$H-NMR ($CDCl_3$) δ: 9.11 (1H, s), 8.96 (1H, d), 8.17 (1H, s), 8.11 (1H, d), 7.85 (1H, d), 7.79 (1H, d), 7.70-7.68 (1H, m).

Present compound 95: $^1$H-NMR ($CDCl_3$) δ: 8.84 (2H, s), 7.93 (2H, d), 7.59-7.51 (3H, m).

Present compound 96: $^1$H-NMR ($CDCl_3$) δ: 8.86 (2H, s), 8.20 (1H, s), 8.11 (1H, d), 7.84 (1H, d), 7.69 (1H, t).

Present compound 97: $^1$H-NMR ($CDCl_3$) δ: 8.85 (2H, s), 7.87 (1H, d), 7.78 (1H, s), 7.58 (1H, t), 7.44 (1H, d).

Present compound 143: $^1$H-NMR ($CDCl_3$) δ: 8.85 (2H, s), 7.79-7.69 (2H, m), 7.38-7..30 (1H, m).

Present compound 146: $^1$H-NMR ($CDCl_3$) δ: 8.85 (2H, s), 7.96-7.92 (2H, m), 7.25-7.21 (2H, m).

Present compound 147: $^1$H-NMR ($CDCl_3$) δ: 8.86 (2H, s), 7.77-7.73 (1H, m), 7.69-7.66 (1H, m), 7.62-7.59 (1H, m).

Present compound 148: $^1$H-NMR ($CDCl_3$) δ: 8.68 (1H, s), 8.64 (1H, s), 8.20 (1H, s), 8.11 (1H, d), 7.86 (1H, d), 7.73-7.67 (1H, m).

Present compound 149: $^1$H-HMR ($CDCl_3$) δ: 8.67 (1H, s), 8.63 (1H, s), 7.78-7.68 (2H, m), 7.39-7.31 (1H, m).

Preparation Example 2

A mixture of the present compound 10 193 mg, 4-fluorophenyl boronic acid 80 mg, and potassium carbonate 212 mg, tris (dibenzylideneacetone) dipalladium (0) 46 mg, 2-dicyclohexylphoshino-2',6'-dimethoxy biphenyl 41 mg, toluene 3 mL, and water 1 mL was stirred at 65°C for 4 hours. Water was added to the resulting mixture, and the mixture was extracted with ethyl acetate. The resulting organic layer was dried over anhydrous sodium sulfate, and concentrated under reduced pressure. The resulting residue was subjected to a silica gel column chromatography (ethyl acetate : hexane = 30 : 70) to obtain the present compound 98 represented by the following formula 90 mg.

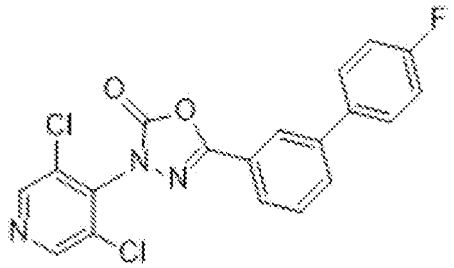

Present compound 98: $^1$H-NMR ($CDCl_3$) δ: 8.72 (2H, s), 8.09 (1H, s), 7.89 (1H, d), 7.75 (1H, d), 7.62-7.57 (3H, m), 7.18-7.15 (2H, m).

Preparation Example 2-1

The compounds which were prepared according to the Preparation Example 2 and their physical property value are shown below.

A compound represented by formula (B-3):

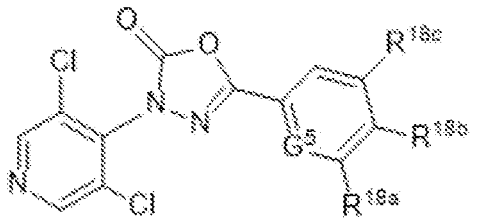 (B-3)

wherein a combination of G, $R^{18a}$, $R^{18b}$, and $R^{18c}$, represents any combinations described in [Table B-3B].

[Table B-3B]

| Comp | $G^5$ | $R^{18a}$ | $R^{18b}$ | $R^{18c}$ |
|---|---|---|---|---|
| 99 | CH | H | H | 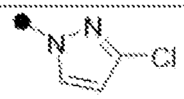 |
| 100 | CH | H | H | c-Pr |

Present compound 99: $^1$H-NMR ($CDCl_3$) δ: 8.72 (2H, s), 8.21 (1H, s), 8.00 (1H, s), 7.89-7.85 (2H, m), 7.69 (1H, s), 7.66-7.64 (1H, m).

Present compound 100: $^1$H-NMR ($CDCl_3$) δ: 8.71 (2H, d), 7.69 (1H, d), 7.60 (1H, s), 7.41-7.39 (1H, m), 7.29-7.27 (1H, m), 1.97-1.95 (1H, m), 1.05-1.04 (2H, m), 0.77-0.75 (2H, m).

Preparation Example 3

A mixture of the present compound 9 387 mg, bis(pinacolato)diboron 508 mg, potassium acetate 2.94 mg, dichloro(1,1'-bis(diphenylphoshino)ferrocene palladium (II) 36 mg, and toluene 3 mL was stirred at 80°C for 6 hours. Water was added to the resulting mixture, and the mixture was extracted with ethyl acetate. The resulting organic layer was dried over anhydrous sodium sulfate, and concentrated under reduced pressure. Sodium acetate 615 mg, water 5 mL and THF 5 mL were added to the resulting mixture successively. The resulting mixture was cooled to 0°C, and 30 % aqueous hydrogen peroxide solution 0.11 mL was added thereto, and the mixture was stirred at room temperature for

9 hours. Aqueous sodium thiosulfate solution was added to the resulting mixture, and the mixture was extracted with chloroform. The resulting organic layer was dried over anhydrous sodium sulfate, and concentrated under reduced pressure to obtain the present compound 101 150 mg as a crude product.

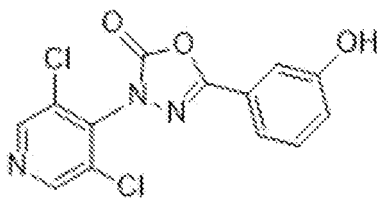

Present compound 101: $^{1}$H-NMR ($CDCl_3$) $\delta$: 8.71 (2H, s), 7.52-7.49 (1H, m), 7.40-7.38 (2H, m), 7.06-7.04 (1H, m), 5.20 (1H, s) .

Preparation Example 3-1

The compounds which were prepared according to the Preparation Example 3 and their physical property value are shown below.

A compound represented by formula (B-3):

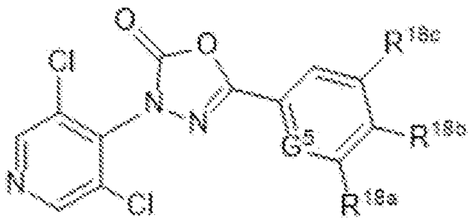

(B-3)

wherein a combination of G, $R^{18a}$, $R^{18b}$ and $R^{18c}$ represents any combinations described in [Table B-4A].

[Table B-4A]

| Comp | $G^5$ | $R^{18a}$ | $R^{18b}$ | $R^{18c}$ |
|---|---|---|---|---|
| 102 | CH | H | OH | H |
| 103 | CH | OH | H | F |
| 104 | CH | OH | H | Cl |
| 105 | C-F | H | F | OH |

Present compound 102: $^{1}$H-NMR ($CDCl_3$) $\delta$: 8.70 (2H, s), 7.82 (2H, d), 6.96 (2H, d), 5.44 (1H, s).
Present compound 103: LCMS: 340 $[M-H]^-$, RT = 1.86 min.
Present compound 104: LCMS: 356 $[M-H]^-$, RT = 1.97 min.
Present compound 105: LCMS: 358 $[M-H]^-$, RT = 1.66 min.

Preparation Example 4

A mixture of the present compound 101 (which was obtained in the Preparation Example 3) as crude product 100 mg cesium carbonate 163 mg, DMF 2 mL and iodomethane 0.04 mL was stirred at room temperature for 1 hour. Water was added to the resulting mixture, and the mixture was extracted with 2-methoxy-2-methylpropane. The resulting organic layer was dried over anhydrous sodium sulfate, and concentrated under reduced pressure. The resulting residue was subjected to a silica gel column chromatography (ethyl acetate : hexane = 30 : 70) to obtain the present compound 106 represented by the following formula 60mg.

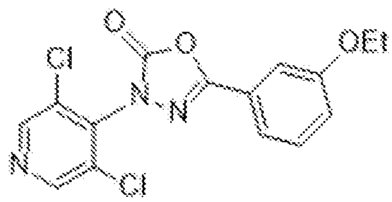

Present compound 106: $^1$H-HMR ($CDCl_3$) δ: 8.71 (2H, s), 7.49 (1H, d), 7.41-7.39 (2H, m), 7.11-7.09 (1H, m), 4.09 (2H, q), 1.45 (3H, t).

Preparation Example 4-1

The compounds which were prepared according to the reparation Example 4 and their physical property value are shown below.

A compound represented by formula (B-3):

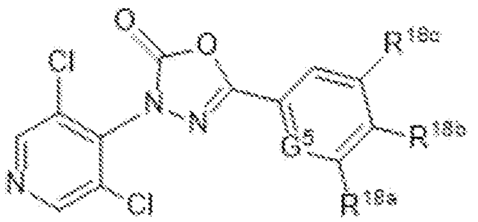

(B-3)

wherein a combination of G, $R^{18a}$, $R^{18b}$, and $R^{18c}$ represents any combinations described in [Table B-5A].

[Table B-5A]

| Comp | $G^5$ | $R^{18a}$ | $R^{18b}$ | $R^{18c}$ |
|---|---|---|---|---|
| 107 | CH | H | OEt | H |
| 108 | CH | $OCH_2CF_3$ | H | H |
| 109 | CH | $OCH_2CF_2CF_3$ | H | H |
| 110 | CH | $OCH_2CF_3$ | H | F |
| 111 | CH | $OCH_2CF_3$ | H | Cl |
| 112 | C-F | H | F | $OCH_2CF_3$ |

Present compound 107: $^1$H-NMR ($CDCl_3$) δ: 8.69 (2H, s), 7.84 (2H, d), 6.99 (2H, d), 4.11 (2H, q), 1.46 (3H, t).
Present compound 108: $^1$H-NMR ($CDCl_3$) δ: 8.72 (2H, s), 7.62 (1H, d), 7.52-7.46 (2H, m), 7.18 (1H, dd), 4.42 (2H, t).
Present compound 109: $^1$H-NMR ($CDCl_3$) δ: 8.72 (2H, s), 7.63 (1H, d), 7.52-7.46 (2H, m), 7.18 (1H, dd), 4.49 (2H, t).
Present compound 110: $^1$H-NMR ($CDCl_3$) δ: 8.72 (2H, s), 7.35-7.33 (1H, m), 7.28-7.27 (1H, m), 6.91-6.89 (1H, m), 4.42 (2H, q)
Present compound 111: $^1$H-NMR ($CDCl_3$) δ: 8.72 (2H, s), 7.62-7.61 (1H, m), 7.36-7.35 (1H, m), 7.18-7.17 (1H, m), 4.42 (2H, q).
Present compound 112: $^1$H-NMR ($CDCl_3$) δ: 8.71 (2H, a), 7.55-7.53 (1H, m), 7.13-7.10 (1H, m), 4.46 (2H, q).

Preparation Example 5

The present compound 31 700 mg was added to a mixture of iron powder 990 mg, ethanol 5 mL and acetic acid 0.29 mL at 90°C, and the mixture was stirred at 90°C for 5 hours. The resulting mixture was filtered through Celite (registered trademark), and aqueous saturated sodium hydrocarbonate solution was added to the filtrates, and the mixture was extracted with ethyl acetate. The resulting organic layer was dried over anhydrous sodium sulfate, and concentrated under reduced pressure to obtain the present compound 113 represented by the following formula 170 mg.

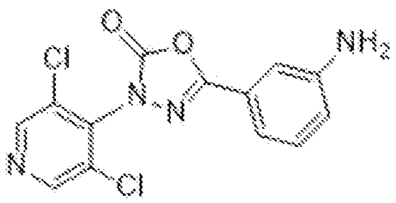

Present compound 113: $^{1}$H-NMR ($CDCl_3$) δ: 8.70 (2H, s), 7.29-7.28 (2H, m), 7.19-7.19 (1H, m), 6.86-6.86 (1H, m), 3.87 (2H, s) .

Preparation Example 5-1

The compounds which were prepared according to the Preparation Example 5 and their physical property value are shown below.

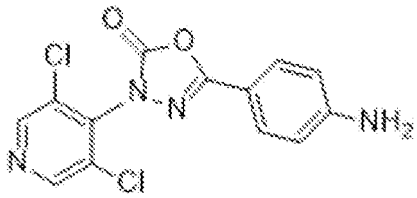

Present compound 114: $^{1}$H-NMR ($CDCl_3$) δ: 8.68 (2H, s), 7.70 (2H, d), 6.73 (2H, d), 4.12 (2H, d).

Preparation Example 6

Acetyl chloride 0.03 mL was added to a mixture of the present compound 113 120 mg, triethylamine 0.06 mL, and chloroform 1 mL at 0°C, and the mixture was stirred at room temperature for 3 hours. Water was added to the resulting mixture, and the mixture was extracted with chloroform. The resulting organic layer was dried over anhydrous sodium sulfate and concentrated under reduced pressure to obtain the present compound 115 represented by the following formula 120 mg.

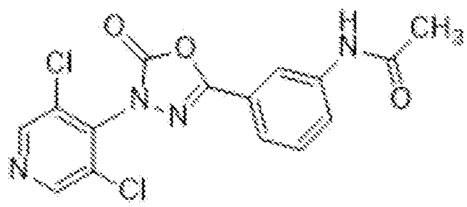

Present compound 115: $^{1}$H-NMR ($CDCl_3$)) δ: 8.71 (2H, s), 8.01 (1H, s), 7.80 (1H, d), 7.66 (1H, d), 7.49-7.47 (1H, m), 7.26 (1H, s), 2.22 (3H, a).

Preparation Example 6-1

The compounds which were prepared according to the Preparation Example 6 and their physical property value are shown below.

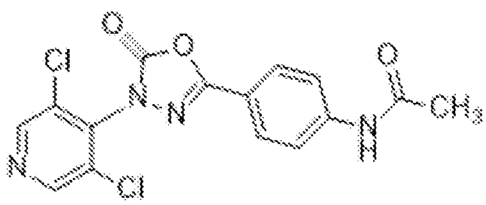

Present compound 116: $^1$H-NMR ($CDCl_3$) $\delta$: 8.70 (2H, s), 7.97 (2H, d), 7.69 (2H, d), 7.30 (1H, s), 2.24 (3H, s).

Preparation Example 7

A mixture or the present compound 92 38.6 mg, 2- (propen-2-yl)-4,4,5,5-tetramethyl-1,3,2-dioxaborolane 300 mg, tripothssium phosphate 424 mg, tris (dibenzylideneacetone) dipalladium (0) 45 mg, toluene 10 mL, and water 1 mL was stirred at 60°C for 6 hours. Water was added to the resulting mixture, and the mixture was extracted with ethyl acetate. The resulting organic layer was dried over anhydrous sodium sulfate, and concentrated under reduced pressure. The resulting residue was subjected to a silica gel column chromatography (ethyl acetate : hexane 30 : 70) to obtain the present compound 117 represented by the following formula 35 mg.

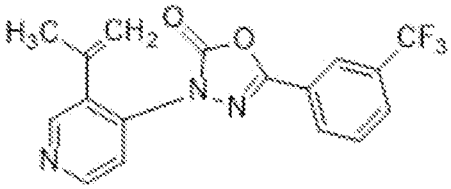

Present compound 117: $^1$H-NMR ($CDCl_3$) $\delta$: 8.65-8.65 (2H, m), 8.16 (1H, s), 8.08 (1H, d), 7.83 (1H, d), 7.69-7.67 (1H, m), 7.56 (1H, d), 5.26 (1H, s), 5.00 (1H, a), 2.13 (3H, s).

Preparation Example 7-1

The compounds which were prepared according to the Preparation Example 7 and their physical property value are shown below.

A compound represented by formula (B-4):

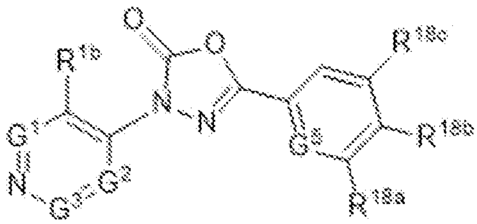

(B-4)

wherein a combination of $G^1$, $R^{1b}$, $G^2$, $G^3$, $G^5$, $R^{18a}$, $R^{18b}$ and $R^{18c}$ represents any combinations described in [Table B-4B]..

[Table B-4B]

| Comp | $G^1$ | $R^{1b}$ | $G^2$ | $G^3$ | $G^5$ | $R^{18a}$ | $R^{18b}$ | $R^{18c}$ |
|---|---|---|---|---|---|---|---|---|
| 118 | CH | $CH=CH_2$ | CH | CH | CH | H | H | $CF_3$ |
| 119 | CH | Me | CH | CH | CH | H | H | $CF_3$ |
| 120 | CH | c-Pr | CH | CH | CH | H | H | $CF_3$ |
| 121 | CH | Ph | CH | CH | CH | H | H | $CF_3$ |
| 122 | CH | 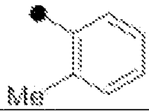 | CH | CH | CH | H | H | $CF_3$ |
| 123 | CH | 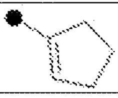 | CH | CH | CH | H | H | $CF_3$ |
| 124 | CH | $CH=CH_2$ | $C-CH=CH_2$ | CH | CH | H | H | $CF_3$ |
| 125 | CH | Me | C-Me | CH | CH | H | H | $CF_3$ |
| 126 | CH | $CMe=CH_2$ | $C-CMe=CH_2$ | CH | CH | H | H | $OCF_3$ |
| 127 | CH | 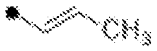 | 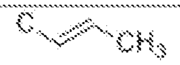 | CH | CH | H | H | $OCF_3$ |
| 128 | CH | Me | C-Me | CH | CH | H | H | $OCF_3$ |

Present compound 118: $^1$H-NMR ($CDCl_3$) δ: 8.92 (1H, s), 8.65 (1H, d), 8.19 (1H, a), 8.11 (1H, d), 7.84 (1H, d), 7.70-7.68 (1H, m), 7.53 (1H, d), 6.97 (1H, dd), 5.87 (1H, d), 5.53 (1H, d) .

Present compound 119: $^1$H-NMR ($CDCl_3$)) δ: 8.63 (1H, s), 8.59 (1H, d), 8.19 (1H, s), 8.11 (1H, d), 7.84 (1H, d), 7.70-7.68 (1H, m), 7.50 (1H, d), 2.47 (3H, s).

Present compound 120: $^1$H-NMR ($CDCl_3$) δ: 8.58 (1H, d), 8.55 (1H, s), 8.20 (1H, s), 8.11 (1H, d), 7.83 (1H, d), 7.71-7.69 (1H, m), 7.44 (1H, d), 2.19-2.16 (1H, m), 1.02-1.00 (2H, m), 0.75-0.72 (2H, m).

Present compound 121: $^1$H-NMR ($CDCl_3$) δ: 8.76-8.75 (2H, m), 7.93 (1H, s), 7.87 (1H, d), 7.78 (1H, d), 7.66 (1H, d), 7.62-7.60 (1H, m), 7.46-7.34 (5H, m).

Present compound 122: $^1$H-NMR ($CDCl_3$) δ: 8.76 (1H, d), 8.64 (1H, s), 7.78-7.75 (4H, m), 7.58-7.56 (1H, m), 7.33-7.28 (2H, **m), 7.22** (1H, dd), 7.16-7.14 (1H, m), 2.14 (3H, s).

Present compound 123: $^1$H-NMR ($CDCl_3$) δ: 8.70 (1H, d), 8.61 (1H, d), 8.17 (1H, s), 8.08 (1H, d), 7.83 (1H, dd), 7.69-7.67 (1H, m), 7.49-7.49 (18, m), 5.94-5.91 (1H, m), 2.64-2.59 (2H, m), 2.53-2.48 (2H, m), 2.02-2.00 (2H, m).

Present compound 124**:** $^1$H-NMR ($CDCl_3$) δ: 8.83 (2H, s), 8.19 (1H, s), 8.10 (1H, d), 7.83 (1H, d), 7.69-7.67 (1H, m), 6.66 (2H, dd), 5.89 (2H, d), 5.52 (2H, d).

Present compound 125: $^1$H-NMR ($CDCl_3$) δ: 8.49 (2H, s), 8.18 (1H, s), 8.10 (1H, d), 7.83 (1H, d), 7.68 (1H, t), 2.31 (6H, s) .

Present compound 126: $^1$H-NMR ($CDCl_3$) δ: 8.71 (2H, s), 7.88 (18, d), 7.79 (1H, s), 7.58 (1H, t), 7.43 (1H, d), 6.45-6.19 (4H, m), 1.89 (6H, dd).

Present compound 127: LCMS: 404 $[M+H]^+$, RT = 2.22 min.

Present compound 128: $^1$H-NMR ($CDCl_3$) δ: 8.49 (2H, s), 7.86 (1H, d), 7.77 (1H, s), 7.58 (1H, t), 7.43 (1H, d), 2.31 (6H, s).

Preparation Example 8

A mixture of the present compound 118 100 mg, ethanol 5 taL, and palladium carbon 5 mg was stirred at room temperature under hydrogen atmosphere for 6 hours. The resulting mixture was filtered, and the filtrates were concentrated under reduced pressure to obtain the present compound 130 represented by the following formula 100 mg.

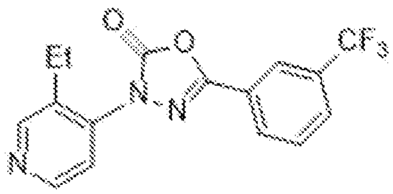

Present compound 130: $^1$H-NMR ($CDCl_3$) δ: 8.67 (1H, s), 8.60 (1H, d), 8.18 (1H, s), 8.10 (1H, d), 7.84 (1H, d), 7.70-7.68 (1H, m), 7.48 (1H, d), 2.87 (2H, q), 1.30 (3H, t).

Preparation Example 8-1

The compounds which were prepared according to the Preparation Example 8 and their physical property value are shown below.
A compound represented by formula (B-5):

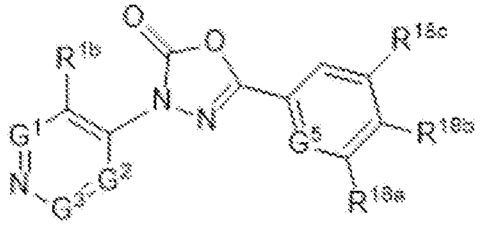

(B-5)

wherein a combination of $G^1$, $R^{1b}$, $G^2$, $G^3$, G, $R^{18a}$, $R^{18b}$ and $R^{18c}$ represents any combinations described in [Table B-5B].

[Table B-5B]

| Comp | $G^1$ | $R^{1b}$ | $G^2$ | $G^3$ | $G^5$ | $R^{18a}$ | $R^{18b}$ | $R^{18c}$ |
|---|---|---|---|---|---|---|---|---|
| 131 | CH | Et | C-Et | CH | CH | H | H | $CF_3$ |
| 132 | CH | Et | C-Et | CH | CH | H | H | $OCF_3$ |
| 133 | CH | iPr | C-iPr | CH | CH | H | H | $OCF_3$ |
| 134 | CH | Pr | Pr | CH | CH | H | H | $OCF_3$ |

Present compound 131: $^1$H-NMR ($CDCl_3$) δ: 8.54 (2H, s), 8.19 (1H, s), 8.10 (1H, d), 7.83 (1H, d), 7.69 (1H, d), 2.66 (4H, q), 1.25 (6H, t).
Present compound 132: $^1$H-NMR ($CDCl_3$) δ: 8.54 (2H, s), 7.86 (1H, d), 7.77 (1H, s), 7.58 (1H, t), 7.43 (1H, d), 2.65 (4H, q), 1.24 (6H, t).
Present compound 133: $^1$H-NMR ($CDCl_3$) δ: 8.61 (2H, s), 7.85 (1H, d), 7.76 (1H, s), 7.57 (1H, t), 7.42 (1H, d), 2.93-2.86 (2H, m), 1.34-1.29 (12H, m).
Present compound 134: $^1$H-NMR ($CDCl_3$) δ: 8.50 (2H, s), 7.86 (1H, d), 7.77 (1H, s), 7.58 (1H, t), 7.43 (1H, d), 2.65-2.53 (4H, m), 1.68-1.57 (4H, m), 0.92 (6H, t).

Preparation Example 9

A mixture of the present compound 12 392 mg, chloroform 5 mL, and m-chloroperoxybenzoic acid 246 mg was stirred at room temperature for 6 hours. The resulting mixture was washed with aqueous sodium thiosulfate solution and aqueous sodium hydrocarbonate solution successively. The resulting organic layer was dried over anhydrous sodium sulfate and concentrated under reduced pressure. The resulting residue was subjected to a silica gel column chromatography (ethyl acetate : hexane = 30 : 70) to obtain the present compound 135 represented by the following formula 240 mg.

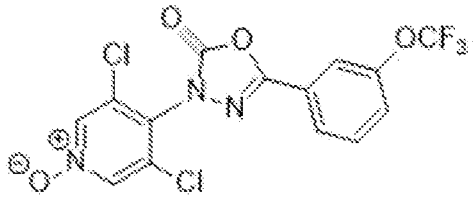

Present compound 135: $^1$H-NMR ($CDCl_3$) δ: 8.29 (2H, s), 7.85 (1H, d), 7.76 (1H, s), 7.59 (1H, t), 7.44 (1H, d).

Preparation Example 10

Thiophosgene 0.15 mL was added to a mixture of the intermediate compound 20 348 mg, triethylamine 0.4 mL, and THF 5 mL, and the mixture was stirred at room temperature for 6 hours. Aqueous saturated sodium hydrocarbonate solution was added to the resulting mixture, and the mixture was extracted with ethyl acetate. The resulting organic layer was dried over anhydrous sodium sulfate and concentrated under reduced pressure. The resulting residue was subjected to a silica gel column chromatography (ethyl acetate : hexane = 30 : 70) to obtain the present compound. 136 represented by the following formula 150 mg.

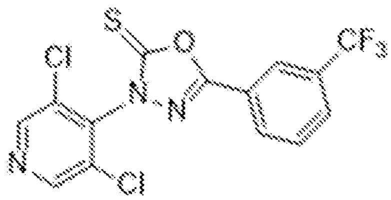

Present compound 136: $^1$H-NMR ($CDCl_3$) δ: 8.76 (2H, s), 8.26 (1H, s), 8.18 (1H, d), 7.88 (1H, d), 7.71 (1H, t).

Next, examples of the present compounds which are prepared according to any one of the Preparation Examples described in Working Examples and the Processes described herein are indicated below.

A compound represented by formula (L-1):

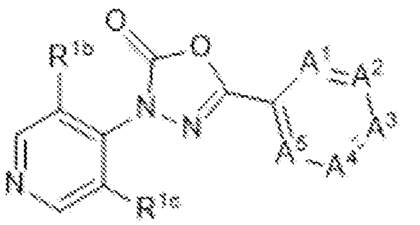

(L-1)

(hereinafter, referred to as "Compound (L-1) ") wherein $R^{1b}$ represents a fluorine atom, R represents a hydrogen atom, and $A^2$, $A^3$, $A^4$, and $A^5$ represent CH, and $A^1$ represents any substituents described in [Table L1] to [Table L3] (hereinafter, referred to as "Compound class SX1").

[Table L1]

| |
|---|
| C-$CF_3$ |
| C-$CHF_2$ |
| C-$CH_2CF_3$ |
| C-$CF_2CF_3$ |
| C-$CH_2CF_2CF_3$ |
| C-$CF_2CF_2CF_3$ |
| C-$CF_2CF_2CF_2CF_3$ |
| C-$CF_2CF_2CF_2CF_2CF_3$ |
| C-$OCF_3$ |
| C-$OCHF_2$ |
| C-$OCH_2CF_3$ |
| C-$OCH_2CHF_2$ |
| C-$OCF_2CF_3$ |
| C-$OCH(CH_3)CF_3$ |
| C-$OCH_2CF_2CHF_2$ |
| C-$OCH_2CF_2CF_3$ |
| C-$OCF_2CF_2CF_3$ |
| C-$OCF_2CHF_2$ |
| C-$OCH_2CF_2CHFCF_3$ |
| C-$OCH_2CF_2CF_2CF_3$ |
| C-$OCF_2CF_2CF_2CF_3$ |
| C-$OCH_2$(2,2-$F_2$-c-Pr) |
| C-$OCH_2$(1-$CF_3$-c-Pr) |
| C-$OS(O)_2CF_3$ |
| C-$OS(O)_2CF_2CF_3$ |
| C-$OS(O)_2CF_2CF_2CF_3$ |

[Table L2]

| |
|---|
| C-$SCF_3$ |
| C-$SCH_2CF_3$ |
| C-$SCF_2CF_3$ |
| C-$SCH_2CF_2CF_3$ |
| C-$SCF_2CF_2CF_3$ |
| C-$SCH_2CF_2CF_2CF_3$ |
| C-$SCF_2CF_2CF_2CF_3$ |
| C-S(O)$CF_3$ |
| C-S(O)$CH_2CF_3$ |
| C-S(O)$CF_2CF_3$ |
| C-S(O)$CH_2CF_2CF_3$ |
| C-S(O)$CF_2CF_2CF_3$ |
| C-S(O)$CH_2CF_2CF_2CF_3$ |
| C-S(O)$CF_2CF_2CF_2CF_3$ |
| C-S(O)$_2CF_3$ |
| C-S(O)$_2CH_2CF_3$ |
| C-S(O)$_2CF_2CF_3$ |
| C-S(O)$_2CH_2CF_2CF_3$ |
| C-S(O)$_2CF_2CF_2CF_3$ |
| C-F |
| C-Cl |
| C-Br |
| C-I |
| C-$OCH_3$ |
| C-$OCH_2CH_3$ |
| C-$OCH(CH_3)_2$ |

[Table L3]

| |
|---|
| $C-OCH_2CH_2CH_3$ |
| $C-OCH_2CH_2CH_2CH_3$ |
| $C-OCH_2CH(CH_3)_2$ |
| $C-CH_3$ |
| $C-CH_2CH_3$ |
| $C-CH(CH_3)_2$ |
| $C-CH_2CH_2CH_3$ |
| $C-CH_2CH_2CH_2CH_3$ |
| $C-C(CH_3)_3$ |
| $C-CH=CH_2$ |
| 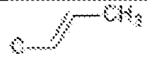 |
| 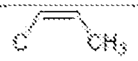 |
| $C-CCH_3=CH_2$ |
| C-2-F-Ph |
| C-3-F-Ph |
| C-4-F-Ph |
| C-2-Cl-Ph |
| C-3-Cl-Ph |
| C-4-Cl-Ph |
| $C-2,6-F_2-Ph$ |
| $C-3,5-F_2-Ph$ |
| $C-2,4-F_2-Ph$ |
| $C-2,4,6-F_3-Ph$ |
| $C-2,6-Cl_2-Ph$ |
| $C-3,5-Cl_2-Ph$ |
| $C-2,4-Cl_2-Ph$ |

A present compound (L-1) wherein $R^{1b}$ and $R^{1c}$ represent a fluorine atom, $A^2$, $A^3$, $A^4$, and $A^5$ represent CH, and $A^1$ represents any one substituent indicated in [Table L1] to [Table L3] (hereinafter, referred to as "Compound class SX2") .

A present compound (L-1) wherein $R^{1b}$ represents a chlorine atom, R represents a hydrogen atom, $A^2$, $A^3$, $A^4$, and $A^5$ represent CH, and $A^1$ represents any one substituent indicated in [Table L1] to [Table L3] (hereinafter, referred to as "Compound class SX3").

A present compound (L-1) wherein $R^{1b}$ and $R^{1c}$ represent a chlorine atom, $A^2$, $A^3$, $A^4$, and $A^5$ represent CH, and $A^1$ represents any one substituent indicated in [Table L1] to [Table L3] (hereinafter, referred to as "Compound class SX4") .

A present compound (L-1) wherein $R^{1b}$ represents a bromine atom, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class SX5").

A present compound (L-1) wherein $R^{1b}$ and $R^{1c}$ represent a bromine atom, $A^2$, $A^3$, $A^4$, and $A^5$ represent CH, and $A^1$ represents any one substituent indicated in [Table L1] to [Table L3] (hereinafter, referred to as "Compound class SX6") .

A present compound (1-1) wherein $R^{1b}$ represents an iodine atom, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class SX7").

A present compound (L-1) wherein $R^{1b}$ and $R^{1c}$ represent an iodine atom, $A^2$, $A^3$, $A^4$, and $A^5$ represent CH, and $A^1$ represents any one substituent indicated in [Table L1] to [Table L3] (hereinafter, referred to as "Compound class

SX8").

A present compound (L-1) wherein $R^{1b}$ represents a methyl group, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class SX9").

A present compound (L-1) wherein $R^{1b}$ and $R^{1c}$ represent a methyl group, $A^2$, $A^3$, $A^4$, and $A^5$ represent CH, and $A^1$ represents any one substituent indicated in [Table L1] to [Table L3] (hereinafter, referred to as "Compound class SX10").

A present compound (L-1) wherein $R^{1b}$ represents an ethyl group, $R^{1c}$ represents a hydrogen atom, $A^2$, $A^3$ $A^4$, and $A^5$ represent CH, and $A^1$ represents any one substituent indicated in [Table L1] to [Table L3] (hereinafter, referred to as "Compound class SX11").

A present compound (L-1) wherein $R^{1b}$ and $R^{1c}$ represent an ethyl group, $A^2$, $A^3$, $A^4$, and $A^5$ represent CH, and $A^1$ represents any one substituent indicated in [Table L1] to [Table L3] (hereinafter, referred to as "Compound class SX12").

A present compound (L-1) wherein $R^{1b}$ represents a propyl group, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class SX13").

A present compound (L-1) wherein $R^{1b}$ and $R^{1c}$ represent a propyl group, $A^2$, $A^3$, $A^4$, and $A^5$ represent CH, and $A^1$ represents any one substituent indicated in [Table L1] to [Table L3] (hereinafter, referred to as "Compound class SX14").

A present compound (L-1) wherein $R^{1b}$ represents an isopropyl group, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class 3X15").

A present compound (L-1) wherein $R^{1b}$ and $R^{1c}$ represent an isopropyl group, $A^2$, $A^3$, $A^4$, and $A^5$ represent CH, and $A^1$ represents any one substituent indicated in [Table L1] to [Table L3] (hereinafter, referred to as "Compound class SX16").

A present compound (L-1) wherein $R^{1b}$ represents a cyclopropyl group, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class 3X17").

A present compound (L-1) wherein $R^{1b}$ and $R^{1c}$ represent a cyclopropyl group, $A^2$, $A^3$, $A^4$, and $A^5$ represent CH, and $A^1$ represents any one substituent indicated in [Table L1] to [Table L3] (hereinafter, referred to as "Compound class SX18") .

A present compound (L-1) wherein $R^{1b}$ represents $CF_3$, $R^{1c}$ represents a hydrogen atom, $A^2$, $A^3$, $A^4$, and $A^5$ represent CH, and $A^1$ represents any one substituent indicated in [Table L1) to [Table L3] (hereinafter, referred to as "Compound class SX19").

A present compound (L-1) wherein $R^{1b}$ and $R^{1c}$ represent $CF_3$, $A^2$, $A^3$, $A^4$, and $A^5$ represent CH, and $A^1$ represents any one substituent indicated in [Table L1] to [Table L3] (hereinafter, referred to as "Compound class SX20").

A present compound (L-1) wherein $R^{1b}$ represents a methylsulfanyl group, $R^{1c}$ represents a hydrogen atom, $A^2$, $A^3$, $A^4$, and $A^5$ represent CH, and $A^1$ represents any one substituent indicated in (Table L1] to [Table L3] (hereinafter, referred to as "Compound class SX21").

A present compound (L-1) wherein $R^{1b}$ represents a methylsulfinyl group, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class SX22").

A present compound (L-1) wherein $R^{1b}$ represents a methylsulfonyl group, $R^{1c}$ represents a hydrogen atom, $A^2$, $A^3$, $A^4$, and $A^5$ represent CH, and $A^1$ represents any one substituent indicated in [Table L1) to [Table L3] (hereinafter, referred. to as "compound class 8X23").

A present compound (L-1) wherein $R^{1b}$ represents a chlorine atom, $R^{1c}$ represents a bromide atom, $A^2$, $A^3$, $A^4$, and $A^5$ represent CH, and $A^1$ represents any one substituent indicated in [Table L1] to [Table L3] (hereinafter, referred to as "Compound class SX24").

A present compound (L-1) wherein $R^{1b}$ represents a chlorine atom, $R^{1c}$ represents an iodine atom, $A^2$, $A^5$ $A^4$, and $A^5$ represent CH, and $A^1$ represents any one substituent indicated in (Table L1] to [Table L3] (hereinafter, referred to as "Compound class SX25").

A present compound (L-1) wherein $R^{1b}$ represents a chlorine atom, $R^{1b}$ represents a methyl group, $A^2$, $A^3$, $A^4$, and $A^5$ represent CH, and $A^1$ represents any one substituent indicated in (Table L1] to [Table L3] (hereinafter, referred to as "Compound class SX26").

A present compound (L-1) wherein $R^{1b}$ represents a chlorine atom, $R^{1c}$ represents an ethyl group, $A^2$, $A^3$, $A^4$, and $A^5$ represent CH, and $A^1$ represents any one substituent indicated in [Table L1] to [Table L3] (hereinafter, referred to as "Compound class SX27").

A present compound (L-1) wherein $R^{1b}$ represents a chlorine atom, $R^{1c}$ represents a propyl group, $A^2$, $A^3$, $A^4$,

and $A^5$ represent CH, and $A^1$ represents any one substituent indicated in [Table L1) to [Table L3] (hereinafter, referred. to as "Compound class SX28").

A present compound (L-1) wherein $R^{1b}$ represents a chlorine atom, $R^{1c}$ represents an isopropyl group, $A^2$, $A^3$, $A^4$, and $A^5$ represent CH, and $A^1$ represents any one substituent indicated in [Table L1] to [Table L3] (hereinafter, referred to as "Compound class SX29").

A present compound (L-1) wherein $R^{1b}$ represents a chlorine atom, $R^{1c}$ represents a cyclopropyl group, $A^2$, $A^3$, $A^4$, and $A^5$ represent CH, and $A^1$ represents any one substituent indicated in (Table L1] to [Table L3] (hereinafter, referred to as "Compound class SX30").

A present compound (L-1) wherein $R^{1b}$ represents a chlorine atom, $R^{1c}$ represents $CF_3$, $A^2$, $A^3$, $A^4$, and $A^5$ represent CH, and $A^1$ represents any one substituent indicated in [Table L1] to [Table L3) (hereinafter, referred to as "Compound class SX31").

A present compound (L-1) wherein $R^{1b}$ represents a bromine atom, $R^1$ represents an iodine atom, $A^2$, $A^3$, $A^4$, and $A^5$ represent CH, and $A^1$ represents any one substituent indicated in [Table L1] to [Table L3] (hereinafter, referred to as "Compound class SX32").

A present compound (L-1) wherein $R^{1b}$ represents a bromine atom, $R^1$ represents a methyl group, $A^2$, $A^3$, $A^4$, and $A^5$ represent CH, and $A^1$ represents any one substituent indicated in [Table L1) to [Table L3] (hereinafter, referred. to as "Compound class SX33").

A present compound (L-1) wherein $R^{1b}$ represents a bromine, atom, $R^{1c}$ represents an ethyl group, $A^2$, $A^3$, $A^4$, and $A^5$ represent CH, and $A^1$ represents any one substituent indicated in [Table L1] to [Table L3] (hereinafter, referred to as "Compound class SX34").

A present compound (L-1) wherein $R^{1b}$ represents a bromine atom, $R^{1c}$ represents a propyl group, $A^2$, $A^3$, $A^4$, and $A^5$ represent CH, and $A^1$ represents any one substituent indicated in (Table L1] to [Table L3] (hereinafter, referred to as "Compound class SX35").

A present compound (L-1) wherein $R^{1b}$ represents a bromine atom, $R^{1c}$ represents an isopropyl group, $A^2$, $A^3$, $A^4$, and $A^5$ represent CH, and $A^1$ represents any one substituent indicated in (Table L1] to [Table L3] (hereinafter, referred to as "Compound class SX36").

A present compound (L-1) wherein $R^{1b}$ represents a bromine atom, $R^{1c}$ represents a cyclopropyl group, $A^2$, $A^3$, $A^4$, and $A^5$ represent CH, and $A^1$ represents any one substituent indicated in [Table L1] to [Table L3] (hereinafter, referred to as "Compound class SX37").

A present compound (L-1) wherein $R^{1b}$ represents a bromine atom, $R^{1c}$ represents $CF_3$, $A^2$, $A^3$, $A^4$, and $A^5$ represent CH, and $A^1$ represents any one substituent indicated in [Table L1] to [Table L3] (hereinafter, referred to as "Compound class SX38").

A present compound (L-1) wherein $R^{1b}$ represents a methyl group, $R^{1c}$ represents an ethyl group, $A^2$, $A^3$, $A^4$, and $A^5$ represent CH, and $A^1$ represents any one substituent indicated in [Table L1] to [Table L3] (hereinafter, referred to as "Compound class SX39").

A present compound (L-1) wherein $R^{1b}$ represents a methyl group, $R^{1c}$ represents a propyl group, $A^2$, $A^3$ $A^4$, and $A^5$ represent CH, and $A^1$ represents any one substituent indicated in [Table L1) to [Table L3] (hereinafter, referred to as "Compound class SX40").

A present compound (L-1) wherein $R^{1b}$ represents an ethyl group, $R^{1c}$ represents a propyl group, $A^2$, $A^3$, $A^4$, and $A^5$ represent CH, and $A^1$ represents any one substituent indicated in [Table L1] to [Table L3] (hereinafter, referred to as "Compound clash SX41"),

A present compound (L-1) wherein $R^{1b}$ represents a fluorine atom, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class 8X42") .

A present compound (L-1) wherein $R^{1b}$ and $R^{1c}$ represent a fluorine atom, $A^1$, $A^3$, $A^4$, and $A^5$ represent CH, and $A^2$ represents any one substituent indicated in [Table L1] to [Table L3] (hereinafter, referred to as "Compound class SX43").

A present compound (L-1) wherein $R^{1b}$ represents a chlorine atom, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class SX44").

A present compound (L-1) wherein $R^{1b}$ and $R^{1c}$ represent a chlorine atom, $A^1$, $A^3$ $A^4$, and $A^5$ represent CH, and $A^2$ represents any one substituent indicated in [Table L1] to [Table L3] (hereinafter, referred to as "Compound class SX45") .

A present compound (L-1) wherein $R^{1b}$ represents a bromine atom, $R^{1c}$ represents a hydrogen atom, $A^3$, $A^3$, $A^4$, and $A^5$ represent CH, and $A^2$ represents any one substituent indicated in [Table L1] to [Table L3] (hereinafter, referred to as "Compound class 8X46") .

A present compound (L-1) wherein $R^{1b}$ and $R^{1c}$ represent a bromine atom, $A^1$, $A^3$, $A^4$, and $A^5$ represent CH, and $A^2$ represents any one substituent indicated in [Table L1] to [Table L3] (hereinafter, referred to as "Compound class

SX47").

A present compound (L-1) wherein $R^{1b}$ represents an iodine atom, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class SX48").

A present compound (L-1) wherein $R^{1b}$ and $R^{1c}$ represent an iodine atom, $A^1$, $A^3$, $A^4$, and $R^5$ represent CH, and $A^2$ represents any one substituent indicated in [Table L1] to [Table L3] (hereinafter, referred to as "Compound class SX49").

A present compound (L-1) wherein $R^{1b}$ represents a methyl group, $R^{1c}$ represents a hydrogen atom, $A^1$, $A^3$, $A^4$, and $A^5$ represent CH, and $A^2$ represents any one substituent indicated in [Table. L1] to [Table L3] (hereinafter, referred to as "Compound class SX50").

A present compound (L-1) wherein $R^{1b}$ and $R^{1c}$ represent a methyl group, $A^1$, $A^3$, $A^4$, and $A^5$ represent CH, and $A^2$ represents any one substituent indicated in [Table L1] to [Table L3] (hereinafter, referred to as "Compound class SX51").

A present compound (L-1) wherein $R^{1b}$ represents an ethyl group, $R^{1c}$ represents a hydrogen atom, $A^1$, $A^3$, $A^4$, and $A^5$ represent CH, and $A^2$ represents any one substituent indicated in [Table L1) to [Table L3] (hereinafter, referred to as "Compound class SX52").

A present compound (L-1) wherein $R^{1b}$ and $R^{1c}$ represent an ethyl group, $A^1$, $A^3$, $A^4$, and $A^5$ represent CH, and $A^2$ represents any one substituent indicated in [Table L1] to [Table L3] (hereinafter, referred to as "Compound class SXS3").

A present compound (L-1) wherein $R^{1b}$ represents a propyl group, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class SX54").

A present compound (L-1) wherein $R^{1b}$ and $R^{1c}$ represent a propyl group, $A^1$, $A^3$, $A^4$, and $A^5$ represent CH, and $A^2$ represents any one substituent indicated in [Table L1] to [Table L3] (hereinafter, referred to as "Compound class 5X55").

A present compound (L-1) wherein $R^{1b}$ represents an isopropyl group, $R^{1c}$ represents a hydrogen atom, $A^1$, $A^3$, $A^4$, and $A^5$ represent CH, and $A^2$ represents any one substituent indicated in [Table L1] to [Table L3) (hereinafter, referred to as "Compound class SX56").

A present compound (L-1) wherein $R^{1b}$ and $R^{1c}$ represent an isopropyl group, $A^1$, $A^3$, $A^4$, and $A^5$ represent CH, and $A^2$ represents any one substituent indicated in [Table L1] to [Table L3] (hereinafter, referred to as "Compound class SX57").

A present compound (L-1) wherein $R^{1b}$ represents a cyclopropyl group, $R^{1c}$ represents a hydrogen atom, $A^1$, $A^3$, $A^4$, and $A^5$ represent CH, and $A^2$ represents any one. substituent indicated in [Table L1] to [Table L3] (hereinafter, referred to as "Compound class SXBS").

A present compound (L-1) wherein $R^{1b}$ and $R^{1c}$ represent a cyclopropyl group, $A^1$, $A^3$, $A^4$, and $A^5$ represent CH, and $A^2$ represents any one substituent indicated in [Table L1] to [Table L3] (hereinafter, referred to as "Compound class SX5.9").

A present compound (L-1) wherein $R^{1b}$ represents $CF_3$, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class SX60').

A present compound (L-1) wherein $R^{1b}$ and $R^{1c}$ represent $CF_3$ $A^1$, $A^3$, $A^4$, and $A^5$ represent CH, and $A^2$ represents any one substituent indicated in [Table L1] to [Table L3] (hereinafter, referred to as "Compound class SX61").

A present compound (L-1) wherein $R^{1b}$ represents a methylsulfanyl group, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class SX62").

A present compound (L-1) wherein $R^{1b}$ represents a methylsulfinyl group, $R^{1c}$ represents a hydrogen atom, $A^1$, $A^3$, $A^4$, and $A^5$ represent CH, and $A^2$ represents any one substituent indicated in [Table L1) to [Table L3] (hereinafter, referred. to as "compound class SX63").

A present compound (L-1) wherein $R^{1b}$ represents a methylsulfonyl group, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class SX64").

A present compound (L-1) wherein $R^{1b}$ represents a chlorine atom, $R^{1c}$ represents a bromine atom, $A^1$, $A^3$, $A^4$, and $A^5$ represent CH, and $A^2$ represents any one substituent indicated in (Table L1] to [Table L3] (hereinafter, referred to as "Compound class SX65").

A present compound (L-1) wherein $R^{1b}$ represents a chlorine atom, $R^{1c}$ represents an iodine atom, $A^1$, $A^3$, $A^4$, and $A^5$ represent CH, and $A^2$ represents any one substituent indicated in (Table L1] to [Table L3] (hereinafter, referred to as "Compound class SX66").

A present compound (L-1) wherein $R^{1b}$ represents a chlorine atom, $R^{1c}$ represents a methyl group, $A^1$, $A^3$, $A^4$,

and $A^5$ represent CH, and $A^2$ represents any one substituent indicated in [Table L1] to [Table L3] (hereinafter, referred to as "Compound class SX67").

A present compound (L-1) wherein $R^{1b}$ represents a chlorine atom, $R^{1c}$ represents an ethyl group, $A^1$, $A^3$, $A^4$, and $A^5$ represent CH, and $A^2$ represents any one substituent indicated in [Table L1) to [Table L3] (hereinafter, referred. to as "Compound class SX68").

A present compound (L-1) wherein $R^{1b}$ represents a chlorine atom, $R^{1c}$ represents a propyl group, $A^1$, $A^3$, $A^4$, and $A^5$ represent CH, and $A^2$ represents any one substituent indicated in [Table L1] to [Table L3] (hereinafter, referred to as "Compound class SX69").

A present compound (L-1) wherein $R^{1b}$ represents a chlorine atom, $R^{1c}$ represents an isopropyl group, $A^1$, $A^3$, $A^4$, and $A^5$ represent CH, and $A^2$ represents any one substituent indicated in (Table L1] to [Table L3] (hereinafter, referred to as "Compound class SX70").

A present compound (L-1) wherein $R^{1b}$ represents a chlorine atom, $R^{1c}$ represents a cyclopropyl group, $A^1$, $A^3$, $A^4$, and $A^5$ represent CH, and $A^2$ represents any one substituent indicated in (Table L1] to [Table L3] (hereinafter, referred to as "Compound class SX71").

A present compound (L-1) wherein $R^{1b}$ represents a chlorine atom, $R^{1c}$ represents $CF_3$, $A^1$, $A^3$, $A^4$, and $A^5$ represent CH, and $A^2$ represents any one substituent indicated in [Table L1] to [Table L3] (hereinafter, referred to as "Compound class 5X72").

A present compound (L-1) wherein $R^{1b}$ represents a bromine atom, $R^{1c}$ represents an iodine atom, $A^1$, $A^3$, $A^4$, and $A^5$ represent CH, and $A^2$ represents any one substituent indicated in [Table L1) to [Table L3] (hereinafter, referred. to as "Compound class SX73").

A present compound (L-1) wherein $R^{1b}$ represents a bromine. atom, $R^{1c}$ represents a methyl group, $A^1$, $A^3$, $A^4$, and $A^5$ represent CH, and $A^2$ represents any one substituent indicated in [Table L1] to [Table L3] (hereinafter, referred to as "Compound class SX74").

A present compound (L-1) wherein $R^{1b}$ represents a bromine atom, $R^{1c}$ represents an ethyl group, $A^1$, $A^3$, $A^4$, and $A^5$ represent CH, and $A^2$ represents any one substituent indicated in (Table L1] to [Table L3) (hereinafter, referred to as "Compound class SX75").

A present compound (L-1) wherein $R^{1b}$ represents a bromine atom, $R^{1c}$ represents a propyl group, $A^1$, $A^3$, $A^4$, and $A^5$ represent CH, and $A^2$ represents any one substituent indicated in (Table L1] to [Table L3] (hereinafter, referred to as "Compound class SX76").

A present compound (L-1) wherein $R^{1b}$ represents a bromine atom, $R^{1c}$ represents an isopropyl group, $A^1$, $A^3$, $A^4$, and $A^5$ represent CH, and $A^2$ represents any one substituent indicated in [Table L1] to [Table L3] (hereinafter, referred to as "Compound class SX77").

A present compound (L-1) wherein $R^{1b}$ represents a bromine atom, $R^{1c}$ represents a cyclopropyl group, $A^1$, $A^3$, $A^4$, and $A^5$ represent CH, and $A^2$ represents any one substituent indicated in [Table L1) to [Table L3] (hereinafter, referred. to as "Compound class SX78").

A present compound (L-1) wherein $R^{1b}$ represents a bromine atom, $R^{1c}$ represents $CF_3$, $A^1$, $A^3$, $A^4$, and $A^5$ represent CH, and $A^2$ represents any one substituent indicated in [Table L1] to [Table L3] (hereinafter, referred to as "Compound class SX79").

A present compound (L-1) wherein $R^{1b}$ represents a methyl group, $R^{1c}$ represents an ethyl group, $A^1$, $A^3$, $A^4$, and $A^5$ represent CH, and $A^2$ represents any one substituent indicated in [Table L1] to [Table L3] (hereinafter, referred to as "Compound class SX80").

A present compound (L-1) wherein $R^{1b}$ represents a methyl group, $R^{1c}$ represents a propyl group, $A^1$, $A^3$, $A^4$, and $A^5$ represent CH, and $A^2$ represents any one substituent indicated in [Table L1] to [Table L3] (hereinafter, referred to as "Compound class SX81"),

A present compound (1-1) wherein $R^{1b}$ represents an ethyl group, $R^{1c}$ represents a propyl group, $A^1$, $A^3$, $A^4$, and $A^5$ represent CH, and $A^2$ represents any one substituent indicated in [Table L1] to [Table L3] (hereinafter, referred to as "Compound class 3X82").

A present compound (L-1) wherein $R^{1b}$ represents a fluorine atom, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class SX83").

A present compound (L-1) wherein $R^{1b}$ and $R^{1c}$ represent a fluorine atom, $A^1$, $A^2$, $A^4$, and $A^5$ represent CH, and $A^3$ represents any one substituent indicated in [Table L1] to [Table L3] (hereinafter, referred to as "Compound class SX84").

A present compound (L-1) wherein $R^{1b}$ represents a chlorine atom, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class SXB5").

A present compound (L-1) wherein $R^{1b}$ and $R^{1c}$ represent a chlorine atom, $A^1$, $A^2$, $A^4$, and $A^5$ represent CH, and $A^3$ represents any one substituent indicated in [Table L1] to [Table L3] (hereinafter, referred to as "Compound class

SX86").

A present compound (L-1) wherein $R^{1b}$ represents a bromine atom, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class SX87").

A present compound (L-1) wherein $R^{1b}$ and $R^{1c}$ represent a bromine atom, $A^1$, $A^2$, $A^4$, and $A^5$ represent CH, and $A^3$ represents any one substituent indicated in [Table L1] to [Table L3] (hereinafter, referred to as "Compound class SX88").

A present compound (L-1) wherein $R^{1b}$ represents an iodine atom, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class SX89").

A present compound (L-1) wherein $R^{1b}$ and $R^{1c}$ represent an iodine atom, $A^1$, $A^2$, $A^4$, and $A^5$ represent CH, and $A^3$ represents any one substituent indicated in [Table L1] to [Table L3] (hereinafter, referred to as "Compound class SX90[11]).

A present compound (L-1) wherein $R^{1b}$ represents a methyl group, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class SX91").

A present compound (L-1) wherein $R^{1b}$ and $R^{1c}$ represent a methyl group, $A^1$, $A^2$, $A^4$, and $A^5$ represent CH, and $A^3$ represents any one substituent indicated in [Table L1] to [Table L3] (hereinafter, referred to as "Compound class SX92").

A present compound (L-1) wherein $R^{1b}$ represents an ethyl group, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class 5X93").

A present compound (L-1) wherein $R^{1b}$ and $R^{1c}$ represent an ethyl group, $A^1$, $A^2$, $A^4$, and $A^5$ represent CH, and $A^3$ represents any one substituent indicated in [Table L1] to [Table L3] (hereinafter, referred to as "Compound class SX94").

A present compound (L-1) wherein $R^{1b}$ represents a propyl group, $R^{1c}$ represents a hydrogen atom, $A^1$, $A^2$, $A^4$, and $A^5$ represent CH, and $A^3$ represents any one substituent indicated in [Table. L1] to [Table L3] (hereinafter, referred to as "Compound class SX95").

A present compound (L-1) wherein $R^{1b}$ and $R^{1c}$ represent a propyl group, $A^1$, $A^2$, $A^4$, and $A^5$ represent CH, and $A^3$ represents any one substituent indicated in [Table L1] to [Table L3] (hereinafter, referred to as "Compound class SX96").

A present compound (L-.1) wherein $R^{1b}$ represents an isopropyl group, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class SX97").

A present compound (L-1) wherein $R^{1b}$ and $R^{1c}$ represent an isopropyl group, $A^1$, $A^2$, $A^4$, and $A^5$ represent CH, and $A^3$ represents any one substituent indicated in [Table L1] to [Table L3] (hereinafter, referred to as "Compound class SX98"),

A present compound (L-1) wherein $R^{1b}$ represents a cyclopropyl group, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class SX99").

A present compound (L-1) wherein $R^{1b}$ and $R^{1c}$ represent a cyclopropyl group, $A^1$, $A^2$, $A^4$, and $A^5$ represent CH, and $A^3$ represents any one substituent indicated in [Table L1] to [Table L3] (hereinafter, referred to as "Compound class SX100").

A present compound (L-1) wherein $R^{1b}$ represents $CF_3$, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class SX101").

A present compound (L-1) wherein $R^{1b}$ and $R^{1c}$ represent $CF_3$, $A^1$, $A^2$, $A^4$, and $A^5$ represent CH, and $A^3$ represents any one substituent indicated in [Table L1] to [Table L3] (hereinafter, referred to as "Compound class SX102").

A present compound (L-1) wherein $R^{1b}$ represents a methylsulfanyl group, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class SX103").

A present compound (L-1) wherein $R^{1b}$ represents a methylsulfinyl group, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class SX104").

A present compound (L-1) wherein $R^{1b}$ represents a methylsulfonyl group, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class SX105").

A present compound (L-1) wherein $R^{1b}$ represents a chlorine atom, $R^{1c}$ represents a bromine atom, $A^1$, $A^2$,

$A^4$, and $A^5$ represent CH, and $A^3$ represents any one substituent indicated in [Table L1] to [Table L3] (hereinafter, referred to as "Compound class SX106").

A present compound (L-1) wherein $R^{1b}$ represents a chlorine atom, $R^{1c}$ represents an iodine atom, $A^1$, $A^2$, $A^4$, and $A^5$ represent CH, and $A^3$ represents any one substituent indicated in [Table L1] to [Table L3] (hereinafter, referred to as "Compound class SX107").

A present compound (L-1) wherein $R^{1b}$ represents a chlorine atom, $R^{1c}$ represents a methyl group, $A^1$, $A^2$, $A^4$, and $A^5$ represent CH, and $A^3$ represents any one substituent indicated in [Table L1] to [Table L3] (hereinafter, referred to as "Compound class SX108").

A present compound (L-1) wherein $R^{1b}$ represents a chlorine atom, $R^{1c}$ represents an ethyl group, $A^1$, $A^2$, $A^4$, and $A^5$ represent CH, and $A^3$ represents any one substituent indicated in [Table L1] to [Table L3] (hereinafter, referred to as "Compound class SX109").

A present compound (L-1) wherein $R^{1b}$ represents a chlorine atom, $R^{1c}$ represents a propyl group, $A^1$, $A^2$, $A^4$, and $A^5$ represent CH, and $A^3$ represents any one substituent indicated in [Table L1] to [Table L3] (hereinafter, referred to as "Compound class SX110"),

A present compound (L-1) wherein $R^{1b}$ represents a chlorine atom, $R^{1c}$ represents an isopropyl group, $A^1$, $A^2$, $A^4$, and $A^5$ represent CH, and $A^3$ represents any one substituent indicated in [Table L1] to [Table L3] (hereinafter, referred to as "Compound class SX111").

A present compound (L-1) wherein $R^{1b}$ represents a chlorine atom, $R^{1c}$ represents a cyclopropyl group, $A^1$, $A^2$, $A^4$, and $A^5$ represent CH, and $A^3$ represents any one substituent indicated in [Table L1] to [Table L3] (hereinafter, referred to as "Compound class SX112").

A present compound (L-1) wherein $R^{1b}$ represents a chlorine atom, $R^{1c}$ represents $CF_3$, $A^1$, $A^2$, $A^4$, and $A^5$ represent CH, and $A^3$ represents any one substituent indicated in [Table L1] to [Table L3] (hereinafter, referred to as "Compound class SX113").

A present compound (L-1) wherein $R^{1b}$ represents a bromine, atom, $R^{1c}$ represents an iodine atom, $A^1$, $A^2$, $A^4$, and $A^5$ represent CH, and $A^3$ represents any one substituent indicated in [Table L1] to [Table L3] (hereinafter, referred to as "Compound class SX114").

A present compound (L-1) wherein $R^{1b}$ represents a bromine atom, $R^{1c}$ represents a methyl group, $A^1$, $A^2$, $A^4$, and $A^5$ represent CH, and $A^3$ represents any one substituent indicated in [Table L1] to [Table L3] (hereinafter, referred to as "Compound class SX115").

A present compound (L-1) wherein $R^{1b}$ represents a bromine atom, $R^{1c}$ represents an ethyl group, $A^1$, $A^2$, $A^4$, and $A^5$ represent CH, and $A^3$ represents any one substituent indicated in [Table L1] to [Table L3] (hereinafter, referred to as "Compound class SX116").

A present compound (L-1) wherein $R^{1b}$ represents a bromine atom, $R^{1c}$ represents a propyl group. $A^1$, $A^2$, $A^4$, and $A^5$ represent CH, and $A^3$ represents any one substituent indicated in [Table L1] to [Table L3] (hereinafter, referred to as "Compound class SX117").

A present compound (L-1) wherein $R^{1b}$ represents a bromine atom, $R^{1c}$ represents an isopropyl group. $A^1$, $A^2$, $A^4$, and $A^5$ represent CH, and $A^3$ represents any one substituent indicated in [Table L1] to [Table L3] (hereinafter, referred to as "Compound class SX118").

A present compound (L-1) wherein $R^{1b}$ represents a bromine. atom, $R^{1c}$ represents a cyclopropyl group, $A^1$, $A^2$, $A^4$, and $A^5$ represent CH, and $A^3$ represents any one substituent indicated in [Table L1] to [Table L3] (hereinafter, referred to as "Compound class SX119").

A present compound (L-1) wherein $R^{1b}$ represents a bromine atom, $R^{1c}$ represents $CF_3$, $A^1$, $A^2$, $A^4$, and $A^5$ represent CH, and $A^3$ represents any one substituent indicated in [Table L1] to [Table L3] (hereinafter, referred to as "Compound class SX120").

A present compound (L-1) wherein $R^{1b}$ represents a methyl group, $R^{1c}$ represents an ethyl group, $A^1$, $A^2$, $A^4$, and $A^5$ represent CH, and $A^3$ represents any one substituent indicated in [Table L1] to [Table L3] (hereinafter, referred to as "Compound class SX121").

A present compound (L-1) wherein $R^{1b}$ represents a methyl group, $R^{1c}$ represents a propyl group, $A^1$, $A^2$, $A^4$, and $A^5$ represent CH, and $A^3$ represents any one substituent indicated in [Table L1] to [Table L3] (hereinafter, referred to as "Compound class SX122").

A present compound (L-1) wherein $R^{1b}$ represents an ethyl group, $R^{1c}$ represents a propyl group, $A^1$, $A^2$, $A^4$, and $A^5$ represent CH, and $A^3$ represents any one substituent indicated in [Table L1] to [Table L3] (hereinafter, referred to as "Compound class SX123").

A present compound (L-2) wherein $R^{1b}$ represents a fluorine atom, $R^{1c}$ represents a hydrogen atom, $A^2$ represents C-F, $A^1$, $A^3$, and $A^5$ represent CH, and $A^4$ represents any one substituent indicated in [Table L1] to [Table L3] (hereinafter, referred to as "Compound class SX124").

A present compound (L-1) wherein $R^{1b}$ represents a fluorine atom, $R^{1c}$ represents a hydrogen atom, $A^2$ represents C-Cl, $A^1$, $A^3$, and $A^5$ represent CH, and $A^4$ represents any one substituent indicated in [Table L1] to [Table. L3]

(hereinafter, referred to as "Compound class SX125").

A present compound (L-1) wherein $R^{1b}$ represents a fluorine atom, $R^{1c}$ represents a hydrogen atom, $A^2$ represents C-Br, $A^1$, $A^3$, and $A^5$ represent CH, and $A^4$ represents any one substituent indicated in [Table L1] to [Table L3] (hereinafter, referred to as "Compound class SX126").

A present compound (L-1) wherein $R^{1b}$ represents a fluorine atom, $R^{1c}$ represents a hydrogen atom, $A^2$ represents C-I, $A^1$, $A^3$, and $A^5$ represent CH, and $A^4$ represents any one substituent indicated in [Table 1.1] to [Table L3] (hereinafter, referred to as "Compound class SX127").

A present compound (L-1) wherein $R^{1b}$ represents a fluorine atom, $R^{1c}$ represents a hydrogen atom, $A^2$ represents C-Me, $A^1$, $A^3$, and $A^5$ represent CH, and $A^4$ represents any one substituent indicated in [Table L1] to [Table L3] (hereinafter, referred to as "Compound class SX128").

A present compound (L-2) wherein $R^{1b}$ represents a fluorine atom, $R^{1c}$ represents a hydrogen atom, $A^2$ represents C-OMe, $A^1$, $A^3$, and $A^5$ represent CH, and $A^4$ represents any one substituent indicated in [Table L1] to [Table L3] (hereinafter, referred to as "Compound class SX129").

A present compound (L-1) wherein $R^{1b}$ represents a fluorine atom, $R^{1c}$ represents a hydrogen atom, $A^2$ represents C-$CF_3$, $A^1$, $A^3$, and $A^5$ represent CH, and $A^4$ represents any one substituent indicated in [Table L1] to [Table. L3] (hereinafter, referred to as "Compound class SX130").

A present compound (L-1) wherein $R^{1b}$ represents a fluorine atom, $R^{1c}$ represents a hydrogen atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX131").

A present compound (L-1) wherein $R^{1b}$ represents a fluorine atom, $R^{1c}$ represents a hydrogen atom, $A^3$ represents C-F, $A^1$, $A^2$, and $A^5$ represent CH, and $A^4$ represents any one substituent indicated in [Table 1.1] to [Table L3] (hereinafter, referred to as "Compound class SX132").

A present compound (L-1) wherein $R^{1b}$ represents a fluorine atom, $R^{1c}$ represents a hydrogen atom, $A^3$ represents C-Cl, $A^1$, $A^2$, and $A^5$ represent CH, and $A^4$ represents any one substituent indicated in [Table L1] to [Table L3] (hereinafter, referred to as "Compound class SX133").

A present compound (L-2) wherein $R^{1b}$ represents a fluorine atom, $R^{1c}$ represents a hydrogen atom, $A^3$ represents C-Br, $A^1$, $A^2$, and $A^5$ represent CH, and $A^4$ represents any one substituent indicated in [Table L1] to [Table L3] (hereinafter, referred to as "Compound class 8X134").

A present compound (L-1) wherein $R^{1b}$ represents a fluorine atom, $R^{1c}$ represents a hydrogen atom, $A^3$ represents C-I, $A^1$, $A^2$, and $A^5$ represent CH, and $A^4$ represents any one substituent indicated in [Table L1] to [Table. L3] (hereinafter, referred to as "Compound class 8X135").

A present compound (L-1) wherein $R^{1b}$ represents a fluorine atom, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter, referred to as "Compound class SX136").

A present compound (L-1) wherein $R^{1b}$ represents a fluorine atom, $R^{1c}$ represents a hydrogen atom, $A^3$ represents C-OMe, $A^1$, $A^2$, and $A^5$ represent CH, and $A^4$ represents any one substituent indicated in [Table 1.1] to [Table L3] (hereinafter, referred to as "Compound class SX137").

A present compound (L-1) wherein $R^{1b}$ represents a fluorine atom, $R^{1c}$ represents a hydrogen atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX138").

A present compound (L-2) wherein $R^{1b}$ represents a fluorine atom, $R^{1c}$ represents a hydrogen atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX139").

A present compound (L-1) wherein $R^{1b}$ represents a chlorine atom, $R^{1c}$ represents a hydrogen atom, $A^2$ represents C-F, $A^1$, $A^3$, and $A^5$ represent CH, and $A^4$ represents any one substituent indicated in [Table L1] to [Table. L3] (hereinafter, referred to as "Compound class SX140").

A present compound (L-1) wherein $R^{1b}$ represents a chlorine atom, $R^{1c}$ represents a hydrogen atom, $A^2$ represents C-Cl, $A^1$, $A^3$, and $A^5$ represent CH, and $A^4$ represents any one substituent indicated in [Table L1] to [Table L3] (hereinafter, referred to as "Compound class SX141").

A present compound (L-1) wherein $R^{1b}$ represents a chlorine atom, $R^{1c}$ represents a hydrogen atom, $A^2$ represents C-Br, $A^1$, $A^3$, and $A^5$ represent CH, and $A^4$ represents any one substituent indicated in [Table 1.1] to [Table L3] (hereinafter, referred to as "Compound class SX142").

A present compound (L-1) wherein $R^{1b}$ represents a chlorine atom, $R^{1c}$ represents a hydrogen atom, $A^2$ represents C-I, $A^1$, $A^3$, and $A^5$ represent CH, and $A^4$ represents any one substituent indicated in [Table L1] to [Table L3] (hereinafter, referred to as "Compound class SX143").

A present compound (L-2) wherein $R^{1b}$ represents a chlorine atom, $R^{1c}$ represents a hydrogen atom, $A^2$ represents C-Me, $A^1$, $A^3$, and $A^5$ represent CH, and $A^4$ represents any one substituent indicated in [Table L1] to [Table L3] (hereinafter, referred to as "Compound class 8X144").

A present compound (L-1) wherein $R^{1b}$ represents a chlorine atom, $R^{1c}$ represents a hydrogen atom, $A^2$ represents C-OMe, $A^1$, $A^3$, and $A^5$ represent CH, and $A^4$ represents any one substituent indicated in [Table L1] to [Table. L3] (hereinafter, referred to as "Compound class SX145").

A present compound (L-1) wherein $R^{1b}$ represents a chlorine atom, $R^{1c}$ represents a hydrogen atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX146").

A present compound (L-1) wherein $R^{1b}$ represents a chlorine atom, $R^{1c}$ represents a hydrogen atom, $A^2$ represents C-$OCF_3$, $A^1$, $A^3$, and $A^5$ represent CH, and $A^4$ represents any one substituent indicated in [Table 1.1] to [Table L3] (hereinafter, referred to as "Compound class SX147").

A present compound (L-1) wherein $R^{1b}$ represents a chlorine atom, $R^{1c}$ represents a hydrogen atom, $A^3$ represents C-F, $A^1$, $A^2$, and $A^5$ represent CH, and $A^4$ represents any one substituent indicated in [Table L1] to [Table L3] (hereinafter, referred to as "Compound class SX148").

A present compound (L-2) wherein $R^{1b}$ represents a chlorine atom, $R^{1c}$ represents a hydrogen atom, $A^3$ represents C.-C1, $A^1$, $A^2$, and $A^5$ represent CH, and $A^4$ represents any one substituent indicated in [Table L1] to [Table L3] (hereinafter, referred to as "Compound class 8X149").

A present compound (L-1) wherein $R^{1b}$ represents a chlorine atom, $R^{1c}$ represents a hydrogen atom, $A^3$ represents C-Br, $A^1$, $A^2$, and $A^5$ represent CH, and $A^4$ represents any one substituent indicated in [Table L1] to [Table. L3] (hereinafter, referred to as "Compound class 8X150").

A present compound (L-1) wherein $R^{1b}$ represents a chlorine atom, $R^{1c}$ represents a hydrogen atom, $A^3$ represents C-I, $A^1$, $A^2$, and $A^5$ represent CH, and $A^4$ represents any one substituent indicated in [Table L1] to [Table L3] (hereinafter, referred to as "Compound class SX151").

A present compound (L-1) wherein $R^{1b}$ represents a chlorine atom, $R^{1c}$ represents a hydrogen atom, $A^3$ represents C-Me, $A^1$, $A^2$, and $A^5$ represent CH, and $A^4$ represents any one substituent indicated in [Table 1.1] to [Table L3] (hereinafter, referred to as "Compound class SX152").

A present compound (L-1) wherein $R^{1b}$ represents a chlorine atom, $R^{1c}$ represents a hydrogen atom, $A^3$ represents C-OMe, $A^1$, $A^2$, and $A^5$ represent CH, and $A^4$ represents any one substituent indicated in [Table L1] to [Table L3] (hereinafter, referred to as "Compound class SX153").

A present compound (L-2) wherein $R^{1b}$ represents a chlorine atom, $R^{1c}$ represents a hydrogen atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class 8X154").

A present compound (L-1) wherein $R^{1b}$ represents a chlorine atom, $R^{1c}$ represents a hydrogen atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class 8X155").

A present compound (L-1) wherein $R^{1b}$ represents a bromine atom, $R^{1c}$ represents a hydrogen atom, $A^2$ represents C-F, $A^1$, $A^3$, and $A^5$ represent CH, and $A^4$ represents any one substituent indicated in [Table L1] to [Table L3] (hereinafter, referred to as "Compound class SX156").

A present compound (L-1) wherein $R^{1b}$ represents a bromine atom, $R^{1c}$ represents a hydrogen atom, $A^2$ represents C.-C1, $A^1$, $A^3$, and $A^5$ represent CH, and $A^4$ represents any one substituent indicated in [Table L1] to [Table L3] (hereinafter, referred to as "Compound class SX157").

A present compound (L-1) wherein $R^{1b}$ represents a bromine atom, $R^{1c}$ represents a hydrogen atom, $A^2$ represents C-Br, $A^1$, $A^3$, and $A^5$ represent CH, and $A^4$ represents any one substituent indicated in [Table L1] to [Table L3] (hereinafter, referred to as "Compound class SX158").

A present compound (L-1) wherein $R^{1b}$ represents a bromine atom, $R^{1c}$ represents a hydrogen atom, $A^2$ represents C-I, $A^1$, $A^3$, and $A^5$ represent CH, and $A^4$ represents any one substituent indicated in [Table L1] to [Table L3] (hereinafter, referred to as "Compound class SX159").

A present compound (L-1) wherein $R^{1b}$ represents a bromine atom, $R^{1c}$ represents a hydrogen atom, $A^2$ represents C-Me, $A^1$, $A^3$, and $A^5$ represent CH, and $A^4$ represents any one substituent indicated in [Table L1] to [Table L3] (hereinafter, referred to as "Compound class SX160").

A present compound (L-1) wherein $R^{1b}$ represents a bromine atom, $R^{1c}$ represents a hydrogen atom, $A^2$ represents C-OMe, $A^1$, $A^3$, and $A^5$ represent CH, and $A^4$ represents any one substituent indicated in [Table L1] to [Table L3] (hereinafter, referred to as "Compound class SX161").

A present compound (L-1) wherein $R^{1b}$ represents a bromine atom, $R^{1c}$ represents a hydrogen atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX162").

A present compound (L-1) wherein $R^{1b}$ represents a bromine atom, $R^{1c}$ represents a hydrogen atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX163").

A present compound (L-1) wherein $R^{1b}$ represents a bromine atom, $R^{1c}$ represents a hydrogen atom, $A^3$

represents C-F, $A^1$, $A^2$, and $A^5$ represent CH, and $A^4$ represents any one substituent indicated in [Table L1] to [Table L3] (hereinafter, referred to as "Compound class SX164").

A present compound (L-1) wherein $R^{1b}$ represents a bromine atom, $R^{1c}$ represents a hydrogen atom, $A^3$ represents C-Cl, $A^1$, $A^2$, and $A^5$ represent CH, and $A^4$ represents any one substituent indicated in [Table L1] to [Table L3] (hereinafter, referred to as "Compound class SX165").

A present compound (L-1) wherein $R^{1b}$ represents a bromine atom, $R^{1c}$ represents a hydrogen atom, $A^3$ represents C-Br, $A^1$, $A^2$, and $A^5$ represent CH, and $A^4$ represents any one substituent indicated in [Table L1] to [Table L3] (hereinafter, referred to as "Compound class SX166").

A present compound (L-1) wherein $R^{1b}$ represents a bromine atom, $R^{1c}$ represents a hydrogen atom, $A^3$ represents C-I, $A^1$, $A^2$, and $A^5$ represent CH, and $A^4$ represents any one substituent indicated in [Table L1] to [Table L3] (hereinafter, referred to as "Compound class SX167").

A present compound (L-1) wherein $R^{1b}$ represents a bromine atom, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter, referred to as "Compound class SX168").

A present compound (L-1) wherein $R^{1b}$ represents a bromine atom, $R^{1c}$ represents a hydrogen atom, $A^3$ represents C-OMe, $A^1$, $A^2$, and $A^5$ represent CH, and $A^4$ represents any one substituent indicated in [Table L1] to [Table L3] (hereinafter, referred to as "Compound class SX169").

A present compound (L-1) wherein $R^{1b}$ represents a bromine atom, $R^{1c}$ represents a hydrogen atom, $A^3$ represents $C-CF_3$, $A^1$, $A^2$, and $A^5$ represent CH, and $A^4$ represents any one substituent indicated in [Table L1] to [Table L3] (hereinafter, referred to as "Compound class SX170").

A present compound (L-1) wherein $R^{1b}$ represents a bromine atom, $R^{1c}$ represents a hydrogen atom, $A^3$ represents $C-OCF_3$, $A^1$, $A^2$, and $A^5$ represent CH, and $A^4$ represents any one substituent indicated in [Table L1] to [Table L3] (hereinafter, referred to as "Compound class SX171").

A present compound (1-1) wherein $R^{1b}$ represents an iodine atom, $R^{1c}$ represents a hydrogen atom, $A^2$ represents C-F, $A^1$, $A^3$, and $A^5$ represent CH, and $A^4$ represents any one substituent indicated in [Table L1] to [Table L3] (hereinafter, referred to as "Compound class SX172").

A present compound (L-1) wherein $R^{1b}$ represents an iodine atom, $R^{1c}$ represents a hydrogen atom, $A^2$ represents C-Cl, $A^1$, $A^3$, and $A^5$ represent CH, and $A^4$ represents any one substituent indicated in [Table L1] to [Table L3] (hereinafter, referred to as "Compound class SX173").

A present compound (L-1) wherein $R^{1b}$ represents an iodine atom, $R^{1c}$ represents a hydrogen atom, $A^2$ represents C-Br, $A^1$, $A^3$, and $A^5$ represent CH, and $A^4$ represents any one substituent indicated in [Table L1] to [Table L3] (hereinafter, referred to as "Compound class SX174").

A present compound (L-1) wherein $R^{1b}$ represents an iodine atom, $R^{1c}$ represents a hydrogen atom, $A^2$ represents C-I, $A^1$, $A^3$, and $A^5$ represent CH, and $A^4$ represents any one substituent indicated in [Table L1] to [Table L3] (hereinafter, referred to as "Compound class SX175").

A present compound (L-1) wherein $R^{1b}$ represents an iodine atom, $R^{1c}$ represents a hydrogen atom, $A^2$ represents C-Me, $A^1$, $A^3$, and $A^5$ represent CH, and $A^4$ represents any one substituent indicated in [Table L1] to [Table L3] (hereinafter, referred to as "Compound class SX176").

A present compound (1-1) wherein $R^{1b}$ represents an iodine atom, $R^{1c}$ represents a hydrogen atom, $A^2$ represents C-OMe, $A^1$, $A^3$, and $A^5$ represent CH, and $A^4$ represents any one substituent indicated in [Table L1] to (Table L3] (hereinafter, referred to as "Compound class SX177") .

A present compound (L-1) wherein $R^{1b}$ represents an iodine atom, $R^{1c}$ represents a hydrogen atom, $A^2$ represents $C-CF_3$, $A^1$, $A^3$, and $A^5$ represent CH, and $A^4$ represents any one substituent indicated in [Table L1] to [Table L3] (hereinafter, referred to as "Compound class SX178").

A present compound (L-1) wherein $R^{1b}$ represents an iodine atom, $R^{1c}$ represents a hydrogen atom, $A^2$ represents $C-OCF_3$, $A^1$, $A^3$, and $A^5$ represent CH, and $A^4$ represents any one substituent indicated in [Table L1] to [Table L3] (hereinafter, referred to as "Compound class SX179").

A present compound (L-1) wherein $R^{1b}$ represents an iodine atom, $R^{1c}$ represents a hydrogen atom, $A^3$ represents C-F, $A^1$, $A^2$, and $A^5$ represent CH, and $A^4$ represents any one substituent indicated in [Table L1] to (Table L3] (hereinafter, referred to as "Compound class SX180").

A present compound (L-1) wherein $R^{1b}$ represents an iodine atom, $R^{1c}$ represents a hydrogen atom, $A^3$ represents C-Cl, $A^1$, $A^2$, and $A^5$ represent CH, and $A^4$ represents any one substituent indicated in [Table L1] to [Table L3] (hereinafter, referred to as "Compound class SX181").

A present compound (1-1) wherein $R^{1b}$ represents an iodine atom, $R^{1c}$ represents a hydrogen atom, $A^3$ represents C-Br, $A^1$, $A^2$, and $A^5$ represent CH, and $A^4$ represents any one substituent indicated in [Table L1] to [Table L3] (hereinafter, referred to as "Compound class SX182").

A present compound (L-1) wherein $R^{1b}$ represents an iodine atom, $R^{1c}$ represents a hydrogen atom, $A^3$ represents C-I, $A^1$, $A^2$, and $A^5$ represent CH, and $A^4$ represents any one substituent indicated in [Table L1] to [Table L3]

(hereinafter, referred to as "Compound class SX183").

A present compound (L-1) wherein $R^{1b}$ represents an iodine atom, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter, referred to as "Compound class SX184").

A present compound (L-1) wherein $R^{1b}$ represents an iodine atom, $R^{1b}$ represents a hydrogen atom, $A^3$ represents C-OMe, $A^1$, $A^2$, and $A^5$ represent CH, and $A^4$ represents any one substituent indicated in [Table L1] to [Table L3] (hereinafter, referred to as "Compound class SX185").

A present compound (L-1) wherein $R^{1b}$ represents an iodine atom, $R^{1c}$ represents a hydrogen atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX186").

A present compound (1-1) wherein $R^{1b}$ represents an iodine atom, $R^{1c}$ represents a hydrogen atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX187").

A present compound (L-1) wherein $R^{1b}$ represents a methyl group, $R^{1c}$ represents a hydrogen atom, $A^2$ represents C-F, $A^1$, $A^3$, and $A^5$ represent CH, and $A^4$ represents any one substituent indicated in [Table L1] to [Table L3] (hereinafter, referred to as "Compound class SX188").

A present compound (L-1) wherein $R^{1b}$ represents a methyl group, $R^{1c}$ represents a hydrogen atom, $A^2$ represents C-Cl, $A^1$, $A^3$, and $A^5$ represent CH, and $A^4$ represents any one substituent indicated in [Table L1] to [Table L3] (hereinafter, referred to as "Compound class SX189").

A present compound (L-1) wherein $R^{1b}$ represents a methyl group, $R^{1c}$ represents a hydrogen atom, $A^2$ represents C-Br, $A^1$, $A^3$, and $A^5$ represent CH, and $A^4$ represents any one substituent indicated in [Table L1] to [Table L3] (hereinafter, referred to as "Compound class 3X190").

A present compound (L-1) wherein $R^{1b}$ represents a methyl group, $R^{1c}$ represents a hydrogen atom, $A^2$ represents C-I, $A^1$, $A^3$, and $A^5$ represent CH, and $A^4$ represents any one substituent indicated in [Table L1] to [Table L3] (hereinafter, referred to as "Compound class SX191").

A present compound (L-1) wherein $R^{1b}$ represents a. methyl group, $R^{1c}$ represents a hydrogen atom, $A^2$ represents C-He, $A^1$, $A^3$, and $A^5$ represent CH, and $A^4$ represents any one substituent indicated in [Table L1] to [Table L3] (hereinafter, referred to as "Compound class SX192").

A present compound (L-1) wherein $R^{1b}$ represents a methyl group, $R^{1c}$ represents a hydrogen atom, $A^2$ represents C-OMe, $A^1$, $A^3$, and $A^5$ represent CH, and $A^4$ represents any one substituent indicated in [Table L1] to [Table L3] (hereinafter, referred to as "Compound class SX193").

A present compound (L-1) wherein $R^{1b}$ represents a methyl group, $R^{1c}$ represents a hydrogen atom, $A^2$ represents C-$CF_3$, $A^1$, $A^3$, and $A^5$ represent CH, and $A^4$ represents any one substituent indicated in [Table 1.1] to [Table L3] (hereinafter, referred to as "Compound class SX194").

A present compound (L-1) wherein $R^{1b}$ represents a methyl group, $R^{1c}$ represents a hydrogen atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX195").

A present compound (L-1) wherein $R^{1b}$ represents a methyl group, $R^{1c}$ represents a hydrogen atom, $A^3$ represents C-F, $A^1$, $A^2$, and $A^5$ represent CH, and $A^4$ represents any one substituent indicated in [Table L1] to [Table L3] (hereinafter, referred to as "Compound class SX196").

A present compound (L-1) wherein $R^{1b}$ represents a methyl group, $R^{1c}$ represents a hydrogen atom, $A^3$ represents C-Cl, $A^1$, $A^2$, and $A^5$ represent CH, and $A^4$ represents any one substituent indicated in [Table L1] to [Table L3] (hereinafter, referred to as "Compound class SX197").

A present compound (L-1) wherein $R^{1b}$ represents a methyl group, $R^{1c}$ represents a hydrogen atom, $A^3$ represents C-Br, $A^1$, $A^2$, and $A^5$ represent CH, and $A^4$ represents any one substituent indicated in [Table L1] to [Table L3] (hereinafter, referred to as "Compound class SX198").

A present compound (L-1) wherein $R^{1b}$ represents a methyl group, $R^{1c}$ represents a hydrogen atom, $A^3$ represents C-I, $A^1$, $A^2$, and $A^5$ represent CH, and $A^4$ represents any one substituent indicated in [Table L1] to [Table L3] (hereinafter, referred to as "Compound class SX199").

A present compound (L-1) wherein $R^{1b}$ represents a methyl group, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter, referred to as "Compound class SX200").

A present compound (L-1) wherein $R^{1b}$ represents a methyl group, $R^{1c}$ represents a hydrogen atom, $A^3$ represents C-OMe, $A^1$, $A^2$, and $A^5$ represent CH, and $A^4$ represents any one substituent indicated in [Table L1] to [Table L3] (hereinafter, referred to as "Compound class SX201").

A present compound (L-1) wherein $R^{1b}$ represents a. methyl group, $R^{1c}$ represents a hydrogen atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX202").

A present compound (L-1) wherein $R^{1b}$ represents a methyl group, $R^{1c}$ represents a hydrogen atom, $A^3$ represents C-$OC_3$, $A^1$, $A^2$, and $A^5$ represent CH, and $A^4$ represents any one substituent indicated in [Table L1] to [Table L3] (hereinafter, referred to as "Compound class SX203").

A present compound (L-1) wherein $R^{1b}$ represents an ethyl group, $R^{1c}$ represents a hydrogen atom, $A^2$ represents C-F, $A^1$, $A^3$, and $A^5$ represent CH, and $A^4$ represents any one substituent indicated in [Table L1] to [Table L3] (hereinafter, referred to as "Compound class SX204").

A present compound (L-1) wherein $R^{1b}$ represents an ethyl group, $R^{1c}$ represents a hydrogen atom, $A^2$ represents C-Cl, $A^1$, $A^3$, and $A^5$ represent CH, and $A^4$ represents any one substituent indicated in [Table L1] to [Table L3] (hereinafter, referred to as "Compound class SX205").

A present compound (L-1) wherein $R^{1b}$ represents an ethyl group, $R^{1c}$ represents a hydrogen atom, $A^2$ represents C-Br, $A^1$, $A^3$, and $A^5$ represent CH, and $A^4$ represents any one substituent indicated in [Table L1] to [Table L3] (hereinafter, referred to as "Compound class SX206"),

A present compound (L-1) wherein $R^{1b}$ represents an ethyl group, $R^{1c}$ represents a hydrogen atom, $A^2$ represents C-I, $A^1$, $A^3$, and $A^5$ represent CH, and $A^4$ represents any one substituent indicated in [Table L1] to [Table L3] (hereinafter, referred to as "Compound class SX207").

A present compound (L-1) wherein $R^{1b}$ represents an ethyl group, $R^{1c}$ represents a hydrogen atom, $A^2$ represents C-Me, $A^1$, $A^3$, and $A^5$ represent CH, and $A^4$ represents any one substituent indicated in [Table L1] to [Table L3] (hereinafter, referred to as "Compound class SX208").

A present compound (L-1) wherein $R^{1b}$ represents an ethyl group, $R^{1c}$ represents a hydrogen atom, $A^2$ represents C-OMe, $A^1$, $A^3$, and $A^5$ represent CH, and $A^4$ represents any one substituent indicated in [Table 1.1] to [Table L3] (hereinafter, referred to as "Compound class 5X209").

A present compound (L-1) wherein $R^{1b}$ represents an ethyl group, $R^{1c}$ represents a hydrogen atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX210") .

A present compound (L-1) wherein $R^{1b}$ represents an ethyl group, $R^{1c}$ represents a hydrogen atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class 8X211").

A present compound (L-1) wherein $R^{1b}$ represents an ethyl group, $R^{1c}$ represents a hydrogen atom, $A^3$ represents C-F, $A^1$, $A^2$, and $A^5$ represent CH, and $A^4$ represents any one substituent indicated in [Table L1] to [Table L3) (hereinafter, referred to as "Compound class SX212").

A present compound (L-1) wherein $R^{1b}$ represents an ethyl group, $R^{1c}$ represents a hydrogen atom, $A^3$ represents C-Cl, $A^1$, $A^2$, and $A^5$ represent CH, and $A^4$ represents any one substituent indicated in [Table L1] to [Table L3] (hereinafter, referred to as "Compound class SX213") .

A present compound (L-1) wherein $R^{1b}$ represents an ethyl group, $R^{1c}$ represents a hydrogen atom, $A^3$ represents C-Br, $A^1$, $A^2$, and $A^5$ represent CH, and $A^4$ represents any one substituent indicated in [Table L1] to [Table L3] (hereinafter, referred to as "Compound class SX214").

A present compound (L-1) wherein $R^{1b}$ represents an ethyl group, $R^{1c}$ represents a hydrogen atom, $A^3$ represents C-I, $A^1$, $A^2$, and $A^5$ represent CH, and $A^4$ represents any one substituent indicated in [Table L1] to [Table L3] (hereinafter, referred to as "Compound class SX215").

A present compound (L-1) wherein $R^{1b}$ represents an ethyl group, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter, referred to as "Compound class SX216"),

A present compound (L-1) wherein $R^{1b}$ represents an ethyl group, $R^{1c}$ represents a hydrogen atom, $A^3$ represents C-OMe, $A^1$, $A^2$, and $A^5$ represent CH, and $A^4$ represents any one substituent indicated in [Table L1] to [Table L3] (hereinafter, referred to as "Compound class SX217").

A present compound (L-1) wherein $R^{1b}$ represents an ethyl group, $R^{1c}$ represents a hydrogen atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX218").

A present compound (L-1) wherein $R^{1b}$ represents an ethyl group, $R^{1c}$ represents a hydrogen atom, $A^3$ represents C-$OCF_3$, $A^1$, $A^2$, and $A^5$ represent CH, and $A^4$ represents any one substituent indicated in [Table 1.1] to [Table L3] (hereinafter, referred to as "Compound class 5X219").

A present compound (L-1) wherein $R^{1b}$ represents $CF_3$, $R^{1c}$ represents a hydrogen atom, $A^2$ represents C-F, $A^1$, $A^3$, and $A^5$ represent CH, and $A^4$ represents any one substituent indicated in [Table L1] to [Table L3] (hereinafter, referred to as "Compound class. SX220").

A present compound (L-1) wherein $R^{1b}$ represents $CF_3$, $R^{1c}$ represents a hydrogen atom, $A^2$ represents C-Cl, $A^1$, $A^3$, and $A^5$ represent CH, and $A^4$ represents any one substituent indicated in [Table L1] to [Table L3] (hereinafter, referred to as "Compound class SX221").

A present compound (L-1) wherein $R^{2b}$ represents $CF_3$, $R^{1c}$ represents a hydrogen atom, $A^2$ represents C-Br,

$A^1$, $A^3$, and $A^5$ represent CH, and $A^4$ represents any one substituent indicated in [Table L1] to [Table L3] (hereinafter, referred to as "Compound class SX222").

A present compound (L-1) wherein $R^{1b}$ represents $CF_3$, $R^{1c}$ represents a hydrogen atom, $A^2$ represents C-I, $A^1$, $A^3$, and $A^5$ represent CH, and $A^4$ represents any one substituent indicated in [Table L1] to [Table L3] (hereinafter, referred to as "Compound class SX223").

A present compound (L-1) wherein $R^{1b}$ represents $CF_3$, $R^{1c}$ represents a hydrogen atom, $A^2$ represents C-Me, $A^1$, $A^3$, and $A^5$ represent CH, and $A^4$ represents any one substituent indicated in [Table L1] to [Table L3] (hereinafter, referred to as "Compound class SX224").

A present compound (L-1) wherein $R^{1b}$ represents $CF_3$, $R^{1c}$ represents a hydrogen atom, $A^2$ represents C-OMe, $A^1$, $A^3$, and $A^5$ represent CH, and $A^4$ represents any one substituent indicated in [Table L1] to [Table. L3] (hereinafter, referred to as "Compound class SX225").

A present compound (L-1) wherein $R^{1b}$ represents $CF_3$, $R^{1c}$ represents a hydrogen atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX226*).

A present compound (L-1) wherein $R^{3b}$ represents $CF_3$, $R^{1c}$ represents a hydrogen atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX227").

A present compound (L-1) wherein $R^{1b}$ represents $CF_3$, $R^{1c}$ represents a hydrogen atom, $A^3$ represents C-F, $A^1$, $A^2$, and $A^5$ represent CH, and $A^4$ represents, any one substituent indicated in [Table L1] to [Table L3] (hereinafter, referred to as "Compound class SX228").

A present compound (L-1) wherein $R^{1b}$ represents $CF_3$, $R^{1c}$ represents a hydrogen atom, $A^3$ represents C-Cl, $A^1$, $A^2$, and $A^5$ represent CH, and $A^4$ represents any one substituent indicated in [Table L1] to [Table L3] (hereinafter, referred to as "Compound class SX229").

A present compound (L-1) wherein $R^{1b}$ represents $CF_3$, $R^{1c}$ represents a hydrogen atom, $A^3$ represents C-Br, $A^1$, $A^2$, and $A^5$ represent CH, and $A^4$ represents any one substituent indicated in [Table L1] to [Table. L3] (hereinafter, referred to as "Compound class SX230").

A present compound (L-1) wherein $R^{1b}$ represents $CF_3$, $R^{1c}$ represents a hydrogen atom, $A^3$ represents C-I, $A^1$, $A^2$, and $A^5$ represent CH, and $A^4$ represents any one substituent indicated in [Table L1] to [Table L3] (hereinafter, referred to as "Compound class SX231") .

A present compound (L-1) wherein $R^{1b}$ represents $CF_3$, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter, referred to as "Compound class SX232").

A present compound (L-1) wherein $R^{1b}$ represents $CF_3$, $R^{1c}$ represents a hydrogen atom, $A^3$ represents C-OMe, $A^1$, $A^2$, and $A^5$ represent CH, and $A^4$ represents any one substituent indicated in [Table L1] to [Table L3] (hereinafter, referred to as "Compound class SX233").

A present compound (L-1) wherein $R^{1b}$ represents $CF_3$, $R^{1c}$ represents a hydrogen atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX234").

A present compound (L-1) wherein $R^{1b}$ represents $CF_3$, $R^{1c}$ represents a hydrogen atom, $A^3$ represents C-$OCF_3$, $A^1$, $A^2$, and $A^5$ represent CH, and $A^4$ represents any one substituent indicated in [Table L1] to [Table. L3] (hereinafter, referred to as "Compound class SX235").

A present Compound (L-1) wherein $R^{1b}$ represents a cyclopropyl group, $R^{1c}$ represents a hydrogen atom, $A^2$ represents C-F, $A^1$, $A^3$, and $A^5$ represent CH, and $A^4$ represents any one substituent indicated in [Table L1] to [Table L3] (hereinafter, referred to as "Compound class SX236").

A present compound (L-1) wherein $R^{1b}$ represents a cyclopropyl group, $R^{1c}$ represents a hydrogen atom, $A^2$ represents C-Cl, $A^1$, $A^3$, and $A^5$ represent CH, and $A^4$ represents any one substituent indicated in [Table L1] to [Table L3] (hereinafter, referred to as "Compound class SX237").

A present compound (L-1) wherein $R^{1b}$ represents a cyclopropyl group, $R^{1c}$ represents a hydrogen atom, $A^2$ represents C-Br, $A^1$, $A^3$, and $A^5$ represent CH, and $A^4$ represents any one substituent indicated in [Table L1] to [Table L3] (hereinafter, referred to as "Compound class SX238").

A present compound (L-1) wherein $R^{1b}$ represents a cyclopropyl group, $R^{1c}$ represents a hydrogen atom, $A^2$ represents C-I, $A^1$, $A^3$, and $A^5$ represent CH, and $A^4$ represents any one substituent indicated in [Table L1] to [Table L3] (hereinafter, referred to as "Compound class SX239").

A present compound (L-1) wherein $R^{1b}$ represents a cyclopropyl group, $R^{1c}$ represents a hydrogen atom, $A^2$ represents C-Me, $A^1$, $A^3$, and $A^5$ represent CH, and $A^4$ represents any one substituent indicated in [Table L1] to [Table L3] (hereinafter, referred to as "Compound class. SX240").

A present compound (L-1) wherein $R^{1b}$ represents a cyclopropyl group, $R^{1c}$ represents a hydrogen atom, $A^2$ represents C-OMe, $A^1$, $A^3$, and $A^5$ represent CH, and $A^4$ represents any one substituent indicated in [Table L1] to [Table

L3] (hereinafter, referred to as "Compound class SX241").

A present compound (L-1) wherein $R^{1b}$ represents a cyclopropyl group, $R^{1c}$ represents a hydrogen atom, $A^2$ represents $C-CF_3$, $A^1$, $A^3$, and $A^5$ represent. CH, and $A^4$ represents any one substituent indicated in [Table L1] to [Table 13] (hereinafter, referred to as "Compound class SX242").

A present compound (L-1) wherein $R^{1b}$ represents a cyclopropyl group, $R^{1c}$ represents a hydrogen atom, $A^2$ represents $C-OCF_3$, $A^1$, $A^3$, and $A^5$ represent CH, and $A^4$ represents any one substituent indicated in [Table L1] to [Table L3] (hereinafter, referred to as "Compound class SX243").

A present compound (L-1) wherein $R^{1b}$ represents a cyclopropyl group, $R^{1c}$ represents a hydrogen atom, $A^3$ represents C-F, $A^1$, $A^2$, and $A^5$ represent CH, and $A^4$ represents any one substituent indicated in [Table L1] to [Table L3] (hereinafter, referred to as "Compound class SX244"),

A present compound (L-1) wherein $R^{1b}$ represents a cyclopropyl group, $R^{1c}$ represents a hydrogen atom, $A^3$ represents C-Cl, $A^1$, $A^2$, and $A^5$ represent CH, and $A^4$ represents any one substituent indicated in [Table L1] to [Table L3] (hereinafter, referred to as "Compound class SX245").

A present compound (L-1) wherein $R^{1b}$ represents a cyclopropyl group, $R^{1c}$ represents a hydrogen atom, $A^3$ represents C-Br, $A^1$, $A^2$, and $A^5$ represent CH, and $A^4$ represents any one substituent indicated in [Table L1] to [Table L3] (hereinafter, referred to as "Compound class SX246").

A present compound (L-1) wherein $R^{1b}$ represents a cyclopropyl group, $R^{1c}$ represents a hydrogen atom, $A^3$ represents C-I, $A^1$, $A^2$, and $A^5$ represent CH, and $A^4$ represents any one substituent indicated in [Table L1] to [Table L3] (hereinafter, referred to as "Compound class SX247").

A present compound (L-1) wherein $R^{1b}$ represents a cyclopropyl group, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter, referred to as "Compound class SX248") .

A present compound (L-2) wherein $R^{1b}$ represents a cyclopropyl group, $R^{1c}$ represents a hydrogen atom, $A^3$ represents C-OMe, $A^1$, $A^2$, and $A^5$ represent CH, and $A^4$ represents any one substituent indicated in [Table L1] to [Table L3] (hereinafter, referred to as "Compound class SX249").

A present compound (L-1) wherein $R^{1b}$ represents a cyclopropyl group, $R^{1c}$ represents a hydrogen atom, $A^3$ represents $C-CF_3$, $A^1$, $A^2$, and $A^5$ represent CH, and $A^4$ represents any one substituent indicated in [Table L1] to [Table L3] (hereinafter, referred to as "Compound class SX250").

A present compound (L-1) wherein $R^{1b}$ represents a cyclopropyl group, $R^{1c}$ represents a hydrogen atom, $A^3$ represents $C-OCF_3$, $A^1$, $A^2$, and $A^5$ represent CH, and $A^4$ represents any one substituent indicated in [Table L1] to [Table L3] (hereinafter, referred to as "Compound class SX251").

A present compound (L-1) wherein $R^{1b}$ represents a methylsulfanyl group, $R^{1c}$ represents a hydrogen atom, $A^2$ represents C-F, $A^1$, $A^3$, and $A^5$ represent CH, and $A^4$ represents any one substituent indicated in [Table L13 to [Table L3] (hereinafter, referred to as "Compound class SX252").

A present compound (L-1) wherein $R^{1b}$ represents a methylsulfanyl group, $R^{1c}$ represents a hydrogen atom, $A^2$ represents C-C1, $A^1$, $A^3$, and $A^5$ represent CH, and $A^4$ represents any one substituent indicated in [Table L1] to [Table L3] (hereinafter, referred to as "Compound class SX253").

A present compound (L-1) wherein $R^{1b}$ represents a methylsulfanyl group, $R^{1c}$ represents a hydrogen atom, $A^2$ represents C-Br, $A^1$, $A^3$, and $A^5$ represent CH, and $A^4$ represents any one substituent indicated in [Table L1] to [Table L3] (hereinafter, referred to as "Compound class SX254").

A present compound (L-1) wherein $R^{1b}$ represents a methylsulfanyl group, $R^{1c}$ represents a hydrogen atom, $A^2$ represents C-I, $A^1$, $A^3$, and $A^5$ represent CH, and $A^4$ represents any one substituent indicated in [Table L1] to [Table L3] (hereinafter, referred to as "Compound class SX255").

A present compound (L-1) wherein $R^{1b}$ represents a methylsulfanyl group, $R^{1c}$ represents a hydrogen atom, $A^2$ represents C-Me, $A^1$, $A^3$, and $A^5$ represent. CH, and $A^4$ represents any one substituent indicated in [Table L1] to [Table L3] (hereinafter, referred to as "Compound class SX256").

A present compound (L-1) wherein $R^{1b}$ represents a methylsulfanyl group, $R^{1c}$ represents a hydrogen atom, $A^2$ represents C-OMe, $A^1$, $A^3$, and $A^3$ represent CH, and $A^4$ represents any one substituent indicated in [Table L1] to [Table L3] (hereinafter, referred to as "Compound class SX257").

A present compound (L-1) wherein $R^{1b}$ represents a methylsulfanyl group, $R^{1c}$ represents a hydrogen atom, $A^2$ represents $C-CF_3$, $A^1$, $A^3$, and $A^5$ represent CH, and $A^4$ represents any one substituent indicated in [Table L1] to [Table L3] (hereinafter, referred to as "Compound class SX258").

A present compound (L-1) wherein $R^{1b}$ represents a methylsulfanyl group, $R^{1c}$ represents a hydrogen atom, $A^2$ represents $C-OCF_5$, $A^1$, $A^3$, and $A^5$ represent CH, and $A^4$ represents any one substituent indicated in [Table L1] to [Table L3] (hereinafter, referred to as "Compound class SX259").

A present compound (L-1) wherein $R^{1b}$ represents a methylsulfanyl group, $R^{1c}$ represents a hydrogen atom. $A^3$ represents C-F, $A^1$, $A^2$, and $A^5$ represent CH, and $A^4$ represents any one substituent indicated in [Table L1] to [Table L3] (hereinafter, referred to as "Compound class SX260").

A present compound (L-1) wherein $R^{1b}$ represents a methylsulfanyl group, $R^{1c}$ represents a hydrogen atom, $A^3$ represents C-Cl, $A^1$, $A^2$, and $A^5$ represent CH, and $A^4$ represents any one substituent indicated in [Table L1] to [Table L3] (hereinafter, referred to as "Compound class SX261").

A present Compound (L-1) wherein $R^{1b}$ represents a methylsulfanyl group, $R^{1c}$ represents a hydrogen atom, $A^3$ represents C-Br, $A^1$, $A^2$, and $A^5$ represent CH, and $A^4$ represents any one substituent indicated in [Table L1] to [Table L3] (hereinafter, referred to as "Compound class SX262") .

A present compound (L-1) wherein $R^{1b}$ represents a methylsulfanyl group, $R^{1c}$ represents a hydrogen atom. $A^3$ represents C-1, $A^1$, $A^2$, and $A^5$ represent CH, and $A^4$ represents any one substituent indicated in [Table L13 to [Table L3] (hereinafter, referred to as "Compound class SX263").

A present compound (L-1) wherein $R^{1b}$ represents a methylsulfanyl group, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter, referred to as "Compound class SX264").

A present compound (L-1) wherein $R^{1b}$ represents a methylsulfanyl group, $R^{1c}$ represents a hydrogen atom, $A^3$ represents C-OMe, $A^1$, $A^2$, and $A^3$ represent CH, and $A^4$ represents any one substituent indicated in [Table L1] to [Table L3] (hereinafter, referred to as "Compound class SX265").

A present compound (L-1) wherein $R^{1b}$ represents a methylsulfanyl group, $R^{1c}$ represents a hydrogen atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX266").

A present compound (L-1) wherein $R^{1b}$ represents a methylsulfanyl group, $R^{1c}$ represents a hydrogen atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX267").

A present compound (L-1) wherein $R^{1b}$ and $R^{1c}$ represent a fluorine atom, $A^2$ represents C-F, $A^1$, $A^3$, and $A^5$ represent CH, and $A^4$ represents any one substituent indicated in [Table L1] to [Table L3] (hereinafter, referred to as "Compound class SX268").

A present compound (L-1) wherein $R^{1b}$ and $R^{1c}$ represent a fluorine atom, $A^2$ represents C-Cl, $A^1$, $A^3$, and $A^5$ represent CH, and $A^4$ represents any one substituent indicated in [Table L1] to [Table L3] (hereinafter, referred to as "Compound class SX269").

A present compound (L-1) wherein $R^{1b}$ and $R^{1c}$ represent a fluorine atom, $A^2$ represents C-Br, $A^1$, $A^3$, and $A^5$ represent CH, and $A^4$ represents any one substituent indicated in [Table L1] to [Table L3] (hereinafter, referred to as "Compound class SX210").

A present compound (L-1) wherein $R^{1b}$ and $R^{1c}$ represent a fluorine atom, $A^2$ represents C-I, $A^1$, $A^3$, and $A^5$ represent CH, and $A^4$ represents any one substituent indicated in [Table L1) to [Table L3] (hereinafter, referred to as "Compound class SX271").

A present compound (L-1) wherein $R^{1b}$ and $R^{1c}$ represent a fluorine atom, $A^2$ represents C-Me, $A^1$, $A^3$, and $A^5$ represent CH, and $A^4$ represents any one substituent indicated in [Table L1] to [Table L3] (hereinafter, referred to as "Compound class SX272").

A present compound (L-1) wherein $R^{1b}$ and $R^{1c}$ represent a fluorine atom, $A^2$ represents C-OMe, $A^1$, $A^3$, and $A^5$ represent CH, and $A^4$ represents any one substituent indicated in [Table L1] to [Table L3] (hereinafter, referred to as "Compound class SX273").

A present compound (L-1) wherein $R^{1b}$ and $R^{1c}$ represent a fluorine atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX274").

A present compound (L-1) wherein $R^{1b}$ and $R^{1c}$ represent a fluorine atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX275").

A present compound (L-1) wherein $R^{1b}$ and $R^{1c}$ represent a fluorine atom, $A^3$ represents C-F, $A^1$, $A^2$, and $A^5$ represent CH, and $A^4$ represents any one substituent indicated in [Table L1) to [Table L3] (hereinafter, referred to as "Compound class SX276") .

A present compound (L-1) wherein $R^{1b}$ and $R^{1c}$ represent a fluorine atom, $A^3$ represents C-Cl, $A^1$, $A^2$, and $A^5$ represent CH, and $A^4$ represents any one substituent indicated in [Table L1] to [Table L3] (hereinafter, referred to as "Compound class SX277").

A present compound (L-1) wherein $R^{1b}$ and $R^{1c}$ represent a fluorine atom, $A^3$ represents C-Br, $A^1$, $A^2$, and $A^5$ represent CH, and $A^4$ represents any one substituent indicated in [Table L1] to [Table L3] (hereinafter, referred to as "Compound class SX278").

A present compound (L-1) wherein $R^{1b}$ and $R^{1c}$ represent a fluorine atom, $A^3$ represents C-1, $A^1$, $A^2$, and $A^5$ represent CH, and $A^4$ represents any one substituent indicated in [Table L1] to [Table L3] (hereinafter, referred to as "Compound class SX279").

A present compound (L-i) wherein $R^{1b}$ and $R^{1c}$ represent a fluorine atom, $A^3$ represents C-Me, $A^1$, $A^2$, and $A^5$

represent CH, and $A^4$ represents any one substituent indicated in [Table L1] to [Table L3] (hereinafter, referred to as "Compound class SX280").

A present compound (L-1) wherein $R^{1b}$ and $R^{1c}$ represent a fluorine atom, $A^3$ represents C-OMe, $A^1$, $A^2$, and $A^5$ represent CH, and $A^4$ represents any one substituent indicated in [Table L1) to [Table L3] (hereinafter, referred to as "Compound class SX281").

A present compound (L-1) wherein $R^{1b}$ and $R^{1c}$ represent a fluorine atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX282").

A present compound (L-1) wherein $R^{1b}$ and $R^{1c}$ represent a fluorine atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX283").

A present Compound (L-1) wherein $R^{1b}$ and $R^{1c}$ represent a chlorine atom. $A^2$ represents C-F, $A^1$, $A^3$, and $A^5$ represent CH, and $A^4$ represents any one substituent indicated in [Table L1] to [Table L3] (hereinafter, referred to as "Compound class SX284").

A present compound (L-1) wherein $R^{1b}$ and $R^{1c}$ represent a chlorine atom, $A^2$ represents C-Cl, $A^1$, $A^3$, and $A^5$ represent CH, and $A^4$ represents any one substituent indicated in [Table L1] to [Table L3) (hereinafter, referred to as "Compound class SX285").

A present compound (L-1) wherein. $R^{1b}$ and $R^{1c}$ represent a chlorine atom, $A^2$ represents C-Br, $A^1$, $A^3$, and $A^5$ represent CH, and $A^4$ represents any one substituent indicated in [Table L1] to [Table L3] (hereinafter, referred to as "Compound class SX286").

A present compound (L-1) wherein $R^{1b}$ and $R^{1c}$ represent a chlorine atom, $A^2$ represents C-I, $A^1$, $A^3$, and $A^5$ represent CH, and $A^4$ represents any one substituent indicated in [Table L1] to [Table L3] (hereinafter, referred to as "Compound class SX281").

A present compound (L-1) wherein $R^{1b}$ and $R^{1c}$ represent a chlorine atom, $A^2$ represents C-Me, $A^1$, $A^3$, and $A^5$ represent CH, and $A^4$ represents any one substituent indicated in [Table L1] to [Table L3] (hereinafter, referred to as "Compound class SX288").

A present compound (L-1) wherein $R^{1b}$ and $R^{1c}$ represent a chlorine atom, $A^2$ represents C-OMe, $A^1$, $A^3$, and $A^5$ represent CH, and $A^4$ represents any one substituent indicated in [Table L1] to [Table L3] (hereinafter, referred to as "Compound class SX289").

A present compound (L-1) wherein $R^{1b}$ and $R^{1c}$ represent a chlorine atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX29.0").

A present compound (L-1) wherein. $R^{1b}$ and $R^{1c}$ represent a chlorine atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX291").

A present compound (L-1) wherein $R^{1b}$ and $R^{1c}$ represent a chlorine atom, $A^3$ represents C-F, $A^1$, $A^2$, and $A^5$ represent CH, and $A^4$ represents any one substituent indicated in [Table L1] to [Table L3] (hereinafter, referred to as "Compound class SX292").

A present compound (L-1) wherein $R^{1b}$ and $R^{1c}$ represent a chlorine atom, $A^3$ represents C-Cl, $A^1$, $A^2$, and $A^5$ represent CH, and $A^4$ represents any one substituent indicated in [Table L1] to [Table L3] (hereinafter, referred to as "Compound class SX293").

A present compound (L-1) wherein $R^{1b}$ and $R^{1c}$ represent a chlorine atom, $A^3$ represents C-Br, $A^1$, $A^2$, and $A^5$ represent CH, and $A^4$ represents any one substituent indicated in [Table L1) to [Table L3] (hereinafter, referred to as "Compound class SX294").

A present compound (L-1) wherein $R^{1b}$ and $R^{1c}$ represent a chlorine atom, $A^3$ represents C-I, $A^1$, $A^2$, and $A^5$ represent CH, and $A^4$ represents any one substituent indicated in [Table L1] to [Table L3] (hereinafter, referred to as "Compound class SX295").

A present compound (L-1) wherein. $R^{1b}$ and $R^{1c}$ represent a chlorine atom, $A^3$ represents C-Me, $A^1$, $A^2$, and $A^5$ represent CH, and $A^4$ represents any one substituent indicated in [Table L1] to [Table L3] (hereinafter, referred to as "Compound class SX296").

A present compound (L-1) wherein $R^{1b}$ and $R^{1c}$ represent a chlorine atom, $A^3$ represents C-OMe, $A^1$, $A^2$, and $A^5$ represent CH, and $A^4$ represents any one substituent indicated in [Table L1] to [Table L3] (hereinafter, referred to as "Compound class SX297").

A present compound (L-1) wherein $R^{1b}$ and $R^{1c}$ represent a chlorine atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX298").

A present compound (L-1) wherein $R^{1b}$ and $R^{1c}$ represent a chlorine atom, $A^3$ represents C-$OCF_3$, $A^1$, $A^2$, and $A^5$ represent CH, and $A^4$ represents any one substituent indicated in [Table L1] to [Table L3] (hereinafter, referred to as

"Compound class SX299").

A present compound (L-1) wherein $R^{1b}$ and $R^{1c}$ represent a bromine atom, $A^2$ represents C-F, $A^1$, $A^3$, and $A^5$ represent CH, and $A^4$ represents any one substituent indicated in [Table L1] to [Table L3] (hereinafter, referred to as "Compound class SX300").

A present compound (L-1) wherein. $R^{1b}$ and $R^{1c}$ represent a bromine atom, $A^2$ represents C-Cl, $A^1$, $A^3$, and $A^5$ represent CH, and $A^4$ represents any one substituent indicated in [Table L1] to [Table L3] (hereinafter, referred to as "Compound class SX301").

A present compound (L-1) wherein $R^{1b}$ and $R^{1c}$ represent a bromine atom, $A^2$ represents C-Br, $A^1$, $A^3$, and $A^5$ represent CH, and $A^4$ represents any one substituent indicated in [Table L1] to [Table L3] (hereinafter, referred to as "Compound class SX302").

A present compound (L-1) wherein $R^{1b}$ and $R^{1c}$ represent a bromine. atom, $A^2$ represents C-I, $A^1$, $A^3$, and $A^5$ represent CH, and $A^4$ represents any one substituent indicated in [Table L1] to [Table L3] (hereinafter, referred to as "Compound class SX303").

A present compound (L-1) wherein $R^{1b}$ and $R^{1c}$ represent a bromine atom, $A^2$ represents C-Me, $A^1$, $A^3$, and $A^5$ represent CH, and $A^4$ represents any one substituent indicated in [Table L1) to [Table L3] (hereinafter, referred to as "Compound class SX304").

A present compound (L-1) wherein $R^{1b}$ and $R^{1c}$ represent a bromine atom, $A^2$ represents C-OMe, $A^1$, $A^3$, and $A^5$ represent CH, and $A^4$ represents any one substituent indicated in [Table L1] to [Table L3] (hereinafter, referred to as "Compound class SX305").

A present compound (L-1) wherein. $R^{1b}$ and $R^{1c}$ represent a bromine atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class 5X306").

A present compound (L-1) wherein $R^{1b}$ and $R^{1c}$ represent a bromine atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound. class SX301").

A present compound (L-1) wherein $R^{1b}$ and $R^{1c}$ represent a bromine. atom, $A^3$ represents C-F, $A^1$, $A^2$, and $A^5$ represent CH, and $A^4$ represents any one substituent indicated in [Table L1] to [Table L3] (hereinafter, referred to as "Compound class SX308").

A present compound (L-1) wherein $R^{1b}$ and $R^{1c}$ represent a bromine atom, $A^3$ represents C-Cl, $A^1$, $A^2$, and $A^5$ represent CH, and $A^4$ represents any one substituent indicated in [Table L1) to [Table L3] (hereinafter, referred to as "Compound class SX309").

A present compound (L-1) wherein $R^{1b}$ and $R^{1c}$ represent a bromine atom, $A^3$ represents C-Br, $A^1$, $A^2$, and $A^5$ represent CH, and $A^4$ represents any one substituent indicated in [Table L1] to [Table L3] (hereinafter, referred to as "Compound class SX310").

A present compound (L-1) wherein. $R^{1b}$ and $R^{1c}$ represent a bromine atom, $A^3$ represents C-I, $A^1$, $A^2$, and $A^5$ represent CH, and $A^4$ represents any one substituent indicated in [Table L1] to [Table L3] (hereinafter, referred to as "Compound class 5X311").

A present compound (L-1) wherein $R^{1b}$ and $R^{1c}$ represent a bromine atom, $A^3$ represents C-Me, $A^1$, $A^2$, and $A^5$ represent CH, and $A^4$ represents any one substituent indicated in [Table L1] to [Table L3] (hereinafter, referred to as "Compound class SX312").

A present compound (L-1) wherein $R^{1b}$ and $R^{1c}$ represent a bromine. atom, $A^3$ represents C-OMe, $A^1$, $A^2$, and $A^5$ represent CH, and $A^4$ represents any one substituent indicated in [Table L1] to [Table L3] (hereinafter, referred to as "Compound class SX313").

A present compound (L-1) wherein $R^{1b}$ and $R^{1c}$ represent a bromine atom, $A^3$ represents C-$CF_3$, $A^1$, $A^4$, and $A^5$ represent CH, and $A^4$ represents any one substituent indicated in [Table L1] to [Table L3] (hereinafter, referred to as "Compound class SX314").

A present compound (L-1) wherein $R^{1b}$ and $R^{1c}$ represent a bromine atom. $A^3$ represents C-$OCF_3$, $A^1$, $A^2$, and $A^5$ represent CH, and $A^4$ represents any one substituent indicated in [Table L1) to [Table L3] (hereinafter, referred to as "Compound class SX315").

A present compound (L-1) wherein $R^{1b}$ and $R^{1c}$ represent an iodine atom, $A^2$ represents C-F, $A^1$, $A^3$, and $A^5$ represent CH, and $A^4$ represents any one substituent indicated in [Table L1] to [Table L3] (hereinafter, referred to as "Compound class SX316").

A present compound (1-1) wherein $R^{1b}$ and $R^{1c}$ represent an iodine atom, $A^2$ represents C-Cl, $A^1$, $A^3$, and $A^5$ represent CH, and $A^4$ represents any one substituent indicated in [Table L1] to [Table L3] (hereinafter, referred to as "Compound class SX3.17").

A present compound (1-1) wherein $R^{1b}$ and $R^{1c}$ represent an iodine atom, $A^2$ represents C-Br, $A^1$, $A^3$, and $A^5$ represent CH, and $A^4$ represents any one substituent indicated in [Table L1) to [Table L3] (hereinafter, referred to as "Compound class SX318").

A present compound (L-1) wherein $R^{1b}$ and $R^{1c}$ represent an iodine atom, $A^2$ represents C-I, $A^1$, $A^3$, and $A^5$ represent CH, and $A^4$ represents any one substituent indicated in [Table L1] to [Table L3] (hereinafter, referred to as "Compound class SX319").

A present compound (L-1) wherein $R^{1b}$ and $R^{1c}$ represent an iodine. atom, $A^2$ represents C-Me, $A^1$, $A^3$, and $A^5$ represent CH, and $A^4$ represents any one substituent indicated in [Table L1] to [Table L3] (hereinafter, referred to as "Compound class SX326").

A present compound (L-1) wherein $R^{1b}$ and $R^{1c}$ represent an iodine atom, $A^2$ represents C-OMe, $A^1$, $A^3$, and $A^5$ represent CH, and $A^4$ represents any one substituent indicated in [Table L1] to [Table L3] (hereinafter, referred to as "Compound class 3X321").

A present compound (L-1) wherein $R^{1b}$ and $R^{1c}$ represent an iodine atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class 3X322").

A present compound (L-1) wherein $R^{1b}$ and $R^{1c}$ represent an iodine atom, $A^2$ represents C-$OCF_3$, $A^1$, $A^3$, and $A^5$ represent CH, and $A^4$ represents any one substituent indicated in [Table L1) to [Table L3] (hereinafter, referred to as "Compound class SX323").

A present compound (L-1) wherein $R^{1b}$ and $R^{1c}$ represent an iodine atom, $A^3$ represents C-F, $A^1$, $A^2$, and $A^5$ represent CH, and $A^4$ represents any one substituent indicated in [Table L1) to [Table L3] (hereinafter, referred to as "Compound class SX324").

A present compound (L-1) wherein $R^{1b}$ and $R^{1c}$ represent an iodine. atom, $A^3$ represents C-Cl, $A^1$, $A^2$, and $A^5$ represent CH, and $A^4$ represents any one substituent indicated in [Table L1] to [Table L3] (hereinafter, referred to as "Compound class SX325").

A present compound (L-1) wherein $R^{1b}$ and $R^{1c}$ represent an iodine atom, $A^3$ represents C-Br, $A^1$, $A^2$, and $A^5$ represent CH, and $A^4$ represents any one substituent indicated in [Table L1] to [Table L3] (hereinafter, referred to as "Compound class SX326").

A present compound (L-1) wherein $R^{1b}$ and $R^{1c}$ represent an iodine atom, $A^3$ represents C-I, $A^1$, $A^2$, and $A^5$ represent CH, and $A^4$ represents any one substituent indicated in [Table L1] to [Table L3] (hereinafter, referred to as "Compound class SX327").

A present compound (L-1) wherein $R^{1b}$ and $R^{1c}$ represent an iodine atom, $A^3$ represents C-Me, $A^1$, $A^2$, and $A^5$ represent CH, and $A^4$ represents any one substituent indicated in [Table L1) to [Table L3] (hereinafter, referred to as "Compound class SX32S").

A present compound (L-1) wherein $R^{1b}$ and $R^{1c}$ represent an iodine atom, $A^3$ represents C-OMe, $A^1$, $A^2$, and $A^5$ represent CH, and $A^4$ represents any one substituent indicated in [Table L1] to [Table L3] (hereinafter, referred to as "Compound class SX329").

A present compound (L-1) wherein $R^{1b}$ and $R^{1c}$ represent an iodine atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX336").

A present compound (L-1) wherein $R^{1b}$ and $R^{1c}$ represent an iodine atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX331").

A present compound (L-1) wherein $R^{1b}$ and $R^{1c}$ represent a methyl group, $A^2$ represents C-F, $A^1$, $A^3$, and $A^5$ represent CH, and $A^4$ represents any one substituent indicated in [Table L1] to [Table L3] (hereinafter, referred to as "Compound class SX3.32"I.

A present compound (L-1) wherein $R^{1b}$ and $R^{1c}$ represent a methyl group, $A^2$ represents C-Cl, $A^1$, $A^3$, and $A^5$ represent CH, and $A^4$ represents any one substituent indicated in [Table L1) to [Table L3] (hereinafter, referred to as "Compound class SX333").

A present compound (L-1) wherein $R^{1b}$ and $R^{1c}$ represent a methyl group, $A^2$ represents C-Br, $A^1$, $A^3$, and $A^5$ represent CH, and $A^4$ represents any one substituent indicated in [Table L1] to [Table L3] (hereinafter, referred to as "Compound class SX334").

A present compound (L-1) wherein $R^{1b}$ and $R^{1c}$ represent a methyl group, $A^2$ represents C-I, $A^1$, $A^3$, and $A^5$ represent CH, and $A^4$ represents any one substituent indicated in [Table L1] to [Table L3] (hereinafter, referred to as "Compound class SX335") .

A present compound (L-1) wherein $R^{1b}$ and $R^{1c}$ represent a methyl group, $A^2$ represents C-Me, $A^1$, $A^3$, and $A^5$ represent CH, and $A^4$ represents any one substituent indicated in [Table L1] to [Table L3] (hereinafter, referred to as "Compound class SX336").

A present compound (L-1) wherein $R^{1b}$ and $R^{1c}$ represent a methyl group, $A^2$ represents C-OMe, $A^1$, $A^3$, and $A^5$ represent CH, and $A^4$ represents any one substituent indicated in [Table L1] to [Table L3] (hereinafter, referred to as "Compound class SX337").

A present compound (L-1) wherein $R^{1b}$ and $R^{1c}$ represent a methyl group, $A^2$ represents C-$CF_3$, $A^1$, $A^3$, and

$R^5$ represent CH, and $A^4$ represents any one substituent indicated in [Table L1]to [Table L3] (hereinafter, referred to as "Compound class SX338").

A present compound (L-1) wherein $R^{1b}$ and $R^{1c}$ represent a methyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX339").

A present compound (L-1) wherein $R^{1b}$ and $R^{1c}$ represent a methyl group, $A^3$ represents C-F, $A^1$, $A^2$, and $A^5$ represent CH, and $A^4$ represents any one substituent indicated in [Table L1] to [Table L3] (hereinafter, referred to as "Compound class SX340").

A present compound (L-1) wherein $R^{1b}$ and $R^{1c}$ represent a methyl group, $A^3$ represents C-Cl, $A^1$, $A^2$, and $A^5$ represent CH, and $A^4$ represents any one substituent indicated in [Table L1] to [Table L3] (hereinafter, referred to as "Compound class 3X341").

A present compound (L-1) wherein $R^{1b}$ and $R^{1c}$ represent a methyl group, $A^3$ represents C-Br, $A^1$, $A^2$, and $A^5$ represent CH, and $A^4$ represents any one substituent indicated in [Table L1] to [Table L3] (hereinafter, referred to as "Compound class SX3.42").

A present compound (L-1) wherein $R^{1b}$ and $R^{1c}$ represent a methyl group, $A^3$ represents C-I, $A^1$, $A^2$, and $A^5$ represent CH, and $A^4$ represents any one substituent indicated in [Table L1) to [Table L3] (hereinafter, referred to as "Compound class SX343").

A present compound (L-1) wherein $R^{1b}$ and $R^{1c}$ represent a methyl group, $A^3$ represents C-Me, $A^1$, $A^2$, and $A^5$ represent CH, and $A^4$ represents any one substituent indicated in [Table L1] to [Table L3] (hereinafter, referred to as "Compound class SX344").

A present compound (L-1) wherein $R^{1b}$ and $R^{1c}$ represent a methyl group, $A^3$ represents C-OMe, $A^1$, $A^2$, and $A^5$ represent CH, and $A^4$ represents any one substituent indicated in [Table L1] to [Table L3] (hereinafter, referred to as "Compound class SX345").

A present compound (L-1) wherein $R^{1b}$ and $R^{1c}$ represent a methyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX346").

A present compound (L-1) wherein $R^{1b}$ and $R^{1c}$ represent a methyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX347").

A present compound (L-1) wherein $R^{1b}$ and $R^{1c}$ represent an ethyl group, $A^2$. represents C-F, $A^1$, $A^3$, and $A^5$ represent CH, and $A^4$ represents any one substituent indicated in [Table L1] to [Table L3] (hereinafter, referred to as "Compound class SX348").

A present compound (L-1) wherein $R^{1b}$ and $R^{1c}$ represent an ethyl group, $A^2$ represents C-Cl, $A^1$, $A^3$, and $A^5$ represent CH, and $A^4$ represents any one substituent indicated in [Table L1] to [Table L3] (hereinafter, referred to as "Compound class SX349").

A present compound (L-1) wherein $R^{1b}$ and $R^{1c}$ represent an ethyl group, $A^2$ represents. C-Br, $A^1$, $A^3$, and $A^5$ represent CH, and $A^4$ represents any one substituent indicated in [Table L1] to [Table L3] (hereinafter, referred to as "Compound class SX350").

A present compound (L-1) wherein $R^{1b}$ and $R^{1c}$ represent an ethyl group, $A^2$ represents C-I, $A^1$, $A^3$, and $A^5$ represent CH, and $A^4$ represents any one substituent indicated in [Table L1] to [Table L3] (hereinafter, referred to as "Compound class SX351").

A present compound (L-1) wherein $R^{1b}$ and $R^{1c}$ represent an ethyl group, $A^2$ represents C-Me, $A^1$, $A^3$, and $A^5$ represent CH, and $A^4$ represents any one substituent indicated in (Table L1] to [Table L3] (hereinafter, referred to as "Compound class SX352").

A present compound (L-1) wherein $R^{1b}$ and $R^{1c}$ represent an ethyl group, $A^2$ represents C-OMe, $A^1$, $A^3$, and $A^5$ represent CH, and $A^4$ represents any one substituent indicated in [Table L1] to [Table L3] (hereinafter, referred to as "Compound class SX353").

A present compound (L-1) wherein $R^{1b}$ and $R^{1c}$ represent an ethyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX354").

A present compound (L-1) wherein $R^{1b}$ and $R^{1c}$ represent an ethyl group, $A^2$ represents C-$QCF_3$, $A^1$, $A^3$, and $A^5$ represent CH, and $A^4$ represents any one substituent indicated in [Table L1] to [Table L3] (hereinafter, referred to as "Compound class SX355").

A present compound (L-1) wherein $R^{1b}$ and $R^{1c}$ represent an ethyl group, $A^3$ represents C-F, $A^1$, $A^2$, and $A^5$ represent CH, and $A^4$ represents any one substituent indicated in [Table L1] to [Table L3] (hereinafter, referred to as "Compound class SX356").

A present compound (L-1) wherein $R^{1b}$ and $R^{1c}$ represent an ethyl group, $A^3$ represents C-Cl, $A^1$, $A^2$, and $A^5$ represent CH, and $A^4$ represents any one substituent indicated in (Table L1] to [Table L3] (hereinafter, referred to as

"Compound class SX357").

A present compound (L-1) wherein $R^{1c}$ and $R^{1c}$ represent an ethyl group, $A^3$ represents C-Br, $A^1$, $A^2$, and $A^5$ represent CH, and $A^4$ represents any one substituent indicated in [Table L1] to [Table L3] (hereinafter, referred to as "Compound class SX358").

A present compound (L-1) wherein $R^{1b}$ and $R^{1c}$ represent an ethyl group, $A^3$ represents C-I, $A^1$, $A^2$, and $A^5$ represent CH, and $A^4$ represents any one substituent indicated in [Table L1] to [Table L3] (hereinafter, referred to as "Compound class SX359").

A present compound (L-1) wherein $R^{1b}$ and $R^{1c}$ represent an ethyl group, $A^3$ represents. C-Me, $A^1$, $A^2$, and $A^3$ represent CH, and $A^4$ represents any one substituent indicated in [Table L1] to [Table L3] (hereinafter, referred to as "Compound class SX350").

A present compound (L-1) wherein $R^{1b}$ and $R^{1c}$ represent an ethyl group, $A^3$ represents C-OMe, $A^1$, $A^2$, and $A^5$ represent CH, and $A^4$ represents any one substituent indicated in [Table L1] to [Table L3] (hereinafter, referred to as "Compound class SX361").

A present compound (L-1) wherein $R^{1b}$ and $R^{1c}$ represent an ethyl group, $A^3$ represents C-$CF_3$, $A^1$, $A^2$, and $A^5$ represent CH, and $A^4$ represents any one substituent indicated in (Table L1] to [Table L3] (hereinafter, referred to as "Compound class SX362").

A present compound (L-1) wherein $R^{1b}$ and $R^{1c}$ represent an ethyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound Class SX363").

A present compound (L-1) wherein $R^{1b}$ and $R^{1c}$ represent $CF_3$, $A^2$ represents C-F, $A^1$, $A^3$, and $A^5$ represent CH, and $A^4$ represents any one substituent indicated in [Table L1] to [Table L3] (hereinafter, referred to as "Compound class SX364").

A present compound (L-1) wherein $R^{1b}$ and $R^{1c}$ represent $CF_3$, $A^2$ represents C-Cl, $A^1$, $A^3$, and $A^5$ represent CH, and $A^4$ represents any one substituent indicated in [Table L1] to [Table L3] (hereinafter, referred to as "Compound class SX365").

A present compound (L-1) wherein $R^{1b}$ and $R^{1c}$ represent $CF_3$, $A^2$ represents C-Br, $A^1$, $A^3$, and $A^5$ represent CH, and $A^4$ represents any one substituent indicated in [Table L1] to [Table L3] (hereinafter, referred to as "Compound class SX366").

A present compound (L-1) wherein $R^{1b}$ and $R^{1c}$ represent $CF_3$, $A^2$ represents C-I, $A^1$, $A^3$, and $A^5$ represent CH, and $A^4$ represents any one substituent indicated in [Table L1] to [Table L3] (hereinafter, referred to as "Compound class SX367").

A present compound (L-1) wherein $R^{1b}$ and $R^{1c}$ represent $CF_3$, $A^2$ represents C-Me, $A^1$, $A^3$, and $A^5$ represent CH, and $A^4$ represents any one substituent indicated in [Table L1] to [Table L3] (hereinafter, referred to as "Compound class SX368").

A present compound (L-1) wherein $R^{1c}$ and $R^{1c}$ represent $CF_3$, $A^2$ represents C-OMe, $A^1$, $A^3$, and $A^5$ represent CH, and $A^4$ represents any one substituent indicated in [Table L1] to [Table L3] (hereinafter, referred to as "Compound class SX369").

A present compound (L-1) wherein $R^{1b}$ and $R^{1c}$ represent $CF_3$, $A^2$ represents C-$GF_3$, $A^1$, $A^3$, and $A^5$ represent CH, and $A^4$ represents any one substituent indicated in [Table L1] to (Table L3) (hereinafter, referred to as "Compound class SX370").

A present compound (L-1) wherein $R^{1b}$ and $R^{1c}$ represent $CF_3$, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX371").

A present compound (L-1) wherein $R^{1b}$ and $R^{1c}$ represent $CF_3$, $A^3$ represents C-F, $A^1$, $A^2$, and $A^3$ represent CH, and $A^4$ represents any one substituent indicated in [Table L1] to [Table L3] (hereinafter, referred to as "Compound class SX372").

A present compound (L-1) wherein $R^{1b}$ and $R^{1c}$ represent $CF_3$, $A^3$ represents C-Cl, $A^1$, $A^2$, and $A^5$ represent CH, and $A^4$ represents any one substituent indicated in [Table L1] to [Table L3] (hereinafter, referred to as "Compound class SX373").

A present compound (L-1) wherein $R^{1b}$ and $R^{1c}$ represent $CF_3$, $A^3$ represents C-Br, $A^1$, $A^2$, and $A^5$ represent CH, and $A^4$ represents any one substituent indicated in [Table L1] to [Table L3] (hereinafter, referred to as "Compound class 3X37-4").

A present compound (L-1) wherein $R^{1b}$ and $R^{1c}$ represent $CF_3$, $A^3$ represents C-I, $A^1$, $A^2$, and $A^5$ represent CH, and $A^4$ represents any one substituent indicated in [Table L1] to [Table L3] (hereinafter, referred to as "Compound class SX375").

A present compound (L-1) wherein $R^{1b}$ and $R^{1c}$ represent $CF_3$, $A^3$ represents C-Me, $A^1$, $A^2$, and $A^5$ represent CH, and $A^4$ represents any one substituent indicated in [Table L1] to [Table L3] (hereinafter, referred to as "Compound class SX316").

A present compound (L-1) wherein $R^{1b}$ and $R^{1c}$ represent $CF_3$, $A^3$ represents C-OMe, $A^1$, $A^2$, and $A^5$ represent CH, and $A^4$ represents any one substituent indicated in [Table L1] to [Table L3] (hereinafter, referred to as "Compound class SX377").

A present compound (L-1) wherein $R^{1b}$ and $R^{1c}$ represent $CF_3$, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX378").

A present compound (L-1) wherein $R^{1b}$ and $R^{1c}$ represent $CF_3$, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX379").

A present compound (L-1) wherein $R^{1b}$ and $R^{1c}$ represent a cyclopropyl group, $A^2$ represents C-F, $A^{31}$, $A^3$, and $A^5$ represent CH, and $A^4$ represents any one substituent indicated in [Table L1] to [Table L3] (hereinafter, referred to as "Compound class SX380").

A present compound (L-1) wherein $R^{1b}$ and $R^{1c}$ represent a cyclopropyl group, $A^2$ represents C-Cl, $A^1$, $A^3$, and $A^5$ represent CH, and $A^4$ represents any one substituent indicated in [Table L1] to [Table L3] (hereinafter, referred to as "Compound class SX381").

A present compound (L-1) wherein $R^{1b}$ and $R^{1c}$ represent a cyclopropyl group, $A^2$ represents C-Br, $A^1$, $A^3$, and $A^5$ represent CH, and $A^4$ represents any one substituent indicated in [Table L1] to [Table L3] (hereinafter, referred to as "Compound class SX382").

A present compound (L-1) wherein $R^{1b}$ and $R^{1c}$ represent a cyclopropyl group. $A^2$ represents C-I, $A^1$, $A^3$, and $A^5$ represent CH, and $A^4$ represents any one substituent indicated in [Table L1] to [Table L3] (hereinafter, referred to as "Compound class SX383").

A present compound (L-1) wherein $R^{1b}$ and $R^{1c}$ represent a cyclopropyl group, $A^2$ represents C-Me, $A^1$, $A^3$, and $A^5$ represent CH, and $A^4$ represents any one substituent indicated in [Table L1] to [Table L3] (hereinafter, referred to as "Compound class SX384").

A present compound (L-1) wherein $R^{1b}$ and $R^{1c}$ represent a cyclopropyl group, $A^2$ represents C-OMe, $A^1$, $A^3$, and $A^5$ represent CH, and $A^4$ represents any one substituent indicated in [Table L1] to [Table L3] (hereinafter, referred to as "Compound class SX305").

A present compound (L-1) wherein $R^{1b}$ and $R^{1c}$ represent a cyclopropyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX386").

A present compound (L-1) wherein $R^{1b}$ and $R^{1c}$ represent a cyclopropyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX387").

A present compound (L-1) wherein $R^{1b}$ and $R^{1c}$ represent a cyclopropyl group, $A^3$ represents C-F, $A^1$, $A^2$, and $A^5$ represent CH, and $A^4$ represents any one substituent indicated in [Table L1] to [Table L3] (hereinafter, referred to as "Compound class 3X388").

A present compound (L-1) wherein $R^{1b}$ and $R^{1c}$ represent a cyclopropyl group, $A^3$ represents C-Cl, $A^1$, $A^2$, and $A^5$ represent CH, and $A^4$ represents any one substituent indicated in [Table L1] to [Table L3] (hereinafter, referred to as "Compound class SX389").

A present compound (L-1) wherein $R^{1b}$ and $R^{1c}$ represent a cyclopropyl group, $A^3$ represents C-Br, $A^1$, $A^2$, and $A^5$ represent CH, and $A^4$ represents any one substituent indicated in [Table L1] to [Table L3] (hereinafter, referred to as "Compound class SX390").

A present compound (L-1) wherein $R^{1b}$ and $R^{1c}$ represent a cyclopropyl group, $A^3$ represents C-I, $A^1$, $A^2$, and $A^5$ represent CH, and $A^4$ represents any one substituent indicated in [Table L1] to [Table L3] (hereinafter, referred to as "Compound class SX391").

A present compound (L-1) wherein $R^{1b}$ and $R^{1c}$ represent a cyclopropyl group, $A^3$ represents C-Me, $A^1$, $A^2$, and $A^5$ represent CH, and $A^4$ represents any one substituent indicated in [Table L1] to [Table L3] (hereinafter, referred to as "Compound class SX392").

A present compound (L-1) wherein $R^{1b}$ and $R^{1c}$ represent a cyclopropyl group, $A^3$ represents C-OMe, $A^1$, $A^2$, and $A^5$ represent CH, and $A^4$ represents any one substituent indicated in [Table L1] to [Table L3] (hereinafter, referred to as "Compound class SX393").

A present compound (L-1) wherein $R^{1b}$ and $R^{1c}$ represent a cyclopropyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX394").

A present compound (L-1) wherein $R^{1b}$ and $R^{1c}$ represent a cyclopropyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX395").

A present compound (L-1) wherein $R^{1b}$ represents a fluorine atom, $R^{1c}$ represents a methyl group, $A^2$ represents

C-F, $A^1$, $A^3$, and $A^5$ represent CH, and $A^4$ represents any one substituent indicated in [Table L1] to [Table L3] (hereinafter, referred to as "Compound class SX396").

A present compound (L-1) wherein $R^{1b}$ represents a fluorine atom, $R^{1c}$ represents a methyl group, $A^2$ represents C-Cl, $A^1$, $A^3$, and $A^5$ represent CH, and $A^4$ represents any one substituent indicated in [Table L1] to [Table L3] (hereinafter, referred to as "Compound class SX397").

A present compound (L-1) wherein $R^{1b}$ represents a fluorine atom, $R^{1c}$ represents a methyl group, $A^2$ represents C-Br, $A^1$, $A^3$, and $A^5$ represent CH, and $A^4$ represents any one substituent indicated in [Table L1] to [Table L3] (hereinafter, referred to as "Compound class SX398").

A present compound (L-1) wherein $R^{1b}$ represents a fluorine atom, $R^{1c}$ represents a methyl group, $A^2$ represents C-I, $A^1$, $A^3$, and $A^5$ represent CH, and $A^4$ represents any one substituent indicated in [Table L1] to [Table L3] (hereinafter, referred to as "Compound class SX399").

A present compound (L-2) wherein $R^{1b}$ represents a fluorine atom, $R^{1c}$ represents a methyl group, $A^2$ represents C-Me, $A^1$, $A^3$, and $A^5$ represent CH, and $A^4$ represents any one substituent indicated in [Table L1] to [Table L3] (hereinafter, referred to as "Compound class SX400").

A present compound (L-1) wherein $R^{1b}$ represents a fluorine atom, $R^{1c}$ represents a methyl group, $A^2$ represents C-OMe, $A^1$, $A^3$, and $A^5$ represent CH, and $A^4$ represents any one substituent indicated in [Table L1] to [Table L3] (hereinafter, referred to as "Compound class SX401").

A present compound (L-1) wherein $R^{1b}$ represents a fluorine atom, $R^{1c}$ represents a methyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX402").

A present compound (L-1) wherein $R^{1b}$ represents a fluorine atom, $R^{1c}$ represents a methyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX403").

A present compound (L-1) wherein $R^{1b}$ represents a fluorine atom, $R^{1c}$ represents a methyl group, $A^3$ represents C-F, $A^1$, $A^2$, and $A^5$ represent CH, and $A^4$ represents any one substituent indicated in [Table L1] to [Table L3] (hereinafter, referred to as "Compound class SX404").

A present compound (L-2) wherein $R^{1b}$ represents a fluorine atom, $R^{1c}$ represents a methyl group, $A^3$ represents C-Cl, $A^1$, $A^2$, and $A^5$ represent CH, and $A^4$ represents any one substituent indicated in [Table L1] to [Table L3] (hereinafter, referred to as "Compound class SX405").

A present compound (L-1) wherein $R^{1b}$ represents a fluorine atom, $R^{1c}$ represents a methyl group, $A^3$ represents C-Br, $A^1$, $A^2$, and $A^5$ represent CH, and $A^4$ represents any one substituent indicated in [Table L1] to [Table L3] (hereinafter, referred to as "Compound class SX406").

A present compound (L-1) wherein $R^{1c}$ represents a fluorine atom, $R^{1c}$ represents a methyl group, $A^3$ represents C-1, $A^1$, $A^2$, and $A^5$ represent CH, and $A^4$ represents any one substituent indicated in [Table L1] to [Table L3] (hereinafter, referred to as "Compound class SX407").

A present compound (L-1) wherein $R^{1b}$ represents a fluorine atom, $R^{1c}$ represents a methyl group, $A^3$ represents C-Me, $A^1$, $A^2$, and $A^5$ represent CH, and $A^4$ represents any one substituent indicated in [Table L1] to [Table L3] (hereinafter, referred to as "Compound class SX408").

A present compound (L-1) wherein $R^{1b}$ represents a fluorine atom, $R^{1c}$ represents a methyl group, $A^3$ represents C-OMe, $A^1$, $A^2$, and $A^5$ represent CH, and $A^4$ represents any one substituent indicated in [Table L1] to [Table L3] (hereinafter, referred to as "Compound class SX409").

A present compound (L-2) wherein $R^{1b}$ represents a fluorine atom, $R^{1c}$ represents a methyl group, $A^3$ represents C-$CF_3$ $A^1$, $A^2$, and $A^5$ represent CH, and $A^4$ represents any one substituent indicated in [Table L1] to [Table L3] (hereinafter, referred to as "Compound class SX410").

A present compound (L-1) wherein $R^{1b}$ represents a fluorine atom, $R^{1c}$ represents a methyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX411").

A present compound (L-1) wherein $R^{1b}$ represents a chlorine atom, $R^{1c}$ represents a methyl group, $A^2$ represents C-F, $A^1$, $A^3$, and $A^5$ represent CH, and $A^4$ represents any one substituent indicated in [Table L1] to [Table L3] (hereinafter, referred to as "Compound class SX412").

A present compound (L-1) wherein $R^{1b}$ represents a chlorine atom, $R^{1c}$ represents a methyl group, $A^2$ represents C-Cl, $A^1$, $A^3$, and $A^5$ represent CH, and $A^4$ represents any one substituent indicated in [Table L1] to [Table L3] (hereinafter, referred to as "Compound class SX413").

A present compound (L-1) wherein $R^{1b}$ represents a chlorine atom, $R^{1c}$ represents a methyl group, $A^2$ represents C-Br, $A^1$, $A^3$, and $A^5$ represent CH, and $A^4$ represents any one substituent indicated in [Table L1] to [Table L3] (hereinafter, referred to as "Compound class SX414").

A present Compound (L-1) wherein $R^{1b}$ represents a chlorine atom, $R^{1c}$ represents a methyl group, $A^2$ represents C-I, $A^1$, $A^3$, and $A^5$ represent CH, and $A^4$ represents any one substituent indicated in [Table L1] to [Table L3] (hereinafter,

referred to as "Compound class SX415").

A present compound (L-1) wherein $R^{1b}$ represents a chlorine atom, $R^{1c}$ represents a methyl group, $A^2$ represents C-Me, $A^1$, $A^3$, and $A^5$ represent CH, and $A^4$ represents any one substituent indicated in [Table L1] to [Table. L3] (hereinafter, referred to as "Compound class SX416").

A present compound (L-1) wherein $R^{1b}$ represents a chlorine atom, $R^{1c}$ represents a methyl group, $A^2$ represents C-OMe, $A^1$, $A^3$, and $A^5$ represent CH, and $A^4$ represents any one substituent indicated in [Table L1] to [Table L3] (hereinafter, referred to as "Compound class SX417").

A present compound (L-1) wherein $R^{1b}$ represents a chlorine atom, $R^{1c}$ represents a methyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX418").

A present compound (L-1) wherein $R^{1b}$ represents a chlorine atom, $R^{1c}$ represents a methyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX419").

A present compound (L-1) wherein $R^{1b}$ represents a chlorine atom, $R^{1c}$ represents a methyl group, $A^3$ represents C-F, $A^1$, $A^2$, and $A^5$ represent CH, and $A^4$ represents any one substituent indicated in [Table L1] to [Table L3] (hereinafter, referred to as "Compound class SX420").

A present compound (L-1) wherein $R^{1b}$ represents a chlorine atom, $R^{1c}$ represents a methyl group, $A^3$ represents C-Cl, $A^1$, $A^2$, and $A^5$ represent CH, and $A^4$ represents any one substituent indicated in [Table L1] to [Table. L3] (hereinafter, referred to as "Compound class SX421").

A present compound (L-1) wherein $R^{1b}$ represents a chlorine atom, $R^{1c}$ represents a methyl group, $A^3$ represents C-Br, $A^1$, $A^2$, and $A^5$ represent CH, and $A^4$ represents any one substituent indicated in [Table L1] to [Table L3] (hereinafter, referred to as "Compound class SX422").

A present compound (L-1) wherein $R^{1b}$ represents a chlorine atom, $R^{1c}$ represents a methyl group. $A^3$ represents C-I, $A^1$, $A^2$, and $A^5$ represent CH, and $A^4$ represents any one substituent indicated in [Table L1] to [Table L3] (hereinafter, referred to as "Compound class SX423").

A present compound (L-1) wherein $R^{1b}$ represents a chlorine atom, $R^{1c}$ represents a methyl group, $A^3$ represents C-Me, $A^1$, $A^2$, and $A^5$ represent CH, and $A^4$ represents any one substituent indicated in [Table L1] to [Table L3] (hereinafter, referred to as "Compound class SX424").

A present compound (L-1) wherein $R^{1b}$ represents a chlorine atom, $R^{1c}$ represents a methyl group, $A^3$ represents C-OMe, $A^1$, $A^2$, and $A^5$ represent CH, and $A^4$ represents any one substituent indicated in [Table L1] to [Table L3] (hereinafter, referred to as "Compound class SX425").

A present compound (L-1) wherein $R^{1b}$ represents a chlorine atom, $R^{1c}$ represents a methyl group, $A^3$ represents C-$CF_3$, $A^1$, $A^2$, and $A^3$ represent CH, and $A^4$ represents any one substituent indicated in [Table L1] to [Table. L3] (hereinafter, referred to as "Compound class SX426").

A present compound (L-1) wherein $R^{1b}$ represents a chlorine atom, $R^{1c}$ represents a methyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX427").

A present compound (L-1) wherein $R^{1b}$ represents a bromine atom, $R^{1c}$ represents a methyl group, $A^2$ represents C-F, $A^1$, $A^3$, and $A^5$ represent CH, and $A^4$ represents any one substituent indicated in [Table L1] to [Table L3] (hereinafter, referred to as "Compound class SX428").

A present compound (L-1) wherein $R^{1b}$ represents a bromine atom, $R^{1c}$ represents a methyl group, $A^2$ represents C-Cl, $A^1$, $A^3$, and $A^5$ represent CH, and $A^4$ represents any one substituent indicated in [Table L1] to [Table L3] (hereinafter, referred to as "Compound class SX429").

A present compound (L-1) wherein $R^{1b}$ represents a bromine atom, $R^{1c}$ represents a methyl group, $A^2$ represents C-Br, $A^1$, $A^3$, and $A^5$ represent CH, and $A^4$ represents any one substituent indicated in [Table L1] to [Table L3] (hereinafter, referred to as "Compound class SX430").

A present compound (L-1) wherein $R^{1b}$ represents a bromine atom, $R^{1c}$ represents a methyl group, $A^2$ represents C-I, $A^1$, $A^3$, and $A^5$ represent CH, and $A^4$ represents any one substituent indicated in [Table L1] to [Table L3] (hereinafter, referred to as "Compound class SX431").

A present compound (L-1) wherein $R^{1b}$ represents a bromine atom, $R^{1c}$ represents a methyl group, $A^2$ represents C-Me, $A^1$, $A^3$, and $A^5$ represent CH, and $A^4$ represents any one substituent indicated in [Table L1] to [Table L3] (hereinafter, referred to as "Compound class SX432").

A present compound (L-1) wherein $R^{1b}$ represents a bromine atom, $R^{1c}$ represents a methyl group, $A^2$ represents C-OMe, $A^1$, $A^3$, and $A^5$ represent CH, and $A^4$ represents any one substituent indicated in [Table L1] to [Table L3] (hereinafter, referred to as "Compound class SX433").

A present compound (L-1) wherein $R^{1b}$ represents a bromine atom, $R^{1c}$ represents a methyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX434").

A present compound (L-1) wherein $R^{1b}$ represents a bromine atom, $R^{1c}$ represents a methyl group, $A^2$ represents C-$OCF_3$, $A^1$, $A^3$, and $A^5$ represent CH, and $A^4$ represents any one substituent indicated in (Table L1] to [Table L3] (hereinafter, referred to as "Compound class SX435").

A present compound (L-1) wherein $R^{1b}$ represents a bromine atom, $R^{1c}$ represents a methyl group, $A^3$ represents C-F, $A^1$, $A^2$, and $A^5$ represent CH, and $A^4$ represents any one substituent indicated in [Table L1] to [Table. L3] (hereinafter, referred to as "Compound class SX436").

A present compound (L-1) wherein $R^{1b}$ represents a bromine atom, $R^{1c}$ represents a methyl group, $A^3$ represents C-Cl, $A^1$, $A^2$, and $A^5$ represent CH, and $A^4$ represents any one substituent indicated in [Table L1] to [Table L3] (hereinafter, referred to as "Compound class SX437").

A present compound (L-1) wherein $R^{1b}$ represents a bromine atom, $R^{1c}$ represents a methyl group, $A^3$ represents C-Br, $A^1$, $A^2$, and $A^5$ represent CH, and $A^4$ represents any one substituent indicated in [Table L1] to [Table L3] (hereinafter, referred to as "Compound class SX438").

A present compound (L-1) wherein $R^{1b}$ represents a bromine atom, $R^{1c}$ represents a methyl group, $A^3$ represents C-I, $A^1$, $A^2$, and $A^5$ represent CH, and $A^4$ represents any one substituent indicated in [Table L1] to [Table L3] (hereinafter, referred to as "Compound class SX439").

A present compound (L-1) wherein $R^{1b}$ represents a bromine atom, $R^{1c}$ represents a methyl group, $A^3$ represents C-Me, $A^1$, $A^2$, and $A^5$ represent CH, and $A^4$ represents any one substituent indicated in [Table L1] to [Table L3] (hereinafter, referred to as "Compound class SX440").

A present compound (L-1) wherein $R^{1b}$ represents a bromine atom, $R^{1c}$ represents a methyl group, $A^3$ represents C-OMe, $A^1$, $A^2$, and $A^5$ represent CH, and $A^4$ represents any one substituent indicated in [Table L1] to [Table L3] (hereinafter, referred to as "Compound class SX441").

A present compound (L-1) wherein $R^{1b}$ represents a bromine atom, $R^{1c}$ represents a methyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX442").

A present compound (L-1) wherein $R^{1b}$ represents a bromine atom, $R^{1c}$ represents a methyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX443").

A present compound (L-1) wherein $R^{1b}$ represents an iodine atom, $R^{1c}$ represents a methyl group, $A^2$ represents C-**F,** $A^1$, $A^3$, and $A^5$ represent CH, and $A^4$ represents any one substituent indicated in [Table L1] to [Table L3] (hereinafter, referred to as "Compound class SX444").

A present compound (L-1) wherein $R^{1b}$ represents an iodine atom, $R^{1c}$ represents a methyl group, $A^2$ represents C-Cl, $A^1$, $A^3$, and $A^5$ represent CH, and $A^4$ represents any one substituent indicated in [Table L1] to [Table L3] (hereinafter, referred to as "Compound class SX445").

A present compound (L-1) wherein $R^{1b}$ represents an iodine atom, $R^{1c}$ represents a methyl group. $A^2$ represents C-Br, $A^1$, $A^3$, and $A^5$ represent CH, and $A^4$ represents any one substituent indicated in [Table L1] to [Table L3] (hereinafter, referred to as "Compound class SX446").

A present compound (L-1) wherein $R^{1b}$ represents an iodine atom, $R^{1c}$ represents a methyl group, $A^2$ represents C-I, $A^1$, $A^3$, and $A^5$ represent CH, and $A^4$ represents any one substituent indicated in [Table L1] to [Table L3] (hereinafter, referred to as "Compound class SX447").

A present compound (L-1) wherein $R^{1b}$ represents an iodine atom, $R^{1c}$ represents a methyl group, $A^2$ represents C-Me, $A^1$, $A^3$, and $A^5$ represent CH, and $A^4$ represents any one substituent indicated in [Table L1] to [Table L3] (hereinafter, referred to as "Compound class SX448").

A present compound (L-1) wherein $R^{1b}$ represents an iodine atom, $R^{1c}$ represents a methyl group, $A^2$ represents C-OMe, $A^1$, $A^3$, and $A^5$. represent CH, and $A^4$ represents any one substituent indicated in [Table L1] to [Table L3] (hereinafter, referred to as "Compound class SX449").

A present compound (L-1) wherein $R^{1b}$ represents an iodine atom, $R^{1c}$ represents a methyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX450").

A present compound (L-1) wherein $R^{1b}$ represents an iodine atom, $R^{1c}$ represents a methyl group. $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX451").

A present compound (L-1) wherein $R^{1b}$ represents an iodine atom, $R^{1c}$ represents a methyl group, $A^3$ represents C-F, $A^1$, $A^2$, and $A^5$ represent CH, and $A^4$ represents any one substituent indicated in [Table L1] to [Table L3] (hereinafter, referred to as "Compound class SX452").

A present compound (L-1) wherein $R^{1b}$ represents an iodine atom, $R^{1c}$ represents a methyl group, $A^3$ represents C-Cl, $A^1$, $A^2$, and $A^5$ represent CH, and $A^4$ represents any one substituent indicated in [Table L1] to [Table L3] (hereinafter, referred to as "Compound class SX453").

A present compound (L-1) wherein $R^{1b}$ represents an iodine atom, $R^{1c}$ represents a methyl group, $A^3$ represents

C-Br, $A^1$, $A^2$, and $A^5$ represent CH, and $A^4$ represents any one substituent indicated in [Table L1] to [Table L3] (hereinafter, referred to as "Compound class SX454").

A present compound (L-1) wherein $R^{1b}$ represents an iodine atom, $R^{1c}$ represents a methyl group, $A^3$ represents C-I, $A^1$, $A^2$, and $A^5$ represent CH, and $A^4$ represents any one substituent indicated in [Table L1] to [Table L3] (hereinafter, referred to as "Compound class SX455").

A present compound (L-1) wherein $R^{1b}$ represents an iodine atom, $R^{1c}$ represents a methyl group. $A^3$ represents C-Me, $A^1$, $A^2$, and $A^5$ represent CH, and $A^4$ represents any one substituent indicated in [Table L1] to [Table L3] (hereinafter, referred to as "Compound class SX456").

A present compound (L-1) wherein $R^{1b}$ represents an iodine atom, $R^{1c}$ represents a methyl group, $A^3$ represents C-OMe, $A^1$, $A^2$, and $A^5$ represent CH, and $A^4$ represents any one substituent indicated in [Table L1] to [Table L3] (hereinafter, referred to as "Compound class SX457").

A present compound (L-1) wherein $R^{1b}$ represents an iodine atom, $R^{1c}$ represents a methyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX458").

A present compound (L-1) wherein $R^{1b}$ represents an iodine atom, $R^{1c}$ represents a methyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX459").

A present compound (L-1) wherein $R^{1b}$ represents an iodine atom, $R^{1c}$ represents an ethyl group, $A^2$ represents C-F, $A^1$, $A^3$, and $A^5$ represent CH, and $A^4$ represents any one substituent indicated in [Table L1] to [Table L3] (hereinafter, referred to as "Compound class SX460").

A present compound (L-1) wherein $R^{1b}$ represents a fluorine atom, $R^{1c}$ represents an ethyl group, $A^2$ represents C-Cl, $A^1$, $A^3$, and $A^5$ represent CH, and $A^4$ represents any one substituent indicated in [Table L1] to [Table L3] (hereinafter, referred to as "Compound class SX461").

A present compound (L-1) wherein $R^{1b}$ represents a fluorine atom, $R^{1c}$ represents an ethyl group, $A^2$ represents C-Br, $A^1$, $A^3$, and $A^5$ represent CH, and $A^4$ represents any one substituent indicated in [Table L1] to [Table L3] (hereinafter, referred to as "Compound class SX462").

A present compound (L-1) wherein $R^{1b}$ represents a fluorine atom, $R^{1c}$ represents an ethyl group, $A^2$ represents C-I, $A^1$, $A^3$, and $A^5$ represent CH, and $A^4$ represents any one substituent indicated in [Table L1] to [Table L3] (hereinafter, referred to as "Compound class SX463").

A present compound (L-1) wherein $R^{1b}$ represents a fluorine atom, $R^{1c}$ represents an ethyl group, $A^2$ represents C-Me, $A^1$, $A^3$, and $A^5$ represent CH, and $A^4$ represents any one substituent indicated in [Table L1] to [Table L3] (hereinafter, referred to as "Compound class SX464").

A present compound (L-1) wherein $R^{1b}$ represents a fluorine atom, $R^{1c}$ represents an ethyl group, $A^2$ represents C-OMe, $A^1$, $A^3$, and $A^5$ represent CH, and $A^4$ represents any one substituent indicated in [Table L1] to [Table L3] (hereinafter, referred to as "Compound class SX465").

A present compound (L-1) wherein $R^{1b}$ represents a fluorine atom, $R^{1c}$ represents an ethyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX466").

A present compound (L-1) wherein $R^{1b}$ represents a fluorine atom, $R^{1c}$ represents an ethyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX467").

A present compound (L-1) wherein $R^{1b}$ represents a fluorine atom, $R^{1c}$ represents an ethyl group, $A^3$ represents C-F, $A^1$, $A^2$, and $A^5$ represent CH, and $A^4$ represents any one substituent indicated in [Table L1] to [Table L3] (hereinafter, referred to as "Compound class SX468").

A present compound (L-1) wherein $R^{1b}$ represents a fluorine atom, $R^{1c}$ represents an ethyl group. $A^3$ represents C-Cl, $A^1$, $A^2$, and $A^5$ represent CH, and $A^4$ represents any one substituent indicated in [Table L1] to [Table L3] (hereinafter, referred to as "Compound class SX469").

A present compound (L-1) wherein $R^{1b}$ represents a fluorine atom, $R^{1c}$ represents an ethyl group, $A^3$ represents C-Br, $A^1$, $A^2$, and $A^5$ represent CH, and $A^4$ represents any one substituent indicated in [Table L1] to [Table L3] (hereinafter, referred to as "Compound class SX410").

A present compound (L-1) wherein $R^{1b}$ represents a fluorine atom, $R^{1c}$ represents an ethyl group, $A^3$ represents C-I, $A^1$, $A^2$, and $A^5$ represent CH, and $A^4$ represents any one substituent indicated in [Table L1] to [Table L3] (hereinafter, referred to as "Compound class SX471").

A present compound (L-1) wherein $R^{1b}$ represents a fluorine atom, $R^{1c}$ represents an ethyl group, $A^3$ represents C-Me, $A^1$, $A^2$, and $A^5$ represent CH, and $A^4$ represents any one substituent indicated in [Table L1] to [Table L3] (hereinafter, referred to as "Compound class SX472").

A present compound (L-1) wherein $R^{1b}$ represents a fluorine atom, $R^{1c}$ represents an ethyl group, $A^3$ represents C-OMe, $A^1$, $A^2$, and $A^5$ represent CH, and $A^4$ represents any one substituent indicated in [Table L1] to [Table L3]

(hereinafter, referred to as "Compound class SX473").

A present compound (L-1) wherein $R^{1b}$ represents a fluorine atom, $R^{1c}$ represents an ethyl group. $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX474").

A present compound (L-1) wherein $R^{1b}$ represents a fluorine atom, $R^{1c}$ represents an ethyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX475").

A present compound (L-1) wherein $R^{1b}$ represents a chlorine atom, $R^{1c}$ represents an ethyl group, $A^2$ represents C-F, $A^1$, $A^3$, and $A^5$ represent CH, and $A^4$ represents any one substituent indicated in [Table L1] to [Table L3] (hereinafter, referred to as "Compound class SX476").

A present compound (L-2) wherein $R^{1b}$ represents a chlorine atom, $R^{1c}$ represents an ethyl group, $A^2$ represents. C-Cl, $A^1$, $A^3$, and $A^5$ represent CH, and $A^4$ represents any one substituent indicated in [Table L1] to [Table L3] (hereinafter, referred to as "Compound class SX477").

A present compound (L-1) wherein $R^{1b}$ represents a chlorine atom, $R^{1c}$ represents an ethyl group, $A^2$ represents C-Br, $A^1$, $A^3$, and $A^5$ represent CH, and $A^4$ represents any one substituent indicated in [Table L1] to [Table. L3] (hereinafter, referred to as "Compound class SX478").

A present compound (L-1) wherein $R^{1b}$ represents a chlorine atom, $R^{1c}$ represents an ethyl group, $A^2$ represents C-I, $A^1$, $A^3$, and $A^5$ represent CH, and $A^4$ represents any one substituent indicated in [Table L1] to [Table L3] (hereinafter, referred to as "Compound class SX479").

A present compound (L-1) wherein $R^{1b}$ represents a chlorine atom, $R^{1c}$ represents an ethyl group, $A^2$ represents C-Me, $A^1$, $A^3$, and $A^5$ represent CH, and $A^4$ represents any one substituent indicated in [Table L1] to [Table L3] (hereinafter, referred to as "Compound class SX480").

A present compound (L-1) wherein $R^{1b}$ represents a chlorine atom, $R^{1c}$ represents an ethyl group, $A^2$ represents C-OMe, $A^1$, $A^3$, and $A^5$ represent CH, and $A^4$ represents any one substituent indicated in [Table L1] to [Table L3] (hereinafter, referred to as "Compound class SX481").

A present compound (L-2) wherein $R^{1b}$ represents a chlorine atom, $R^{1c}$ represents an ethyl group, $A^2$ represents. C-$CF_3$, $A^1$, $A^3$, and $A^5$ represent CH, and $A^4$ represents any one substituent indicated in [Table L1] to [Table L3] (hereinafter, referred to as "Compound class SX482").

A present compound (L-1) wherein $R^{1b}$ represents a chlorine atom, $R^{1c}$ represents an ethyl group, $A^2$ represents C-$OCF_3$, $A^1$, $A^3$, and $A^5$ represent CH, and $A^4$ represents any one substituent indicated in [Table L1] to [Table. L3] (hereinafter, referred to as "Compound class SX483").

A present compound (L-1) wherein $R^{1b}$ represents a chlorine atom, $R^{1c}$ represents an ethyl group, $A^3$ represents C-F, $A^1$, $A^2$, and $A^5$ represent CH, and $A^4$ represents any one substituent indicated in [Table L1] to [Table L3] (hereinafter, referred to as "Compound class SX484").

A present compound (L-1) wherein $R^{1b}$ represents a chlorine atom, $R^{1c}$ represents an ethyl group, $A^3$ represents C-Cl, $A^1$, $A^2$, and $A^5$ represent CH, and $A^4$ represents any one substituent indicated in [Table L1] to [Table L3] (hereinafter, referred to as "Compound class SX485").

A present compound (L-1) wherein $R^{1b}$ represents a chlorine atom, $R^{1c}$ represents an ethyl group, $A^3$ represents C-Br, $A^1$, $A^2$, and $A^5$ represent CH, and $A^4$ represents any one substituent indicated in [Table L1] to [Table L3] (hereinafter, referred to as "Compound class SX486").

A present compound (L-2) wherein $R^{1b}$ represents a chlorine atom, $R^{1c}$ represents an ethyl group, $A^3$ represents. C-I, $A^1$, $A^2$, and $A^5$ represent CH, and $A^4$ represents any one substituent indicated in [Table L1] to [Table L3] (hereinafter, referred to as "Compound class SX487").

A present compound (L-1) wherein $R^{1b}$ represents a chlorine atom, $R^{1c}$ represents an ethyl group, $A^3$ represents C-Me, $A^1$, $A^2$, and $A^5$ represent CH, and $A^4$ represents any one substituent indicated in [Table L1] to [Table. L3] (hereinafter, referred to as "Compound class SX488").

A present compound (L-1) wherein $R^{1b}$ represents a chlorine atom, $R^{1c}$ represents an ethyl group, $A^3$ represents C-OMe, $A^1$, $A^2$, and $A^5$ represent CH, and $A^4$ represents any one substituent indicated in [Table L1] to [Table L3] (hereinafter, referred to as "Compound class SX489").

A present compound (L-1) wherein $R^{1b}$ represents a chlorine atom, $R^{1c}$ represents an ethyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX490").

A present compound (L-1) wherein $R^{1b}$ represents a chlorine atom, $R^{1c}$ represents an ethyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX491").

A present compound (L-2) wherein $R^{1b}$ represents a bromine atom, $R^{1c}$ represents an ethyl group, $A^2$ represents C-F, $A^1$, $A^3$, and $A^5$ represent CH, and $A^4$ represents any one substituent indicated in [Table L1] to [Table L3] (hereinafter, referred to as "Compound class 8X492").

A present compound (L-1) wherein $R^{1b}$ represents a bromine atom, $R^{1c}$ represents an ethyl group, $A^2$ represents C-Cl, $A^1$, $A^3$, and $A^5$ represent CH, and $A^4$ represents any one substituent indicated in [Table L1] to [Table. L3] (hereinafter, referred to as "Compound class SX493").

A present compound (L-1) wherein $R^{1b}$ represents a bromine atom, $R^{1c}$ represents an ethyl group, $A^2$ represents C-Br, $A^1$, $A^3$, and $A^5$ represent CH, and $A^4$ represents any one substituent indicated in [Table L1] to [Table L3] (hereinafter, referred to as "Compound class SX494").

A present compound (L-1) wherein $R^{1b}$ represents a bromine atom, $R^{1c}$ represents an ethyl group, $A^2$ represents C-I, $A^3$, $A^3$, and $A^5$ represent CH, and $A^4$ represents any one substituent indicated in [Table L1] to [Table L3] (hereinafter, referred to as "Compound class SX495").

A present compound (L-1) wherein $R^{1b}$ represents a bromine atom, $R^{1c}$ represents an ethyl group, $A^2$ represents C-Me, $A^1$, $A^3$, and $A^5$ represent CH, and $A^4$ represents any one substituent indicated in [Table L1] to [Table L3] (hereinafter, referred to as "Compound class SX496").

A present compound (L-2) wherein $R^{1b}$ represents a bromine atom, $R^{1c}$ represents an ethyl group, $A^2$ represents C-OMe, $A^1$, $A^3$, and $A^5$ represent CH, and $A^4$ represents any one substituent indicated in [Table L1] to [Table L3] (hereinafter, referred to as "Compound class SX497").

A present compound (L-1) wherein $R^{1b}$ represents a bromine atom, $R^{1c}$ represents an ethyl group, $A^2$ represents C-$CF_3$, $A^1$, $A^3$, and $A^5$ represent CH, and $A^4$ represents any one substituent indicated in [Table L1] to [Table. L3] (hereinafter, referred to as "Compound class SX498").

A present compound (L-1) wherein $R^{1b}$ represents a bromine atom, $R^{1c}$ represents an ethyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX499").

A present compound (L-1) wherein $R^{1b}$ represents a bromine atom, $R^{1c}$ represents an ethyl group, $A^3$ represents C-F, $A^1$, $A^2$, and $A^5$ represent CH, and $A^4$ represents any one substituent indicated in [Table L1] to [Table L3] (hereinafter, referred to as "Compound class SX500").

A present compound (L-1) wherein $R^{1b}$ represents a bromine atom, $R^{1c}$ represents an ethyl group, $A^3$ represents C-Cl, $A^1$, $A^2$, and $A^5$ represent CH, and $A^4$ represents any one substituent indicated in [Table L1] to [Table L3] (hereinafter, referred to as "Compound class 5X501").

A present compound (L-2) wherein $R^{1b}$ represents a bromine atom, $R^{1c}$ represents an ethyl group, $A^3$ represents C-Br, $A^1$, $A^2$, and $A^5$ represent CH, and $A^4$ represents any one substituent indicated in [Table L1] to [Table L3] (hereinafter, referred to as "Compound class SX502").

A present compound (L-1) wherein $R^{1b}$ represents a bromine atom, $R^{1c}$ represents an ethyl group, $A^3$ represents C-I, $A^1$, $A^2$, and $A^5$ represent CH, and $A^4$ represents any one substituent indicated in [Table L1] to [Table. L3] (hereinafter, referred to as "Compound class SX503").

A present compound (L-1) wherein $R^{1b}$ represents a bromine atom, $R^{1c}$ represents an ethyl group, $A^3$ represents C-Me, $A^1$, $A^2$, and $A^5$ represent CH, and $A^4$ represents any one substituent indicated in [Table L1] to [Table L3] (hereinafter, referred to as "Compound class SX504").

A present compound (L-1) wherein $R^{1b}$ represents a bromine atom, $R^{1c}$ represents an ethyl group, $A^3$ represents C-OMe, $A^1$, $A^2$, and $A^5$ represent CH, and $A^4$ represents any one substituent indicated in [Table L1] to [Table L3] (hereinafter, referred to as "Compound class SX505").

A present compound (L-1) wherein $R^{1b}$ represents a bromine atom, $R^{1c}$ represents an ethyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX506").

A present compound (L-2) wherein $R^{1b}$ represents a bromine atom, $R^{1c}$ represents an ethyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX507").

A present compound (L-1) wherein $R^{1b}$ represents an iodine atom, $R^{1c}$ represents an ethyl group. $A^2$ represents C-F, $A^1$, $A^3$, and $A^5$ represent CH, and $A^4$ represents any one substituent indicated in [Table L1] to [Table L3] (hereinafter, referred to as "Compound class SX508").

A present compound (L-1) wherein $R^{1b}$ represents an iodine atom, $R^{1c}$ represents an ethyl group, $A^2$ represents C-Cl, $A^1$, $A^3$, and $A^5$ represent CH, and $A^4$ represents any one substituent indicated in [Table L1] to [Table L3] (hereinafter, referred to as "Compound class SX509").

A present compound (L-1) wherein $R^{1b}$ represents an iodine atom, $R^{1c}$ represents an ethyl group, $A^2$ represents C-Br, $A^1$, $A^3$, and $A^5$ represent CH, and $A^4$ represents any one substituent indicated in [Table L1] to [Table L3] (hereinafter, referred to as "Compound class SX510").

A present compound (L-1) wherein $R^{1b}$ represents an iodine atom, $R^{1c}$ represents an ethyl group, $A^2$ represents C-I, $A^1$, $A^3$, and $A^5$ represent CH, and $A^4$ represents any one substituent indicated in [Table L1] to [Table D3] (hereinafter, referred to as "Compound class SX511").

A present compound (L-1) wherein $R^{1b}$ represents an iodine atom, $R^{1c}$ represents an ethyl group, $A^2$ represents

C-Me, $A^1$, $A^3$, and $A^5$ represent CH, and $A^4$ represents any one substituent indicated in [Table L1] to [Table L3] (hereinafter, referred to as "Compound class SX512").

A present compound (L-1) wherein $R^{1b}$ represents an iodine atom, $R^{1c}$ represents an ethyl group, $A^2$ represents C-OMe, $A^1$, $A^3$, and $A^5$ represent CH, and $A^4$ represents any one substituent indicated in [Table L1] to [Table L3] (hereinafter, referred to as "Compound class SX513").

A present compound (L-1) wherein $R^{1b}$ represents an iodine atom, $R^{1c}$ represents an ethyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX514").

A present compound (L-1) wherein $R^{1b}$ represents an iodine atom, $R^{1c}$ represents an ethyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX515").

A present compound (L-1) wherein $R^{1b}$ represents an iodine atom, $R^{1c}$ represents an ethyl group, $A^3$ represents C-F, $A^1$, $A^2$, and $A^5$ represent CH, and $A^4$ represents any one substituent indicated in [Table L1] to [Table L3] (hereinafter, referred to as "Compound class SX516").

A present compound (L-1) wherein $R^{1b}$ represents an iodine atom, $R^{1c}$ represents an ethyl group, $A^3$ represents C-Cl, $A^1$, $A^2$, and $A^5$ represent CH, and $A^4$ represents any one substituent indicated in [Table L1] to [Table L3] (hereinafter, referred to as "Compound class SX517").

A present compound (L-1) wherein $R^{1b}$ represents an iodine atom, $R^{1c}$ represents an ethyl group. $A^3$ represents C-Br, $A^1$, $A^2$, and $A^5$ represent CH, and $A^4$ represents any one substituent indicated in [Table L1] to [Table L3] (hereinafter, referred to as "Compound class SX518").

A present compound (L-1) wherein $R^{1b}$ represents an iodine atom, $R^{1c}$ represents an ethyl group, $A^3$ represents C-I, $A^1$, $A^2$, and $A^5$ represent CH, and $A^4$ represents any one substituent indicated in [Table L1] to [Table L3] (hereinafter, referred to as "Compound class SX519").

A present compound (L-1) wherein $R^{1b}$ represents an iodine atom, $R^{1c}$ represents an ethyl group, $A^3$ represents C-Me, $A^1$, $A^2$, and $A^5$ represent CH, and $A^4$ represents any one substituent indicated in [Table L1] to [Table L3] (hereinafter, referred to as "Compound class SX520").

A present compound (L-1) wherein $R^{1b}$ represents an iodine atom, $R^{1c}$ represents an ethyl group, $A^3$ represents C-OMe, $A^1$, $A^2$, and $A^5$ represent CH, and $A^4$ represents any one substituent indicated in [Table L1] to [Table L3] (hereinafter, referred to as "Compound class SX521").

A present compound (L-1) wherein $R^{1b}$ represents an iodine atom, $R^{1c}$ represents an ethyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX522").

A present compound (L-1) wherein $R^{1b}$ represents an iodine atom, $R^{1c}$ represents an ethyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX523").

A present compound (L-1) wherein $R^{1b}$ represents a methyl group, $R^{1c}$ represents an ethyl group, $A^2$ represents C-F, $A^1$, $A^3$, and $A^5$ represent CH, and $A^4$ represents any one substituent indicated in [Table L1] to [Table L3] (hereinafter, referred to as "Compound class SX524").

A present compound (L-1) wherein $R^{1b}$ represents a methyl group,. $R^{1c}$ represents an ethyl group, $A^2$ represents C-Cl, $A^1$, $A^3$, and $A^5$ represent CH, and $A^4$ represents any one substituent indicated in [Table L1] to [Table L3] (hereinafter, referred to as "Compound class SX525").

A present compound (L-1) wherein $R^{1b}$ represents a methyl group, $R^{1c}$ represents an ethyl group, $A^2$ represents C-Br, $A^1$, $A^3$, and $A^5$ represent CH, and $A^4$ represents any one substituent indicated in [Table L1] to [Table. L3] (hereinafter, referred to as "Compound class SX526").

A present compound (L-1) wherein $R^{1b}$ represents a methyl group, $R^{1c}$ represents an ethyl group, $A^2$ represents C-I, $A^1$, $A^3$, and $A^5$ represent CH, and $A^4$ represents any one substituent indicated in [Table L1] to [Table L3] (hereinafter, referred to as "Compound class SX527").

A present compound (L-1) wherein $R^{1b}$ represents a methyl group, $R^{1c}$ represents an ethyl group, $A^2$ represents C-Me, $A^1$, $A^3$, and $A^5$ represent CH, and $A^4$ represents any one substituent indicated in [Table L1] to [Table L3] (hereinafter, referred. to as "Compound class SX528").

A present compound (L-1) wherein $R^{1b}$ represents a methyl group, $R^{1c}$ represents an ethyl group, $A^2$ represents C-OMe, $A^1$, $A^3$, and $A^5$ represent CH, and $A^4$ represents any one substituent indicated in [Table L1] to [Table L3] (hereinafter, referred to as "Compound class SX529").

A present compound (L-1) wherein $R^{1b}$ represents a methyl group, $R^{1c}$ represents an ethyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX530").

A present compound (L-1) wherein $R^{1b}$ represents a methyl group, $R^{1c}$ represents an ethyl group, $A^2$ represents C-$OCF_3$, $A^1$, $A^3$, and $A^5$ represent CH, and $A^4$ represents any one substituent indicated in [Table L1] to [Table L3]

(hereinafter, referred to as "Compound class SX531").

A present compound (L-1) wherein $R^{1b}$ represents a methyl group, $R^{1c}$ represents an ethyl group, $A^3$ represents C-F, $A^1$, $A^2$, and $A^5$ represent CH, and $A^4$ represents any one substituent indicated in [Table L1] to [Table L3] (hereinafter, referred to as "Compound class SX532").

A present compound (L-1) wherein $R^{1b}$ represents a methyl group, $R^{1c}$ represents an ethyl group, $A^3$ represents C-Cl, $A^1$, $A^2$, and $A^5$ represent CH, and $A^4$ represents any one substituent indicated in [Table L1] to [Table L3] (hereinafter, referred. to as "Compound class SX533").

A present compound (L-1) wherein $R^{1b}$ represents a methyl group, $R^{1c}$ represents an ethyl group, $A^3$ represents C-Br, $A^1$, $A^2$, and $A^5$ represent CH, and $A^4$ represents any one substituent indicated in [Table L1] to [Table L3] (hereinafter, referred to as "Compound class SX534").

A present compound (L-1) wherein $R^{1b}$ represents a methyl group, $R^{1c}$ represents an ethyl group, $A^3$ represents C-I, $A^1$, $A^2$, and $A^5$ represent CH, and $A^4$ represents any one substituent indicated in [Table L1] to [Table L3] (hereinafter, referred to as "Compound class SX535").

A present compound (L-1) wherein $R^{1b}$ represents a methyl group, $R^{1c}$ represents an ethyl group, $A^3$ represents C-Me, $A^1$, $A^2$, and $A^5$ represent CH, and $A^4$ represents any one substituent indicated in [Table L1] to [Table. L3] (hereinafter, referred to as "Compound class SX536").

A present compound (L-1) wherein $R^{1b}$ represents a methyl group, $R^{1c}$ represents an ethyl group, $A^3$ represents C-OMe, $A^1$, $A^2$, and $A^5$ represent CH, and $A^4$ represents any one substituent indicated in [Table L1] to [Table L3] (hereinafter, referred to as "Compound class SX537").

A present compound (L-1) wherein $R^{1b}$ represents a methyl group, $R^{1c}$ represents an ethyl group, $A^3$ represents C-$CF_3$, $A^1$, $A^2$, and $A^5$ represent CH, and $A^4$ represents any one substituent indicated in [Table L1] to [Table L3] (hereinafter, referred. to as "Compound class SX538").

A present compound (L-1) wherein $R^{1b}$ represents a methyl group, $R^{1c}$ represents an ethyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX539").

A present compound (L-1) wherein $R^{1b}$ represents a chlorine atom, $R^{1c}$ represents a bromine atom, $A^2$ represents C-F, $A^1$, $A^3$, and $A^5$ represent CH, and $A^4$ represents any one substituent indicated in [Table L1] to [Table L3] (hereinafter, referred to as "Compound class SX540").

A present compound (L-1) wherein $R^{1b}$ represents a chlorine atom, $R^{1c}$ represents a bromine atom, $A^2$ represents C-Cl, $A^1$, $A^3$, and $A^5$ represent CH, and $A^4$ represents any one substituent indicated in [Table L1] to [Table L3] (hereinafter, referred to as "Compound class SX541").

A present compound (L-1) wherein $R^{1b}$ represents a chlorine atom, $R^{1c}$ represents an ethyl group, $A^2$ represents C-Br, $A^1$, $A^3$, and $A^5$ represent CH, and $A^4$ represents any one substituent indicated in [Table L1] to [Table L3] (hereinafter, referred to as "Compound class SX542").

A present compound (L-1) wherein $R^{1b}$ represents a chlorine atom, $R^{1c}$ represents a bromine atom, $A^2$ represents C-I, $A^1$, $A^3$, and $A^5$ represent CH, and $A^4$ represents any one substituent indicated in [Table L1] to [Table L3] (hereinafter, referred to as "Compound class SX543").

A present compound (L-2) wherein $R^{1b}$ represents a chlorine atom, $R^{1c}$ represents a bromine atom, $A^2$ represents C-Me, $A^1$, $A^3$, and $A^5$ represent CH, and $A^4$ represents any one substituent indicated in [Table L1] to [Table L3] (hereinafter, referred to as "Compound class SX544").

A present compound (L-1) wherein $R^{1b}$ represents a chlorine atom, $R^{1c}$ represents a bromine atom, $A^2$ represents C-OMe, $A^1$, $A^3$, and $A^5$ represent CH, and $A^4$ represents any one substituent indicated in [Table L1] to [Table L3] (hereinafter, referred to as "Compound class SX545").

A present compound (L-1) wherein $R^{1b}$ represents a chlorine atom, $R^{1c}$ represents a bromine atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX546").

A present compound (L-1) wherein $R^{1b}$ represents a chlorine atom, $R^{1c}$ represents a bromine atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX547").

A present compound (L-1) wherein $R^{1b}$ represents a chlorine atom, $R^{1c}$ represents a bromine atom, $A^3$ represents C-F, $A^1$, $A^2$, and $A^5$ represent CH, and $A^4$ represents any one substituent indicated in [Table L1] to [Table L3] (hereinafter, referred to as "Compound class SX548").

A present compound (L-2) wherein $R^{1b}$ represents a chlorine atom, $R^{1c}$ represents a bromine atom, $A^3$ represents C-Cl, $A^1$, $A^2$, and $A^5$ represent CH, and $A^4$ represents any one substituent indicated in [Table L1] to [Table L3] (hereinafter, referred to as "Compound class SX549").

A present compound (L-1) wherein $R^{1b}$ represents a chlorine atom, $R^{1c}$ represents a bromine atom, $A^3$ represents C-Br, $A^1$, $A^2$, and $A^5$ represent CH, and $A^4$ represents any one substituent indicated in [Table L1] to [Table L3] (hereinafter, referred to as "Compound class SX550").

A present compound (L-1) wherein $R^{1b}$ represents a chlorine atom, $R^{1c}$ represents a bromine atom, $A^3$ represents C-1, $A^1$, $A^2$, and $A^5$ represent CH, and $A^4$ represents any one substituent indicated in [Table L1] to [Table L3] (hereinafter, referred to as "Compound class SX551").

A present compound (L-1) wherein $R^{1b}$ represents a chlorine atom, $R^{1c}$ represents a bromine atom, $A^3$ represents C-Me, $A^1$, $A^2$, and $A^5$ represent CH, and $A^4$ represents any one substituent indicated in [Table L1] to [Table L3] (hereinafter, referred to as "Compound class SX552").

A present compound (L-1) wherein $R^{1b}$ represents a chlorine atom, $R^{1c}$ represents a bromine atom, $A^3$ represents C-OMe, $A^1$, $A^2$, and $A^5$ represent CH, and $A^4$ represents any one substituent indicated in [Table L1] to [Table L3] (hereinafter, referred to as "Compound class SX553").

A present compound (L-2) wherein $R^{1b}$ represents a chlorine atom, $R^{1c}$ represents a bromine atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX554").

A present compound (L-1) wherein $R^{1b}$ represents a chlorine atom, $R^{1c}$ represents a bromine atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SXS5S").

A present compound (L-1) wherein $R^{1b}$ represents a chlorine atom, $R^{1c}$ represents any one group selected from a hydrogen atom, a fluorine atom, a chlorine atom, a bromine atom, an iodine atom, a methyl group, an ethyl group, a propyl group, an isopropyl group, a cyclopropyl group, or $CF_3$, $A^1$, $A^2$, and $A^3$ represent CH, and $A^4$ represents any one substituent indicated in [Table L1] to [Table L3] (hereinafter, referred to as "Compound class SX556").

A present compound (L-1) wherein $R^{1b}$ represents a fluorine atom, $R^{1c}$ represents a hydrogen atom, $A^1$ represents a nitrogen atom, $A^2$, $A^3$, and $A^4$ represent CH, and $A^5$ represents any one substituent indicated in [Table L13 to [Table L3] (hereinafter, referred to as "Compound class SX557").

A present compound (L-1) wherein $R^{1b}$ represents a chlorine atom, $R^{1c}$ represents a hydrogen atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX558").

A present compound (L-1) wherein $R^{1b}$ represents a bromine atom, $R^{1c}$ represents a hydrogen atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX559").

A present compound (L-1) wherein $R^{1b}$ represents an iodine atom, $R^{1c}$ represents a hydrogen atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX560").

A present compound (L-1) wherein $R^{1b}$ represents a methyl group, $R^{1c}$ represents a hydrogen atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX561").

A present compound (L-1) wherein $R^{1b}$ represents an ethyl group, $R^{1c}$ represents a hydrogen atom, $A^1$ represents a nitrogen atom, $A^2$, $A^3$, and $A^4$ represent CH, and $A^5$ represents any one substituent indicated in [Table L13 to [Table L3] (hereinafter, referred to as "Compound class SX562").

A present compound (L-1) wherein $R^{1b}$ represents a propyl group, $R^{1c}$ represents a hydrogen atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX563").

A present compound (L-1) wherein $R^{1b}$ represents an isopropyl group, $R^{1c}$ represents a hydrogen atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX564").

A present compound (L-1) wherein $R^{1b}$ represents a cyclopropyl group, $R^{1c}$ represents a hydrogen atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX565").

A present compound (L-1) wherein $R^{1b}$ represents $CF_3$, $R^{1c}$ represents a hydrogen atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX566").

A present compound (L-1) wherein $R^{1b}$ and $R^{1c}$ represents a fluorine atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX567").

A present compound (L-1) wherein $R^{1b}$ and $R^{1c}$ represents a chlorine atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX568").

A present compound (L-1) wherein $R^{1b}$ and $R^{1c}$ represents a bromine atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX569").

A present compound (L-1) wherein $R^{1b}$ and $R^{1c}$ represents an iodine atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX570").

A present compound (L-1) wherein $R^{1b}$ and $R^{1c}$ represents a methyl group, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX571").

A present compound (L-1) wherein $R^{1b}$ and $R^{1c}$ represents an ethyl group, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SXS72").

A present compound (L-1) wherein $R^{1b}$ and $R^{1c}$ represents a propyl group, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX573").

A present compound (L-1) wherein $R^{1b}$ and $R^{1c}$ represents an isopropyl group, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX574"),

A present compound (L-1) wherein $R^{1b}$ and $R^{1c}$ represents a cyclopropyl group, $A^1$ represents a nitrogen atom, $A^2$, $A^3$, and $A^4$ represent CH, and $A^5$ represents any one substituent indicated in [Table L1] to [Table L3] (hereinafter, referred to as "Compound class 3X575").

A present compound (L-1) wherein $R^{1b}$ and $R^{1c}$ represents $CF_3$, $A^1$ represents a nitrogen atom, $A^2$, $A^3$, and $A^4$ represent CH, and $A^5$ represents any one substituent indicated in [Table L1] to [Table L3] (hereinafter, referred to as "Compound class SX516").

A present compound (L-1) wherein $R^{1b}$ and $R^{1c}$ represents a fluorine atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class SX517").

A present compound (1-1) wherein $R^{1b}$ and $R^{1c}$ represents a chlorine atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class SX578").

A present compound (L-1) wherein $R^{1b}$ and $R^{1c}$ represents a bromine atom, $A^1$ represents a nitrogen atom, $A^2$, $A^3$, and $A^5$ represent CH, and $A^4$ Represents any one substituent indicated in [Table L1] to [Table L3] (hereinafter, referred to as "Compound class SX579").

A present compound (L-1.) wherein $R^{1b}$ and $R^{1c}$ represents an iodine atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class SX580").

A present compound (L-1) wherein $R^{1b}$ and $R^{1c}$ represents a methyl group, $A^1$ represents a nitrogen atom, $A^2$, $A^3$, and $A^5$ represent CH, and $A^4$ represents any one substituent indicated in [Table L1] to [Table L3] (hereinafter, referred to as "Compound class SX581").

A present compound (L-1) wherein $R^{1b}$ and $R^{1c}$ represents an ethyl group, $A^1$ represents a nitrogen atom, $A^2$, $A^{3,}$, and $A^5$ represent CH, and $A^4$ represents any one substituent indicated in [Table L1] to [Table L3] (hereinafter, referred to as "Compound class SXS82").

A present compound (L-1) wherein $R^{1b}$ and $R^{1c}$ represents an propyl group, $A^1$ represents a nitrogen atom, $A^2$, $A^3$, and $A^5$ represent CH, and $A^4$ represents any one substituent indicated in [Table L1] to [Table L3] (hereinafter, referred to as "Compound class SX583").

A present compound (L-1) wherein $R^{1b}$ and $R^{1c}$ represents an isopropyl group, $A^1$ represents a nitrogen atom, $A^2$, $A^3$, and $A^5$ represent CH, and $A^4$ represents any one substituent indicated in [Table L1] to [Table L3] (hereinafter, referred to as "Compound class SX584").

A present compound (L-1) wherein $R^{1b}$ and $R^{1c}$ represents a cyclopropyl group, $A^1$ represents a nitrogen atom, $A^2$, $A^3$, and $A^5$ represent CH, and $A^4$ represents any one substituent indicated in [Table L1] to [Table L3] (hereinafter, referred to as "Compound class SX585").

A present compound (L-1) wherein $R^{1b}$ and $R^{1c}$ represents $CF_3$, $A^1$ represents a nitrogen atom, $A^2$, $A^3$, and $A^5$ represent CH, and $A^4$ represents any one substituent indicated in [Table L1] to [Table L3] (hereinafter, referred to as "Compound class SX586").

A present compound (L-1) wherein $R^{1b}$ represents a fluorine atom, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class SX587").

A present compound (L-1) wherein $R^{1b}$ represents a chlorine atom, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class SX588").

A present compound (L-1) wherein $R^{1b}$ represents a bromine atom, $R^{1c}$ represents a hydrogen atom, $A^1$

represents a nitrogen atom, $A^2$, $A^3$, and $A^5$ represent CH, and $A^4$ represents any one substituent indicated in [Table L1] to [Table L3] (hereinafter, referred to as "Compound class SX589").

A present compound (L-1) wherein $R^{1b}$ represents an iodine atom, $R^{1c}$ represents a hydrogen atom, $A^1$ represents a nitrogen atom, $A^2$, $A^2$, and $A^5$ represent CH, and $A^4$ represents any one substituent indicated in [Table L1] to [Table L3] (hereinafter, referred to as "Compound class SX590").

A present compound (L-1) wherein $R^{1b}$ represents a methyl group, $R^{1c}$ represents a hydrogen atom, $A^1$ represents a nitrogen atom, $A^2$, $A^3$, and $A^5$ represent CH, and $A^4$ represents any one substituent, indicated in [Table L1] to [Table L3] (hereinafter, referred to as "Compound class 5X591").

A present compound (L-1) wherein $R^{1b}$ represents an ethyl group, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class SX592").

A present compound (L-1) wherein $R^{1b}$ represents a propyl group, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class SX593").

A present compound (L-1) wherein $R^{1b}$ represents an isopropyl group, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class SX594").

A present compound (L-1) wherein $R^{1b}$ represents a cyclopropyl group, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class SX595").

A present compound (L-1) wherein $R^{1b}$ represents $CF_3$, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class SX596").

A present compound (L-1) wherein $R^{1b}$ and $R^{1c}$ represent a fluorine atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class SXS97").

A present compound (L-1) wherein. $R^{1b}$ and $R^{1c}$ represent a chlorine atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class SX598").

A present compound (L-1) wherein $R^{1b}$ and $R^{1c}$ represent a bromine atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class 8X599").

A present compound (L-1) wherein $R^{1b}$ and $R^{1c}$ represent an iodine atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class SX600").

A present compound (L-1) wherein $R^{1b}$ and $R^{1c}$ represent a methyl group, $A^1$ represents a nitrogen atom, $A^2$, $A^4$, and $A^5$ represent CH, and $A^3$ represents any one substituent indicated in [Table L1] to [Table L3] (hereinafter, referred to as "Compound class SX601").

A present compound (L-1) wherein $R^{1b}$ and $R^{1c}$ represent an ethyl group, $A^1$ represents a nitrogen atom, $A^2$, $A^4$, and $A^5$ represent CH, and $A^3$ represents any one substituent indicated in [Table L1] to [Table L3] (hereinafter, referred to as "Compound class SX602").

A present compound (L-1) wherein. $R^{1b}$ and $R^{1c}$ represent a propyl group. $A^1$ represents a nitrogen atom, $A^2$, $A^4$, and $A^5$ represent CH, and $A^3$ represents any one substituent indicated in [Table L1] to [Table L3] (hereinafter, referred to as "Compound class SX603").

A present compound (L-1) wherein $R^{1b}$ and $R^{1c}$ represent an isopropyl group, $A^1$ represents a nitrogen atom, $A^2$, $A^4$, and $A^5$ represent CH, and $A^3$ represents any one substituent indicated in [Table L1] to [Table L3] (hereinafter, referred to as Ccompound class SX604").

A present compound (L-1) wherein $R^{1b}$ and $R^{1c}$ represent a cyclopropyl group, $A^1$ represents a nitrogen atom. $A^2$, $A^4$, and $A^5$ represent CH, and $A^3$ represents any one substituent indicated in [Table L1] to [Table L3] (hereinafter, referred to as "Compound class SX605").

A present compound (L-1) wherein $R^{1b}$ and $R^{1c}$ represent $CF_3$, $A^1$ represents a nitrogen atom, $A^2$, $A^4$, and $A^5$ represent CH, and $A^3$ represents any one substituent indicated in [Table L1] to [Table L3] (hereinafter, referred to as "Compound class SX606") .

A present compound (L-1) wherein $R^{1b}$ represents a fluorine atom, $R^{1c}$ represent a hydrogen atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class SX607").

A present compound (L-1) wherein $R^{1b}$ represents a chlorine atom, $R^{1c}$ represent a hydrogen atom, $A^1$ represents a nitrogen atom, $A^2$, $A^4$, and $A^5$ represent CH, and $A^3$ represents any one substituent indicated in [Table L1] to

[Table L3] (hereinafter, referred to as "Compound class SX608").

A present compound (L-1) wherein $R^{1b}$ represents a bromine atom, $R^{1c}$ represent a hydrogen atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class 8X609").

A present compound (L-1) wherein $R^{1b}$ represents an iodine atom, $R^{1c}$ represent a hydrogen atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class 5X610").

A present compound (L-1) wherein $R^{1b}$ represents a methyl group, $R^{1c}$ represent a hydrogen atom, $A^1$ represents a nitrogen atom, $A^2$, $A^4$, and $A^5$ represent CH, and $A^3$, represents any one substituent indicated in [Table L1] to [Table L3] (hereinafter, referred to as "Compound class SX611").

A present compound (L-1) wherein $R^{1b}$ represents an ethyl group, $R^{1c}$ represent a hydrogen atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class SX612").

A present compound (L-1) wherein $R^{1b}$ represents a propyl group, $R^{1c}$ represent a hydrogen atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class SX613").

A present compound (L-1) wherein $R^{1b}$ represents an isopropyl group, $R^{1c}$ represent a. hydrogen atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class SX614").

A present compound (L-1) wherein $R^{1b}$ represents a cyclopropyl group, $R^{1c}$ represent a hydrogen atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class SX615").

A present compound (L-1) wherein $R^{1b}$ represents $CF_3$, $R^{1c}$ represent a hydrogen atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class SX616").

A present compound (L-1) wherein $R^{1b}$ and $R^{1c}$ represent a fluorine atom, $A^1$ represents a nitrogen atom, $A^3$, $A^4$, and $A^5$ represent CH, and $A^2$ represents, any one substituent indicated in [Table L1] to [Table L3] (hereinafter, referred to as "Compound class SX617").

A present compound (L-1) wherein $R^{1b}$ and $R^{1c}$ represent a chlorine atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class SX618").

A present compound (L-1) wherein $R^{1b}$ and $R^{1c}$ represent a bromine atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class SX619").

A present compound (L-1) wherein $R^{1b}$ and $R^{1c}$ represent an iodine atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class SX620").

A present compound (L-1) wherein $R^{1b}$ and $R^{1c}$ represent a methyl group, $A^1$ represents a nitrogen atom, $A^3$, $A^4$, and $A^5$ represent CH, and $A^2$ represents any one substituent indicated in [Table. L1] to [Table L3] (hereinafter, referred to as "Compound class SX621").

A present compound (L-1) wherein $R^{1b}$ and $R^{1c}$ represent an ethyl group, $A^1$ represents a nitrogen atom, $A^3$, $A^4$, and $A^5$ represent CH, and $A^2$ represents, any one substituent indicated in [Table L1] to [Table L3] (hereinafter, referred to as "Compound class SX622").

A present compound (L-1) wherein $R^{1b}$ and $R^{1c}$ represent a propyl group, $A^1$ represents a nitrogen atom, $A^3$, $A^4$, and $A^5$ represent CH, and $A^2$ represents any one substituent indicated in [Table L1] to [Table L3] (hereinafter, referred to as "Compound class SX623").

A present compound (L-1) wherein $R^{1b}$ and $R^{1c}$ represent an isopropyl group, $A^1$ represents a nitrogen atom, $A^3$, $A^4$, and $A^5$ represent CH, and $A^2$ represents any one substituent indicated in [Table L1] to [Table L3] (hereinafter, referred to as "Compound class SX624").

A present compound (L-1) wherein $R^{1b}$ and $R^{1c}$ represent a cyclopropyl group, $A^1$ represents a nitrogen atom, $A^3$ $A^4$, and $A^5$ represent CH, and $A^2$ represents any one substituent indicated in [Table L1] to [Table L3] (hereinafter, referred to as "Compound class SX625").

A present compound (L-1) wherein $R^{1b}$ and $R^{1c}$ represent $CF_3$, $A^1$ represents a nitrogen atom, $A^3$, $A^4$, and $A^5$ represent CH, and $A^2$ represents any one substituent indicated in [Table L1] to [Table L3] (hereinafter, referred to as "Compound class SX626").

A present compound (L-1) wherein $R^{1b}$ represents a fluorine atom, $R^{1c}$ represents a hydrogen atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class SX627").

A present compound (L-1) wherein $R^{1b}$ represents a chlorine atom, $R^{1c}$ represents a hydrogen atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class SX628").

A present compound (L-1) wherein $R^{1b}$ represents a bromine atom, $R^{1c}$ represents a hydrogen atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class SX629").

A present compound (L-1) wherein $R^{1b}$ represents an iodine atom, $R^{1c}$ represents a hydrogen atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class 8X630").

A present compound (L-1) wherein $R^{1b}$ represents a methyl group, $R^{1c}$ represents a hydrogen atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class 8X631").

A present compound (L-1) wherein $R^{1b}$ represents an ethyl group, $R^{1c}$ represents a hydrogen atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class SX632").

A present compound (L-1) wherein $R^{1b}$ represents a propyl group, $R^{1c}$ represents a hydrogen atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class SX633").

A present compound (L-1) wherein $R^{1b}$ represents an isopropyl group, $R^{1c}$ represents a hydrogen atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class SX634").

A present compound (L-1) wherein $R^{1b}$ represents a cyclopropyl group, $R^{1c}$ represents a hydrogen atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class SX635").

A present compound (L-1) wherein $R^{1b}$ represents $CF_3$ $R^{1c}$ represents a hydrogen atom. $A^1$ represents a nitrogen atom, $A^3$, $A^4$, and $A^5$ represent CH, and $A^2$ represents any one substituent indicated in [Table L1] to [Table L3] (hereinafter, referred to as "Compound class SX636").

A present compound (L-1) wherein $R^{1b}$ represents a chlorine atom, $R^{1c}$ represents any one group selected from a hydrogen atom, a fluorine atom, a chlorine atom, a bromine atom, an iodine atom, a methyl group, an ethyl group, a propyl group, an isopropyl group, a cyclopropyl group, or $CF_3$, $A^1$ represents a nitrogen atom, and $A^2$, $A^3$, $A^4$, and $A^5$ represent CH (hereinafter, referred to as "Compound class SX637").

A compound represented by formula (L-2):

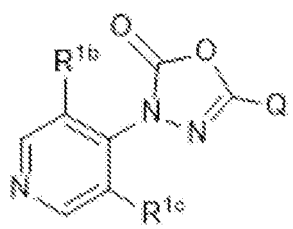

(L-2)

(hereinafter, referred to as "Compound (L-2)")
wherein $R^{1b}$ represents a fluorine atom, $R^{1c}$ represents a hydrogen atom, and Q represents any one substituent indicated in [Table L4] to [Table L9] (hereinafter, referred to as "Compound class SX638").

[Table L4]

| |
|---|
| 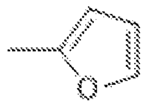 |
| 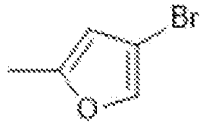 |
| 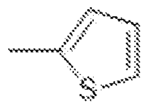 |
| 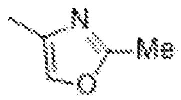 |
| 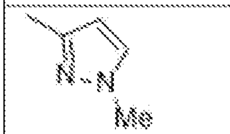 |
| 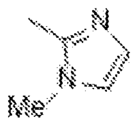 |

[Table L5]

| |
|---|
| 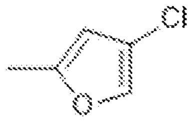 |
| 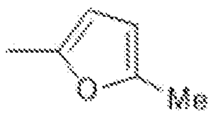 |
| 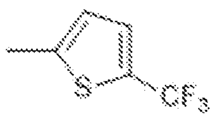 |
| 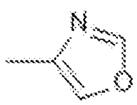 |
| 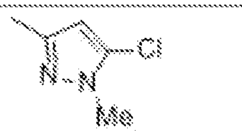 |
| 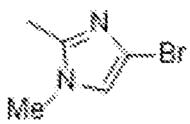 |

[Table L6]

| |
|---|
| 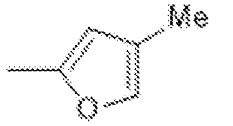 |
| 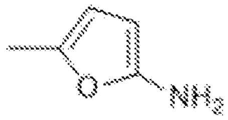 |
| 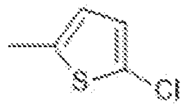 |
| 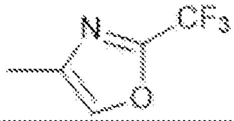 |
| 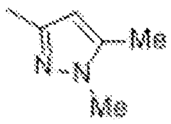 |
| 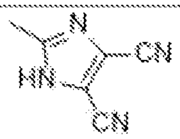 |

[Table L7]

[Table L8]

[Table L9]

| |
|---|
| 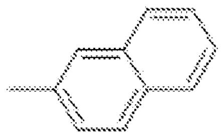 |
| 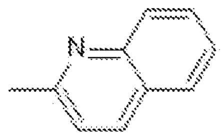 |
| 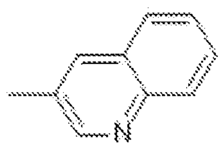 |
| 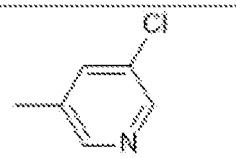 |
| 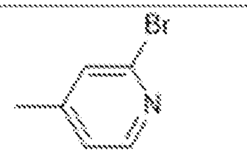 |
| 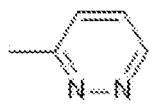 |
| 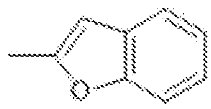 |
| 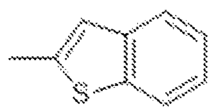 |

| |
|---|
| 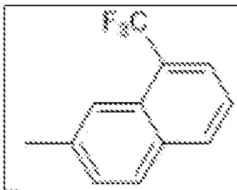 |
| 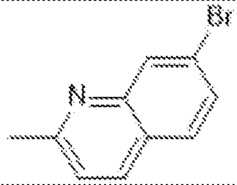 |
| 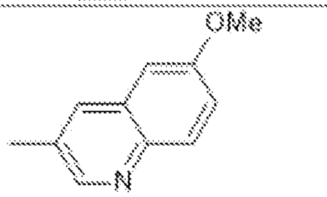 |
| 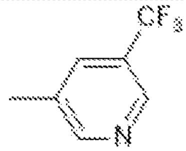 |
| 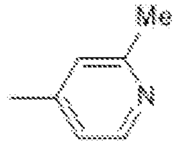 |
| 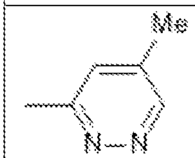 |
| 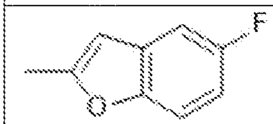 |
| 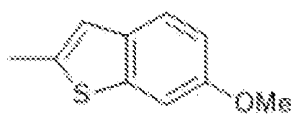 |

| |
|---|
| 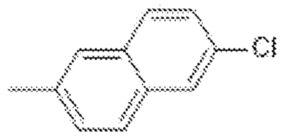 |
| 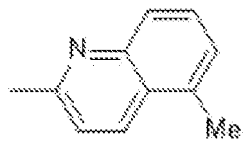 |
| 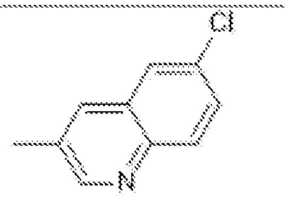 |
| 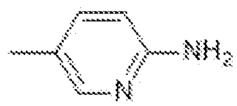 |
| 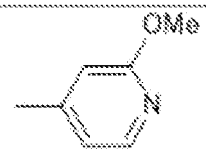 |
| 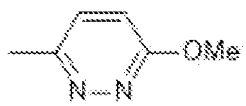 |
| 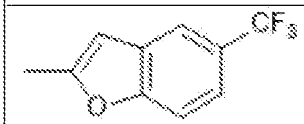 |
| 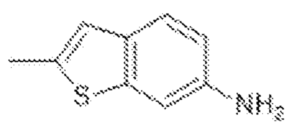 |

A present compound (L-2) wherein $R^{1b}$ represents a chlorine atom, $R^{1c}$ represents a hydrogen atom, and Q represents any one substituent indicated in [Table L4] to [Table L9] (hereinafter, referred to as "Compound class SX639").

A present compound (L-2) wherein $R^{1b}$ represents a bromine atom, $R^{1c}$ represents a hydrogen atom, and Q represents any one substituent indicated in [Table L4] to [Table L9] (hereinafter, referred to as "Compound class SX640").

A present compound (L-2) wherein $R^{1b}$ represents an iodine atom, $R^{1c}$ represents a hydrogen atom, and Q represents any one substituent indicated in [Table L4] to [Table L9] (hereinafter, referred to as "Compound class SX641").

A present compound (L:-2) wherein $R^{1b}$ represents a methyl group, $R^{1c}$ represents a hydrogen atom, and Q represents any one substituent indicated in [Table L4] to [Table L9] (hereinafter, referred to as "Compound class SX642").

A present compound (L-2) wherein $R^{1b}$ represents an ethyl group, $R^{1c}$ represents a hydrogen atom, and Q represents any one substituent indicated in [Table L4] to [Table L9] (hereinafter, referred to as "Compound class 3X643").

A present compound (L-2) wherein $R^{1b}$ represents a propyl group, $R^{1c}$ represents a hydrogen atom, and Q represents any one substituent indicated in [Table L4] to [Table L9] (hereinafter, referred to as "Compound class SX644").

A present compound (L-2) wherein $R^{1b}$ represents an isopropyl group, $R^{1c}$ represents a hydrogen atom, and Q represents any one substituent indicated in [Table L4] to [Table L9] (hereinafter, referred to as "Compound class SX645").

A present compound (L-2) wherein $R^{1b}$ represents a cyclopropyl group, $R^{1c}$ represents a hydrogen atom, and Q represents any one substituent indicated in [Table L4] to [Table L9] (hereinafter, referred to as "Compound class SX646").

A present compound (L-2) wherein $R^{1b}$ represents $CF_3$, $R^{1c}$ represents a hydrogen atom, and Q represents any one substituent indicated in [Table L4] to [Table L9] (hereinafter, referred to as "Compound class SX647").

A present compound (L-2) wherein $R^{1b}$ represents a methylsulfanyl group, $R^{1c}$ represents a hydrogen atom, and Q represents any one substituent indicated in [Table L4] to [Table L9] (hereinafter, referred to as "Compound class SX648").

A present compound (L-2) wherein $R^{1b}$ represents a methylsulfinyl group, $R^{1c}$ represents a hydrogen atom, and Q represents any one substituent indicated in [Table L4] to [Table L9] (hereinafter, referred to as "Compound class SX649").

A present compound (L-2) wherein $R^{1b}$ represents a methylsulfonyl group, $R^{1c}$ represents a hydrogen atom, and Q represents any one substituent indicated in [Table L4] to [Table L9] (hereinafter, referred to as "Compound class SX650").

A present compound (L-2) wherein $R^{1b}$ and $R^{1c}$ represents a fluorine atom, and Q represents any one substituent indicated in [Table L4] to [Table L9] (hereinafter, referred to as "Compound class SX651").

A present compound (L-2) wherein $R^{1b}$ and $R^{1c}$ represents a chlorine atom, and Q represents any one substituent indicated in [Table L4] to [Table L9] (hereinafter, referred to as "Compound class SX652").

A present compound (L-2) wherein $R^{1b}$ and $R^{1c}$ represents a bromine atom, and Q represents any one substituent indicated in [Table L4] to [Table. L9] (hereinafter, referred to as "Compound class SX653").

A present compound (L-2) wherein $R^{1b}$ and $R^{1c}$ represents an iodine atom, and Q represents any one substituent indicated in [Table L4] to [Table L9] (hereinafter, referred to as "Compound class SX654").

A present compound (L-2) wherein $R^{1b}$ and $R^{1c}$ represents a methyl group, and Q represents any one substituent indicated in [Table L4] to [Table L9] (hereinafter, referred to as "Compound class SX655").

A present compound (L-2) wherein $R^{1b}$ and $R^{1c}$ represents an ethyl group, and Q represents any one substituent indicated in [Table L4] to [Table L9] (hereinafter, referred to as "Compound class SX6S6").

A present compound (L-2) wherein $R^{1b}$ and $R^{1c}$ represents a propyl group, and Q represents any one substituent indicated in [Table L4] to [Table L9] (hereinafter, referred to as "Compound class SX657").

A present compound (L-2) wherein $R^{1b}$ and $R^{1c}$ represents an isopropyl group, and Q represents any one substituent indicated in [Table L4] to [Table L9] (hereinafter, referred to as "Compound class SX658").

A present compound (L-2) wherein $R^{1b}$ and $R^{1c}$ represents a cyclopropyl group, and Q represents any one substituent indicated in [Table L4] to [Table L9] (hereinafter, referred to as "Compound class SX6S9").

A present compound (L-2) wherein $R^{1b}$ and $R^{1c}$ represents $CF_3$, and Q represents any one substituent indicated in [Table L4] to [Table L9] (hereinafter, referred to as "Compound class SX660").

A compound represented by formula (L-3):

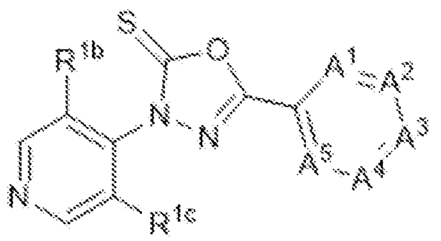

(L-3)

(hereinafter, referred to as "Compound (L-3)")
wherein $R^{1b}$ and $R^{1c}$ represent a chlorine atom, $A^2$, $A^3$, $A^4$, and $A^5$ represent CH, and $A^1$ represents any one substituent indicated in [Table L1] to [Table L3] (hereinafter, referred to as "Compound class SX661").

A compound (L-3) wherein $R^{1b}$ and $R^{1c}$ represent a chlorine atom, $A^1$, $A^3$, $A^4$, and $A^5$ represent CH, and $A^2$ represents any one substituent indicated in [Table L1] to [Table L3] (hereinafter, referred to as "Compound class SX662").

A compound (L-3) wherein $R^{1b}$ and $R^{1c}$ represent a chlorine atom, $A^1$, $A^2$, $A^4$, and $A^5$ represent CH, and $A^3$ represents any one substituent indicated in [Table L1] to [Table L3] (hereinafter, referred to as "Compound class SX663").

A compound (L-3) wherein $R^{1b}$ and $R^{1c}$ represent a chlorine atom, $A^2$ represents C-F, $A^1$, $A^3$, and $A^5$ represent CH, and $A^4$ represents any one substituent indicated in [Table L1] to [Table L3] (hereinafter, referred to as "Compound class SX664") .

A compound (L-3) wherein $R^{1b}$ and $R^{1c}$ represent a chlorine atom, $A^2$ represents C-Cl, $A^1$, $A^3$, and $A^5$ represent CH, and $A^4$ represents any one substituent indicated in [Table L1] to [Table L3] (hereinafter, referred to as "Compound class SX665").

A compound (L-3) wherein $R^{1b}$ and $R^{1c}$ represent a chlorine atom, $A^2$ represents C-Br, $A^1$, $A^3$, and $A^5$ represent CH, and $A^4$ represents any one substituent indicated in [Table L1] to [Table L3] (hereinafter, referred to as "Compound class SX666").

A compound (L-3) wherein $R^{1b}$ and $R^{1c}$ represent a chlorine atom, $A^2$ represents C-I, $A^1$, $A^3$, and $A^5$ represent CH, and $A^4$ represents any one substituent indicated in [Table L1] to [Table L3] (hereinafter, referred to as "Compound class SX667") .

A compound (L-3) wherein $R^{1b}$ and $R^{1c}$ represent a chlorine atom, $A^2$ represents C-Me, $A^1$, $A^3$, and $A^5$ represent CH, and $A^4$ represents any one substituent indicated in [Table L1] to [Table L3] (hereinafter, referred to as "Compound class SX668").

A compound (L-3) wherein $R^{1b}$ and $R^{1c}$ represent a chlorine atom, $A^2$ represents C-OMe, $A^1$, $A^3$, and $A^5$ represent CH, and $A^4$ represents any one substituent indicated in [Table L1] to [Table L3] (hereinafter, referred to as "Compound class SX669") .

A compound (L-3) wherein $R^{1b}$ and $R^{1c}$ represent a chlorine atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX670").

A compound (L-3) wherein $R^{1b}$ and $R^{1c}$ represent a chlorine atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX671").

A compound (L-3) wherein $R^{1b}$ and $R^{1c}$ represent a chlorine atom, $A^3$ represents C-F, $A^1$, $A^2$, and $A^5$ represent CH, and $A^4$ represents any one substituent indicated in [Table L1] to [Table L3] (hereinafter, referred to as "Compound class SX672") .

A compound (L-3) wherein $R^{1b}$ and $R^{1c}$ represent a chlorine atom, $A^3$ represents C-Cl, $A^{31}$, $A^2$, and $A^5$ represent CH, and $A^4$ represents any one substituent indicated in [Table L1] to [Table L3] (hereinafter, referred to as "Compound class SX673").

A compound (L-3) wherein $R^{1b}$ and $R^{1c}$ represent a chlorine atom, $A^3$ represents C-Br, $A^1$, $A^2$, and $A^5$ represent CH, and $A^4$ represents any one substituent indicated in [Table L1] to [Table L3] (hereinafter, referred to as "Compound class SX674") .

A compound (L-3) wherein $R^{1b}$ and $R^{1c}$ represent a chlorine atom, $A^3$ represents C-I, $A^1$, $A^2$, and $A^5$ represent CH, and $A^4$ represents any one substituent indicated in [Table L1] to [Table L3] (hereinafter, referred to as "Compound class SX675").

A compound (L-3) wherein $R^{1b}$ and $R^{1c}$ represent a chlorine atom, $A^3$ represents C-Me, $A^1$, $A^2$, and $A^5$ represent CH, and $A^4$ represents any one substituent indicated in [Table L1] to [Table L3] (hereinafter, referred to as "Compound class SX676").

A compound (L-3) wherein $R^{1b}$ and $R^{1c}$ represent a chlorine atom, $A^3$ represents C-OMe, $A^1$, $A^2$, and $A^5$ represent CH, and $A^4$ represents any one substituent indicated in [Table L1] to [Table L3] (hereinafter, referred to as "Compound class SX677") .

A compound (L-3) wherein $R^{1b}$ and $R^{1c}$ represent a chlorine atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX678").

A compound (L-3) wherein $R^{1b}$ and $R^{1c}$ represent a chlorine atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class SX679") .

A compound (L-3) wherein $R^{1b}$ and $R^{1c}$ represent a chlorine atom, $A^2$ represents a nitrogen atom, $A^1$, $A^3$, and $A^4$ represent CH, and $A^5$ represents any one substituent indicated in [Table L1] to [Table L3] (hereinafter, referred to as "Compound class SX680").

A compound (L-3) wherein $R^{1b}$ and $R^{1c}$ represent a chlorine atom, $A^2$ represents a nitrogen atom, $A^1$, $A^3$, and $A^5$ represent CH, and $A^4$ represents any one substituent indicated in [Table L1] to [Table L3] (hereinafter, referred to as "Compound class SX681").

A compound (L-3) wherein $R^{1b}$ and $R^{1c}$ represent a chlorine atom, $A^2$ represents a nitrogen atom, $A^1$, $A^4$, and $A^5$ represent CH, and $A^3$ represents any one substituent indicated in [Table L1] to [Table L3] (hereinafter, referred to as "Compound class SX682") .

A compound (L-3) wherein $R^{1b}$ and $R^{1c}$ represent a chlorine atom, $A^2$ represents a nitrogen atom, $A^3$, $A^4$, and $A^5$ represent CH, and $A^1$ represents any one substituent indicated in [Table L1] to [Table L3] (hereinafter, referred to as "Compound class SX683").

A present compound (L-3) wherein $R^{1b}$ represents a chlorine atom, $R^{1c}$ represents any one group selected from a hydrogen atom, a fluorine atom, a chlorine atom, a bromine atom, an iodine atom, a methyl group, an ethyl group, a propyl group, an isopropyl group, a cyclopropyl group, or $CF_3$, and $A^1$, $A^2$, $A^3$, $A^4$, and $A^5$ represent CH (hereinafter, referred to as "Compound class SX684").

A compound represented by formula (L-4):

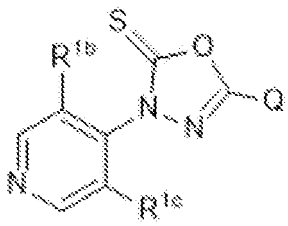

(L-4)

(hereinafter, referred to as "Compound (L-4)")
wherein $R^{1b}$ and $R^{1c}$ represent a chlorine atom, and Q represents any one substituent indicated in [Table L4] to [Table L9] (hereinafter, referred to as "Compound class SX685").

A compound represented by formula (L-5):

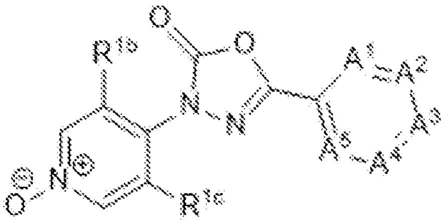

(L-5)

(hereinafter, referred to as "Compound (L-5)")
wherein $R^{1b}$ and $R^{1c}$ represent a chlorine atom, and $A^2$, $A^3$, $A^4$, and $A^5$ represent CH, and $A^1$ represents any one substituent indicated in [Table L4] to [Table L9] (hereinafter, referred to as "Compound class SX686").

A compound (L-5) wherein $R^{1b}$ and $R^{1c}$ represent a chlorine atom, and $A^1$, $A^3$, $A^4$, and $A^5$ represent CH, and $A^2$ represents any one substituent indicated in [Table L1] to [Table L3] (hereinafter, referred to as "Compound class SX687").

A compound (L-5) wherein $R^{1b}$ and $R^{1c}$ represent a chlorine atom, and $A^1$, $A^2$, $A^4$, and $A^5$ represent CH, and $A^3$ represents any one substituent indicated in [Table L1] to [Table L3] (hereinafter, referred to as "Compound class SX688").

A compound (L-5) wherein $R^{1b}$ and $R^{1c}$ represent a chlorine atom, $A^2$ represents C-F, $A^1$, $A^3$, and $A^5$ represent CH, and $A^4$ represents any one substituent indicated in [Table L1] to [Table L3] (hereinafter, referred to as "Compound class SX689") .

A compound (L-5) wherein $R^{1b}$ and $R^{1c}$ represent a chlorine atom, $A^2$ represents C-Cl, $A^2$, $A^3$, and $A^5$ represent CH, and $A^4$ represents any one substituent indicated in [Table L1] to [Table L3] (hereinafter, referred to as "Compound class SX690").

A compound (L-5) wherein $R^{1b}$ and $R^{1c}$ represent a chlorine atom, $A^2$ represents C-Br, $A^1$, $A^3$, and $A^5$ represent CH, and $A^4$ represents any one substituent indicated in [Table L1] to [Table L3] (hereinafter, referred to as "Compound class SX691").

A compound (L-5) wherein $R^{1b}$ and $R^{1c}$ represent a chlorine atom, $A^2$ represents C-I, $A^1$, $A^3$, and $A^5$ represent CH, and $A^4$ represents any one substituent indicated in [Table L1] to [Table L3] (hereinafter, referred to as "Compound class SX692").

A compound (L-5) wherein $R^{1b}$ and $R^{1c}$ represent a chlorine atom, $A^2$ represents C-Me, $A^1$, $A^3$, and $A^5$ represent CH, and $A^4$ represents any one substituent indicated in [Table L1] to [Table L3] (hereinafter, referred to as "Compound class SX693").

A compound (L-5) wherein $R^{1b}$ and $R^{1c}$ represent a chlorine atom, $A^2$ represents C-QMe, $A^1$, $A^3$, and $A^5$ represent CH, and $A^4$ represents any one substituent indicated in [Table L1] to [Table L3] (hereinafter, referred to as "Compound class SX694") .

A compound (L-5) wherein $R^{1b}$ and $R^{1c}$ represent a chlorine atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class SX695").

A compound (L-5) wherein $A^{1b}$ and $R^{1c}$ represent a chlorine atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class SX696").

A compound (L-5) wherein $R^{1b}$ and $R^{1c}$ represent a chlorine atom, $A^3$ represents C-F, $A^1$, $A^2$, and $A^5$ represent CH, and $A^4$ represents any one substituent indicated in [Table L1] to [Table L3] (hereinafter, referred to as "Compound class SX697").

A compound (L-5) wherein $R^{1b}$ and $R^{1c}$ represent a chlorine atom, $A^3$ represents C-Cl, $A^1$, $A^2$, and $A^5$ represent CH, and $A^4$ represents any one substituent indicated in [Table L1] to [Table L3] (hereinafter, referred to as "Compound class SX69S").

A compound (L-5) wherein $R^{1b}$ and $R^{1c}$ represent a chlorine atom, $A^3$ represents C-Br, $A^1$, $A^2$, and $A^5$ represent CH, and $A^4$ represents any one substituent indicated in [Table L1] to [Table L3] (hereinafter, referred to as "Compound class SX699") .

A compound (L-5) wherein $R^{1b}$ and $R^{1c}$ represent a chlorine atom, $A^3$ represents C-I, $A^1$, $A^2$, and $A^5$ represent CH, and $A^4$ represents any one substituent indicated in [Table L1] to [Table L3] (hereinafter, referred to as "Compound class SX700").

A compound (L-5) wherein $R^{1b}$ and $R^{1c}$ represent a chlorine atom, $A^3$ represents C-Me, $A^1$, $A^2$, and $A^5$ represent CH, and $A^4$ represents any one substituent indicated in [Table L1] to [Table L3] (hereinafter, referred to as "Compound class SX701").

A compound (L-5) wherein $R^{1b}$ and $R^{1c}$ represent a chlorine atom, $A^3$ represents C-OMe, $A^1$, $A^2$, and $A^5$ represent CH, and $A^4$ represents any one substituent indicated in [Table L1] to [Table L3] (hereinafter, referred to as "Compound class SX702").

A compound (L-5) wherein $R^{1b}$ and $R^{1c}$ represent a chlorine atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class SX703").

A compound (L-5) wherein $R^{1b}$ and $R^{1c}$ represent a chlorine atom, $A^3$ represents C-$OCF_3$, $A^1$, $A^2$, and $A^3$ represent CH, and $A^4$ represents any one substituent indicated in [Table L1] to [Table L3] (hereinafter, referred to as "Compound class SX704").

A compound (L-5) wherein $R^{1b}$ and $R^{1c}$ represent a chlorine atom, $A^2$ represents a nitrogen atom. $A^1$, $A^3$, and $A^4$ represent CH, and $A^5$ represents any one substituent indicated in [Table L1] to [Table L3] (hereinafter, referred to as "Compound class SX705").

A compound (L-5) wherein $R^{1b}$ and $R^{1c}$ represent a chlorine atom, $A^2$ represents a nitrogen atom, $A^1$, $A^3$, and $A^5$ represent CH, and $A^4$ represents any one substituent indicated in [Table L1] to [Table L3] (hereinafter, referred to as "Compound class SX706").

A compound (L-5) wherein $R^{1b}$ and $R^{1c}$ represent a chlorine atom, $A^2$ represents a nitrogen atom, $A^1$, $A^4$, and $A^5$ represent CH, and $A^3$ represents any one substituent indicated in [Table L1] to [Table L3] (hereinafter, referred to as "Compound class SX707").

A compound (L-5) wherein $R^{1b}$ and $R^{1c}$ represent a chlorine atom, $A^2$ represents a nitrogen atom, $A^3$, $A^4$, and $A^5$ represent CH, and $A^1$ represents any one substituent indicated in [Table L1] to [Table L3] (hereinafter, referred to as "Compound class SX708").

A present compound (L-5) wherein $R^{1b}$ represents a chlorine atom, $R^{1c}$ represents any one group selected from a hydrogen atom, a fluorine atom, a chlorine atom, a bromine atom, an iodine atom, a methyl group, an ethyl group, a propyl group, an isopropyl group, a cyclopropyl group, or $CF_3$, and $A^1$, $A^2$, $A^3$, $A^4$, and $A^5$ represent CH (hereinafter, referred to as "Compound class SX709").

A compound represented by formula (L-6):

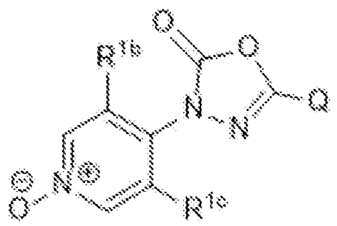

(L-6)

(hereinafter, referred to as "Compound (L-6)")
wherein $R^{1b}$ and $R^{1c}$ represent a chlorine atom, and Q represents any one substituent indicated in [Table L4] to [Table L9] (hereinafter, referred to as "Compound class SX710") .

A compound represented by formula (K-1):

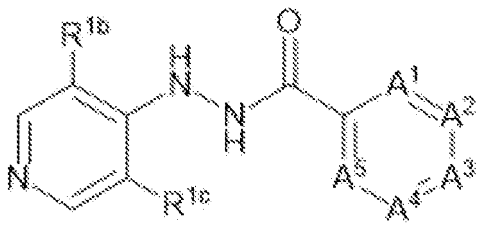

(K-1)

(hereinafter, referred to as "Compound (K-1)")
wherein $R^{1b}$ represents a fluorine atom, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class CX1") .

A compound (K-1) wherein $R^{1b}$ and $R^{1c}$ represent a fluorine atom, $A^2$, $A^3$, $A^4$, and $A^5$ represent CH, and $A^1$ represents any one substituent indicated in [Table L1] to [Table L3] (hereinafter, referred to as "Compound class CX2").

A compound (K-1) wherein $R^{1b}$ represents a chlorine atom, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class CX3").

A compound (K-1) wherein $R^{1b}$ and $R^{1c}$ represent a chlorine atom, $A^2$, $A^3$, $A^4$, and $A^5$ represent CH, and $A^1$ represents any one substituent indicated in [Table L1] to [Table L3] (hereinafter, referred to as "Compound class CX4").

A compound (K-1) wherein $R^{1b}$ represents a bromine atom, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class CX5").

A compound (K-1) wherein $R^{1b}$ and $R^{1c}$ represent a bromine atom, $A^2$, $A^3$, $A^4$, and $A^5$ represent CH, and $A^1$ represents any one substituent indicated in [Table L1] to [Table L3] (hereinafter, referred to as "Compound class CX6").

A compound (K-1) wherein $R^{1b}$ represents an iodine atom, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class CX7").

A compound (K-1) wherein $R^{1b}$ and $R^{1c}$ represent an iodine atom, $A^2$, $A^3$, $A^4$, and $A^5$ represent CH, and $A^1$ represents any one substituent indicated in [Table L1] to [Table L3] (hereinafter, referred to as "Compound class CX8").

A compound (K-1) wherein $R^{1b}$ represents a methyl group, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class CX9").

A compound (K-1) wherein $R^{1b}$ and $R^{1c}$ represent a methyl group, $A^2$, $A^3$, $A^4$, and $A^5$ represent CH, and $A^1$ represents any one substituent indicated in [Table L1] to [Table L3] (hereinafter, referred to as "Compound class CX10").

A compound (K-1) wherein $R^{1b}$ represents an ethyl group, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class CX11").

A compound (K-1) wherein $R^{1b}$ and $R^{1c}$ represent an ethyl group, $A^2$, $A^3$, $A^4$, and $A^5$ represent CH, and $A^1$ represents any one substituent indicated in [Table L1] to [Table L3] (hereinafter, referred to as "Compound class CX12").

A compound (K-1) wherein $R^{1b}$ represents a propyl group, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class CX13").

A compound (K-1) wherein $R^{1b}$ and $R^{1c}$ represent a propyl group, $A^2$, $A^3$, $A^4$, and $A^5$ represent CH, and $A^1$ represents any one substituent indicated in [Table L1] to [Table L3] (hereinafter, referred to as "Compound class CX14").

A compound (K-1) wherein $R^{1b}$ represents an isopropyl group, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class CX15").

A compound (K-1) wherein $R^{1b}$ and $R^{1c}$ represent an isopropyl group, $A^2$, $A^3$, $A^4$, and $A^5$ represent CH, and $A^1$ represents any one substituent indicated in [Table L1] to [Table L3] (hereinafter, referred to as "Compound class CX16") .

A compound (K-1) wherein $R^{1b}$ represents a cyclopropyl group, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class CX17").

A compound (K-1) wherein $R^{1b}$ and $R^{1c}$ represent a cyclopropyl group, $A^2$, $A^3$, $A^4$, and $A^5$ represent CH, and $A^1$ represents any one substituent indicated in [Table L1] to [Table L3] (hereinafter, referred to as "Compound class CX18"),

A compound (K-1) wherein $R^{1b}$ represents $CF_3$, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound

class CX19").

A compound (R-1) wherein $R^{1b}$ and $R^{1c}$ represent $CF_3$, $A^2$, $A^3$, $A^4$, and $A^5$ represent CH, and $A^1$ represents any one substituent indicated in [Table L1] to [Table L3] (hereinafter, referred to as "Compound class CX20").

A compound (K-1) wherein $R^{1b}$ represents a methyl sulfanyl group, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class CX21").

A compound (K-1) wherein $R^{1b}$ represents a methylsulfinyl group, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class CX22").

A compound (R-1) wherein $R^{1b}$ represents a methylsulfonyl group, $R^{1c}$ represents a hydrogen atom, $A^2$, $A^3$, $A^4$, and $A^5$ represent CH, and $A^1$ represents any one substituent indicated in [Table L1] to [Table L3] (hereinafter, referred to as "Compound class. CX23").

A compound (K-1) wherein $R^{1b}$ represents a chlorine atom, $R^{1c}$ represents a bromine atom, $A^2$, $A^3$, $A^4$, and $A^5$ represent CH, and $A^1$ represents any one substituent indicated in [Table L1) to [Table L3] (hereinafter, referred to as "Compound class CX24"),

A compound (K-1) wherein $R^{1b}$ represents a chlorine atom, $R^{1c}$ represents an iodine atom. $A^2$, $A^3$, $A^4$, and $A^5$ represent CH, and $A^1$ represents any one substituent indicated in [Table L1] to [Table L3] (hereinafter, referred to as "Compound class CX25").

A compound (K-1) wherein $R^{1b}$ represents a chlorine atom, $R^{1c}$ represents a methyl group, $A^2$, $A^3$, $A^4$, and $A^5$ represent CH, and $A^1$ represents any one substituent indicated in [Table L1] to [Table L3] (hereinafter, referred to as "Compound class CX26").

A compound (K-1) wherein $R^{1b}$ represents a chlorine atom, $R^{1c}$ represents an ethyl group, $A^2$, $A^4$, $A^4$, and $A^5$ represent CH, and $A^1$ represents any one substituent indicated in [Table L1] to [Table L3] (hereinafter, referred to as "Compound class CX27").

A compound (K-1) wherein $R^{1b}$ represents a chlorine atom, $R^{1c}$ represents a propyl group, $A^2$, $A^3$, $A^4$, and $A^5$ represent CH, and $A^1$ represents any one substituent indicated in [Table L1] to [Table L3] (hereinafter, referred to as "Compound class CX28").

A compound (K-1) wherein $R^{1b}$ represents a chlorine atom, $R^{1c}$ represents an isopropyl group, $A^2$, $A^3$, $A^4$, and $A^5$ represent CH, and $A^1$ represents any one substituent indicated in [Table L1] to [Table L3] (hereinafter, referred to as "Compound class CX29").

A compound (K-1) wherein $R^{1b}$ represents a chlorine atom, $R^{1c}$ represents a cycloptopyl group, $A^4$, $A^3$, $A^4$, and $A^5$ represent CH, and $A^1$ represents any one substituent indicated in [Table. L1] to [Table L3] (hereinafter, referred to as "Compound class CX30").

A compound (K-1) wherein $R^{1b}$ represents a chlorine atom, $R^{1c}$ represents $CF_3$, $A^2$, $A^3$, $A^4$, and $A^5$ represent CH, and $A^1$ represents any one substituent indicated in [Table L1] to [Table L3] (hereinafter, referred to as "Compound class. CX31").

A compound (K-1) wherein $R^{1b}$ represents a bromine atom, $R^{1c}$ represents an iodine atom, $A^2$, $A^3$, $A^4$, and $A^5$ represent CH, and $A^1$ represents any one substituent indicated in [Table L1] to [Table L3] (hereinafter, referred to as "Compound class CX32").

A compound (K-1) wherein $R^{1b}$ represents a bromine atom, $R^{1c}$ represents a methyl group, $A^2$, $A^3$, $A^4$, and $A^5$ represent CH, and $A^1$ represents any one substituent indicated in [Table L1] to [Table L3] (hereinafter, referred to as "Compound class CX33").

A compound (K-1) wherein $R^{1b}$ represents a bromine atom, $R^{1c}$ represents an ethyl group, $A^2$, $A^3$, $A^4$, and $A^5$ represent CH, and $A^1$ represents any one substituent indicated in [Table L1] to [Table L3] (hereinafter, referred to as "Compound class CX34").

A compound (K-1) wherein $R^{1b}$ represents a bromine atom, $R^{1c}$ represents a propyl group, $A^2$, $A^3$, $A^4$, and $A^5$ represent CH, and $A^1$ represents any one substituent indicated in [Table L1] to [Table L3] (hereinafter, referred to as "Compound class CX35").

A compound (K-1) wherein $R^{1b}$ represents a bromine atom, $R^{1c}$ represents an isopropyl group, $A^2$, $A^3$, $A^4$, and $A^5$ represent CH, and $A^1$ represents any one substituent indicated in [Table L1] to [Table L3] (hereinafter, referred to as "Compound class CX36").

A compound (R-1) wherein $R^{1b}$ represents a bromine atom, $R^{1c}$ represents a cyclopropyl group, $A^2$, $A^3$, $A^4$, and $A^5$ represent CH, and $A^1$ represents any one substituent indicated in [Table L1] to [Table L3] (hereinafter, referred to as "Compound class CX37").

A compound (K-1) wherein $R^{1b}$ represents a bromine atom, $R^{1c}$ represents $CF_3$, $A^2$, $A^3$, $A^4$, and $A^5$ represent CH, and $A^1$ represents any one substituent indicated in [Table L1] to [Table L3] (hereinafter, referred to as "Compound class CX38").

A compound (K-1) wherein $R^{1b}$ represents a methyl group, $R^{1c}$ represents an ethyl group, $A^2$, $A^3$, $A^4$, and $A^5$

represent CH, and $A^1$ represents any one substituent indicated in [Table L1] to [Table L3] (hereinafter, referred to as "Compound class CX39").

A compound (K-1) wherein $R^{1b}$ represents a methyl group, $R^{1c}$ represents a propyl group, $A^2$, $A^3$, $A^4$, and $A^5$ represent CH, and $A^1$ represents any one substituent indicated in [Table L1] to [Table L3] (hereinafter, referred to as "Compound class CX40").

A compound (K-1) wherein $R^{1b}$ represents an ethyl group, $R^{1c}$ represents a propyl group, $A^2$, $A^3$, $A^4$, and $A^5$ represent CH, and $A^1$ represents any one substituent indicated in [Table L1] to [Table L3] (hereinafter, referred to as "Compound class CX41").

A compound (K-1) wherein $R^{1b}$ represents a fluorine atom, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class CX42").

A compound (K-1) wherein $R^{1b}$ and $R^{1c}$ represent a fluorine atom, $A^1$, $A^3$, $A^4$, and $A^5$ represent CH, and $A^2$ represents any one substituent indicated in [Table L1] to [Table L3] (hereinafter, referred to as "Compound class CX43").

A compound (K-1) wherein $R^{1b}$ represents a chlorine atom, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class CX44").

A compound (K-1) wherein $R^{1b}$ and $R^{1c}$ represent a chlorine atom, $A^1$, $A^3$, $A^4$, and $A^5$ represent CH, and $A^2$ represents any one substituent indicated in [Table L1] to [Table L3] (hereinafter, referred to as "Compound class CX45").

A compound (K-1) wherein $R^{1b}$ represents a bromine atom, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class CX46").

A compound (K-1) wherein $R^{1b}$ and $R^{1c}$ represent a bromine atom, $A^1$, $A^3$, $A^4$, and $A^5$ represent CH, and $A^2$ represents any one substituent indicated in [Table L1] to [Table L3] (hereinafter, referred to as "Compound class CX47").

A compound (K-1) wherein $R^{1b}$ represents an iodine atom, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class CX48").

A compound (K-1) wherein $R^{1b}$ and $R^{1c}$ represent an iodine atom, $A^1$, $A^3$, $A^4$, and $A^5$ represent CH, and $A^2$ represents any one substituent indicated in [Table L1] to [Table L3] (hereinafter, referred to as "Compound class CX49").

A compound (K-1) wherein $R^{1b}$ represents a methyl group, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class CX50") .

A compound (K-1) wherein $R^{1b}$ and $R^{1c}$ represent a methyl group, $A^1$, $A^3$, $A^4$, and $A^5$ represent CH, and $A^2$ represents any one substituent indicated in [Table L1] to [Table L3] (hereinafter, referred to as "Compound class CX51").

A compound (K-1) wherein $R^{1b}$ represents an ethyl group, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class CX52").

A compound (K-1) wherein $R^{1b}$ and $R^{1c}$ represent an ethyl group, $A^1$, $A^3$, $A^4$, and $A^5$ represent CH, and $A^2$ represents any one substituent indicated in [Table L1] to [Table L3] (hereinafter, referred to as "Compound class CX53").

A compound (K-1) wherein $R^{1b}$ represents a propyl group, $R^{1c}$ represents a hydrogen atom, $A^1$, $A^3$, $A^4$, and $A^5$. represent CH, and $A^2$ represents any one substituent indicated in [Table L1] to [Table L3] (hereinafter, referred to as "Compound class CX54").

A compound (K-1) wherein $R^{1b}$ and $R^{1c}$ represent a propyl group, $A^1$, $A^3$, $A^4$, and $A^5$ represent CH, and $A^2$ represents any one substituent indicated in [Table L1] to [Table L3] (hereinafter, referred to as "Compound class CX55").

A compound (K-1) wherein $R^{1b}$ represents an isopropyl group, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class CX56"),

A compound (K-1) wherein $R^{1b}$ and $R^{1c}$ represent an isopropyl group, $A^1$, $A^3$, $A^4$, and $A^5$ represent CH, and $A^2$ represents any one substituent indicated in [Table L1] to [Table L3] (hereinafter, referred to as "Compound class CX57").

A compound (K-1) wherein $R^{1b}$ represents a cyclopropyl group, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class CX58").

A compound (K-1) wherein $R^{1b}$ and $R^{1c}$ represent a cyclopropyl group, $A^1$, $A^3$, $A^4$, and $A^5$ represent CH, and $A^2$ represents any one substituent indicated in [Table L1] to [Table L3] (hereinafter, referred to as "Compound class CX59").

A compound (K-1) wherein $R^{1b}$ represents $CF_3$, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class CX60").

A compound (K-1) wherein $R^{1b}$ and $R^{1c}$ represent $CF_3$, $A^1$, $A^3$, $A^4$, and $A^5$ represent CH, and $A^2$ represents any one substituent indicated in [Table L1] to [Table L3] (hereinafter, referred to as "Compound class CX61").

A compound (K-1) wherein $R^{1b}$ represents a methylsulfanyl group, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class. CX62").

A compound (K-1) wherein $R^{1b}$ represents a methylsulfinyl group, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class CX63").

A compound (K-1) wherein $R^{1b}$ represents a methylsulfonyl group, $R^{1c}$ represents a hydrogen atom, $A^1$, $A^3$, $A^4$, and $A^5$ represent CH, and $A^2$ represents any one substituent indicated in [Table L1] to [Table L3] (hereinafter, referred to as "Compound class CX64").

A compound (K-1) wherein $R^{1b}$ represents a chlorine atom, $R^{1c}$ represents a bromine atom, $A^1$, $A^3$, $A^4$, and $A^5$ represent CH, and $A^2$ represents any one substituent indicated in [Table L1] to [Table L3] (hereinafter, referred to as "Compound class CX65").

A compound (K-1) wherein $R^{1b}$ represents a chlorine atom, $R^{1c}$ represents an iodine atom, $A^1$, $A^3$, $A^4$, and $A^5$ represent CH, and $A^2$ represents any one substituent indicated in [Table L1] to [Table L3] (hereinafter, referred to as "Compound class CX66").

A compound (K-1) wherein $R^{1b}$ represents a chlorine atom, $R^{1c}$ represents a methyl group, $A^1$, $A^3$, $A^4$, and $A^5$ represent CH, and $A^2$ represents any one substituent indicated in [Table L1] to [Table L3] (hereinafter, referred to as "Compound class CX67").

A compound (K-1) wherein $R^{1b}$ represents a chlorine atom, $R^{1c}$ represents an ethyl group, $A^1$, $A^3$, $A^4$, and $A^5$ represent CH, and $A^2$ represents any one substituent indicated in [Table L1] to [Table L3] (hereinafter, referred to as "Compound class GX68").

A compound (K-1) wherein $R^{1b}$ represents a chlorine atom, $R^{1c}$ represents a propyl group, $A^1$, $A^3$, $A^4$, and $A^5$ represent CH, and $A^2$ represents any one substituent indicated in [Table L1] to [Table L3] (hereinafter, referred to as "Compound class CX69").

A compound (K-1) wherein $R^{1b}$ represents a chlorine atom, $R^{1c}$ represents an isopropyl group, $A^1$, $A^3$, $A^4$, and $A^5$ represent CH, and $A^2$ represents any one substituent indicated in [Table L1] to [Table L3] (hereinafter, referred to as "Compound class CX70").

A compound (K-1) wherein $R^{1b}$ represents a chlorine atom, $R^{1c}$ represents a cyclopropyl group, $A^1$, $A^3$, $A^4$, and $A^5$ represent CH, and $A^2$ represents any one substituent indicated in [Table L1] to [Table L3] (hereinafter, referred to as "Compound class CX71").

A compound (K-1) wherein $R^{1b}$ represents a chlorine atom, $R^{1c}$ represents $CF_3$, $A^1$, $A^3$, $A^4$, and $A^5$ represent CH, and $A^2$ represents any one substituent indicated in [Table L1] to [Table L3] (hereinafter, referred to as "Compound class CX72").

A compound (K-1) wherein $R^{1b}$ represents a bromine atom, $R^{1c}$ represents an iodine atom, $A^1$, $A^3$, $A^4$, and $A^5$ represent CH, and $A^2$ represents any one substituent indicated in [Table L1] to [Table L3] (hereinafter, referred to as "Compound class CX73").

A compound (K-1) wherein $R^{1b}$ represents a bromine atom, $R^{1c}$ represents a methyl group, $A^1$, $A^3$, $A^4$, and $A^5$ represent CH, and $A^2$ represents any one substituent indicated in [Table L1] to [Table L3] (hereinafter, referred to as "Compound class CX74").

A compound (K-1) wherein $R^{1b}$ represents a bromine atom, $R^{1c}$ represents an ethyl group, $A^1$, $A^3$, $A^4$, and $A^5$ represent CH, and $A^2$ represents any one substituent indicated in [Table L1] to [Table L3] (hereinafter, referred to as "Compound class CX75").

A compound (R-1) wherein $R^{1b}$ represents a bromine atom, $R^{1c}$ represents a propyl group, $A^1$, $A^3$, $A^4$, and $A^5$ represent CH, and $A^2$ represents any one substituent indicated in [Table L1] to [Table L3] (hereinafter, referred to as "Compound class CX76").

A compound (K-1) wherein $R^{1b}$ represents a bromine atom, $R^{1c}$ represents an isopropyl group, $A^1$, $A^3$, $A^4$, and $A^5$ represent CH, and $A^2$ represents any one substituent indicated in [Table L1] to [Table L3] (hereinafter, referred to as "Compound class CX77").

A compound (K-1) wherein $R^{1b}$ represents a bromine atom, $R^{1c}$ represents a cyclopropyl group, $A^1$, $A^3$, $A^4$, and $A^5$ represent CH, and $A^2$ represents any one substituent indicated in [Table L1] to [Table L3] (hereinafter, referred to as "Compound class CX78").

A compound (K-1) wherein $R^{1b}$ represents a bromine atom, $R^{1c}$ represents $CF_3$, $A^1$, $A^3$, $A^4$, and $A^5$ represent CH, and $A^2$ represents any one substituent indicated in [Table L1] to [Table L3] (hereinafter, referred to as "Compound class CX79").

A compound (K-1) wherein $R^{1b}$ represents a methyl group, $R^{1c}$ represents an ethyl group, $A^1$, $A^3$, $A^4$, and $A^5$ represent CH, and $A^2$ represents any one substituent indicated in [Table L1] to [Table L3] (hereinafter, referred to as

"Compound class CX80").

A compound (R-1) wherein $R^{1b}$ represents a methyl group, $R^{1c}$ represents a propyl group, $A^1$, $A^3$, $A^4$, and $A^5$ represent CH, and $A^2$ represents any one substituent indicated in [Table L1] to [Table L3] (hereinafter, referred to as "Compound class CX81").

A compound (K-1) wherein $R^{1b}$ represents an ethyl group, $R^{1c}$ represents a propyl group, $A^1$, $A^3$, $A^4$, and $A^5$ represent CH, and $A^2$ represents any one substituent indicated in [Table L1] to [Table L3] (hereinafter, referred to as "Compound class CX82").

A compound (K-1) wherein $R^{1b}$ represents a fluorine atom, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class CX83") .

A compound (K-1) wherein $R^{1b}$ and $R^{1c}$ represent a fluorine atom, $A^1$, $A^2$, $A^4$, and $A^5$ represent CH, and $A^3$ represents any one substituent indicated in [Table L1] to [Table L3] (hereinafter, referred to as "Compound class CX84").

A compound (K-1) wherein $R^{1b}$ represents a chlorine atom, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class CX85").

A compound (K-1) wherein $R^{1b}$ and $R^{1c}$ represent a chlorine atom, $A^1$, $A^2$, $A^4$, and $A^5$ represent CH, and $A^3$ represents any one substituent indicated in [Table L1] to [Table L3] (hereinafter, referred to as "Compound class CX86").

A compound (K-1) wherein $R^{1b}$ represents a bromine atom, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class CX87").

A compound (K-1) wherein $R^{1b}$ and $R^{1c}$ represent a bromine atom, $A^1$, $A^2$, $A^4$, and $A^5$ represent CH, and $A^3$ represents any one substituent indicated in [Table L1] to [Table L3] (hereinafter, referred to as "Compound class CX88").

A Compound (K-1) wherein $R^{1b}$ represents an iodine atom, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class CX89").

A compound (K-1) wherein $R^{1b}$ and $R^{1c}$ represent an iodine atom, $A^1$, $A^2$, $A^4$, and $A^5$ represent CH, and $A^3$ represents any one substituent indicated in [Table L1] to [Table L3] (hereinafter, referred to as "Compound class CX9D").

A compound (K-1) wherein $R^{1b}$ represents a methyl group, $R^{1c}$ represents a hydrogen atom, $A^1$, $A^2$, $A^4$, and $A^5$ represent CH, and $A^3$ represents any one substituent indicated in [Table L1) to [Table L3] (hereinafter, referred to as "Compound class CX91").

A compound (K-1) wherein $R^{1b}$ and $R^{1c}$ represent a methyl group, $A^1$, $A^2$, $A^4$, and $A^5$ represent CH, and $A^3$ represents any one substituent indicated in [Table L1] to [Table L3] (hereinafter, referred to as "Compound class CX92").

A compound (K-1) wherein $R^{1b}$ represents an ethyl group, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class CX93").

A compound (K-1), wherein $R^{1b}$ and $R^{1c}$ represent an ethyl group, $A^1$, $A^2$, $A^4$, and $A^5$ represent CH, and $A^3$ represents any one substituent indicated in [Table L1] to [Table L3] (hereinafter, referred to as "Compound class CX94").

A compound (K-1) wherein $R^{1b}$ represents a propyl group, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class CX95").

A compound (K-1) wherein $R^{1b}$ and $R^{1c}$ represent a propyl group, $A^1$, $A^2$, $A^4$, and $A^5$ represent CH, and $A^3$ represents any one substituent indicated in [Table L1] to [Table L3] (hereinafter, referred to as "Compound class CX96").

A compound (K-1) wherein $R^{1b}$ represents an isopropyl group, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class CX97").

A compound (K-1) wherein $R^{1b}$ and $R^{1c}$ represent an isopropyl group, $A^1$, $A^2$, $A^4$, and $A^5$ represent CH, and $A^3$ represents any one substituent indicated in [Table L1] to [Table L3] (hereinafter, referred to as "Compound class CX98"),

A compound (K-1) wherein $R^{1b}$ represents a cyclopropyl group, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class CX99").

A compound (K-1) wherein $R^{1b}$ and $R^{1c}$ represent a cyclopropyl group, $A^1$, $A^2$, $A^4$, and $A^5$ represent CH, and $A^3$ represents any one substituent indicated in [Table L1] to [Table L3] (hereinafter, referred to as "Compound class CX100").

A compound (K-1) wherein $R^{1b}$ represents $CF_3$, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as *Compound class CX101").

A compound (K-1) wherein $R^{1b}$ and $R^{1c}$ represent $CF_3$, $A^1$, $A^2$, $A^4$, and $A^5$ represent CH, and $A^3$ represents

any one substituent indicated in [Table L1] to [Table L3] (hereinafter, referred to as "Compound class CX102") .

A compound (K-1) wherein $R^{1b}$ represents a methylsulfanyl group, $R^{1c}$ represents a hydrogen atom, $A^1$, $A^2$, $A^4$ and $A^5$ represent CH, and $A^3$ represents any one substituent indicated in [Table L1] to [Table L3] (hereinafter, referred to as "Compound class CX103").

A compound (K-1) wherein $R^{1b}$ represents a methylsulfinyl group, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class CX104").

A compound (K-1) wherein $R^{1b}$ represents a methylsulfonyl group, $R^{1c}$ represents a hydrogen atom, $A^1$, $A^2$, $A^4$, and $A^5$ represent CH, and $A^3$ represents any one substituent indicated in [Table L1] to [Table L3] (hereinafter, referred to as "Compound class CX105").

A compound (K-1) wherein $R^{1b}$ represents a chlorine atom, $R^{1c}$ represents a bromine atom, $A^1$, $A^2$, $A^4$, and $A^5$ represent CH, and $A^3$ represents any one substituent indicated in [Table L1] to [Table L3] (hereinafter, referred to as "Compound class CX106").

A compound (K-1) wherein $R^{1b}$ represents a chlorine atom, $R^{1c}$ represents an iodine atom, $A^1$, $A^2$, $A^4$, and $A^5$ represent CH, and $A^3$ represents any one substituent indicated in [Table L1] to [Table L3] (hereinafter, referred to as "Compound class CX107").

A compound (K-1) wherein $R^{1b}$ represents a chlorine atom, $R^{1c}$ represents a methyl group, $A^1$, $A^2$, $A^4$, and $A^5$ represent CH, and $A^3$ represents any one substituent indicated in [Table L1] to [Table L3] (hereinafter, referred to as "Compound class CX108").

A compound (K-1) wherein $R^{1b}$ represents a chlorine atom, $R^{1c}$ represents an ethyl group, $A^1$, $A^2$, $A^4$, and $A^5$ represent CH, and $A^3$ represents any one substituent indicated in [Table L1] to [Table L3] (hereinafter, referred to as "Compound class CX109") .

A compound (K-1) wherein $R^{1b}$ represents a chlorine atom, $R^{1c}$ represents a propyl group, $A^1$, $A^2$, $A^4$, and $A^5$ represent CH, and $A^3$ represents any one substituent indicated in [Table L1] to [Table L3] (hereinafter, referred to as "Compound class CX110").

A compound (K-1) wherein $R^{1b}$ represents a chlorine atom, $R^{1c}$ represents an isopropyl group, $A^1$, $A^2$, $A^4$, and $A^5$ represent CH, and $A^3$ represents any one substituent indicated in [Table L1] to [Table L3] (hereinafter, referred to as "Compound class CX111").

A compound (K-1) wherein $R^{1b}$ represents a chlorine atom, $R^{1c}$ represents a cyclopropyl group, $A^1$, $A^2$, $A^4$, and $A^5$ represent CH, and $A^3$ represents any one substituent indicated in [Table L1] to [Table L3] (hereinafter, referred to as "Compound class CX112"),

A compound (K-1) wherein $R^{1b}$ represents a chlorine atom, $R^{1c}$ represents $CF_3$, $A^1$, $A^2$, $A^4$, and $A^5$ represent CH, and $A^3$ represents any one substituent indicated in [Table L1] to [Table L3) (hereinafter, referred to as "Compound class CX113").

A compound (K-1) wherein $R^{1b}$ represents a bromine atom, $R^{1c}$ represents an iodine atom, $A^1$, $A^2$, $A^4$, and $A^5$ represent CH, and $A^3$ represents any one substituent indicated in [Table L1] to [Table L3] (hereinafter, referred to as "Compound class CX114").

A compound (K-1) wherein $R^{1b}$ represents a bromine atom, $R^{1c}$ represents a methyl group, $A^1$, $A^2$, $A^4$, and $A^5$ represent CH, and $A^3$ represents any one substituent indicated in [Table L1] to [Table L3] (hereinafter, referred to as "Compound class CX115").

A compound (K-1) wherein $R^{1b}$ represents a bromine atom, $R^{1c}$ represents an ethyl group, $A^1$, $A^2$, $A^4$, and $A^5$ represent CH, and $A^3$ represents any one substituent indicated in [Table L1] to [Table L3) (hereinafter, referred to as "Compound class CX116").

A compound (K-1) wherein $R^{1b}$ represents a bromine atom, $R^{1c}$ represents a propyl group, $A^1$, $A^2$, $A^4$, and $A^5$ represent CH, and $A^3$ represents any one substituent indicated in [Table L1] to [Table L3] (hereinafter, referred to as "Compound class CX117").

A compound (K-1) wherein $R^{1b}$ represents a bromine atom, $R^{1c}$ represents an isopropyl group, $A^1$, $A^2$, $A^4$, and $A^5$ represent CH, and $A^3$ represents any one substituent indicated in [Table L1] to [Table L3] (hereinafter, referred to as "Compound class CX118").

A compound (K-1) wherein $R^{1b}$ represents a bromine atom, $R^{1c}$ represents a cyclopropyl group, $A^1$, $A^2$, $A^4$, and $A^5$ represent CH, and $A^3$ represents any one substituent indicated in [Table L1] to [Table L3] (hereinafter, referred to as "Compound class CX119").

A compound (K-1) wherein $R^{1b}$ represents a bromine atom, $R^{1c}$ represents $CF_3$, $A^1$, $A^2$, $A^4$, and $A^5$ represent CH, and $A^3$ represents any one substituent indicated in [Table L1] to [Table L3] (hereinafter, referred to as "Compound class CX120").

A compound (K-1) wherein $R^{1b}$ represents a methyl group, $R^{1c}$ represents an ethyl group, $A^1$, $A^2$, $A^4$, and $A^5$ represent CH, and $A^3$ represents any one substituent indicated in [Table L1] to [Table L3] (hereinafter, referred to as "Compound class CX121"),

A compound (K-1) wherein $R^{1b}$ represents a methyl group, $R^{1c}$ represents a propyl group, $A^1$, $A^2$, $A^4$, and $A^5$ represent CH, and $A^3$ represents any one substituent indicated in [Table L1] to [Table L3] (hereinafter, referred to as "Compound class CX122").

A compound (K-1) wherein $R^{1b}$ represents an ethyl group, $R^{1c}$ represents a propyl group, $A^1$, $A^2$, $A^4$, and $A^5$ represent CH, and $A^3$ represents any one substituent indicated in [Table L1] to [Table L3] (hereinafter, referred to as "Compound. class CX123").

A compound (K-1) wherein $R^{1b}$ represents a fluorine atom, $R^{1c}$ represents a hydrogen atom, $A^2$ represents C-F, $A^1$, $A^3$, and $A^5$ represent CH, and $A^4$ represents any one substituent indicated in [Table L1] to [Table L3] (hereinafter, referred to as "Compound class CX124").

A compound (N-1) wherein $R^{1b}$ represents a fluorine atom, $R^{1c}$ represents a hydrogen atom, $A^2$ represents C-Cl, $A^1$, $A^3$, and $A^5$ represent CH, and $A^4$ represents any one substituent indicated in [Table L1] to [Table L3] (hereinafter, referred to as "Compound class CX125").

A compound (K-1) wherein $R^{1b}$ represents a fluorine atom, $R^{1c}$ represents a hydrogen atom, $A^2$ represents C-Br, $A^1$, $A^3$, and $A^5$ represent CH, and $A^4$ represents any one substituent indicated in [Table L1] to [Table L3] (hereinafter, referred to as "Compound class CX126").

A compound (K-1) wherein $R^{1b}$ represents a fluorine atom, $R^{1c}$ represents a hydrogen atom. $A^2$ represents C-I, $A^1$, $A^3$, and $A^5$ represent CH, and $A^4$ represents any one substituent indicated in [Table L1] to [Table L3] (hereinafter, referred to as "Compound class CX127").

A compound (K-1) wherein $R^{1b}$ represents a fluorine atom, $R^{1c}$ represents a hydrogen atom, $A^2$ represents C-Me, $A^1$, $A^3$, and $A^5$ represent CH, and $A^4$ represents any one. substituent indicated in [Table L1] to [Table L3] (hereinafter, referred to as "Compound class CX128").

A compound (K-1) wherein $R^{1b}$ represents a fluorine atom, $R^{1c}$ represents a hydrogen atom, $A^2$ represents C-OMe, $A^1$, $A^3$, and $A^5$ represent CH, and $A^4$ represents any one substituent indicated in [Table L1] to [Table L3] (hereinafter, referred to as "Compound class CX129").

A compound (N-1) wherein $R^{1b}$ represents a fluorine atom, $R^{1c}$ represents a hydrogen atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX130").

A compound (K-1) wherein $R^{1b}$ represents a fluorine atom, $R^{1c}$ represents a hydrogen atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class GX131").

A compound (K-1) wherein $R^{1b}$ represents a fluorine atom, $R^{1c}$ represents a hydrogen atom. $A^3$ represents C-F, $A^1$, $A^2$, and $A^5$ represent CH, and $A^4$ represents any one substituent indicated in [Table L1] to [Table L3] (hereinafter, referred to as "Compound class CX132").

A compound (K-1) wherein $R^{1b}$ represents a fluorine atom, $R^{1c}$ represents a hydrogen atom, $A^3$ represents C-C1, $A^1$, $A^2$, and $A^5$ represent CH, and $A^4$ represents any one substituent indicated in [Table L1] to [Table L3] (hereinafter, referred to as "Compound class CX133").

A compound (K-1) wherein $R^{1b}$ represents a fluorine atom, $R^{1c}$ represents a hydrogen atom, $A^3$ represents C-Br, $A^1$, $A^2$, and $A^5$ represent CH, and $A^4$ represents any one substituent indicated in [Table L1] to [Table L3] (hereinafter, referred to as "Compound class CX134").

A compound (N-1) wherein $R^{1b}$ represents a fluorine atom, $R^{1c}$ represents a hydrogen atom, $A^3$ represents C-I, $A^1$, $A^2$, and $A^5$ represent CH, and $A^4$ represents any one substituent indicated in [Table L1] to [Table L3] (hereinafter, referred to as "Compound class CX135").

A compound (K-1) wherein $R^{1b}$ represents a fluorine atom, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter, referred to as "Compound class CX136").

A compound (ΣC-41) wherein $R^{1b}$ represents a fluorine atom, $R^{1c}$ represents a hydrogen atom, $A^3$ represents C-QMe, $A^1$, $A^2$, and $A^5$ represent CH, and $A^4$ represents any one substituent indicated in [Table L1] to [Table L3] (hereinafter, referred to as "Compound class CX137").

A compound (K-1) wherein $R^{1b}$ represents a fluorine atom, $R^{1c}$ represents a hydrogen atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX138").

A compound (K-1) wherein $R^{1b}$ represents a fluorine atom, $R^{1c}$ represents a hydrogen atom, $A^3$ represents C-$OCF_3$, $A^1$, $A^3$, and $A^5$ represent CH, and $A^4$ represents any one substituent indicated in [Table L1] to [Table L3] (hereinafter, referred to as "Compound class CX139").

A compound (N-1) wherein $R^{1b}$ represents a chlorine atom, $R^{1c}$ represents a hydrogen atom, $A^2$ represents C-F, $A^1$, $A^3$, and $A^5$ represent CH, and $A^4$ represents any one substituent indicated in [Table L1] to [Table L3] (hereinafter, referred to as "Compound class CX140").

A compound (K-1) wherein $R^{1b}$ represents a chlorine atom, $R^{1c}$ represents a hydrogen atom, $A^2$ represents

C-Cl, $A^1$, $A^3$, and $A^5$ represent CH, and $A^4$ represents any one substituent indicated in [Table L1] to [Table L3] (hereinafter, referred to as "Compound class CX141").

A compound (K-1) wherein $R^{1b}$ represents a chlorine atom, $R^{1c}$ represents a hydrogen atom, $A^2$ represents C-Br, $A^1$, $A^3$, and $A^5$ represent CH, and $A^4$ represents any one substituent indicated in [Table L1] to [Table L3] (hereinafter, referred to as "Compound class CX142").

A compound (K-1) wherein $R^{1b}$ represents a chlorine atom, $R^{1c}$ represents a hydrogen atom, $A^2$ represents C-I, $A^1$, $A^3$, and $A^5$ represent CH, and $A^4$ represents any one substituent indicated in [Table L1] to [Table L3] (hereinafter, referred to as "Compound class CX1.43").

A compound (K-1) wherein $R^{1b}$ represents a chlorine atom, $R^{1c}$ represents a hydrogen atom, $A^2$ represents C-Me, $A^1$, $A^3$, and $A^5$ represent CH, and $A^4$ represents any one substituent indicated in [Table L1] to [Table L3] (hereinafter, referred to as "Compound class CX144").

A compound (N-1) wherein $R^{1b}$ represents a chlorine atom, $R^{1c}$ represents a hydrogen atom, $A^2$ represents C-OMe, $A^1$, $A^3$, and $A^5$ represent CH, and $A^4$ represents any one substituent indicated in [Table L1] to [Table L3] (hereinafter, referred to as "Compound class CX145").

A compound (K-1) wherein $R^{1b}$ represents a chlorine atom, $R^{1c}$ represents a hydrogen atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX146").

A compound (K-1) wherein $R^{1b}$ represents a chlorine atom, $R^{1c}$ represents a hydrogen atom, $A^2$ represents C-Q$CF_3$, $A^1$, $A^3$, and $A^5$ represent CH, and $A^4$ represents any one substituent indicated in [Table L1] to [Table L3] (hereinafter, referred to as "Compound class CX147").

A compound (K-1) wherein $R^{1b}$ represents a chlorine atom, $R^{1c}$ represents a hydrogen atom, $A^3$ represents C-F, $A^1$, $A^2$, and $A^5$ represent CH, and $A^4$ represents any one substituent indicated in [Table L1] to [Table L3] (hereinafter, referred to as "Compound class CX148").

A compound (K-1) wherein $R^{1b}$ represents a chlorine atom, $R^{1c}$ represents a hydrogen atom, $A^3$ represents C-Cl, $A^1$, $A^2$, and $A^5$ represent CH, and $A^4$ represents any one substituent indicated in [Table L1] to [Table L3] (hereinafter, referred to as "Compound class CX149").

A compound (N-1) wherein $R^{1b}$ represents a chlorine atom, $R^{1c}$ represents a hydrogen atom, $A^3$ represents C-Br, $A^1$, $A^2$, and $A^5$ represent CH, and $A^4$ represents any one substituent indicated in [Table L1] to [Table L3] (hereinafter, referred to as "Compound class CX150").

A compound (K-1) wherein $R^{1b}$ represents a chlorine atom, $R^{1c}$ represents a hydrogen atom, $A^3$ represents C-I, $A^1$, $A^2$, and $A^5$ represent CH, and $A^4$ represents any one substituent indicated in [Table L1] to [Table L3] (hereinafter, referred to as "Compound class CX151").

A compound (K-1) wherein $R^{1b}$ represents a chlorine atom, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter, referred to as "Compound class CX152").

A compound (K-1) wherein $R^{1b}$ represents a chlorine atom, $R^{1c}$ represents a hydrogen atom, $A^3$ represents C-OMe, $A^1$, $A^2$, and $A^5$ represent CH, and $A^4$ represents any one substituent indicated in [Table L1] to [Table L3] (hereinafter, referred to as "Compound class CX153").

A compound (K-1) wherein $R^{1b}$ represents a chlorine atom, $R^{1c}$ represents a hydrogen atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX154").

A compound (N-1) wherein $R^{1b}$ represents a chlorine atom, $R^{1c}$ represents a hydrogen atom, $A^3$ represents C-O$CF_3$, $A^1$, $A^2$, and $A^5$ represent CH, and $A^4$ represents any one substituent indicated in [Table L1] to [Table L3] (hereinafter, referred to as "Compound class CX155").

A compound (K-1) wherein $R^{1b}$ represents a bromine atom, $R^{1c}$ represents a hydrogen atom, $A^2$ represents C-F, $A^1$, $A^3$, and $A^5$ represent CH, and $A^4$ represents any one substituent indicated in [Table L1] to [Table L3] (hereinafter, referred to as "Compound class CX156").

A compound (K-1) wherein $R^{1b}$ represents a bromine atom, $R^{1c}$ represents a hydrogen atom, $A^2$ represents C-Cl, $A^1$, $A^3$, and $A^5$ represent CH, and $A^4$ represents any one substituent indicated in [Table L1] to [Table L3] (hereinafter, referred to as "Compound class CX157").

A compound (K-1) wherein $R^{1b}$ represents a bromine atom, $R^{1c}$ represents a hydrogen atom, $A^2$ represents C-Br, $A^1$, $A^3$, and $A^5$ represent CH, and $A^4$ represents any one substituent indicated in [Table L1] to [Table L3] (hereinafter, referred to as "Compound class CX158").

A compound (K-1) wherein $R^{1b}$ represents a bromine atom, $R^{1c}$ represents a hydrogen atom, $A^2$ represents C-I, $A^1$, $A^3$, and $A^5$ represent CH, and $A^4$ represents any one substituent indicated in [Table L1] to [Table L3] (hereinafter, referred to as "Compound class CX159").

A compound (K-1) wherein $R^{1b}$ represents a bromine atom, $R^{1c}$ represents a hydrogen atom, $A^2$ represents C-Me, $A^1$, $A^3$, and $A^5$ represent CH, and $A^4$ represents any one substituent indicated in [Table L1] to [Table L3] (hereinafter,

referred to as "Compound class CX160").

A compound (K-1) wherein $R^{1b}$ represents a bromine atom, $R^{1c}$ represents a hydrogen atom, $A^2$ represents C-OMe, $A^1$, $A^3$, and $A^5$ represent CH, and $A^4$ represents any one substituent indicated in [Table L1] to [Table L3] (hereinafter, referred to as "Compound class CX161").

A compound (K-1) wherein $R^{1b}$ represents a bromine atom, $R^{1c}$ represents a hydrogen atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX162").

A compound (R-1) wherein $R^{1b}$ represents a bromine atom, $R^{1c}$ represents a hydrogen atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX163").

A compound (K-1) wherein $R^{1b}$ represents a bromine atom, $R^{1c}$ represents a hydrogen atom, $A^3$ represents C-F, $A^1$, $A^2$, and $A^5$ represent CH, and $A^4$ represents any one substituent indicated in [Table L1] to [Table L3] (hereinafter, referred to as "Compound class CX164").

A compound (K-1) wherein $R^{1b}$ represents a bromine atom, $R^{1c}$ represents a hydrogen atom, $A^3$ represents C-Cl, $A^1$, $A^2$, and $A^5$ represent CH, and $A^4$ represents any one substituent indicated in [Table L1] to [Table L3] (hereinafter, referred. to as "Compound class CX165").

A compound (K-1) wherein $R^{1b}$ represents a bromine atom, $R^{1c}$ represents a hydrogen atom, $A^3$ represents C-Br, $A^1$, $A^2$, and $A^5$ represent CH, and $A^4$ represents any one substituent indicated in [Table 11] to [Table L3] (hereinafter, referred to as "Compound class CX166").

A compound (K-1) wherein $R^{1b}$ represents a bromine atom, $R^{1c}$ represents a hydrogen atom, $A^3$ represents C-I, $A^1$, $A^2$, and $A^5$ represent CH, and $A^4$ represents any one substituent indicated in [Table L1] to [Table L3] (hereinafter, referred to as "Compound class CX167").

A compound (K-1) wherein $R^{1b}$ represents a bromine atom, $R^{1c}$ represents a hydrogen atom, $A^3$ represents C-Me, $A^1$, $A^2$, and $A^5$ represent CH, and $A^4$ represents any one substituent indicated in [Table L1] to [Table. L3] (hereinafter, referred to as "Compound class CX168").

A compound (K-1) wherein $R^{1b}$ represents a bromine atom, $R^{1c}$ represents a hydrogen atom, $A^3$ represents C-OMe, $A^1$, $A^2$, and $A^5$ represent CH, and $A^4$ represents any one substituent indicated in [Table L1] to [Table L3] (hereinafter, referred to as "Compound class CX169").

A compound (K-1) wherein $R^{1b}$ represents a bromine atom, $R^{1c}$ represents a hydrogen atom, $A^3$ represents C-$CF_3$, $A^1$, $A^2$, and $A^5$ represent CH, and $A^4$ represents any one substituent indicated in [Table L1) to [Table L3] (hereinafter, referred. to as "Compound class CX170").

A compound (K-1) wherein $R^{1b}$ represents a bromine atom, $R^{1c}$ represents a hydrogen atom, $A^3$ represents C-$OCF_3$, $A^1$, $A^2$, and $A^5$ represent CH, and $A^4$ represents any one substituent indicated in [Table L1) to [Table L3] (hereinafter, referred to as "Compound class CX171").

A compound (R-1) wherein $R^{1b}$ represents an iodine atom, $R^{1c}$ represents a hydrogen atom, $A^2$ represents C-.F, $A^1$, $A^3$, and $A^5$ represent CH, and $A^4$ represents any one substituent indicated in [Table L1] to [Table L3] (hereinafter, referred to as "Compound class CX172").

A compound (N-1) wherein $R^{1b}$ represents an iodine atom, $R^{1c}$ represents a hydrogen atom, $A^2$ represents C-Cl, $A^1$, $A^3$, and $A^5$ represent CH, and $A^4$ represents any one substituent indicated in [Table L1] to [Table. L3] (hereinafter, referred to as "Compound class CX173").

A compound (K-1) wherein $R^{1b}$ represents an iodine atom, $R^{1c}$ represents a hydrogen atom, $A^2$ represents C-Br, $A^1$, $A^3$, and $A^5$ represent CH, and $A^4$ represents any one substituent indicated in [Table L1] to [Table L3] (hereinafter, referred to as "Compound class CX174").

A compound (K-1) wherein $R^{1b}$ represents an iodine atom, $R^{1c}$ represents a hydrogen atom, $A^2$ represents C-I, $A^1$, $A^3$, and $A^5$ represent CH, and $A^4$ represents any one substituent indicated in [Table L1) to [Table L3] (hereinafter, referred. to as "Compound class CX175").

A compound (K-1) wherein $R^{1b}$ represents an iodine atom, $R^{1c}$ represents a hydrogen atom, $A^2$ represents C-Me, $A^1$, $A^3$, and $A^5$ represent CH, and $A^4$ represents any one substituent indicated in [Table 11] to [Table L3] (hereinafter, referred to as "Compound class CX176").

A compound (R-1) wherein $R^{1b}$ represents an iodine atom, $R^{1c}$ represents a hydrogen atom, $A^2$ represents C-OMe, $A^1$, $A^3$, and $A^5$ represent CH, and $A^4$ represents any one substituent indicated in [Table L1] to [Table L3] (hereinafter, referred to as "Compound class CX177").

A compound (K-1) wherein $R^{1b}$ represents an iodine atom, $R^{1c}$ represents a hydrogen atom, $A^2$ represents C-$CF_3$, $A^1$, $A^3$, and $A^5$ represent CH, and $A^4$ represents any one substituent indicated in [Table L1] to [Table. L3] (hereinafter, referred to as "Compound class CX178").

A compound (K-1) wherein $R^{1b}$ represents an iodine atom, $R^{1c}$ represents a hydrogen atom, $A^2$ represents C-OCF3, $A^1$, $A^3$, and $A^5$ represent CH, and $A^4$ represents any one substituent indicated in [Table L1] to [Table L3] (hereinafter, referred to as "Compound class CX179").

A compound (K-1) wherein $R^{1b}$ represents an iodine atom, $R^{1c}$ represents a hydrogen atom, $A^3$ represents C-F, $A^1$, $A^2$, and $A^5$ represent CH, and $A^4$ represents any one substituent indicated in [Table L1) to [Table L3] (hereinafter, referred. to as "Compound class CX180").

A compound (K-1) wherein $R^{1b}$ represents an iodine atom, $R^{1c}$ represents a hydrogen atom, $A^3$ represents C-Cl, $A^1$, $A^2$, and $A^5$ represent CH, and $A^4$ represents any one substituent indicated in [Table L1] to [Table L3] (hereinafter, referred to as "Compound class CX181").

A compound (R-1) wherein $R^{1b}$ represents an iodine atom, $R^{1c}$ represents a hydrogen atom, $A^3$ represents C-Br, $A^1$, $A^2$, and $A^5$ represent CH, and $A^4$ represents any one substituent indicated in [Table L1] to [Table L3] (hereinafter, referred to as "Compound class CX182").

A compound (N-1) wherein $R^{1b}$ represents an iodine atom, $R^{1c}$ represents a hydrogen atom, $A^3$ represents C-I, $A^1$, $A^2$, and $A^5$ represent CH, and $A^4$ represents any one substituent indicated in [Table L1] to [Table. L3] (hereinafter, referred to as "Compound class CX183").

A compound (K-1) wherein $R^{1b}$ represents an iodine atom, $R^{1c}$ represents a hydrogen atom, $A^3$ represents C-I, $A^1$, $A^2$, and $A^5$ represent CH, and $A^4$ represents any one substituent indicated in [Table L1] to [Table L3] (hereinafter, referred to as "Compound class CX183").

A compound (K-1) wherein $R^{1b}$ represents an iodine atom, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter, referred. to as "Compound class CX184"),

A compound (K-1) wherein $R^{1b}$ represents an iodine atom, $R^{1c}$ represents a hydrogen atom, $A^3$ represents C-OMe, $A^1$, $A^2$, and $A^5$ represent CH, and $A^4$ represents any one substituent indicated in [Table 11] to [Table L3] (hereinafter, referred to as "Compound class CX185").

A compound (R-1) wherein $R^{1b}$ represents an iodine atom, $R^{1c}$ represents a hydrogen atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX186").

A compound (N-1) wherein $R^{1b}$ represents an iodine atom, $R^{1c}$ represents a hydrogen atom, $A^3$ represents C-$OCF_3$, $A^1$, $A^2$, and $A^5$ represent CH, and $A^4$ represents any one substituent indicated in [Table L1] to [Table. L3] (hereinafter, referred to as "Compound class CX187").

A compound (K-1) wherein $R^{1b}$ represents a methyl group, $R^{1c}$ represents a hydrogen atom, $A^2$ represents C-F, $A^1$, $A^3$, and $A^5$ represent CH, and $A^4$ represents any one substituent indicated in [Table L1] to [Table L3] (hereinafter, referred to as "Compound class CX188").

A compound (K-1) wherein $R^{1b}$ represents a methyl group, $R^{1c}$ represents a hydrogen atom, $A^2$ represents C-Cl, $A^1$, $A^3$, and $A^5$ represent CH, and $A^4$ represents any one substituent indicated in [Table L1] to [Table L3] (hereinafter, referred. to as "Compound class CX189").

A compound (K-1) wherein $R^{1b}$ represents a methyl group, $R^{1c}$ represents a hydrogen atom, $A^2$ represents C-Br, $A^1$, $A^3$, and $A^5$ represent CH, and $A^4$ represents any one substituent indicated in [Table 11] to [Table L3] (hereinafter, referred to as "Compound class CX190").

A compound (R-1) wherein $R^{1b}$ represents a methyl group, $R^{1c}$ represents a hydrogen atom, $A^2$ represents C-I, $A^1$, $A^3$, and $A^5$ represent CH, and $A^4$ represents any one substituent indicated in [Table L1] to [Table L3] (hereinafter, referred to as "Compound class CX191").

A compound (N-1) wherein $R^{1b}$ represents a methyl group, $R^{1c}$ represents a hydrogen atom, $A^2$ represents C-I, $A^1$, $A^3$, and $A^5$ represent CH, and $A^4$ represents any one substituent indicated in [Table L1] to [Table. L3] (hereinafter, referred to as "Compound class CX191").

A compound (K-1) wherein $R^{1b}$ represents a methyl group, $R^{1c}$ represents a hydrogen atom, $A^2$ represents C-Me, $A^1$, $A^3$, and $A^5$ represent CH, and $A^4$ represents any one substituent indicated in [Table L1] to [Table L3] (hereinafter, referred to as "Compound class CX192").

A compound (K-1) wherein $R^{1b}$ represents a methyl group, $R^{1c}$ represents a hydrogen atom, $A^2$ represents C-OMe, $A^1$, $A^3$, and $A^5$ represent CH, and $A^4$ represents any one substituent indicated in [Table L1] to [Table L3] (hereinafter, referred. to as "Compound class CX193").

A compound (K-1) wherein $R^{1b}$ represents a methyl group, $R^{1c}$ represents a hydrogen atom, $A^2$ represents C-$CF_3$, $A^1$, $A^3$, and $A^5$ represent CH, and $A^4$ represents any one substituent indicated in [Table 11] to [Table L3] (hereinafter, referred to as "Compound class CX104").

A compound (R-1) wherein $R^{1b}$ represents a methyl group, $R^{1c}$ represents a hydrogen atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX195").

A compound (N-1) wherein $R^{1b}$ represents a methyl group, $R^{1c}$ represents a hydrogen atom, $A^3$ represents C-F, $A^1$, $A^2$, and $A^5$ represent CH, and $A^4$ represents any one substituent indicated in [Table L1] to [Table. L3] (hereinafter, referred to as "Compound class C.X196").

A compound (K-1) wherein $R^{1b}$ represents a methyl group, $R^{1c}$ represents a hydrogen atom, $A^3$ represents C-

Cl, $A^1$, $A^2$, and $A^5$ represent CH, and $A^4$ represents any one substituent indicated in [Table L1] to [Table L3] (hereinafter, referred to as "Compound class CX197").

A compound (K-1) wherein $R^{1b}$ represents a methyl group, $R^{1c}$ represents a hydrogen atom, $A^3$ represents C-Br, $A^1$, $A^2$, and $A^5$ represent CH, and $A^4$ represents any one substituent indicated in [Table L1] to [Table L3] (hereinafter, referred. to as "Compound class CX198").

A compound (K-1) wherein $R^{1b}$ represents a methyl group, $R^{1c}$ represents a hydrogen atom, $A^3$ represents C-I, $A^1$, $A^2$, and $A^5$ represent CH, and $A^4$ represents any one substituent indicated in [Table 11] to [Table L3] (hereinafter, referred to as "Compound class CX199").

A compound (R-1) wherein $R^{1b}$ represents a methyl group, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter, referred to as "Compound class CX200").

A compound (N-1) wherein $R^{1b}$ represents a methyl group, $R^{1c}$ represents a hydrogen atom, $A^3$ represents C-QMe, $A^1$, $A^2$, and $A^5$ represent CH, and $A^4$ represents any one substituent indicated in [Table L1] to [Table. L3] (hereinafter, referred to as "Compound class C.X201").

A compound (K-1) wherein $R^{1b}$ represents a methyl group, $R^{1c}$ represents a hydrogen atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX202").

A compound (K-1) wherein $R^{1b}$ represents a methyl group, $R^{1c}$ represents a hydrogen atom, $A^3$ represents C-$OCF_3$, $A^1$, $A^2$, and $A^5$ represent CH, and $A^4$ represents any one substituent indicated in [Table L1] to [Table L3] (hereinafter, referred. to as "Compound class CX203").

A compound (K-1) wherein $R^{1b}$ represents an ethyl group, $R^{1c}$ represents a hydrogen atom, $A^2$ represents C-F, $A^1$, $A^3$, and $A^5$ represent CH, and $A^4$ represents any one substituent indicated in [Table L1] to [Table L3] (hereinafter, referred to as "Compound class CX204").

A compound (R-1) wherein $R^{1b}$ represents an ethyl group, $R^{1c}$ represents a hydrogen atom, $A^2$ represents C-Cl, $A^1$, $A^3$, and $A^5$ represent CH, and $A^4$ represents any one substituent indicated in [Table L1] to [Table L3] (hereinafter, referred to as "Compound class CX205").

A compound (N-1) wherein $R^{1b}$ represents an ethyl group, $R^{1c}$ represents a hydrogen atom, $A^2$ represents C-Br, $A^1$, $A^3$, and $A^5$ represent CH, and $A^4$ represents any one substituent indicated in [Table L1] to [Table. L3] (hereinafter, referred to as "Compound class C.X206").

A compound (K-1) wherein $R^{1b}$ represents an ethyl group, $R^{1c}$ represents a hydrogen atom, $A^2$ represents C-I, $A^1$, $A^3$, and $A^5$ represent CH, and $A^4$ represents any one substituent indicated in [Table L1] to [Table L3] (hereinafter, referred to as "Compound class CX207").

A compound (K-1) wherein $R^{1b}$ represents an ethyl group, $R^{1c}$ represents a hydrogen atom, $A^2$ represents C-Me, $A^1$, $A^3$, and $A^5$ represent CH, and $A^4$ represents any one substituent indicated in [Table L1] to [Table L3] (hereinafter, referred. to as "Compound class CX208).

A compound (K-1) wherein $R^{1b}$ represents an ethyl group, $R^{1c}$ represents a hydrogen atom, $A^2$ represents C-OMe, $A^1$, $A^3$, and $A^5$ represent CH, and $A^4$ represents any one substituent indicated in [Table 11] to [Table L3] (hereinafter, referred to as "Compound class CX209).

A compound (R-1) wherein $R^{1b}$ represents an ethyl group, $R^{1c}$ represents a hydrogen atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX210).

A compound (N-1) wherein $R^{1b}$ represents an ethyl group, $R^{1c}$ represents a hydrogen atom, $A^2$ represents C-$OCF_3$, $A^1$, $A^3$, and $A^5$ represent CH, and $A^4$ represents any one substituent indicated in [Table L1] to [Table. L3] (hereinafter, referred to as "Compound class CX211).

A compound (K-1) wherein $R^{1b}$ represents an ethyl group, $R^{1c}$ represents a hydrogen atom, $A^3$ represents C-F, $A^1$, $A^2$, and $A^5$ represent CH, and $A^4$ represents any one substituent indicated in [Table L1] to [Table L3] (hereinafter, referred to as "Compound class CX212).

A compound (K-1) wherein $R^{1b}$ represents an ethyl group. $R^{1c}$ represents a hydrogen atom, $A^3$ represents C-C1, $A^1$, $A^2$, and $A^5$ represent CH, and $A^4$ represents any one substituent indicated in [Table L1] to [Table L3] (hereinafter, referred to as "Compound class CX213).

A compound (K-1) wherein $R^{1b}$ represents an ethyl group, $R^{1c}$ represents a hydrogen atom, $A^3$ represents C-Br, $A^1$, $A^2$, and $A^5$ represent CH, and $A^4$ represents any one substituent indicated in [Table L1] to [Table L3] (hereinafter, referred to as "Compound class CX214).

A compound (K-1) wherein $R^{1b}$ represents an ethyl group, $R^{1c}$ represents a hydrogen atom, $A^3$ represents C-I, $A^1$, $A^2$, and $A^5$ represent CH, and $A^4$ represents any one substituent indicated in [Table L1] to [Table L3] (hereinafter, referred to as "Compound class CX215).

A compound (K-1) wherein $R^{1b}$ represents an ethyl group, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter,

referred to as "Compound class CX216).

A compound (K-1) wherein $R^{1b}$ represents an ethyl group, $R^{1c}$ represents a hydrogen atom, $A^3$ represents C-QHe, $A^1$, $A^2$, and $A^5$ represent CH, and $A^4$ represents any one substituent indicated in [Table L1] to [Table L3] (hereinafter, referred to as "Compound class CX217).

A compound (K-1) wherein $R^{1b}$ represents an ethyl group, $R^{1c}$ represents a hydrogen atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX218).

A compound (K-1) wherein $R^{1b}$ represents an ethyl group, $R^{1c}$ represents a hydrogen atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX219).

A compound (K-1) wherein $R^{1b}$ represents $CF_3$, $R^{1c}$ represents a hydrogen atom, $A^2$ represents C-F, $A^1$, $A^3$, and $A^5$ represent CH, and $A^4$ represents any one substituent indicated in [Table L1] to [Table L3] (hereinafter, referred to as "Compound class CX220).

A compound (N-2) wherein $R^{1b}$ represents $CF_3$, $R^{1c}$ represents a hydrogen atom, $A^2$ represents C-Cl, $A^1$, $A^3$, and $A^5$ represent CH, and $A^4$ represents any one substituent indicated in [Table L1] to [Table L3] (hereinafter, referred to as "Compound class CX221).

A compound (K-1) wherein $R^{1b}$ represents $CF_3$, $R^{1c}$ represents a hydrogen atom, $A^2$ represents C-Br, $A^1$, $A^3$, and $A^5$ represent CH, and $A^4$ represents any one substituent indicated in [Table L1] to [Table L3] (hereinafter, referred to as "Compound class CX222).

A compound (K-1) wherein $R^{1b}$ represents $CF_3$, $R^{1c}$ represents a hydrogen atom, $A^2$ represents C-I, $A^1$, $A^3$, and $A^5$ represent CH, and $A^4$ represents any one substituent indicated in [Table L1] to [Table L3] (hereinafter, referred to as "Compound class CX223).

A compound (K-1) wherein $R^{1b}$ represents $CF_3$, $R^{1c}$ represents a hydrogen atom, $A^2$ represents C-Me, $A^1$, $A^3$, and $A^5$ represent CH, and $A^4$ represents any one substituent indicated in [Table L1] to [Table L3] (hereinafter, referred to as "Compound class CX224).

A compound (K-1) wherein $R^{1b}$ represents $CF_3$, $R^{1c}$ represents a hydrogen atom, $A^2$ represents C-OMe, $A^1$, $A^3$, and $A^5$ represent CH, and $A^4$ represents any one substituent indicated in [Table L1] to [Table L3] (hereinafter, referred to as "Compound class CX225).

A compound (N-2) wherein $R^{1b}$ represents $CF_3$, $R^{1c}$ represents a hydrogen atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX226).

A compound (K-1) wherein $R^{1b}$ represents $CF_3$, $R^{1c}$ represents a hydrogen atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX227).

A compound (K-1) wherein $R^{1b}$ represents $CF_3$, $R^{1c}$ represents a hydrogen atom, $A^3$ represents C-F, $A^1$, $A^2$, and $A^5$ represent CH, and $A^4$ represents any one substituent indicated in [Table L1] to [Table L3] (hereinafter, referred to as "Compound class CX228).

A compound (K-1) wherein $R^{1b}$ represents $CF_3$, $R^{1c}$ represents a hydrogen atom, $A^3$ represents C-Cl, $A^1$, $A^2$, and $A^5$ represent CH, and $A^4$ represents any one substituent indicated in [Table L1] to [Table L3] (hereinafter, referred to as "Compound class CX229).

A compound (K-1) wherein $R^{1b}$ represents $CF_3$, $R^{1c}$ represents a hydrogen atom, $A^3$ represents C-Br, $A^1$, $A^2$, and $A^5$ represent CH, and $A^4$ represents any one substituent indicated in [Table L1] to [Table L3] (hereinafter, referred to as "Compound class CX230).

A compound (N-2) wherein $R^{1b}$ represents $CF_3$, $R^{1c}$ represents a hydrogen atom, $A^3$ represents C-I, $A^1$, $A^2$, and $A^5$ represent CH, and $A^4$ represents any one substituent indicated in [Table L1] to [Table L3] (hereinafter, referred to as "Compound class CX231).

A compound (K-1) wherein $R^{1b}$ represents $CF_3$, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter, referred to as "Compound class CX232).

A compound (K-1) wherein $R^{1b}$ represents $CF_3$, $R^{1c}$ represents a hydrogen atom, $A^3$ represents C-OMe, $A^1$, $A^2$, and $A^5$ represent CH, and $A^4$ represents any one substituent indicated in [Table L1] to [Table L3] (hereinafter, referred to as "Compound class CX233).

A compound (K-1) wherein $R^{1b}$ represents $CF_3$, $R^{1c}$ represents a hydrogen atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX234).

A compound (K-1) wherein $R^{1b}$ represents $CF_3$, $R^{1c}$ represents a hydrogen atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX235).

A compound (K-1) wherein $R^{1b}$ represents a cyclopropyl group, $R^{1c}$ represents a hydrogen atom, $A^2$ represents C-F, $A^1$, $A^3$, and $A^5$ represent CH, and $A^4$ represents any one substituent indicated in [Table L1] to [Table L3] (hereinafter, referred to as "Compound class CX236).

A compound (K-1) wherein $R^{1b}$ represents a cyclopropyl group, $^{1c}$ represents a hydrogen atom, $A^2$ represents C-Cl, $A^1$, $A^3$, and $A^5$ represent CH, and $A^4$ represents any one substituent indicated in [Table L1] to [Table L3] (hereinafter, referred to as "Compound class CX237).

A compound (K-1) wherein $R^{1b}$ represents a cyclopropyl group, $R^{1c}$ represents a hydrogen atom, $A^2$ represents C-Br, $A^1$, $A^3$, and $A^5$ represent CH, and $A^4$ represents any one substituent indicated in [Table L1] to [Table L3] (hereinafter, referred to as "Compound class CX238).

A compound (K-1) wherein $R^{1b}$ represents a cyclopropyl group, $R^{1c}$ represents a hydrogen atom, $A^2$ represents C-I, $A^1$, $A^3$, and $A^5$ represent CH, and $A^4$ represents any one substituent indicated in [Table L1] to [Table L3] (hereinafter, referred to as "Compound class CX239).

A compound (K-1) wherein $R^{1b}$ represents a cyclopropyl group, $R^{1c}$ represents a hydrogen atom, $A^2$ represents C-Me, $A^1$, $A^3$, and $A^5$ represent CH, and $A^4$ represents any one substituent indicated in [Table L1] to [Table L3] (hereinafter, referred to as "Compound class CX240).

A compound (K-1) wherein $R^{1b}$ represents a cyclopropyl group, $R^{1c}$ represents a hydrogen atom, $A^2$ represents C-OMe, $A^1$, $A^3$, and $A^5$ represent CH, and $A^4$ represents any one substituent indicated in [Table L1] to [Table L3] (hereinafter, referred to as "Compound class CX241).

A compound (K-1) wherein $R^{1b}$ represents a cyclopropyl group, $R^{1c}$ represents a hydrogen atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX242).

A compound (K-1) wherein $R^{1b}$ represents a cyclopropyl group, $R^{1c}$ represents a hydrogen atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX243).

A compound (K-1) wherein $R^{1b}$ represents a cyclopropyl group, $R^{1c}$ represents a hydrogen atom, $A^3$ represents C-F, $A^1$, $A^2$, and $A^5$ represent CH, and $A^4$ represents any one substituent indicated in [Table L1] to [Table L3] (hereinafter, referred to as "Compound class CX244).

A compound (K-1) wherein $R^{1b}$ represents a cyclopropyl group, $R^{1c}$ represents a hydrogen atom, $A^3$ represents C-Cl, $A^1$, $A^2$, and $A^5$ represent CH, and $A^4$ represents any one substituent indicated in [Table L1] to [Table L3] (hereinafter, referred to as "Compound class CX245).

A compound (K-1) wherein $R^{1b}$ represents a cyclopropyl group, $R^{1c}$ represents a hydrogen atom, $A^3$ represents C-Br, $A^1$, $A^2$, and $A^5$ represent CH, and $A^4$ represents any one substituent indicated in [Table L1] to [Table L3] (hereinafter, referred to as "Compound class CX246).

A compound (K-1) wherein $R^{1b}$ represents a cyclopropyl group, $R^{1c}$ represents a hydrogen atom, $A^3$ represents C-I, $A^1$, $A^2$, and $A^5$, represent CH, and $A^4$ represents any one substituent indicated in [Table L1] to [Table L3] (hereinafter, referred to as "Compound class CX247).

A compound (K-1) wherein $R^{1b}$ represents a cyclopropyl group, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter, referred to as "Compound class CX248).

A compound (K-1) wherein $R^{1b}$ represents a cyclopropyl group, $R^{1c}$ represents a hydrogen atom, $A^3$ represents C-OMe, $A^1$, $A^2$, and $A^5$ represent CH, and $A^4$ represents any one substituent indicated in [Table L1] to [Table L3] (hereinafter, referred to as "Compound class CX249).

A compound (K-1) wherein $R^{1b}$ represents a cyclopropyl group, $R^{1c}$ represents a hydrogen atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX250).

A compound (K-1) wherein $R^{1b}$ represents a cyclopropyl group, $R^{1c}$ represents a hydrogen atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX251).

A compound (K-1) wherein $R^{1b}$ represents a methylsulfanyl group, $R^{1c}$ represents a hydrogen atom, $A^2$ represents C-F, $A^1$, $A^3$, and $A^5$ represent CH, and $A^4$ represents any one substituent indicated in [Table L1] to [Table L3] (hereinafter, referred to as "Compound class CX252).

A compound (R-1) wherein $R^{1b}$ represents a methylsulfanyl group, $R^{1c}$ represents a hydrogen atom, $A^2$ represents C-Cl, $A^1$, $A^3$, and $A^5$ represent CH, and $A^4$ represents any one substituent indicated in [Table L1] to [Table. L3] (hereinafter, referred to as "Compound class CX253).

A compound (R-1) wherein $R^{1b}$ represents a methylsulfanyl group, $R^{1c}$ represents a hydrogen atom, $A^2$ represents C-Br, $A^1$, $A^3$, and $A^5$ represent CH, and $A^4$ represents any one substituent indicated in [Table L1] to [Table L3] (hereinafter, referred to as "Compound class CX254).

A compound (N-1) wherein $R^{1b}$ represents a methylsulfanyl group, $K^{1c}$ represents a hydrogen atom, $A^2$ rep-

resents C-I, $A^1$, $A^3$, and $A^5$ represent CH, and $A^4$ represents any one substituent indicated in [Table L1] to [Table L3] (hereinafter, referred to as "Compound class CX255).

A compound (K-1) wherein $R^{1b}$ represents a methylsulfanyl group, $R^{1c}$ represents a hydrogen atom, $A^2$ represents C-Me, $A^1$, $A^3$, and $A^5$ represent CH, and $A^4$ represents any one substituent indicated in [Table L1] to [Table L3] (hereinafter, referred to as "Compound class CX256).

A compound (K-1) wherein $R^{1b}$ represents a methylsulfanyl group, $R^{1c}$ represents a hydrogen atom, $A^2$ represents C-OMe, $A^1$, $A^3$, and $A^5$ represent CH, and $A^4$ represents any one substituent indicated in [Table L1] to [Table L3] (hereinafter, referred to as "Compound class CX257),

A compound (R-1) wherein $R^{1b}$ represents a methylsulfanyl group, $R^{1c}$ represents a hydrogen atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX258).

A compound (R-1) wherein $R^{1b}$ represents a methylsulfanyl group, $R^{1c}$ represents a hydrogen atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX259).

A compound (N-1) wherein $R^{1b}$ represents a methylsulfanyl group, $R^{1c}$ represents a hydrogen atom, $A^3$ represents C-F, $A^1$, $A^2$, and $A^5$ represent CH, and $A^4$ represents any one substituent indicated in [Table L1] to [Table L3] (hereinafter, referred to as "Compound class CX260).

A compound (K-1) wherein $R^{1b}$ represents a methylsulfanyl group, $R^{1c}$ represents a hydrogen atom, $A^3$ represents C-Cl, $A^1$, $A^2$, and $A^5$ represent CH, and $A^4$ represents any one substituent indicated in [Table L1] to [Table L3] (hereinafter, referred to as "Compound class CX261).

A compound (K-1) wherein $R^{1b}$ represents a methylsulfanyl group, $R^{1c}$ represents a hydrogen atom, $A^3$ represents C-Br, $A^1$, $A^2$, and $A^5$ represent CH, and $A^4$ represents any one substituent indicated in [Table L1] to [Table L3] (hereinafter, referred to as "Compound class CX262).

A compound. (R-1) wherein $R^{1b}$ represents a methylsulfanyl group, $R^{1c}$ represents a hydrogen atom, $A^3$ represents C-I, $A^1$, $A^2$, and $A^5$ represent CH, and $A^4$ represents any one substituent indicated in [Table L1] to [Table. L3] (hereinafter, referred to as "Compound class CX263).

A compound (R-1) wherein $R^{1b}$ represents a methylsulfanyl group, $R^{1c}$ represents a hydrogen atom, $A^3$ represents C-Me, $A^1$, $A^2$, and $A^5$ represent CH, and $A^4$ represents any one substituent indicated in [Table L1] to [Table L3] (hereinafter, referred to as "Compound class CX264).

A compound (N-1) wherein $R^{1b}$ represents a methylsulfanyl group, $R^{1c}$ represents a hydrogen atom, $A^3$ represents C-OMe, $A^1$, $A^2$, and $A^5$ represent CH, and $A^4$ represents any one substituent indicated in [Table L1] to [Table L3] (hereinafter, referred to as "Compound class CX265).

A compound (K-1) wherein $R^{1b}$ represents a methyl sulfanyl group, $R^{1c}$ represents a hydrogen atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX266).

A compound (K-1) wherein $R^{1b}$ represents a methylsulfanyl group, $R^{1c}$ represents a hydrogen atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX267),

A compound (R-1) wherein $R^{1b}$ and $R^{1c}$ represent a fluorine atom, $A^2$ represents C-F, $A^1$, $A^3$, and $A^5$ represent CH, and $A^4$ represents any one substituent indicated in [Table L1] to [Table L3] (hereinafter, referred to as "Compound class CX268).

A compound (R-1) wherein $R^{1b}$ and $R^{1c}$ represent a fluorine atom, $A^2$ represents C-Cl, $A^1$, $A^3$, and $R^5$ represent CH, and $A^4$ represents any one substituent indicated in [Table L1] to [Table L3] (hereinafter, referred to as "Compound class CX269).

A compound (N-1) wherein $R^{1b}$ and $R^{1c}$ represent a fluorine atom, $A^2$ represents C-Br, $A^1$, $A^3$, and $A^5$ represent CH, and $A^4$ represents any one substituent indicated in [Table L1] to [Table L3] (hereinafter, referred to as "Compound class CX270).

A compound (K-1) wherein $R^{1b}$ and $R^{1c}$ represent a fluorine atom, $A^2$ represents C-I, $A^1$, $A^3$, and $A^5$ represent CH, and $A^4$ represents any one substituent indicated in [Table L1] to [Table L3] (hereinafter, referred to as "Compound class. CX271).

A compound (K-1) wherein $R^{1b}$ and $R^{1c}$ represent a fluorine atom, $A^2$ represents C-Me, $A^1$, $A^3$, and $A^5$ represent CH, and $A^4$ represents any one substituent indicated in [Table L1] to [Table L3] (hereinafter, referred to as "Compound class CX272).

A compound (R-1) wherein $R^{1b}$ and $R^{1c}$ represent a fluorine atom, $A^2$ represents C-OMe, $A^1$, $A^3$, and $A^5$ represent CH, and $A^4$ represents any one substituent indicated in [Table L1] to [Table L3] (hereinafter, referred to as "Compound class CX273).

A compound (K-1) wherein $R^{1b}$ and $R^{1c}$ represent a fluorine atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound

class CX2.74) .

A compound (N-1) wherein $R^{1b}$ and $R^{1c}$ represent a fluorine atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX275).

A compound (K-1) wherein $R^{1b}$ and $R^{1c}$ represent a fluorine atom, $A^3$ represents C-F, $A^1$, $A^2$, and $A^5$ represent CH, and $A^4$ represents any one substituent indicated in [Table L1] to [Table L3] (hereinafter, referred to as "Compound class CX276) .

A compound (K-1) wherein $R^{1b}$ and $R^{1c}$ represent a fluorine atom, $A^3$ represents C-Cl, $A^1$, $A^2$, and $A^5$ represent CH, and $A^4$ represents any one substituent indicated in [Table L1] to [Table L3] (hereinafter, referred to as "Compound class CX277) .

A compound (K-1) wherein $R^{1b}$ and $R^{1c}$ represent a fluorine atom, $A^3$ represents C-Br, $A^1$, $A^2$, and $A^5$ represent CH, and $A^4$ represents any one substituent indicated in [Table L1] to [Table L3] (hereinafter, referred to as "Compound class CX278).

A compound (K-1) wherein $R^{1b}$ and $R^{1c}$ represent a fluorine atom, $A^3$ represents C-I, $A^1$, $A^2$, and $A^5$ represent CH, and $A^4$ represents any one substituent indicated in [Table L1] to [Table L3] (hereinafter, referred to as "Compound class CX27 9) .

A compound (K-1) wherein $R^{1b}$ and $R^{1c}$ represent a fluorine atom, $A^3$ represents C-Me, $A^1$, $A^2$, and $A^5$ represent CH, and $A^4$ represents any one substituent indicated in [Table L1] to [Table L3] (hereinafter, referred to as "Compound class CX280).

A compound (K-1) wherein $R^{1b}$ and $R^{1c}$ represent a fluorine atom, $A^3$ represents C-OMe, $A^1$, $A^2$, and A* represent CH, and $A^4$ represents any one substituent indicated in [Table L1] to [Table L3] (hereinafter, referred to as "Compound class. CX281).

A compound (K-1) wherein $R^{1b}$ and $R^{1c}$ represent a fluorine atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX282).

A compound (R-1) wherein $R^{1b}$ and $R^{1c}$ represent a fluorine atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX283).

A compound (R-1) wherein $R^{1b}$ and $R^{1c}$ represent a chlorine atom, $A^2$ represents C-F, $A^1$, $A^3$, and $A^5$ represent CH, and $A^4$ represents any one substituent indicated in [Table L1] to [Table L3] (hereinafter, referred to as "Compound class CX284).

A compound (N-1) wherein $R^{1b}$ and $R^{1c}$ represent a chlorine atom, $A^2$ represents C-Cl, $A^1$, $A^3$, and $A^5$ represent CH, and $A^4$ represents any one substituent indicated in [Table L1] to [Table L3] (hereinafter, referred to as "Compound class CX285).

A compound (K-1) wherein $R^{1b}$ and $R^{1c}$ represent a chlorine atom, $A^2$ represents C-Br, $A^1$, $A^3$, and $A^5$ represent CH, and $A^4$ represents any one substituent indicated in [Table L1] to [Table L3] (hereinafter, referred to as "Compound class. CX286) .

A compound (K-1) wherein $R^{1b}$ and $R^{1c}$ represent a chlorine atom, $A^2$ represents C-I, $A^1$, $A^3$, and $A^5$ represent CH, and $A^4$ represents any one substituent indicated in [Table L1] to [Table L3] (hereinafter, referred to as "Compound class CX287).

A compound (R-1) wherein $R^{1b}$ and $R^{1c}$ represent a chlorine atom, $A^2$ represents C-Me, $A^1$, $A^3$, and $A^5$ represent CH, and $A^4$ represents any one substituent indicated in [Table L1] to [Table L3] (hereinafter, referred to as "Compound class CX288).

A compound (R-1) wherein $R^{1b}$ and $R^{1c}$ represent a chlorine atom, $A^2$ represents C-OMe, $A^1$, $A^3$, and $A^5$ represent CH, and $A^4$ represents any one substituent indicated in [Table L1] to [Table L3] (hereinafter, referred to as "Compound class CX289).

A compound (K-1) wherein $R^{1b}$ and $R^{1c}$ represent a chlorine atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX290).

A compound (K-1) wherein $R^{1b}$ and $R^{1c}$ represent a chlorine atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX291) .

A compound (K-1) wherein $A^{1b}$ and $R^{1c}$ represent a chlorine atom, $A^3$ represents C-F, $A^1$, $A^2$, and $A^5$ represent CH, and $A^4$ represents any one substituent indicated in [Table L1] to [Table L3] (hereinafter, referred to as "Compound class CX2 92) .

A compound (K-1) wherein $R^{1b}$ and $R^{1c}$ represent a chlorine atom, $A^3$ represents C-Cl, $A^1$, $A^2$, and $A^5$ represent CH, and $A^4$ represents any one substituent indicated in [Table L1] to [Table L3] (hereinafter, referred to as "Compound class CX293).

A compound (K-1) wherein $R^{1b}$ and $R^{1c}$ represent a chlorine atom, $A^3$ represents C-Br, $A^1$, $A^2$, and $A^5$ represent CH, and $A^4$ represents any one substituent indicated in [Table L1] to [Table L3] (hereinafter, referred to as "Compound class CX294).

A compound (K-1) wherein $R^{1b}$ and $R^{1c}$ represent a chlorine atom, $A^3$ represents C-I, $A^1$, $A^2$, and $A^5$ represent CH, and $A^4$ represents any one substituent indicated in [Table L1] to [Table L3] (hereinafter, referred to as "Compound class CX295).

A compound (K-1) wherein $R^{1b}$ and $R^{1c}$ represent a chlorine atom, $A^3$ represents C-Me, $A^1$, $A^2$, and $A^5$ represent CH, and $A^4$ represents any one substituent indicated in [Table L1] to [Table L3] (hereinafter, referred to as "Compound class CX296).

A compound (K-1) wherein $R^{1b}$ and $R^{1c}$ represent a chlorine atom, $A^3$ represents C-QMe, $A^1$, $A^2$, and $A^5$ represent CH, and $A^4$ represents any one substituent indicated in [Table L1] to [Table L3] (hereinafter, referred to as "Compound class CX297) .

A compound (K-1) wherein $R^{1b}$ and $R^{1c}$ represent a chlorine atom, $A^3$ represents C-CFj, $A^1$, $A^2$, and $A^5$ represent CH, and $A^4$ represents any one substituent indicated in [Table L1] to [Table L3] (hereinafter, referred to as "Compound class CX298),

A compound (K-1) wherein $R^{1b}$ and $R^{1c}$ represent a chlorine atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX299).

A compound (K-1) wherein $R^{1b}$ and $R^{1c}$ represent a bromine atom, $A^2$ represents C-F, $A^1$, $A^3$, and $A^5$ represent CH, and $A^4$ represents any one substituent indicated in [Table L1] to [Table L3] (hereinafter, referred to as "Compound class CX300).

A compound (K-1) wherein $R^{1b}$ and $R^{1c}$ represent a bromine atom, $A^2$ represents C-Cl, $A^1$, $A^3$, and $A^5$ represent CH, and $A^4$ represents any one substituent indicated in [Table L1] to [Table L3] (hereinafter, referred to as "Compound class CX301).

A compound (K-1) wherein $R^{1b}$ and $R^{1c}$ represent a bromine atom, $A^2$ represents C-Br, $A^1$, $A^3$, and $A^5$ represent CH, and $A^4$ represents any one substituent indicated in [Table L1] to [Table L3] (hereinafter, referred to as "Compound class CX302) .

A compound (K-1) wherein $R^{1b}$ and $R^{1c}$ represent a bromine atom, $A^2$ represents C-I, $A^1$, $A^3$, and $A^5$ represent CH, and $A^4$ represents any one substituent indicated in [Table L1] to [Table L3] (hereinafter, referred to as "Compound class CX303),

A compound (K-1) wherein $R^{1b}$ and $R^{1c}$ represent a bromine atom, $A^2$ represents C-Me, $A^1$, $A^3$, and $A^5$ represent CH, and $A^4$ represents any one substituent indicated in [Table L1] to [Table L3] (hereinafter, referred to as "Compound class CX304) .

A compound (K-1) wherein $R^{1b}$ and $R^{1c}$ represent a bromine atom, $A^2$ represents C-OMe, $A^1$, $A^3$, and $A^5$ represents CH, and $A^4$ represents any one substituent indicated in [Table L1] to [Table L3] (hereinafter, referred to as "Compound class CX305).

A compound (K-1) wherein $R^{1b}$ and $R^{1c}$ represent a bromine atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX306).

A compound (K-1) wherein $R^{1b}$ and $R^{1c}$ represent a. bromine atom, $A^2$ represents C-$QCF_3$, $A^1$, $A^3$, and $A^5$ represent CH, and $A^4$ represents any one substituent indicated in [Table L1] to [Table L3] (hereinafter, referred to as "Compound class CX307).

A compound (K-1) wherein $R^{1b}$ and $R^{1c}$ represent a bromine atom, $A^3$ represents C-F, $A^1$, $A^2$, and $A^5$ represent CH, and $A^4$ represents any one substituent indicated in [Table L1] to [Table L3] (hereinafter, referred to as "Compound class CX308).

A compound (K-1) wherein $R^{1b}$ and $R^{1c}$ represent a bromine atom, $A^3$ represents C-C1, $A^1$, $A^2$, and $A^5$ represent CH, and $A^4$ represents any one substituent indicated in [Table L1] to [Table L3] (hereinafter, referred to as "Compound class CR309).

A compound (K-1) wherein $R^{1b}$ and $R^{1c}$ represent a bromine atom, $A^3$ represents C-Br, $A^1$, $A^2$, and $A^5$ represent CH, and $A^4$ represents any one substituent indicated in [Table L1] to [Table L3] (hereinafter, referred to as "Compound class CX310).

A compound (K-1) wherein $R^{1b}$ and $R^{1c}$ represent a bromine atom, $A^3$ represents C-I, $A^1$, $A^2$, and $A^5$ represent CH, and $A^4$ represents any one substituent indicated in [Table L1] to [Table L3] (hereinafter, referred to as "Compound class CX311).

A compound (K-1) wherein $R^{1b}$ and $R^{1c}$ represent a. bromine atom, $A^3$ represents C-Me, $A^1$, $A^2$, and $A^5$ represent CH, and $A^4$ represents any one substituent indicated in [Table L1] to [Table L3] (hereinafter, referred to as "Compound class CX312).

A compound (K-1) wherein $R^{1b}$ and $R^{1c}$ represent a bromine atom, $A^3$ represents C-OMe, $A^1$, $A^2$, and $A^5$

represent CH, and $A^4$ represents any one substituent indicated in [Table L1] to [Table L3] (hereinafter, referred to as "Compound class CX313),

A compound (K-1) wherein $R^{1b}$ and $R^{1c}$ represent a bromine atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX314).

A compound (K-1) wherein $R^{1b}$ and $R^{1c}$ represent a bromine atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX315).

A compound (K-1) wherein $R^{1b}$ and $R^{1c}$ represent a bromine atom, $A^2$ represents C-F, $A^1$, $A^3$, and $A^5$ represent CH, and $A^4$ represents any one substituent indicated in [Table L1] to [Table L3] (hereinafter, referred to as "Compound class CX316).

A compound (K-1) wherein $R^{1b}$ and $R^{1c}$ represent an iodine atom, $A^2$ represents C-Cl, $A^1$, $A^3$, and $A^5$ represent CH, and $A^4$ represents any one substituent indicated in [Table L1] to [Table L3] (hereinafter, referred to as "Compound class CX3.17) .

A compound (K-1) wherein $R^{1b}$ and $R^{1c}$ represent an iodine atom, $A^2$ represents C-Br, $A^1$, $A^3$, and $A^5$ represent CH, and $A^4$ represents any one substituent indicated in [Table L1] to [Table L3] (hereinafter, referred to as "Compound class CX318).

A compound (K-1) wherein $R^{1b}$ and $R^{1c}$ represent an iodine atom, $A^2$ represents C-I, $A^1$, $A^3$, and $A^5$ represent CH, and $A^4$ represents any one substituent indicated in [Table L1] to [Table L3] (hereinafter, referred to as "Compound class CX319).

A compound (K-1) wherein $R^{1b}$ and $R^{1c}$ represent an iodine atom, $A^2$ represents C-Me, $A^1$, $A^3$, and $A^5$ represent CH, and $A^4$ represents any one substituent indicated in [Table L1] to [Table L3] (hereinafter, referred to as "Compound class CX320).

A compound (K-1) wherein $R^{1b}$ and $R^{1c}$ represent an iodine atom, $A^2$ represents C-OMe, $A^1$, $A^3$, and $A^5$ represent CH, and $A^4$ represents any one substituent indicated in [Table L1] to [Table L3] (hereinafter, referred to as "Compound class CX321).

A compound (K-1) wherein $R^{1b}$ and $R^{1c}$ represent an iodine atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX322).

A compound (K-1) wherein $R^{1b}$ and $R^{1c}$ represent an iodine atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX323),

A compound (K-1) wherein $R^{1b}$ and $R^{1c}$ represent an iodine atom, $A^3$ represents C-F, $A^1$, $A^2$, and $A^5$ represent CH, and $A^4$ represents any one substituent indicated in [Table L1] to [Table L3] (hereinafter, referred to as "Compound class CX324).

A compound (K-1) wherein $R^{1b}$ and $R^{1c}$ represent an iodine atom, $A^3$ represents C-Cl, $A^1$, $A^2$, and $A^5$ represent CH, and $A^4$ represents any one substituent indicated in [Table L1] to [Table L3] (hereinafter, referred to as "Compound class CX325).

A compound (K-1) wherein $R^{1b}$ and $R^{1c}$ represent an iodine atom, $A^3$ represents C-Br, $A^1$, $A^2$, and $A^5$ represent CH, and $A^4$ represents any one substituent indicated in [Table L1] to [Table L3] (hereinafter, referred to as "Compound class CX326).

A compound (K-1) wherein $R^{1b}$ and $R^{1c}$ represent an iodine atom, $A^3$ represents C-I, $A^1$, $A^2$, and $A^5$ represent CH, and $A^4$ represents any one substituent indicated in [Table L1] to [Table L3] (hereinafter, referred to as "Compound class CX327) .

A compound (K-1) wherein $R^{1b}$ and $R^{1c}$ represent an iodine atom, $A^3$ represents C-Me, $A^1$, $A^2$, and $A^5$ represent CH, and $A^4$ represents any one substituent indicated in [Table L1] to [Table L3] (hereinafter, referred to as "Compound class CX328),

A compound (K-1) wherein $R^{1b}$ and $R^{1c}$ represent an iodine atom, $A^3$ represents C-OMe, $A^1$, $A^2$, and $A^5$ represent CH, and $A^4$ represents any one substituent indicated in [Table L1] to [Table L3] (hereinafter, referred to as "Compound class CX329).

A compound (K-1) wherein $R^{1b}$ and $R^{1c}$ represent an iodine atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX330).

A compound (K-1) wherein $R^{1b}$ and $R^{1c}$ represent an iodine atom, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX331).

A compound (K-1) wherein $R^{1b}$ and $R^{1c}$ represent a methyl group, $A^2$ represents C-F, $A^1$, $A^3$, and $A^5$ represent CH, and $A^4$ represents any one substituent indicated in [Table L1] to [Table L3] (hereinafter, referred to as "Compound

class CX332).

A compound (K-1) wherein $R^{1b}$ and $R^{1c}$ represent a methyl group, $A^2$ represents C-Cl, $A^1$, $A^3$, and $A^5$ represent CH, and $A^4$ represents any one substituent indicated in [Table L1] to [Table L3] (hereinafter, referred to as "Compound class CX333).

A compound (K-1) wherein $R^{1b}$ and $R^{1c}$ represent a methyl group, $A^2$ represents C-Br, $A^1$, $A^3$, and $A^5$ represent CH, and $A^4$ represents any one substituent indicated in [Table L1] to [Table L3] (hereinafter, referred to as "Compound class CX334).

A compound (K-1) wherein $R^{1b}$ and $R^{1c}$ represent a methyl group, $A^2$ represents C-I, $A^1$, $A^3$, and $A^5$ represent CH, and $A^4$ represents any one substituent indicated in [Table L1] to [Table L3] (hereinafter, referred to as "Compound class CX335).

A compound (K-1) wherein $R^{1b}$ and $R^{1c}$ represent a methyl group, $A^2$ represents C-Me, $A^1$, $A^3$, and $A^5$ represent CH, and $A^4$ represents any one substituent indicated in [Table L1] to [Table L3] (hereinafter, referred to as "Compound class CX336).

A compound (K-1) wherein $R^{1b}$ and $R^{1c}$ represent a methyl group, $A^2$ represents C-OMe, $A^1$, $A^3$, and $A^5$ represent CH, and $A^4$ represents any one substituent indicated in [Table L1] to [Table L3] (hereinafter, referred to as "Compound class CX337) .

A compound (K-1) wherein $R^{1b}$ and $R^{1c}$ represent a methyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX338).

A compound (K-1) wherein $R^{1b}$ and $R^{1c}$ represent a methyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX339) .

A compound (K-1) wherein $R^{1b}$ and $R^{1c}$ represent a methyl group, $A^3$ represents C-F, $A^1$, $A^2$, and $A^5$ represent CH, and $A^4$ represents any one substituent indicated in [Table L1] to [Table L3] (hereinafter, referred to as "Compound class CX340).

A compound (K-1) wherein $R^{1b}$ and $R^{1c}$ represent a methyl group, $A^3$ represents C-Cl, $A^1$, $A^2$, and $A^5$ represent CH, and $A^4$ represents any one substituent indicated in [Table L1] to [Table L3] (hereinafter, referred to as "Compound class CX341).

A compound (K-1) wherein $R^{1b}$ and $R^{1c}$ represent a methyl group, $A^3$ represents C-Br, $A^1$, $A^2$, and $A^5$ represent CH, and $A^4$ represents any one substituent indicated in [Table L1] to [Table L3] (hereinafter, referred to as "Compound class CX342).

A compound (K-1) wherein $R^{1b}$ and $R^{1c}$ represent a methyl group, $A^3$ represents C-I, $A^1$, $A^2$, and $A^5$ represent CH, and $A^4$ represents any one substituent indicated in [Table L1] to [Table L3] (hereinafter, referred to as "Compound class CX343).

A compound (K-1) wherein $R^{1b}$ and $R^{1c}$ represent a methyl group, $A^3$ represents C-Me, $A^1$, $A^2$, and $A^5$ represent CH, and $A^4$ represents any one substituent indicated in [Table L1] to [Table L3] (hereinafter, referred to as "Compound class CX344).

A compound (K-1) wherein $R^{1b}$ and $R^{1c}$ represent a methyl group, $A^3$ represents C-OMe, $A^1$, $A^2$, and $A^5$ represent CH, and $A^4$ represents any one substituent indicated in [Table L1] to [Table L3] (hereinafter, referred to as "Compound class CX345).

A compound (K-1) wherein $R^{1b}$ and $R^{1c}$ represent a methyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX346).

A compound (K-1) wherein $R^{1b}$ and $R^{1c}$ represent a methyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX347).

A compound (K-1) wherein $R^{1b}$ and $R^{1c}$ represent an ethyl group, $A^2$ represents C-F, $A^1$, $A^3$, and $A^5$ represent CH, and $A^4$ represents any one substituent indicated in [Table L1] to [Table L3] (hereinafter, referred to as "Compound class CX348).

A compound (K-1) wherein $R^{1b}$ and $R^{1c}$ represent an ethyl group, $A^2$ represents C-Cl, $A^1$, $A^3$, and $A^5$ represent CH, and $A^4$ represents any one substituent indicated in [Table L1] to [Table L3] (hereinafter, referred to as "Compound class CX349).

A compound (K-1) wherein $R^{1b}$ and $R^{1c}$ represent an ethyl group, $A^2$ represents C-Br, $A^1$, $A^3$, and $A^5$ represent CH, and $A^4$ represents any one substituent indicated in [Table L1] to [Table L3] (hereinafter, referred to as "Compound class CX350).

A compound (K-1) wherein $R^{1b}$ and $R^{1c}$ represent an ethyl group, $A^2$ represents C-I, $A^1$, $A^3$, and $A^5$ represent CH, and $A^4$ represents any one substituent indicated in [Table L1] to [Table L3] (hereinafter, referred to as "Compound class CX351).

A compound (K-1) wherein $R^{1b}$ and $R^{1c}$ represent an ethyl group, $A^2$ represents C-Me, $A^1$, $A^3$, and $A^5$ represent CH, and $A^4$ represents any one substituent indicated in [Table L1] to [Table L3] (hereinafter, referred to as "Compound class CX352).

A compound (K-1) wherein $R^{1b}$ and $R^{1c}$ represent an ethyl group, $A^2$ represents C-OMe, $A^1$, $A^3$, and $A^5$ represent CH, and $A^4$ represents any one substituent indicated in [Table L1] to [Table L3] (hereinafter, referred to as "Compound class CX353).

A compound (K-1) wherein $R^{1b}$ and $R^{1c}$ represent an ethyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX354).

A compound (K-1) wherein $R^{1b}$ and $R^{1c}$ represent an ethyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX35S).

A compound (K-1) wherein $R^{1b}$ and $R^{1c}$ represent an ethyl group, $A^3$ represents C-F, $A^1$, $A^2$, and $A^5$ represent CH, and $A^4$ represents any one substituent indicated in [Table L1] to [Table L3] (hereinafter, referred to as "Compound class CX356).

A compound (K-1) wherein $R^{1b}$ and $R^{1c}$ represent an ethyl group, $A^3$ represents C-Cl, $A^1$, $A^2$, and $A^5$ represent CH, and $A^4$ represents any one substituent indicated in [Table L1] to [Table L3] (hereinafter, referred to as "Compound class CX357).

A compound (K-1) wherein $R^{1b}$ and $R^{1c}$ represent an ethyl group, $A^3$ represents C-Br, $A^1$, $A^2$, and $A^5$ represent CH, and $A^4$ represents any one substituent indicated in [Table L1] to [Table L3] (hereinafter, referred to as "Compound class CX358).

A compound (K-1) wherein $R^{1b}$ and $R^{1c}$ represent an ethyl group, $A^3$ represents C-I, $A^1$, $A^2$, and $A^5$ represent CH, and $A^4$ represents any one substituent indicated in [Table L1] to (Table L3] (hereinafter, referred to as "Compound class CX359).

A compound (K-1) wherein $R^{1b}$ and $R^{1c}$ represent an ethyl group, $A^3$ represents C-Me, $A^1$, $A^2$, and $A^5$ represent CH, and $A^4$ represents any one substituent indicated in [Table L1] to (Table L3] (hereinafter, referred to as "Compound class CX360).

A compound (K-1) wherein $R^{1b}$ and $R^{1c}$ represent an ethyl group, $A^3$ represents C-OMe, $A^1$, $A^2$, and $A^5$ represent CH, and $A^4$ represents any one substituent indicated in [Table L11 to [Table L3] (hereinafter, referred to as "Compound class CX361).

A compound (K-1) wherein $R^{1b}$ and $R^{1c}$ represent an ethyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX362).

A compound (K-1) wherein $R^{1b}$ and $R^{1c}$ represent an ethyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX363).

A compound (K-1) wherein $R^{1b}$ and $R^{1c}$ represent $CF_3$, $A^2$ represents C-F, $A^1$, $A^3$, and $A^5$ represent CH, and $A^4$ represents any one substituent indicated in [Table L1] to [Table L3] (hereinafter, referred to as "Compound class CX364).

A compound (K-1) wherein $R^{1b}$ and $R^{1c}$ represent $CF_3$, $A^2$ represents C-Cl, $A^1$, $A^3$, and $A^5$ represent CH, and $A^4$ represents any one substituent indicated in [Table L1] to [Table L3] (hereinafter, referred to as "Compound class CX365).

A compound (K-1) wherein $R^{1b}$ and $R^{1c}$ represent $CF_3$, $A^2$ represents C-Br, $A^1$, $A^3$, and $A^5$ represent CH, and $A^4$ represents any one substituent indicated in [Table L1] to [Table L3] (hereinafter, referred to as "Compound class CX366).

A compound (K-1) wherein $R^{1b}$ and $R^{1c}$ represent $CF_3$, $A^2$ represents C-I, $A^1$, $A^3$, and $A^5$ represent CH, and $A^4$ represents any one substituent indicated in [Table L1] to [Table L3] (hereinafter, referred to as "Compound class CX367).

A compound (K-1) wherein $R^{1b}$ and $R^{1c}$ represent $CF_3$, $A^2$ represents C-Me, $A^1$, $A^3$, and $A^5$ represent CH, and $A^4$ represents any one substituent indicated in [Table L1] to [Table L3] (hereinafter, referred to as "Compound class CX368).

A compound (K-1) wherein $R^{1b}$ and $R^{1c}$ represent $CF_3$, $A^2$ represents C-OMe, $A^1$, $A^3$, and $A^5$ represent CH, and $A^4$ represents any one substituent indicated in [Table L1] to [Table L3] (hereinafter, referred to as "Compound class CX369).

A compound (K-1) wherein $R^{1b}$ and $R^{1c}$ represent $CF_3$, $A^2$ represents C-CFs, $A^1$, $A^3$, and $A^5$ represent CH, and $A^4$ represents any one substituent indicated in [Table L1] to [Table L3] (hereinafter, referred to as "Compound class CX370).

A compound (K-1) wherein $R^{1b}$ and $R^{1c}$ represent $CF_3$, $A^2$ represents C-$OCF_3$, $A^1$, $A^3$, and $A^5$ represent CH,

and $A^4$ represents any one substituent indicated in [Table L1] to [Table L3] (hereinafter, referred to as "Compound class CX371).

A compound (K-1) wherein $R^{1b}$ and $R^{1c}$ represent $CF_3$, $A^3$ represents C-F, $A^1$, $A^2$, and $A^5$ represent CH, and $A^4$ represents any one substituent indicated in [Table L1] to [Table L3] (hereinafter, referred to as "Compound class CX372).

A compound (K-1) wherein $R^{1b}$ and $R^{1c}$ represent $CF_3$, $A^3$ represents C-Cl, $A^1$, $A^2$, and $A^5$ represent CH, and $A^4$ represents any one substituent indicated in [Table L1] to [Table L3] (hereinafter, referred to as "Compound class CX373).

A compound (K-1) wherein $R^{1b}$ and $R^{1c}$ represent $CF_3$, $A^3$ represents C-Br, $A^1$, $A^2$, and $A^5$ represent CH, and $A^4$ represents any one substituent indicated in [Table L1] to [Table L3] (hereinafter, referred to as "Compound class CX374).

A compound (K-1) wherein $R^{1b}$ and $R^{1c}$ represent $CF_3$, $A^3$ represents C-I, $A^1$, $A^2$, and $A^5$ represent CH, and $A^4$ represents any one substituent indicated in [Table L1] to [Table L3] (hereinafter, referred to as "Compound class CX375).

A compound (K-1) wherein $R^{1b}$ and $R^{1c}$ represent $CF_1$, $A^3$ represents C-Me, $A^1$, $A^2$, and $A^5$ represent CH, and $A^4$ represents any one substituent indicated in [Table L1] to [Table L3] (hereinafter, referred to as "Compound class CX376) .

A compound (N-1) wherein $R^{1b}$ and $R^{1c}$ represent $CF_3$, $A^3$ represents C-OMe, $A^1$, $A^2$, and $A^5$ represent CH, and $A^4$ represents any one substituent indicated in [Table L1] to [Table L3] (hereinafter, referred to as "Compound class CX377).

A compound (K-1) wherein $R^{1b}$ and $R^{1c}$ represent $CF_3$, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX378).

A compound (K-1) wherein $R^{1b}$ and $R^{1c}$ represent $CF_3$, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX379).

A compound (K-1) wherein $R^{1b}$ and $R^{1c}$ represent a cyclopropyl group, $A^2$ represents C-F, $A^1$, $A^3$, and $A^5$ represent CH, and $A^4$ represents any one substituent indicated in [Table L1] to [Table L3] (hereinafter, referred to as "Compound class CX380).

A compound (K-1) wherein $R^{1b}$ and $R^{1c}$ represent a cyclopropyl group, $A^2$ represents C-Cl, $A^1$, $A^3$, and $A^5$ represent CH, and $A^4$ represents any one substituent indicated in [Table L1] to [Table L3] (hereinafter, referred to as "Compound class CX381).

A compound (K-1) wherein $R^{1b}$ and $R^{1c}$ represent a cyclopropyl group, $A^2$ represents C-Br, $A^1$, $A^3$, and $A^5$ represent CH, and $A^4$ represents any one substituent indicated in [Table L1] to [Table L3] (hereinafter, referred to as "Compound class CX382).

A compound (K-1) wherein $R^{1b}$ and $R^{1c}$ represent a cyclopropyl group, $A^2$ represents C-I, $A^1$, $A^3$, and $A^5$ represent CH, and $A^4$ represents any one substituent indicated in [Table LI] to [Table L3] (hereinafter, referred to as "Compound class CX383).

A compound (X-1) wherein $R^{1b}$ and $R^{1c}$ represent a cyclopropyl group, $A^2$ represents C-Me, $A^1$, $A^3$, and $A^5$ represent CH, and $A^4$ represents any one substituent indicated in [Table L1] to [Table L3] (hereinafter, referred to as "Compound class CX394).

A compound (K-1) wherein $R^{1b}$ and $R^{1c}$ represent a cyclopropyl group, $A^2$ represents C-OMe, $A^1$, $A^3$, and $A^5$ represent CH, and $A^4$ represents any one substituent indicated in [Table L1] to [Table L3] (hereinafter, referred to as "Compound class CX385).

A compound (K-1) wherein $R^{1b}$ and $R^{1c}$ represent a cyclopropyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX386).

A compound (K-1) wherein $R^{1b}$ and $R^{1c}$ represent a cyclopropyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX387).

A compound (K-1) wherein $R^{1b}$ and $R^{1c}$ represent a cyclopropyl group, $A^3$ represents C-F, $A^1$, $A^2$, and $A^5$ represent CH, and $A^4$ represents any one substituent indicated in [Table L1] to [Table L3] (hereinafter, referred to as "Compound class CX388).

A compound (X-1) wherein $R^{1b}$ and $R^{1c}$, represent a cyclopropyl group, $A^3$ represents C-Cl, $A^1$, $A^2$, and $A^5$ represent CH, and $A^4$ represents any one substituent indicated in [Table L1] to [Table L3] (hereinafter, referred to as "Compound class CX309).

A compound (K-1) wherein $R^{1b}$ and $R^{1c}$ represent a cyclopropyl group, $A^3$ represents C-Br, $A^1$, $A^2$, and $A^5$ represent CH, and $A^4$ represents any one substituent indicated in [Table L1] to [Table L3] (hereinafter, referred to as

"Compound class CX390).

A compound (K-1) wherein $R^{1b}$ and $R^{1c}$ represent a cyclopropyl group, $A^3$ represents C-I, $A^1$, $A^2$, and $A^5$ represent CH, and $A^4$ represents any one substituent indicated in [Table L1] to [Table L3] (hereinafter, referred to as "Compound class CX391).

A compound (K-1) wherein $R^{1b}$ and $R^{1c}$ represent a cyclopropyl group, $A^3$ represents C-Me, $A^1$, $A^2$, and $A^5$ represent CH, and $A^4$ represents any one substituent indicated in [Table L1] to [Table L3] (hereinafter, referred to as "Compound class CX302).

A compound (K-1) wherein $R^{1b}$ and $R^{1c}$ represent a cyclopropyl group, $A^3$ represents C-OMe, $A^1$, $A^2$, and $A^5$ represent CH, and $A^4$ represents any one substituent indicated in (Table L1] to [Table L3] (hereinafter, referred to as "Compound class CX393).

A compound (X-1) wherein $R^{1b}$ and $R^{1c}$, represent a cyclopropyl group. $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX394).

A compound (K-1) wherein $R^{1b}$ and $R^{1c}$ represent a cyclopropyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX395).

A compound (K-1) wherein $R^{1b}$ represents a fluorine atom, $R^{1c}$ represents a methyl group, $A^2$ represents C-F, $A^1$, $A^3$, and $A^5$ represent CH, and $A^4$ represents any one substituent indicated in [Table L1] to [Table L3] (hereinafter, referred to as "Compound class CX396).

A compound (K-1) wherein $R^{1b}$ represents a fluorine atom, $R^{1c}$ represents a methyl group, $A^2$ represents C-Cl, $A^1$, $A^3$, and $A^5$ represent CH, and $A^4$ represents any one substituent indicated in [Table L1] to [Table L3] (hereinafter, referred to as "Compound class CX397).

A compound (K~1) wherein $R^{1b}$ represents a fluorine atom, $R^{1c}$ represents a methyl group, $A^2$ represents C-Br, $A^1$, $A^3$, and $A^5$ represent CH, and $A^4$ represents any one substituent indicated in [Table L1] to [Table L3] (hereinafter, referred to as "Compound class CX398).

A compound (K-1) wherein $R^{1b}$ represents a fluorine atom, $R^{1c}$ represents a methyl group, $A^2$ represents C-I, $A^1$, $A^3$, and $A^5$ represent CH, and $A^4$ represents any one substituent indicated in [Table L1] to [Table L3] (hereinafter, referred to as "Compound class CX399).

A compound (K-1) wherein $R^{1b}$ represents a fluorine atom, $R^{1c}$ represents a methyl group, $A^2$ represents C-Me, $A^1$, $A^3$, and $A^5$ represent CH, and $A^4$ represents any one substituent indicated in [Table L1] to [Table L3] (hereinafter, referred to as "Compound class CX400).

A compound (K-1) wherein $R^{1b}$ represents a fluorine atom, $R^{1c}$ represents a methyl group, $A^2$ represents C-OMe, $A^1$, $A^3$, and $A^5$ represent CH, and $A^4$ represents any one substituent indicated in [Table L1] to [Table L3] (hereinafter, referred to as "Compound class CX401) .

A compound (K-1) wherein $R^{1b}$ represents a fluorine atom, $R^{1c}$ represents a methyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class Cr402).

A compound (K~1) wherein $R^{1b}$ represents a fluorine atom, $R^{1c}$ represents a methyl group, $A^2$ represents C-$OCF_3$, $A^1$, $A^3$, and $A^5$ represent CH, and $A^4$ represents any one. substituent indicated in [Table L1] to [Table L3] (hereinafter, referred to as "Compound class CX403).

A compound (K-1) wherein $R^{1b}$ represents a fluorine atom, $R^{1c}$ represents a methyl group, $A^3$ represents C-F, $A^1$, $A^2$, and $A^5$ represent CH, and $A^4$ represents any one substituent indicated in [Table L1] to [Table L3] (hereinafter, referred to as "Compound class CX404).

A compound (K-1) wherein $R^{1b}$ represents a fluorine atom, $R^{1c}$ represents a methyl group, $A^3$ represents C-Cl, $A^1$, $A^2$, and $A^5$ represent CH, and $A^4$ represents any one substituent indicated in [Table L1] to [Table L3] (hereinafter, referred to as "Compound class CX405).

A compound (K-1) wherein $R^{1b}$ represents a fluorine atom, $R^{1c}$ represents a methyl group, $A^3$ represents C-Br, $A^1$, $A^2$, and $A^5$ represent CH, and $A^4$ represents any one substituent indicated in [Table L1] to [Table L3] (hereinafter, referred to as "Compound class CX406).

A compound (K-1) wherein $R^{1b}$ represents a fluorine atom, $R^{1c}$ represents a methyl group, $A^3$ represents C-I, $A^1$, $A^2$, and $A^5$ represent CH, and $A^4$ represents any one substituent indicated in [Table L1] to [Table L3] (hereinafter, referred to as "Compound class Cr407).

A compound (K~1) wherein $R^{1b}$ represents a fluorine atom, $R^{1c}$ represents a methyl group, $A^3$ represents C-Me, $A^1$, $A^2$, and $A^5$ represent CH, and $A^4$ represents any one substituent indicated in [Table L1] to [Table L3] (hereinafter, referred to as "Compound class CX408).

A compound (K-1) wherein $R^{1b}$ represents a fluorine atom, $R^{1c}$ represents a methyl group, $A^3$ represents C-OMe, $A^1$, $A^2$, and $A^5$ represent CH, and $A^4$ represents any one substituent indicated in [Table L1] to [Table L3] (hereinafter, referred to as "Compound class CX409).

A compound (K-1) wherein $R^{1b}$ represents a fluorine atom, $R^{1c}$ represents a methyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX410).

A compound (K-1) wherein $R^{1b}$ represents a fluorine atom, $R^{1c}$ represents a methyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX411).

A compound (K-1) wherein $R^{1b}$ represents a chlorine atom, $R^{1c}$ represents a methyl group. $A^2$ represents C-F, $A^1$, $A^3$, and $A^5$ represent CH, and $A^4$ represents any one substituent indicated in [Table L1] to [Table L3] (hereinafter, referred to as "Compound class CX412).

A compound (N-1) wherein $R^{1b}$ represents a chlorine atom, $R^{1c}$ represents a methyl group, $A^2$ represents C-Cl, $A^1$, $A^3$, and $A^5$ represent CH, and $A^4$ represents any one substituent indicated in [Table L1] to [Table. L3] (hereinafter, referred to as "Compound class CX413).

A compound (N-1) wherein $R^{1b}$ represents a chlorine atom, $R^{1c}$ represents a methyl group, $A^2$ represents C-Br, $A^1$, $A^3$, and $A^5$ represent CH, and $A^4$ represents any one substituent indicated in [Table L1] to [Table L3] (hereinafter, referred to as "Compound class CX414).

A compound (K-1) wherein $R^{1b}$ represents a chlorine atom, $R^{1c}$ represents a methyl group, $A^2$ represents C-I, $A^1$, $A^3$, and $A^5$ represent CH, and $A^4$ represents any one substituent indicated in [Table L1] to [Table L3] (hereinafter, referred to as "Compound class CX415).

A compound (K-1) wherein $R^{1b}$ represents a chlorine atom, $R^{1c}$ represents a methyl group, $A^2$ represents C-Me, $A^1$, $A^3$, and $A^5$ represent CH, and $A^4$ represents any one substituent indicated in [Table L1] to [Table L3] (hereinafter, referred to as "Compound class CX416).

A compound (K-1) wherein $R^{1b}$ represents a chlorine atom, $R^{1c}$ represents a methyl group, $A^2$ represents C-OMe, $A^1$, $A^3$, and $A^5$ represent CH, and $A^4$ represents any one substituent indicated in [Table L1] to [Table L3] (hereinafter, referred to as "Compound class CX417).

A compound (K-1) wherein $R^{1b}$ represents a chlorine atom, $R^{1c}$ represents a methyl group, $A^2$ represents C-$CF_3$, $A^1$, $A^3$, and $A^5$, represent CH, and $A^4$ represents any one substituent indicated in [Table L1] to [Table. L3] (hereinafter, referred to as "Compound class CX418).

A compound (K-1) wherein $R^{1b}$ represents a chlorine atom, $R^{1c}$ represents a methyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX419).

A compound (K-1) wherein $R^{1b}$ represents a chlorine atom, $R^{1c}$ represents a methyl group, $A^3$ represents C-F, $A^1$, $A^2$, and $A^5$ represent CH, and $A^4$ represents any one substituent indicated in [Table L1] to [Table L3] (hereinafter, referred to as "Compound class CX420).

A compound (K-1) wherein $R^{1b}$ represents a chlorine atom, $R^{1c}$ represents a methyl group, $A^3$ represents C-Cl, $A^1$, $A^2$, and $A^5$ represent CH, and $A^4$ represents any one substituent indicated in [Table L1] to [Table L3] (hereinafter, referred to as "Compound class CX421).

A compound (K-1) wherein $R^{1b}$ represents a chlorine atom, $R^{1c}$ represents a methyl group, $A^3$ represents C-Br, $A^1$, $A^2$, and $A^5$ represent CH, and $A^4$ represents any one substituent indicated in [Table L1] to [Table L3] (hereinafter, referred to as "Compound class CX422).

A compound (K-1) wherein $R^{1b}$ represents a chlorine atom, $R^{1c}$ represents a methyl group, $A^3$ represents C-I, $A^1$, $A^2$, and $A^5$ represent CH, and $A^4$ represents any one substituent indicated in [Table L1] to [Table. L3] (hereinafter, referred to as "Compound class CX423).

A compound (K-1) wherein $R^{1b}$ represents a chlorine atom, $R^{1c}$ represents a methyl group, $A^3$ represents C-Me, $A^1$, $A^2$, and $A^5$ represent CH, and $A^4$ represents any one substituent indicated in [Table L1] to [Table L3] (hereinafter, referred to as "Compound class CX424).

A compound (K-1) wherein $R^{1b}$ represents a chlorine atom, $R^{1c}$ represents a methyl group, $A^3$ represents C-OMe, $A^1$, $A^2$, and $A^5$ represent CH, and $A^4$ represents any one substituent indicated in [Table L1] to [Table L3] (hereinafter, referred to as "Compound class CX425).

A compound (K-1) wherein $R^{1b}$ represents a chlorine atom, $R^{1c}$ represents a methyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX426).

A compound (K-1) wherein $R^{1b}$ represents a chlorine atom, $R^{1c}$ represents a methyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX427).

A compound (K-1) wherein $R^{1b}$ represents a bromine atom, $R^{1c}$ represents a methyl group, $A^2$ represents C-F, $A^1$, $A^3$, and $A^5$ represent CH, and $A^4$ represents any one substituent indicated in [Table L1] to [Table. L3] (hereinafter, referred to as "Compound class CX428).

A compound (K-1) wherein $R^{1b}$ represents a bromine atom, $R^{1c}$ represents a methyl group, $A^2$ represents C-

Cl, $A^1$, $A^3$, and $A^5$ represent CH, and $A^4$ represents any one substituent indicated in [Table L1] to [Table L3] (hereinafter, referred to as "Compound class CX429).

A compound (K-1) wherein $R^{1b}$ represents a bromine atom, $R^{1c}$ represents a methyl group, $A^2$ represents C-Br, $A^1$, $A^3$, and $A^5$ represent CH, and $A^4$ represents any one substituent indicated in [Table L1] to [Table L3] (hereinafter, referred to as "Compound class CX430).

A compound (K-1) wherein $R^{1b}$ represents a bromine atom, $R^{1c}$represents a methyl group, $A^2$ represents C-I, $A^1$, $A^3$, and $A^5$ represent CH, and $A^4$ represents any one substituent indicated in [Table L1] to [Table L3] (hereinafter, referred to as "Compound class CX431).

A compound (K-1) wherein $R^{1b}$ represents a bromine atom, $R^{1c}$ represents a methyl group, $A^2$ represents C-Me, $A^1$, $A^3$, and $A^5$ represent CH, and $A^4$ represents any one substituent indicated in [Table L1] to [Table L3] (hereinafter, referred to as "Compound class CX432).

A compound (K-1) wherein $R^{1b}$ represents a bromine atom, $R^{1c}$ represents a methyl group, $A^2$ represents C-OMe, $A^1$, $A^3$, and $A^5$ represent CH, and $A^4$ represents any one substituent indicated in [Table L1] to [Table. L3] (hereinafter, referred to as "Compound class CX433).

A compound (K-1) wherein $R^{1b}$ represents a bromine atom, $R^{1c}$ represents a methyl group, $A^2$ represents C-$CF_3$ $A^1$, $A^3$, and $A^5$ represent CH, and $A^4$ represents any one substituent indicated in [Table L1] to [Table L3] (hereinafter, referred to as "Compound class CX434).

A compound (K-1) wherein $R^{1b}$ represents a bromine atom, $R^{1c}$ represents a methyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX435).

A compound (K-1) wherein $R^{1b}$ represents a bromine atom, $R^{1c}$ represents a methyl group, $A^3$ represents C-F, $A^1$, $A^2$, and $A^5$ represent CH, and $A^4$ represents any one substituent indicated in [Table L1] to [Table L3] (hereinafter, referred to as "Compound class CX436).

A compound (R-1) wherein $R^{1b}$ represents a bromine atom, $R^{1c}$ represents a methyl group, $A^3$ represents C-Cl, $A^1$, $A^2$, and $A^5$ represent CH, and $A^4$ represents any one substituent indicated in [Table L1] to [Table L3] (hereinafter, referred to as "Compound class CX437).

A compound (K-1) wherein $R^{1b}$ represents a bromine atom, $T^{1c}$ represents a methyl group, $A^3$ represents C-Br, $A^1$, $A^2$, and $A^5$ represent CH, and $A^4$ represents any one substituent indicated in [Table L1] to [Table. L3] (hereinafter, referred to as "Compound class CX438).

A compound (K-1) wherein $R^{1b}$ represents a bromine atom, $R^{1c}$ represents a methyl group, $A^3$ represents C-I, $A^1$, $A^2$, and $A^5$ represent CH, and $A^4$ represents any one substituent indicated in [Table L1] to [Table L3] (hereinafter, referred to as "Compound class CX439).

A compound (K-1) wherein $R^{1b}$ represents a bromine atom, $R^{1c}$ represents a methyl group, $A^3$ represents C-Me, $A^1$, $A^2$, and $A^5$ represent CH, and $A^4$ represents any one substituent indicated in [Table L1] to [Table L3] (hereinafter, referred to as "Compound class CX440).

A compound (K-1) wherein $R^{1b}$ represents a bromine atom, $R^{1c}$ represents a methyl group, $A^3$ represents C-OMe, $A^1$, $A^2$, and $A^5$ represent CH, and $A^4$ represents any one substituent indicated in [Table L1] to [Table L3] (hereinafter, referred to as "Compound class CX441).

A compound (R-1) wherein $R^{1b}$ represents a bromine atom, $R^{1c}$ represents a methyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX442).

A compound (K-1) wherein $R^{1b}$ represents a bromine atom, $R^{1c}$ represents a methyl group, $A^3$ represents C-$OCF_3$, $A^1$, $A^2$, and $A^5$ represent CH, and $A^4$ represents any one substituent indicated in [Table L1] to [Table. L3] (hereinafter, referred to as "Compound class CX443).

A compound (K-1) wherein $R^{1b}$ represents an iodine atom, $R^{1c}$ represents a methyl group, $A^2$ represents C-F**,** $A^1$, $A^3$, and $A^5$ represent CH, and $A^4$ represents any one substituent indicated in [Table L1] to [Table L3] (hereinafter, referred to as "Compound class CX444).

A compound (K-1) wherein $R^{1b}$ represents an iodine atom, $R^{1c}$ represents a methyl group, $A^2$ represents C-Cl, $A^1$, $A^3$, and $A^5$ represent CH, and $A^4$ represents any one substituent indicated in [Table L1] to [Table L3] (hereinafter, referred to as "Compound class CX445).

A compound (K-1) wherein $R^{1b}$ represents an iodine atom, $R^{1c}$ represents a methyl group, $A^2$ represents C-Br, $A^1$, $A^3$, and $A^5$ represent CH, and $A^4$ represents any one substituent indicated in [Table L1] to [Table L3] (hereinafter, referred to as "Compound class CX446).

A compound (R-1) wherein $R^{1b}$ represents an iodine atom, $R^{1c}$ represents a methyl group, $A^2$ represents C-I, $A^1$, $A^3$, and $A^5$ represent CH, and $A^4$ represents any one substituent indicated in [Table L1] to [Table L3] (hereinafter, referred to as "Compound class CX447).

A compound (N-1) wherein $R^{1b}$ represents an iodine atom, $T^{1c}$ represents a methyl group, $A^2$ represents C-Me, $A^1$, $A^3$, and $A^5$ represent CH, and $A^4$ represents any one substituent indicated in [Table L1] to [Table. L3] (hereinafter,

referred to as "Compound class CX448).

A compound (K-1) wherein $R^{1b}$ represents an iodine atom, $R^{1c}$ represents a methyl group, $A^2$ represents C-OMe, $A^1$, $A^3$, and $A^5$ represent CH, and $A^4$ represents any one substituent indicated in [Table L1] to [Table L3] (hereinafter, referred to as "Compound class CX449).

A compound (K-1) wherein $R^{1b}$ represents an iodine atom, $R^{1c}$ represents a methyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX450).

A compound (K-1) wherein $R^{1b}$ represents an iodine atom, $R^{1c}$ represents a methyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX451).

A compound (K-1) wherein $R^{1b}$ represents an iodine atom, $R^{1c}$ represents a methyl group, $A^3$ represents C-F, $A^1$, $A^2$, and $A^5$ represent CH, and $A^4$ represents any one substituent indicated in [Table L1] to [Table L3] (hereinafter, referred to as "Compound class CX452).

A compound (K-1) wherein $R^{1b}$ represents an iodine atom, $R^{1c}$ represents a methyl group, $A^3$ represents C-C1, $A^1$, $A^2$, and $A^5$ represent CH, and $A^4$ represents any one substituent indicated in [Table L1] to [Table L3] (hereinafter, referred to as "Compound class CX453).

A compound (K-1) wherein $R^{1b}$ represents an iodine atom, $R^{1c}$ represents a methyl group, $A^3$ represents C-Br, $A^1$, $A^2$, and $A^5$ represent CH, and $A^4$ represents any one substituent indicated in [Table L1] to [Table L3] (hereinafter, referred to as "Compound class CX454).

A compound (K-1) wherein $R^{1b}$ represents an iodine atom, $R^{1c}$ represents a methyl group, $A^3$ represents C-I, $A^1$, $A^2$, and $A^5$ represent CH, and $A^4$ represents any one substituent indicated in [Table L1] to [Table L3] (hereinafter, referred to as "Compound class CX455).

A compound (K-1) wherein $R^{1b}$ represents an iodine atom, $R^{1c}$ represents a methyl group, $A^3$ represents C-Me, $A^1$, $A^2$, and $A^5$ represent CH, and $A^4$ represents any one substituent indicated in [Table L1] to [Table L3] (hereinafter, referred to as "Compound class CX456).

A compound (K-1) wherein $R^{1b}$ represents an iodine atom, $R^{1c}$ represents a methyl group, $A^3$ represents C-OMe, $A^1$, $A^2$, and $A^5$ represent CH, and $A^4$ represents any one substituent indicated in [Table L1] to [Table L3] (hereinafter, referred to as "Compound class CX457).

A compound (K-1) wherein $R^{1b}$ represents an iodine atom, $R^{1c}$ represents a methyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX458).

A compound (K-1) wherein $R^{1b}$ represents an iodine atom, $R^{1c}$ represents a methyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX459).

A compound (K-1) wherein $R^{1b}$ represents a fluorine atom, $R^{1c}$ represents an ethyl group, $A^2$ represents C-F, $A^1$, $A^3$, and $A^5$ represent CH, and $A^4$ represents any one substituent indicated in [Table L1] to [Table L3] (hereinafter, referred to as "Compound class CX460).

A compound (K-1) wherein $R^{1b}$ represents a fluorine atom, $R^{1c}$ represents an ethyl group, $A^2$ represents C-Cl, $A^1$, $A^3$, and $A^5$ represent CH, and $A^4$ represents any one substituent indicated in [Table L1] to [Table L3] (hereinafter, referred to as "Compound class CX461).

A compound (K-1) wherein $R^{1b}$ represents a fluorine atom, $R^{1c}$ represents an ethyl group, $A^2$ represents C-Br, $A^1$, $A^3$, and $A^5$ represent CH, and $A^4$ represents any one substituent indicated in [Table L1] to [Table L3] (hereinafter, referred to as "Compound class CX462).

A compound (K-1) wherein $R^{1b}$ represents a fluorine atom, $R^{1c}$ represents an ethyl group, $A^2$ represents C-I, $A^1$, $A^3$, and $A^5$ represent CH, and $A^4$ represents any one substituent indicated in [Table L1] to [Table L3] (hereinafter, referred to as "Compound class CX463).

A compound (K-1) wherein $R^{1b}$ represents a fluorine atom, $R^{1c}$ represents an ethyl group, $A^2$ represents C-Me, $A^1$, $A^3$, and $A^5$ represent CH, and $A^4$ represents any one substituent indicated in [Table L1] to [Table L3] (hereinafter, referred to as "Compound class CX464).

A compound (K-1) wherein $R^{1b}$ represents a fluorine atom, $R^{1c}$ represents an ethyl group, $A^2$ represents C-OMe, $A^1$, $A^3$, and $A^5$ represent CH, and $A^4$ represents any one substituent indicated in [Table L1] to [Table L3] (hereinafter, referred to as "Compound class CX465).

A compound (K-1) wherein $R^{1b}$ represents a fluorine atom, $R^{1c}$ represents an ethyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX466).

A compound (K-1) wherein $R^{1b}$ represents a fluorine atom, $R^{1c}$ represents an ethyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX467).

A compound (K-1) wherein $R^{1b}$ represents a fluorine atom, $R^{1c}$ represents an ethyl group, $A^3$ represents C-F, $A^1$, $A^2$, and $A^5$ represent CH, and $A^4$ represents any one substituent indicated in [Table L1] to [Table L3] (hereinafter, referred to as "Compound class CX468).

A compound (K-1) wherein $R^{1b}$ represents a fluorine atom, $R^{1c}$ represents an ethyl group, $A^3$ represents C-Cl, $A^1$, $A^2$, and $A^5$ represent CH, and $A^4$ represents any one substituent indicated in [Table L1] to [Table L3] (hereinafter, referred to as "Compound class CX469).

A compound (K-1) wherein $R^{1b}$ represents a fluorine atom, $R^{1c}$ represents an ethyl group, $A^3$ represents C-Br, $A^1$, $A^2$, and $A^5$ represent CH, and $A^4$ represents any one substituent indicated in [Table L1] to [Table L3] (hereinafter, referred to as "Compound class CX470).

A compound (K-1) wherein $R^{1b}$ represents a fluorine atom, $R^{1c}$ represents an ethyl group, $A^3$ represents C-I, $A^1$, $A^2$, and $A^5$ represent CH, and $A^4$ represents any one substituent indicated in [Table L1] to [Table L3] (hereinafter, referred to as "Compound class CX471).

A compound (K-1) wherein $R^{1b}$ represents a fluorine atom, $R^{1c}$ represents an ethyl group, $A^3$ represents C-Me, $A^1$, $A^2$, and $A^5$ represent CH, and $A^4$ represents any one substituent indicated in [Table L1] to [Table L3] (hereinafter, referred to as "Compound class CX472).

A compound (K-1) wherein $R^{1b}$ represents a fluorine atom, $R^{1c}$ represents an ethyl group, $A^3$ represents C-OMe, $A^1$, $A^2$, and $A^5$ represent CH, and $A^4$ represents any one substituent indicated in [Table L1] to [Table L3] (hereinafter, referred to as "Compound class CX473).

A compound (K-1) wherein $R^{1b}$ represents a fluorine atom, $R^{1c}$ represents an ethyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX474).

A compound (K-1) wherein $R^{1b}$ represents a fluorine atom, $R^{1c}$ represents an ethyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX475).

A compound (K-1) wherein $R^{1b}$ represents a chlorine atom, $R^{1c}$ represents an ethyl group, $A^2$ represents C-F, $A^1$, $A^3$, and $A^5$ represent CH, and $A^4$ represents any one substituent indicated in [Table L1] to [Table L3] (hereinafter, referred to as "Compound class CX476).

A compound (K-1) wherein $R^{1b}$ represents a chlorine atom, $R^{1c}$ represents an ethyl group, $A^2$ represents C-Cl, $A^1$, $A^3$, and $A^5$ represent CH, and $A^4$ represents any one substituent indicated in [Table L1] to [Table L3] (hereinafter, referred to as "Compound class CX477).

A compound (K-1) wherein $R^{1b}$ represents a chlorine atom, $R^{1c}$ represents an ethyl group, $A^2$ represents C-Br, $A^1$, $A^3$, and $A^5$ represent CH, and $A^4$ represents any one substituent indicated in [Table L1] to [Table L3] (hereinafter, referred to as "Compound class CX478).

A compound (K-1) wherein $R^{1b}$ represents a chlorine atom, $R^{1c}$ represents an ethyl group, $A^2$ represents C-I, $A^1$, $A^3$, and $A^5$ represent CH, and $A^4$ represents any one substituent indicated in [Table L1] to [Table L3] (hereinafter, referred to as "Compound class CX479).

A compound (K-1) wherein $R^{1b}$ represents a chlorine atom, $R^{1c}$ represents an ethyl group, $A^2$ represents C-Me, $A^1$, $A^3$, and $A^5$ represent CH, and $A^4$ represents any one substituent indicated in [Table L1] to [Table L3] (hereinafter, referred to as "Compound class CX480).

A compound (K-1) wherein $R^{1b}$ represents a chlorine atom, $R^{1c}$ represents an ethyl group, $A^2$ represents C-OMe, $A^1$, $A^3$, and $A^5$ represent CH, and $A^4$ represents any one substituent indicated in [Table L1] to [Table L3] (hereinafter, referred to as "Compound class CX481).

A compound (K-1) wherein $R^{1b}$ represents a chlorine atom, $R^{1c}$ represents an ethyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX482).

A compound (K-1) wherein $R^{1b}$ represents a chlorine atom, $R^{1c}$ represents an ethyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX483).

A compound (K-1) wherein $R^{1b}$ represents a chlorine atom, $R^{1c}$ represents an ethyl group, $A^3$ represents C-F, $A^1$, $A^2$, and $A^5$ represent CH, and $A^4$ represents any one substituent indicated in [Table L1] to [Table L3] (hereinafter, referred to as "Compound class CX494).

A compound (K-1) wherein $R^{1b}$ represents a chlorine atom, $R^{1c}$ represents an ethyl group, $A^3$ represents C-Cl, $A^1$, $A^2$, and $A^5$ represent CH, and $A^4$ represents any one substituent indicated in [Table L1] to [Table L3] (hereinafter, referred to as "Compound class CX485).

A compound (K-1) wherein $R^{1b}$ represents a chlorine atom, $R^{1c}$ represents an ethyl group, $A^3$ represents C-Br, $A^1$, $A^2$, and $A^5$ represent CH, and $A^4$ represents any one substituent indicated in [Table L1] to [Table L3] (hereinafter, referred to as "Compound class CX486).

A compound (K-1) wherein $R^{1b}$ represents a chlorine atom, $R^{1c}$ represents an ethyl group, $A^3$ represents C-I,

$A^1$, $A^2$, and $A^5$ represent CH, and $A^4$ represents any one substituent indicated in [Table L1] to [Table L3] (hereinafter, referred to as "Compound class CX487).

A compound (K-1) wherein $R^{1b}$ represents a chlorine atom, $R^{1c}$ represents an ethyl group, $A^3$ represents C-Me, $A^1$, $A^2$, and $A^5$ represent CH, and $A^4$ represents any one substituent indicated in [Table L1] to [Table L3] (hereinafter, referred to as "Compound class CX488).

A compound (K-1) wherein $R^{1b}$ represents a chlorine atom, $R^{1c}$ represents an ethyl group, $A^3$ represents C-OMe, $A^1$, $A^2$, and $A^5$ represent CH, and $A^4$ represents any one substituent indicated in [Table L1] to [Table L3] (hereinafter, referred to as "Compound class CX499).

A compound (K-1) wherein $R^{1b}$ represents a chlorine atom, $R^{1c}$ represents an ethyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX490).

A compound (K-1) wherein $R^{1b}$ represents a chlorine atom, $R^{1c}$ represents an ethyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX491).

A compound (K-1) wherein $R^{1b}$ represents a bromine atom, $R^{1c}$ represents an ethyl group, $A^2$ represents C-F, $A^1$, $A^3$, and $A^5$ represent CH, and $A^4$ represents any one substituent indicated in [Table L1] to [Table L3] (hereinafter, referred to as "Compound class CX492).

A compound (K-1) wherein $R^{1b}$ represents a bromine atom, $R^{1c}$ represents an ethyl group, $A^2$ represents C-Cl, $A^1$, $A^3$, and $A^5$ represent CH, and $A^4$ represents any one substituent indicated in [Table L1] to [Table L3] (hereinafter, referred to as "Compound class CX493).

A compound (K-1) wherein $R^{1b}$ represents a bromine atom, $R^{1c}$ represents an ethyl group, $A^2$ represents C-Br, $A^1$, $A^3$, and $A^5$ represent CH, and $A^4$ represents any one substituent indicated in [Table L1] to [Table L3] (hereinafter, referred to as "Compound class CX494).

A compound (K-1) wherein $R^{1b}$ represents a bromine atom, $R^{1c}$ represents an ethyl group, $A^2$ represents C-I, $A^1$, $A^3$, and $A^5$ represent CH, and $A^4$ represents any one substituent indicated in [Table L1] to [Table L3] (hereinafter, referred to as "Compound class CX495).

A compound (K-1) wherein $R^{1b}$ represents a bromine atom, $R^{1c}$ represents an ethyl group, $A^2$ represents C-Me, $A^1$, $A^3$, and $A^5$ represent CH, and $A^4$ represents any one substituent indicated in [Table L1] to [Table L3] (hereinafter, referred to as "Compound class CX496).

A compound (K-1) wherein $R^{1b}$ represents a bromine atom, $R^{1c}$ represents an ethyl group, $A^2$ represents C-OMe, $A^1$, $A^3$, and $A^5$ represent CH, and $A^4$ represents any one substituent indicated in [Table L1] to [Table L3] (hereinafter, referred to as "Compound class CX497).

A compound (K-1) wherein $R^{1b}$ represents a bromine atom, $R^{1c}$ represents an ethyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX498).

A compound (K-1) wherein $R^{1b}$ represents a bromine atom, $R^{1c}$ represents an ethyl group, $A^2$ represents C-$OCE_3$, $A^1$, $A^3$, and $A^5$ represent CH, and $A^4$ represents any one substituent indicated in [Table L1] to [Table L3] (hereinafter, referred to as "Compound class CX499).

A compound (R-1) wherein $R^{1b}$ represents a bromine atom, $R^{1c}$ represents an ethyl group, $A^3$ represents C-F, $A^1$, $A^2$, and $A^5$ represent CH, and $A^4$ represents any one substituent indicated in [Table L1] to [Table L3] (hereinafter, referred to as "Compound class CX500).

A compound (K-1) wherein $R^{1b}$ represents a bromine atom, $R^{1c}$ represents an ethyl group, $A^3$ represents C-Cl, $A^1$, $A^2$, and $A^5$ represent CH, and $A^4$ represents any one substituent indicated in [Table L1] to [Table L3] (hereinafter, referred to as "Compound class CX501).

A compound (K-1) wherein $R^{1b}$ represents a bromine atom, $R^{1c}$ represents an ethyl group, $A^3$ represents C-Br, $A^1$, $A^2$, and $A^5$ represent CH, and $A^4$ represents any one substituent indicated in [Table L1] to [Table L3] (hereinafter, referred to as "Compound class CX502).

A compound (K-1) wherein $R^{1b}$ represents a bromine atom, $R^{1c}$ represents an ethyl group, $A^3$ represents C-I, $A^1$, $A^2$, and $A^5$ represent CH, and $A^4$ represents any one substituent indicated in [Table L1] to [Table L3] (hereinafter, referred to as "Compound class CX5Q3).

A compound (K-1) wherein $R^{1b}$ represents a bromine atom, $R^{1c}$ represents an ethyl group, $A^3$ represents C-Me, $A^1$, $A^2$, and $A^5$ represent CH, and $A^4$ represents any one substituent indicated in [Table L1] to [Table L3] (hereinafter, referred to as "Compound class CX504).

A compound (K-1) wherein $R^{1b}$ represents a bromine atom, $R^{1c}$ represents an ethyl group, $A^3$ represents C-OMe, $A^1$, $A^2$, and $A^5$ represent CH, and $A^4$ represents any one substituent indicated in [Table L1] to [Table L3] (hereinafter, referred to as "Compound class CX505).

A compound (K-1) wherein $R^{1b}$ represents a bromine atom, $R^{1c}$ represents an ethyl group, $A^3$ represents C-$CF_3$, $A^1$, $A^2$, and $A^5$ represent CH, and $A^4$ represents any one substituent indicated in [Table L1] to [Table L3] (hereinafter,

referred to as "Compound class CX506).

A compound (N-1) wherein $R^{1b}$ represents a bromine atom, $R^{1c}$ represents an ethyl group. $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX507).

A compound (K-1) wherein $R^{1b}$ represents an iodine atom, $R^{1c}$ represents an ethyl group, $A^2$ represents C-F, $A^1$, $A^3$, and $A^5$ represent CH, and $A^4$ represents any one substituent indicated in [Table L1] to [Table L3] (hereinafter, referred to as "Compound class CX5Q8).

A compound (K-1) wherein $R^{1b}$ represents an iodine atom, $R^{1c}$ represents an ethyl group, $A^2$ represents C-Cl, $A^1$, $A^3$, and $A^5$ represent CH, and $A^4$ represents any one substituent indicated in [Table L1] to [Table L3] (hereinafter, referred to as "Compound class CX509).

A compound (K-1) wherein $R^{1b}$ represents an iodine atom, $R^{1c}$ represents an ethyl group, $A^2$ represents C-Br, $A^1$, $A^3$, and $A^5$ represent CH, and $A^4$ represents any one substituent indicated in [Table L1] to [Table L3] (hereinafter, referred to as "Compound class CX510).

A compound (K-1) wherein $R^{1b}$ represents an iodine atom, $R^{1c}$ represents an ethyl group, $A^2$ represents C-I, $A^1$, $A^3$, and $A^5$ represent CH, and $A^4$ represents any one substituent indicated in [Table L1] to [Table L3] (hereinafter, referred to as "Compound class CX511).

A compound (K-1) wherein $R^{1b}$ represents an iodine atom, $R^{1c}$ represents an ethyl group. $A^2$ represents C-Me, $A^1$, $A^3$, and $A^5$ represent CH, and $A^4$ represents any one substituent indicated in [Table L1] to [Table L3] (hereinafter, referred to as "Compound class CX512).

A compound (K-1) wherein $R^{1b}$ represents an iodine atom, $R^{1c}$ represents an ethyl group, $A^2$ represents C-OMe, $A^1$, $A^3$, and $A^5$ represent CH, and $A^4$ represents any one substituent indicated in [Table L1] to [Table L3] (hereinafter, referred to as "Compound class CX513).

A compound (N-1) wherein $R^{1b}$ represents an iodine atom, $R^{1c}$ represents an ethyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX514).

A compound (K-1) wherein $R^{1b}$ represents an iodine atom, $R^{1c}$ represents an ethyl group, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX515).

A compound (K-1) wherein $R^{1b}$ represents an iodine atom, $R^{1c}$ represents an ethyl group, $A^3$ represents C-F, $A^1$, $A^2$, and $A^5$ represent CH, and $A^4$ represents any one substituent indicated in [Table L1] to [Table L3] (hereinafter, referred to as "Compound class CX516).

A compound (K-1) wherein $R^{1b}$ represents an iodine atom, $R^{1c}$ represents an ethyl group. $A^3$ represents C-Cl, $A^1$, $A^2$, and $A^5$ represent CH, and $A^4$ represents any one substituent indicated in [Table L1] to [Table L3] (hereinafter, referred to as "Compound class CX517).

A compound (K-1) wherein $R^{1b}$ represents an iodine atom, $R^{1c}$ represents an ethyl group, $A^3$ represents C-Br, $A^1$, $A^2$, and $A^5$ represent CH, and $A^4$ represents any one substituent indicated in [Table L1] to [Table L3] (hereinafter, referred to as "Compound class CX518).

A compound (N-1) wherein $R^{1b}$ represents an iodine atom, $R^{1c}$ represents an ethyl group, $A^3$ represents C-I, $A^1$, $A^2$, and $A^5$ represent CH, and $A^4$ represents any one substituent indicated in [Table L1] to [Table L3] (hereinafter, referred to as "Compound class CX519).

A compound (K-1) wherein $R^{1b}$ represents an iodine atom, $R^{1c}$ represents an ethyl group, $A^3$ represents C-Me, $A^1$, $A^2$, and $A^5$ represent CH, and $A^4$ represents any one substituent indicated in [Table L1] to [Table L3] (hereinafter, referred to as "Compound class CX520).

A compound (K-1) wherein $R^{1b}$ represents an iodine atom, $R^{1c}$ represents an ethyl group, $A^3$ represents C-OMe, $A^1$, $A^2$, and $A^5$ represent CH, and $A^4$ represents any one substituent indicated in [Table L1] to [Table L3] (hereinafter, referred to as "Compound class CX521).

A compound (K-1) wherein $R^{1b}$ represents an iodine atom, $R^{1c}$ represents an ethyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX522).

A compound (K-1) wherein $R^{1b}$ represents an iodine atom, $R^{1c}$ represents an ethyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX523).

A compound (K-1) wherein $R^{1b}$ represents a methyl group, $R^{1c}$ represents an ethyl group, $A^2$ represents C-F, $A^1$, $A^3$, and $A^5$ represent CH, and $A^4$ represents any one substituent indicated in [Table L1] to [Table L3] (hereinafter, referred to as "Compound class CX524).

A compound (K-1) wherein $R^{1b}$ represents a methyl group, $R^{1c}$ represents an ethyl group, $A^2$ represents C-Cl, $A^1$, $A^3$, and $A^5$ represent CH, and $A^4$ represents any one substituent indicated in [Table L1] to [Table L3] (hereinafter, referred to as "Compound class CX52S).

A compound (K-1) wherein $R^{1b}$ represents a methyl group, $R^{1c}$ represents an ethyl group, $A^2$ represents C-Br, $A^1$, $A^3$, and $A^5$ represent CH, and $A^4$ represents any one substituent indicated in [Table L1] to [Table L3] (hereinafter, referred to as "Compound class CX526) .

A compound (K-1) wherein $R^{1b}$ represents a methyl group, $R^{1c}$ represents an ethyl group, $A^2$ represents C-I, $A^1$, $A^3$, and $A^5$ represent CH, and $A^4$ represents any one substituent indicated in [Table L1] to [Table L3] (hereinafter, referred to as "Compound class CX527).

A compound (X-1) wherein $R^{1b}$ represents a methyl group, $R^{1c}$ represents an ethyl group, $A^2$ represents C-Me, $A^1$, $A^3$, and $A^5$ represent CH, and $A^4$ represents any one substituent indicated in [Table L1] to [Table L3] (hereinafter, referred to as "Compound class CX528).

A compound (K-1) wherein $R^{1b}$ represents a methyl group, $R^{1c}$ represents an ethyl group, $A^2$ represents C-OMe, $A^1$, $A^3$, and $A^5$ represent CH, and $A^4$ represents any one substituent indicated in [Table L1] to [Table L3] (hereinafter, referred to as "Compound class CX529).

A compound (K-1) wherein $R^{1b}$ represents a methyl group, $R^{1c}$ represents an ethyl group, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX530).

A compound (K-1) wherein $R^{1b}$ represents a methyl group, $R^{1c}$ represents an ethyl group. $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX531).

A compound (K-1) wherein $R^{1b}$ represents a methyl group, $R^{1c}$ represents an ethyl group, $A^3$ represents C-F, $A^1$, $A^2$, and $A^5$ represent CH, and $A^4$ represents any one substituent indicated in [Table L1) to [Table L3] (hereinafter, referred to as "Compound class CX532).

A compound (K-1) wherein $R^{1b}$ represents a methyl group, $R^{1c}$ represents an ethyl group, $A^3$ represents C-Cl, $A^1$, $A^2$, and $A^5$ represent CH, and $A^4$ represents any one substituent indicated in [Table L1] to [Table L3] (hereinafter, referred to as "Compound class CX533).

A compound (R-1) wherein $R^{1b}$ represents a methyl group, $R^{1c}$ represents an ethyl group, $A^3$ represents C-Br, $A^1$, $A^2$, and $A^5$ represent CH, and $A^4$ represents any one substituent indicated in [Table L1] to [Table L3] (hereinafter, referred to as "Compound class CX534).

A compound (N-1) wherein $R^{1b}$ represents a methyl group, $R^{1c}$ represents an ethyl group, $A^3$ represents C-I, $A^1$, $A^2$, and $A^5$ represent CH, and $A^4$ represents any one substituent indicated in [Table L1] to [Table. L3] (hereinafter, referred to as "Compound class CX535).

A compound (K-1) wherein $R^{1b}$ represents a methyl group, $R^{1c}$ represents an ethyl group, $A^3$ represents C-Me, $A^1$, $A^2$, and $A^5$ represent CH, and $A^4$ represents any one substituent indicated in [Table L1] to [Table L3] (hereinafter, referred to as "Compound class CX536).

A compound (K-1) wherein $R^{1b}$ represents a methyl group, $R^{1c}$ represents an ethyl group, $A^3$ represents C-OMe, $A^1$, $A^2$, and $A^5$ represent CH, and $A^4$ represents any one substituent indicated in [Table L1) to [Table L3] (hereinafter, referred to as "Compound class CX537).

A compound (K-1) wherein $R^{1b}$ represents a methyl group, $R^{1c}$ represents an ethyl group, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX538).

A compound (R-1) wherein $R^{1b}$ represents a methyl group, $R^{1c}$ represents an ethyl group, $A^3$ represents C-$OCF_3$, $A^1$, $A^2$, and $A^5$ represent CH, and $A^4$ represents any one substituent indicated in [Table L1] to [Table L3] (hereinafter, referred to as "Compound class CX539).

A compound (N-1) wherein $R^{1b}$ represents a chlorine atom, $R^{1c}$ represents a bromine atom, $A^2$ represents C-F, $A^1$, $A^3$, and $A^5$ represent CH, and $A^4$ represents any one substituent indicated in [Table L1] to [Table. L3] (hereinafter, referred to as "Compound class CX540).

A compound (K-1) wherein $R^{1b}$ represents a chlorine atom, $R^{1c}$ represents a bromine atom, $A^2$ represents C-Cl, $A^1$, $A^3$, and $A^5$ represent CH, and $A^4$ represents any one substituent indicated in [Table L1] to [Table L3] (hereinafter, referred to as "Compound class CX541).

A compound (K-1) wherein $R^{1b}$ represents a chlorine atom, $R^{1c}$ represents an ethyl group, $A^2$ represents C-Br, $A^1$, $A^3$, and $A^5$ represent CH, and $A^4$ represents any one substituent indicated in [Table L1] to [Table L3] (hereinafter, referred to as "Compound class CX542).

A compound (K-1) wherein $R^{1b}$ represents a chlorine atom, $R^{1c}$ represents a bromine atom, $A^2$ represents C-I, $A^1$, $A^3$, and $A^5$ represent CH, and $A^4$ represents any one substituent indicated in [Table L1] to [Table L3] (hereinafter, referred to as "Compound class CX543).

A compound (K-1) wherein $R^{1b}$ represents a chlorine atom, $R^{1c}$ represents a bromine atom, $A^2$ represents C-Me, $A^1$, $A^3$, and $A^5$ represent CH, and $A^4$ represents any one substituent indicated in [Table L1] to [Table L3] (hereinafter, referred to as "Compound class CX544).

A compound (N-1) wherein $R^{1b}$ represents a chlorine atom, $R^{1c}$ represents a bromine atom, $A^2$ represents C-

OMe, $A^1$, $A^3$, and $A^5$ represent CH, and $A^4$ represents any one substituent indicated in [Table L1] to [Table. L3] (hereinafter, referred to as "Compound class CX545).

A compound (K-1) wherein $R^{1b}$ represents a chlorine atom, $R^{1c}$ represents a bromine atom, $A^2$ represents C-$CF_3$, $A^1$, $A^3$, and $A^5$ represent CH, and $A^4$ represents any one substituent indicated in [Table L1] to [Table L3] (hereinafter, referred to as "Compound class CX546).

A compound (K-1) wherein $R^{1b}$ represents a chlorine atom, $R^{1c}$ represents a bromine atom, $A^2$ represents C-$OCF_3$, $A^1$, $A^3$, and $A^5$ represent CH, and $A^4$ represents any one substituent indicated in [Table L1] to [Table L3] (hereinafter, referred to as "Compound class CX547).

A compound (K-1) wherein $R^{1b}$ represents a chlorine atom, $R^{1c}$ represents a bromine atom, $A^3$ represents C-F, $A^1$, $A^2$, and $A^5$ represent CH, and $A^4$ represents any one substituent indicated in [Table L1] to [Table L3] (hereinafter, referred to as "Compound class CX548).

A compound (K-1) wherein $R^{1b}$ represents a chlorine atom, $R^{1c}$ represents a bromine atom, $A^3$ represents C-Cl, $A^1$, $A^2$, and $A^5$ represent CH, and $A^4$ represents any one substituent indicated in [Table L1] to [Table L3] (hereinafter, referred to as "Compound class CX549).

A compound (N-1) wherein $R^{1b}$ represents a chlorine atom, $R^{1c}$ represents a bromine atom, $A^3$ represents C-Br, $A^1$, $A^2$, and $A^5$ represent CH, and $A^4$ represents any one substituent indicated in [Table L1] to [Table. L3] (hereinafter, referred to as "Compound class CX550).

A compound (K-1) wherein $R^{1b}$ represents a chlorine atom, $R^{1c}$ represents a bromine atom, $A^3$ represents C-I, $A^1$, $A^2$, and $A^5$ represent CH, and $A^4$ represents any one substituent indicated in [Table L1] to [Table L3] (hereinafter, referred to as "Compound class CX551).

A compound (K-1) wherein $R^{1b}$ represents a chlorine atom, $R^{1c}$ represents a bromine atom, $A^3$ represents C-Me, $A^1$, $A^2$, and $A^5$ represent CH, and $A^4$ represents any one substituent indicated in [Table L1] to [Table L3] (hereinafter, referred to as "Compound class CX552).

A compound (K-1) wherein $R^{1b}$ represents a chlorine atom, $R^{1c}$ represents a bromine atom, $A^3$ represents C-OMe, $A^1$, $A^2$, and $A^5$ represent CH, and $A^4$ represents any one substituent indicated in [Table L1] to [Table L3] (hereinafter, referred to as "Compound class CX553).

A compound (K-1) wherein $R^{1b}$ represents a chlorine atom, $R^{1c}$ represents a bromine atom, $A^3$ represents C-$CF_3$, $A^1$, $A^2$, and $A^5$ represent CH, and $A^4$ represents any one substituent indicated in [Table L1] to [Table L3] (hereinafter, referred to as "Compound class CX554).

A compound (N-1) wherein $R^{1b}$ represents a chlorine atom, $R^{1c}$ represents a bromine atom, $A^3$ represents C-$OCF_3$, $A^1$, $A^2$, and $A^5$ represent CH, and $A^4$ represents any one substituent indicated in [Table L1] to [Table. L3] (hereinafter, referred to as "Compound class CX555).

A compound (K-1) wherein $R^{1b}$ represents a chlorine atom, $R^{1c}$ represents any one of a hydrogen atom, a fluorine atom, a chlorine atom, a bromine atom, an iodine atom, a methyl group, an ethyl group, a propyl group, an isopropyl group, a cyclopropyl group, or $CF_3$, and $A^1$, $A^2$, $A^3$, $A^4$, and $A^5$ represent CH (hereinafter, referred to as "Compound class CX556).

A compound (K-1) wherein $R^{1b}$ represents a fluorine atom, $R^{1c}$ represents a hydrogen atom, $A^1$ represents a nitrogen atom, $A^2$, $A^3$, and $A^4$, represent CH, and $A^5$ represents any one substituent indicated in [Table L1] to [Table L3] (hereinafter, referred to as "Compound class CX557).

A compound (K-1) wherein $R^{1b}$ represents a chlorine atom, $R^{1c}$ represents a hydrogen atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class CX558).

A compound (K-1) wherein $R^{1b}$ represents a bromine atom, $R^{1c}$ represents a hydrogen atom, $A^1$ represents a nitrogen atom, $A^2$, $A^3$, and $A^4$ represent CH, and $A^5$ represents any one substituent indicated in [Table L1] to [Table. L3] (hereinafter, referred to as "Compound class CX559).

A compound (K-1) wherein $R^{1b}$ represents an iodine atom, $R^{1c}$ represents a hydrogen atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class CX560).

A compound (R-1) wherein $R^{1b}$ represents a methyl group, $R^{1c}$ represents a hydrogen atom, $A^1$ represents a nitrogen atom, $A^2$, $A^3$, and $A^4$ represent CH, and $A^5$ represents any one substituent indicated in [Table L1] to (Table L3] (hereinafter, referred to as "Compound class CX561).

A compound (K-1) wherein $R^{1b}$ represents an ethyl group, $R^{1c}$ represents a hydrogen atom, $A^1$ represents a nitrogen atom, $A^2$, $A^3$, and $A^4$, represent CH, and $A^5$ represents any one substituent indicated in [Table L1] to [Table L3] (hereinafter, referred to as "Compound class CX562).

A compound (K-1) wherein $R^{1b}$ represents a propyl group, $R^{1c}$ represents a hydrogen atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class CX563).

A compound (K-1) wherein $R^{1b}$ represents an isopropyl group, $R^{1c}$ represents a hydrogen atom, $A^1$ represents

a nitrogen atom, $A^2$, $A^3$, and $A^4$ represent CH, and $A^5$ represents any one substituent indicated in [Table L1] to [Table L3] (hereinafter, referred to as "Compound class CX564).

A compound (N-1) wherein $R^{1b}$ represents a cyclopropyl group, $R^{1c}$ represents a hydrogen atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class CX565).

A compound (K-1) wherein $R^{1b}$ represents $CF_3$, $R^{1c}$ represents a hydrogen atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class CX566).

A compound (K-1) wherein $R^{1b}$ and $R^{1c}$ represent a fluorine atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class CX567).

A compound (R-1) wherein $R^{1b}$ and $R^{1c}$ represent a chlorine atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class CX568).

A compound (R-1) wherein $R^{1b}$ and $R^{1c}$ represent a bromine atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class CX569).

A compound (K-1) wherein $R^{1b}$ and $R^{1c}$ represent an iodine atom, $A^1$ represents a nitrogen atom, $A^2$, $A^3$, and $A^4$ represent CH, and $A^5$ represents any one substituent indicated in [Table L1] to [Table L3] (hereinafter, referred to as "Compound class CX570).

A compound (K-1) wherein $R^{1b}$ and $R^{1c}$ represent a methyl group, $A^1$ represents a nitrogen atom, $A^2$, $A^3$, and $A^4$ represent CH, and $A^5$ represents any one substituent indicated in [Table L1] to [Table L3] (hereinafter, referred to as "Compound class CX571).

A compound (K-1) wherein $R^{1b}$ and $R^{1c}$ represent an ethyl group, $A^1$ represents a nitrogen atom, $A^2$, $A^3$, and $A^4$ represent CH, and $A^5$ represents any one substituent indicated in [Table L1] to [Table L3] (hereinafter, referred to as "Compound class CX572).

A compound (K-1) wherein $R^{1b}$ and $R^{1c}$ represent a propyl group, $A^1$ represents a nitrogen atom, $A^2$, $A^3$, and $A^4$ represent CH, and $A^5$ represents any one substituent indicated in [Table L1] to [Table L3] (hereinafter, referred to as "Compound class CX573).

A compound (N-1) wherein $R^{1b}$ and $R^{1c}$ represent an isopropyl group, $A^1$ represents a nitrogen atom, $A^2$, $A^3$, and $A^4$ represent CH, and $A^5$ represents any one substituent indicated in [Table L1] to [Table L3] (hereinafter, referred to as "Compound class CX574).

A compound (N-1) wherein $R^{1b}$ and $R^{1c}$ represent a cyclopropyl group, $A^1$ represents a nitrogen atom, $A^2$, $A^3$, and $A^4$ represent CH, and $A^5$ represents any one substituent indicated in [Table L1] to [Table L3] (hereinafter, referred to as "Compound class CX575).

A compound (R-1) wherein $R^{1b}$ and $R^{1c}$ represent $CF_3$, $A^1$ represents a nitrogen atom, $A^2$, $A^3$, and $A^4$ represent CH, and $A^5$ represents any one substituent indicated in [Table L1] to [Table L3] (hereinafter, referred to as "Compound class CX576).

A compound (K-1) wherein $R^{1b}$ and $R^{1c}$ represent a chlorine atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class CX578).

A compound (K-1) wherein $R^{1b}$ and $R^{1c}$ represent a bromine atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class GX579).

A compound (K-1) wherein $R^{1b}$ and $R^{1c}$ represent an iodine atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound. class CX580).

A compound (K-1) wherein $R^{1b}$ and $R^{1c}$ represent a methyl group, $A^1$ represents a nitrogen atom, $A^2$, $A^3$, and $A^5$ represent CH, and $A^4$ represents any one substituent indicated in [Table L1] to [Table L3] (hereinafter, referred to as "Compound class CX581).

A compound (K-1) wherein $R^{1b}$ and $R^{1c}$ represent an ethyl group, $A^1$ represents a nitrogen atom, $A^2$, $A^3$, and $A^5$ represent CH, and $A^4$ represents any one substituent indicated in [Table L1] to [Table L3] (hereinafter, referred to as "Compound class CX582).

A compound (N-1) wherein $R^{1b}$ and $R^{1c}$ represent a propyl group, $A^1$ represents a nitrogen atom, $A^2$, $A^3$, and $A^5$ represent CH, and $A^4$ represents any one substituent indicated in [Table L1] to [Table 13] (hereinafter, referred to as "Compound class CX583).

A compound (K-1) wherein $R^{1b}$ and $R^{1c}$ represent an isopropyl group, $A^1$ represents a nitrogen atom, $A^2$, $A^3$, and $A^5$ represent CH, and $A^4$ represents any one substituent indicated in [Table L1] to [Table L3] (hereinafter, referred

to as "Compound class CX584).

A compound (K-1) wherein $R^{1b}$ and $R^{1c}$ represent a cyclopropyl group, $A^1$ represents a nitrogen atom, $A^2$, $A^3$, and $A^5$ represent CH, and $A^4$ represents any one substituent indicated in [Table L1] to [Table L3] (hereinafter, referred to as "compound class CX585).

A compound (K-1) wherein $R^{1b}$ and $R^{1c}$ represent $CF_3$, $A^1$ represents a nitrogen atom, $A^2$, $A^3$, and $A^5$ represent CH, and $A^4$ represents any one substituent indicated in [Table L1] to [Table L3] (hereinafter, referred to as "Compound class CX586).

A compound (K-1) wherein $R^{1b}$ represents a fluorine atom, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class CX587).

A compound (K-1) wherein $R^{1b}$ represents a chlorine atom, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class CXS88).

A compound (K-1) wherein $R^{1b}$ represents a bromine atom, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class CX589).

A compound (K-1) wherein $R^{1b}$ represents an iodine atom, $R^{1c}$ represents a hydrogen atom, $A^1$ represents a nitrogen atom, $A^2$, $A^3$, and $A^5$, represent CH, and $A^4$ represents any one substituent indicated in [Table L1] to [Table L3] (hereinafter, referred to as "Compound class CX590).

A compound (K-1) wherein $R^{1b}$ represents a methyl group, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class CX591).

A compound (K-1) wherein $R^{1b}$ represents an ethyl group, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class CX592).

A compound (K-1) wherein $R^{1b}$ represents a propyl group, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class CXS93).

A compound (K-1) wherein $R^{1b}$ represents an isopropyl group, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class CX594).

A compound (K-1) wherein $R^{1b}$ represents a cyclopropyl group, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class CX595).

A compound (K-1) wherein $R^{1b}$ represents $CF_3$, $R^{1c}$ represents a hydrogen atom, $A^1$ represents a nitrogen atom, $A^2$, $A^3$, and $A^5$ represent CH, and $A^4$ represents any one substituent indicated in [Table L1] to [Table L3] (hereinafter, referred to as "Compound class CX596).

A compound (K-1) wherein $R^{1b}$ and $R^{1c}$ represent a fluorine atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX597).

A compound (K-1) wherein $R^{1b}$ and $R^{1c}$ represent a chlorine atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX598).

A compound (K-1) wherein $R^{1b}$ and $R^{1c}$ represent a bromine atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX590).

A compound (K-1) wherein $R^{1b}$ and $R^{1c}$ represent an iodine atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound. class CX600).

A compound (K-1) wherein $R^{1b}$ and $R^{1c}$ represent a methyl group, $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX601).

A compound (N-1) wherein $R^{1b}$ and $R^{1c}$ represent an ethyl group, $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX602).

A compound (N-1) wherein $R^{1b}$ and $R^{1c}$ represent a propyl group, $A^1$ represents a nitrogen atom, $A^2$, $A^4$, and $A^5$ represent CH, and $A^3$ represents any one substituent indicated in [Table L1] to [Table 13) (hereinafter, referred to as "Compound class CX603).

A compound (K-1) wherein $R^{1b}$ and $R^{1c}$ represent an isopropyl group, $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX604).

A compound (K-1) wherein $R^{1b}$ and $R^{1c}$ represent a cyclopropyl group, $A^1$ represents a nitrogen atom, $A^2$, $A^4$, and $A^5$ represent CH, and $A^3$ represents any one substituent indicated in [Table L1] to [Table L3] (hereinafter, referred to as "compound class CX605).

A compound (K-1) wherein $R^{1b}$ and $R^{1c}$ represent $CF_3$, $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX606).

A compound (K-1) wherein $R^{1b}$ represents a fluorine atom, $R^{1c}$ represents a hydrogen atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX607).

A compound (K-1) wherein $R^{1b}$ represents a chlorine atom, $R^{1c}$ represents a hydrogen atom. $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX608).

A compound (K-1) wherein $R^{1b}$ represents a bromine atom, $R^{1c}$ represents a hydrogen atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX609).

A compound (K-1) wherein $R^{1b}$ represents an iodine atom, $R^{1c}$ represents a hydrogen atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX610).

A compound (K-1) wherein $R^{1b}$ represents a methyl group, $R^{1c}$ represents a hydrogen atom, $A^1$ represents a nitrogen atom. $A^2$, $A^4$, and $A^5$ represent CH, and $A^3$ represents any one substituent indicated in [Table L1] to [Table L3] (hereinafter, referred to as "Compound class CX611).

A compound (K-1) wherein $R^{1b}$ represents an ethyl group, $R^{1c}$ represents a hydrogen atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX612).

A compound (K-1) wherein $R^{1b}$ represents a propyl group, $R^{1c}$ represents a hydrogen atom. $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX613).

A compound (K-1) wherein $R^{1b}$ represents an isopropyl group, $R^{1c}$ represents a hydrogen atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX614).

A compound (K-1) wherein $R^{1b}$ represents a cyclopropyl group, $R^{1c}$ represents a hydrogen atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX615).

A compound (K-1) wherein $R^{1b}$ represents $CF_3$, $R^{1c}$ represents a hydrogen atom, $A^1$ represents a nitrogen atom, $A^2$, $A^4$, and $A^5$ represent CH, and $A^3$ represents any one substituent indicated in [Table L1] to [Table L3] (hereinafter, referred to as "Compound class CX616).

A compound (N-1) wherein $R^{1b}$ and $R^{1c}$ represent a fluorine atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class CX617).

A compound (R-1) wherein $R^{1b}$ and $R^{1c}$ represent a chlorine atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class CX618).

A compound (K-1) wherein $R^{1b}$ and $R^{1c}$ represent a bromine atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class CX619).

A compound (K-1) wherein $R^{1b}$ and $R^{1c}$ represent an iodine atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class CX620).

A compound (K-1) wherein $R^{1b}$ and $R^{1c}$ represent a methyl group, $A^1$ represents a nitrogen atom, $A^3$, $A^4$, and $A^5$ represent CH, and $A^2$ represents any one substituent indicated in [Table L1] to [Table L3] (hereinafter, referred to as "Compound class CX621).

A compound (K-1) wherein $R^{1b}$ and $R^{1c}$ represent an ethyl group, $A^1$ represents a nitrogen atom, $A^3$, $A^4$, and $A^5$ represent CH, and $A^2$ represents any one substituent indicated in [Table L1] to [Table L3] (hereinafter, referred to as "Compound class CX622).

A compound (K-1) wherein $R^{1b}$ and $R^{1c}$ represent a propyl group, $A^1$ represents a nitrogen atom, $A^3$, $A^4$, and

$A^5$ represent CH, and $A^2$ represents any one substituent indicated in [Table L1] to [Table L3] (hereinafter, referred to as "Compound class CX623).

A compound (K-1) wherein $R^{1b}$ and $R^{1c}$ represent an isopropyl group, $A^1$ represents a nitrogen atom, $A^3$, $A^4$, and $A^5$ represent CH, and $A^2$ represents any one substituent indicated in [Table L1] to [Table L3] (hereinafter, referred to as "Compound class CX624).

A compound (K-1) wherein $R^{1b}$ and $R^{1c}$ represent a cyclopropyl group, $A^1$ represents a nitrogen atom, $A^3$, $A^4$, and $A^5$ represent CH, and $A^2$ represents any one substituent indicated in [Table L1] to [Table L3] (hereinafter, referred to as "Compound class CX625).

A compound (K-1) wherein $R^{1b}$ and $R^{1c}$ represent $CF_3$, $A^1$ represents a nitrogen atom, $A^3$, $A^4$, and $A^5$ represent CH, and $A^2$ represents any one substituent indicated in [Table L1] to [Table L3] (hereinafter, referred to as "Compound class CX626).

A compound (K-1) wherein $R^{1b}$ represents a fluorine atom, $R^{1c}$ represent a hydrogen atom, $A^1$ represents a nitrogen atom, $A^3$, $A^4$, and $A^5$ represent CH, and $A^2$ represents any one substituent indicated in [Table L1] to [Table. L3] (hereinafter, referred to as "Compound class CX627).

A compound (K-1) wherein $R^{1b}$ represents a chlorine atom, $R^{1c}$ represent a hydrogen atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class CX628).

A compound (K-1) wherein $R^{1b}$ represents a bromine atom, $R^{1c}$ represent a hydrogen atom, $A^1$ represents a nitrogen atom, $A^3$, $A^4$, and $A^5$ represent CH, and $A^2$ represents any one substituent indicated in [Table 1.1] to [Table L3] (hereinafter, referred to as "Compound class CX629).

A compound (R-1) wherein $R^{1b}$ represents an iodine atom, $R^{1c}$ represent a hydrogen atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class CX630).

A compound (K-1) wherein $R^{1b}$ represents a methyl group, $R^{1c}$ represent a hydrogen atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class CX631).

A compound (K-1) wherein $R^{1b}$ represents an ethyl group, $R^{1c}$ represent a hydrogen atom, $A^1$ represents a nitrogen atom, $A^3$, $A^4$, and $A^5$ represent CH, and $A^2$ represents any one substituent indicated in [Table L1] to [Table. L3] (hereinafter, referred to as "Compound class CX632).

A compound (K-1) wherein $R^{1b}$ represents a propyl group, $R^{1c}$ represent a hydrogen atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class CX633).

A compound (K-1) wherein $R^{1b}$ represents an isopropyl group, $R^{1c}$ represent a hydrogen atom, $A^1$ represents a nitrogen atom, $A^3$, $A^4$, and $A^5$ represent CH, and $A^2$ represents any one substituent indicated in [Table 1.1] to [Table L3] (hereinafter, referred to as "Compound class CX634).

A compound (K-1) wherein $R^{1b}$ represents a cyclopropyl group, $R^{1c}$ represent a hydrogen atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class CX635).

A compound (K-1) wherein $R^{1b}$ represents $CF_3$, $R^{1c}$ represent a hydrogen atom, $A^1$ represents a nitrogen atom, $A^3$, $A^4$, and $A^5$ represent CH, and $A^2$ represents any one substituent indicated in [Table L1] to [Table L3] (hereinafter, referred to as "Compound class CX636).

A compound (K-1) wherein $R^{1b}$ represents chlorine atom, $R^{1c}$ represents any one of a hydrogen atom, a fluorine atom, a chlorine atom, a bromine atom, an iodine atom, a methyl group, an ethyl group, a propyl group, an isopropyl group, a cyclopropyl group, or $CF^3$, $A^1$ represents a nitrogen atom, and $A^2$, $A^3$, $A^4$, and $A^5$ represent CH (hereinafter, referred to as "Compound class CX637).

A compound represented by formula (K-2):

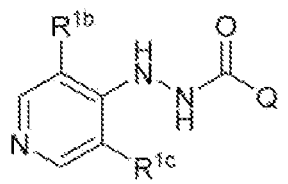

(K-2)

(hereinafter, referred to as "compound (K-2) ")
wherein $R^{1b}$ represents a fluorine atom, $R^{1c}$ represents a hydrogen atom, and Q represents any one substituent indicated in [Table L4] to [Table L9] (hereinafter, referred to as "Compound class CX638).

A compound (K-2) wherein $R^{1b}$ represents a chlorine atom, $a^{1c}$ represent a hydrogen atom, and Q represents

any one substituent indicated in [Table L4] to [Table L9] (hereinafter, referred to as "Compound class CX639).

A compound (K-2) wherein $R^{1b}$ represents a bromine atom, $R^{1c}$ represent a hydrogen atom, and Q represents any one substituent indicated in [Table L4] to [Table L9] (hereinafter, referred to as "Compound class CX640).

A compound (K-2) wherein $R^{1b}$ represents an iodine atom, $R^{1c}$ represent a hydrogen atom, and Q represents any one substituent indicated in [Table L4] to [Table L9.] (hereinafter, referred to as "Compound class CX641).

A compound (K-2) wherein $R^{1b}$ represents a methyl group, $R^{1c}$ represent a hydrogen atom, and Q represents any one substituent indicated in [Table L4] to [Table L9] (hereinafter, referred to as "Compound class CX642).

A compound (K-2) wherein $R^{1b}$ represents an ethyl group, $R^{1c}$ represent a hydrogen atom, and Q represents any one substituent indicated in [Table L4] to [Table L9] (hereinafter, referred to as "Compound class CX643).

A compound (K-2) wherein $R^{1b}$ represents a propyl group, $R^{1c}$ represent a hydrogen atom, and Q represents any one substituent indicated in [Table L4] to [Table L9] (hereinafter, referred to as "Compound class CX644).

A compound (K-2) wherein $R^{1b}$ represents an isopropyl group, $R^{1c}$ represent a hydrogen atom, and Q represents any one substituent indicated in [Table L4] to [Table L9] (hereinafter, referred to as "Compound class CX645).

A compound (K-2) wherein $R^{1b}$ represents a cyclopropyl group, $R^{1c}$ represent a hydrogen atom, and Q represents any one substituent indicated in [Table L4] to [Table L9] (hereinafter, referred to as "Compound class CX646).

A compound (K-2) wherein $R^{1b}$ represents $CF_3$, $R^{1c}$ represent a hydrogen atom, and Q represents any one substituent indicated in [Table L4] to [Table L9] (hereinafter, referred to as "Compound class CX647),

A compound (K-2) wherein $R^{1b}$ represents a methylsulfanyl group, $R^{1c}$ represent a hydrogen atom, and Q represents any one substituent indicated in [Table L41 to [Table L9] (hereinafter, referred to as "Compound class CX648).

A compound (K-2) wherein $R^{1b}$ represents a methylsulfinyl group, $R^{1c}$ represent a hydrogen atom, and Q represents any one substituent indicated in [Table L4] to [Table L9] (hereinafter, referred to as "Compound class CX649).

A compound (K-2) wherein $R^{1b}$ represents a methylsulfonyl group, $R^{1c}$ represent a hydrogen atom, and Q. represents any one substituent indicated in [Table L4] to [Table L9] (hereinafter, referred to as "Compound class CX650).

A compound (K-2) wherein $R^{1b}$ and $R^{1c}$ represent a fluorine atom, and Q represents any one substituent indicated in [Table L4] to [Table L9] (hereinafter, referred to as "Compound class CX651).

A compound (K-2) wherein $R^{1b}$ and $R^{1c}$ represent, a chlorine atom, and Q represents any one substituent indicated in [Table L4] to [Table L9] (hereinafter, referred to as "Compound class CX652).

A compound (K-2) wherein $R^{1b}$ and $R^{1c}$ represent a bromine atom, and Q represents any one substituent indicated in [Table L4] to [Table L9] (hereinafter, referred to as "Compound class CX653).

A compound (K-2) wherein $R^{1b}$ and $R^{1c}$ represent an iodine atom, and Q represents any one substituent indicated in [Table L4] to [Table L9] (hereinafter, referred to as "Compound class CX654).

A compound (K-2) wherein $R^{1b}$ and $R^{1c}$ represent a methyl group, and Q represents any one substituent indicated in [Table L4] to [Table L9] (hereinafter, referred to as "Compound class CX655).

A compound (K-2) wherein $R^{1b}$ and $R^{1c}$ represent an ethyl group, and Q represents any one substituent indicated in [Table L4] to [Table L9] (hereinafter, referred to as "Compound class CX656).

A compound (K-2) wherein $R^{1b}$ and $R^{1c}$ represent a propyl group, and Q represents any one substituent indicated in [Table L4] to [Table L9] (hereinafter, referred to as "Compound class CX657).

A compound (K-2) wherein $R^{1b}$ and $R^{1c}$ represent an isopropyl group, and Q represents any one substituent indicated in [Table L4] to [Table L9] (hereinafter, referred to as "Compound class CX658).

A compound (K-2) wherein $R^{1b}$ and $R^{1c}$ represent a cyclopropyl group, and Q represents any one substituent indicated in [Table L4] to [Table L9] (hereinafter, referred to as "Compound class CX659).

A compound (K-2) wherein $R^{1b}$ and $R^{1c}$ represent $CF_3$, and Q represents any one substituent indicated in [Table L4] to [Table L9] (hereinafter, referred to as "Compound class CX660).

A compound represented by formula (L-1) wherein $R^{1b}$ represents a methoxy group, $R^{1c}$ represent a hydrogen atom, $A^2$, $A^3$, $A^4$, and $A^5$ represent CH, and $A^1$ represents any one substituent indicated in [Table L1] to [Table L3] (hereinafter, referred to as "Compound class SX711").

A compound represented by formula (L-1) wherein $R^{1b}$ represents an ethoxy group, $R^{1c}$ represent a hydrogen atom, $A^2$, $A^3$, $A^4$, and $A^5$ represent CH, and $A^1$ represents any one substituent indicated in [Table L1] to [Table L3] (hereinafter, referred to as "Compound class SX712").

A compound represented by formula (L-1) wherein $R^{1b}$ represents a methoxy group, $R^{1c}$ represent a fluorine atom, $A^2$, $A^3$, $A^4$, and $A^5$ represent CH, and $A^1$ represents any one substituent indicated in [Table L1] to [Table L3] (hereinafter, referred to as "Compound class SX713").

A compound represented by formula (L-1) wherein $R^{1b}$ represents an ethoxy group, $R^{1c}$ represent a fluorine atom, $A^2$, $A^3$, $A^4$, and $A^5$ represent CH, and $A^1$ represents any one substituent indicated in [Table L1] to [Table L3] (hereinafter, referred to as "Compound class SX714").

A compound represented by formula (L-1) wherein $R^{1b}$ represents a methoxy group, $R^{1c}$ represent a chlorine atom, $A^2$, $A^3$, $A^4$, and $A^5$ represent CH, and $A^1$ represents any one substituent indicated in [Table L1] to [Table L3] (hereinafter, referred to as "Compound class SX715").

A compound represented by formula (L-1) wherein $R^{1b}$ represents an ethoxy group, $R^{1c}$ represent a chlorine atom, $A^2$, $A^3$, $A^4$, and $A^5$ represent CH, and $A^1$ represents any one substituent indicated in [Table L1] to [Table L3] (hereinafter, referred to as "Compound class SX716").

A compound represented by formula (L-1) wherein $R^{1b}$ represents a methoxy group, $R^{1c}$ represent a bromine atom, $A^2$, $A^3$, $A^4$, and $A^5$ represent CH, and $A^1$ represents any one substituent indicated in [Table L1J to [Table L3] (hereinafter, referred to as "Compound class SX717").

A compound represented by formula (L-1) wherein $R^{1b}$ represents an ethoxy group, $R^{1c}$ represent a bromine atom, $A^2$, $A^3$, $A^4$, and $A^5$ represent CH, and $A^1$ represents any one substituent indicated in [Table L1] to [Table L3] (hereinafter, referred to as "Compound class SX718").

A compound represented by formula (L-1) wherein $R^{1b}$ represents a methylsulfanyl group, $R^{1c}$ represent a fluorine atom, $A^2$, $A^3$, $A^4$, and $A^5$ represent CH, and $A^1$ represents any one substituent indicated in [Table L1] to [Table L3] (hereinafter, referred to as "Compound class SX719").

A compound represented by formula (L-1) wherein $R^{1b}$ represents a methylsulfinyl group, $R^{1c}$ represent a fluorine atom, $A^2$, $A^3$, $A^4$, and $A^5$ represent CH, and $A^1$ represents any one substituent indicated in [Table L13 to [Table L3] (hereinafter, referred to as "Compound class SK720").

A compound represented by formula (L-1) wherein $R^{1b}$ represents a methylsulfonyl group, $R^{1c}$ represent a fluorine atom, $A^2$, $A^3$, $A^4$, and $A^5$ represent CH, and $A^1$ represents any one substituent indicated in [Table L1] to [Table L3] (hereinafter, referred to as "Compound class SX721").

A compound represented by formula (L-1) wherein $R^{1b}$ represents a methylsulfanyl group, $R^{1c}$ represent a chlorine atom, $A^2$, $A^3$, $A^4$, and $A^5$ represent CH, and $A^1$ represents any one substituent indicated in [Table L1] to [Table L3] (hereinafter, referred to as "Compound class SX722").

A compound represented by formula (L-1) wherein $R^{1b}$ represents a methylsulfinyl group, $R^{1c}$ represent a chlorine atom, $A^2$, $A^3$, $A^4$, and $A^5$ represent CH, and $A^1$ represents any one substituent indicated in [Table L1] to [Table L3] (hereinafter, referred to as "Compound class SX723").

A compound represented by formula (L-1) wherein $R^{1b}$ represents a methylsulfonyl group, $R^{1c}$ represent a chlorine atom, $A^2$, $A^3$, $A^4$, and $A^5$ represent CH, and $A^1$ represents any one substituent indicated in [Table L1] to [Table L3] (hereinafter, referred to as "Compound class SX724").

A compound represented by formula (L-1) wherein $R^{1b}$ represents a methylsulfanyl group, $R^{1c}$ represent a bromine atom, $A^2$, $A^3$, $A^4$, and $A^5$ represent CH, and $A^1$ represents any one substituent indicated in [Table L1] to [Table L3] (hereinafter, referred to as "Compound class SX725").

A compound represented by formula (L-1) wherein $R^{1b}$ represents a methylsulfinyl group, $R^{1c}$ represent a bromine atom, $A^2$, $A^3$, $A^4$, and $A^5$ represent CH, and $A^1$ represents any one substituent indicated in [Table L1] to [Table L3] (hereinafter, referred to as "Compound class SX726").

A compound represented by formula (L-1) wherein $R^{1b}$ represents a methylsulfonyl group, $R^{1c}$ represent a bromine atom, $A^2$, $A^3$, $A^4$, and $A^5$ represent CH, and $A^1$ represents any one substituent indicated in [Table L1] to [Table L3] (hereinafter, referred to as "Compound class SX727").

A compound represented by formula (L-1) wherein $R^{1b}$ represents a methoxy group, $R^{1c}$ represent a hydrogen atom, $A^1$, $A^3$, $A^4$, and $A^5$ represent CH, and $A^2$ represents any one substituent indicated in [Table L1] to [Table L3] (hereinafter, referred to as "Compound class SX728").

A compound represented by formula (L-1) wherein $R^{1b}$ represents an ethoxy group, $R^{1c}$ represent a hydrogen atom, $A^1$, $A^3$, $A^4$, and $A^5$ represent CH, and $A^2$ represents any one substituent indicated in [Table L1] to [Table L3] (hereinafter, referred to as "Compound class SX729").

A compound represented by formula (L-1) wherein $R^{1b}$ represents a methoxy group, $R^{1c}$ represent a fluorine atom, $A^1$, $A^3$, $A^4$, and $A^5$ represent CH, and $A^2$ represents any one substituent indicated in [Table L1] to [Table L3] (hereinafter, referred to as "Compound class SX730").

A compound represented by formula (L-1) wherein $R^{1b}$ represents an ethoxy group, $R^{1c}$ represent a fluorine atom, $A^1$, $A^3$, $A^4$, and $A^5$ represent CH, and $A^2$ represents any one substituent indicated in [Table L1] to [Table L3] (hereinafter, referred to as "Compound class SX731").

A compound represented by formula (L-1) wherein $R^{1b}$ represents a methoxy group, $R^{1c}$ represent a chlorine atom, $A^1$, $A^3$, $A^4$, and $A^5$ represent CH, and $A^2$ represents any one substituent indicated in [Table L1J to [Table L3] (hereinafter, referred to as "Compound class SX732").

A compound represented by formula (L-1) wherein $R^{1b}$ represents an ethoxy group, $R^{1c}$ represent a chlorine atom, $A^1$, $A^3$, $A^4$, and $A^5$ represent CH, and $A^2$ represents any one substituent indicated in [Table L1] to [Table L3] (hereinafter, referred to as "Compound class SX733").

A compound represented by formula (L-1) wherein $R^{1b}$ represents a methoxy group, $R^{1c}$ represent a bromine atom, $A^1$, $A^3$, $A^4$, and $A^5$ represent CH, and $A^2$ represents any one substituent indicated in [Table L1] to [Table L3] (hereinafter, referred to as "Compound class SX734").

A compound represented by formula (L-1) wherein $R^{1b}$ represents an ethoxy group, $R^{1c}$ represent a bromine

atom, $A^1$, $A^3$, $A^4$, and $A^5$, represent CH, and $A^2$ represents any one substituent indicated in [Table L1] to [Table L3] (hereinafter, referred to as "Compound class SX735").

A compound represented by formula (L-1) wherein $R^{1b}$ represents a methylsulfanyl group, $R^{1c}$ represent a fluorine atom, $A^1$, $A^3$, $A^4$, and $A^5$ represent CH, and $A^2$ represents any one substituent indicated in [Table L1] to [Table L3] (hereinafter, referred to as "Compound class SX736").

A compound represented by formula (L-1) wherein $R^{1b}$ represents a methylsulfinyl group, $R^{1c}$ represent a fluorine atom, $A^1$, $A^3$, $A^4$, and $A^5$ represent CH, and $A^2$ represents any one substituent indicated in [Table L1] to [Table L3] (hereinafter, referred to as "Compound class SX737").

A compound represented by formula (L-1) wherein $R^{1b}$ represents a methylsulfonyl group, $R^{1c}$ represent a fluorine atom, $A^1$, $A^3$, $A^4$, and $A^5$ represent CH, and $A^2$ represents any one substituent indicated in [Table L1] to [Table L3] (hereinafter, referred to as "Compound class SX738").

A compound represented by formula (L-1) wherein $R^{1b}$ represents a methylsulfanyl group, $R^{1c}$ represent a chlorine atom, $A^1$, $A^3$, $A^4$, and $A^5$ represent CH, and $A^2$ represents any one substituent indicated in [Table L1] to [Table L3] (hereinafter, referred to as "Compound class SX739").

A compound represented by formula (L-1) wherein $R^{1b}$ represents a methylsulfinyl group, $R^{1c}$ represent a chlorine atom, $A^1$, $A^3$, $A^4$, and $A^5$ represent CH, and $A^2$ represents any one substituent indicated in [Table L1] to [Table L3] (hereinafter, referred to as "Compound class SX740").

A compound represented by formula (L-1) wherein $R^{1b}$ represents a methylsulfonyl group, $R^{1c}$ represent a chlorine atom, $A^1$, $A^3$, $A^4$, and $A^5$ represent CH, and $A^2$ represents any one substituent indicated in [Table L1] to [Table L3] (hereinafter, referred to as "Compound class SX741").

A compound represented by formula (L-1) wherein $R^{1b}$ represents a methylsulfanyl group, $R^{1c}$ represent a bromine atom, $A^1$, $A^3$, $A^4$, and $A^5$ represent CH, and $A^2$ represents any one substituent indicated in [Table L1] to [Table L3] (hereinafter, referred to as "Compound class SX742").

A compound represented by formula (L-1) wherein $R^{1b}$ represents a methylsulfinyl group, $R^{1c}$ represent a bromine atom, $A^1$, $A^3$, $A^4$, and $A^5$ represent CH, and $A^2$ represents any one substituent indicated in [Table L1] to [Table L3] (hereinafter, referred to as "Compound class SX743").

A compound represented by formula (L-1) wherein $R^{1b}$ represents a methylsulfanyl group, $R^{1c}$ represent a bromine atom, $A^1$, $A^3$, $A^4$, and $A^5$ represent CH, and $A^2$ represents any one substituent indicated in [Table L1] to [Table L3] (hereinafter, referred to as "Compound class SX744").

A compound represented by formula (L-1) wherein $R^{1b}$ represents a methoxy group, $R^{1c}$ represent a hydrogen atom, $A^1$, $A^2$, $A^4$, and $A^5$ represent CH, and $A^3$ represents any one substituent indicated in [Table L1] to [Table L3] (hereinafter, referred to as "Compound class SX745").

A compound represented by formula (L-1) wherein $R^{1b}$ represents an ethoxy group, $R^{1c}$ represent a hydrogen atom, $A^1$, $A^2$, $A^4$, and $A^5$ represent CH, and $A^3$ represents any one substituent indicated in [Table L1] to [Table L3] (hereinafter, referred to as "Compound class SX746").

A compound represented by formula (L-1) wherein $R^{1b}$ represents a methoxy group, $R^{1c}$ represent a fluorine atom, $A^1$, $A^2$, $A^4$, and $A^5$ represent CH, and $A^3$ represents any one substituent indicated in [Table L1] to [Table L3] (hereinafter, referred to as "Compound class SX747").

A compound represented by formula (L-1) wherein $R^{1b}$ represents an ethoxy group, $R^{1c}$ represent a fluorine atom, $A^1$, $A^2$, $A^4$, and $A^5$ represent CH, and $A^3$ represents any one substituent indicated in [Table L1] to [Table L3] (hereinafter, referred to as "Compound class SX748").

A compound represented by formula (L-1) wherein $R^{1b}$ represents a methoxy group, $R^{1c}$ represent a chlorine atom, $A^1$, $A^2$, $A^4$, and $A^5$ represent CH, and $A^3$ represents any one substituent indicated in [Table L1] to [Table L3] (hereinafter, referred to as "Compound class SX749").

A compound represented by formula (L-1) wherein $R^{1b}$ represents an ethoxy group, $R^{1c}$ represent a chlorine atom, $A^1$, $A^2$, $A^4$, and $A^5$ represent CH, and $A^3$ represents any one substituent indicated in [Table L1] to [Table L3] (hereinafter, referred to as "Compound class SX750").

A compound represented by formula (L-1) wherein $R^{1b}$ represents a methoxy group, $R^{1c}$ represent a bromine atom, $A^1$, $A^2$, $A^4$, and $A^5$ represent CH, and $A^3$ represents any one substituent indicated in [Table L1] to [Table L3] (hereinafter, referred to as "Compound class SX751").

A compound represented by formula (L-1) wherein $R^{1b}$ represents an ethoxy group, $R^{1c}$ represent a bromine atom, $A^1$, $A^2$, $A^4$, and $A^5$ represent CH, and $A^3$ represents any one substituent indicated in [Table L1] to [Table L3] (hereinafter, referred to as "Compound class SX752").

A compound represented by formula (L-1) wherein $R^{1b}$ represents a methylsulfanyl group, $R^{1c}$ represent a fluorine atom, $A^1$, $A^2$, $A^4$, and $A^5$ represent CH, and $A^3$ represents any one substituent indicated in [Table L1] to [Table L3] (hereinafter, referred to as "Compound class SX753").

A compound represented by formula (L-1) wherein $R^{1b}$ represents a methylsulfinyl group, $R^{1c}$ represent a fluorine atom, $A^1$, $A^2$, $A^4$, and $A^5$ represent CH, and $A^3$ represents any one substituent indicated in [Table L1] to [Table

L3] (hereinafter, referred to as "Compound class SX754").

A compound represented by formula (L-1) wherein $R^{1b}$ represents a methylsulfonyl group, $R^{1c}$ represent a fluorine atom, $A^1$, $A^2$, $A^4$, and $A^5$ represent CH, and $A^3$ represents any one substituent indicated in [Table L1] to [Table L3] (hereinafter, referred to as "Compound class SX755").

A compound represented by formula (L-1) wherein $R^{1b}$ represents a methylsulfanyl group, $R^{1c}$ represent a chlorine atom, $A^1$, $A^2$, $A^4$, and $A^5$ represent CH, and $A^3$ represents any one substituent indicated in [Table L1] to [Table L3] (hereinafter, referred to as "Compound class SX756").

A compound represented by formula (L-1) wherein $R^{1b}$ represents a methylsulfinyl group, $R^{1c}$ represent a chlorine atom, $A^1$, $A^2$, $A^4$, and $A^5$ represent CH, and $A^3$ represents any one substituent indicated in [Table L1] to [Table L3] (hereinafter, referred to as "Compound class SX757").

A compound represented by formula (L-1) wherein $R^{1b}$ represents a methylsulfonyl group, $R^{1c}$ represent a chlorine atom, $A^1$, $A^2$, $A^4$, and $A^5$ represent CH, and $A^3$ represents any one substituent indicated in [Table L1] to [Table L3] (hereinafter, referred to as "Compound class SX758").

A compound represented by formula (L-1) wherein $R^{1b}$ represents a methylsulfanyl group, $R^{1c}$ represent a bromine atom, $A^1$, $A^2$, $A^4$, and $A^5$ represent CH, and $A^3$ represents any one substituent indicated in [Table L1] to [Table L3] (hereinafter, referred to as "Compound class SX759").

A compound represented by formula (L-1) wherein $R^{1b}$ represents a methylsulfinyl group, $R^{1c}$ represent a bromine atom, $A^1$, $A^2$, $A^4$, and $A^5$ represent CH, and $A^3$ represents any one substituent indicated in [Table L1] to [Table L3] (hereinafter, referred to as "Compound class SX760").

A compound represented by formula (L-1) wherein $R^{1b}$ represents a methylsulfonyl group, $R^{1c}$ represent a bromine atom, $A^1$, $A^2$, $A^4$, and $A^5$ represent CH, and $A^3$ represents any one substituent indicated in [Table L1] to [Table L3] (hereinafter, referred to as "Compound class SX761").

A compound represented by formula (L-1) wherein $R^{1b}$ represents a methoxy group, $R^{1c}$ represent a hydrogen atom, $A^1$, $A^4$, and $A^5$ represent CH, $A^2$ represents C-F, and $A^3$ represents any one substituent indicated in [Table L1] to [Table L3] (hereinafter, referred to as "Compound class SX762").

A compound represented by formula (L-1) wherein $R^{1b}$ represents an ethoxy group, $R^{1c}$ represent a hydrogen atom, $A^1$, $A^4$, and $A^5$ represent CH, $A^2$ represents C-F, and $A^3$ represents any one substituent indicated in [Table L1] to [Table L3] (hereinafter, referred to as "Compound class SX763").

A compound represented by formula (L-1) wherein $R^{1b}$ represents a methoxy group, $R^{1c}$ represent a fluorine atom, $A^1$, $A^4$, and $A^5$ represent CH, $A^4$ represents C-F, and $A^3$ represents any one substituent indicated in [Table L1] to [Table L3] (hereinafter, referred to as "Compound class SX764").

A compound represented by formula (L-1) wherein $R^{1b}$ represents an ethoxy group, $R^{1c}$ represent a fluorine atom, $A^1$, $A^4$, and $A^5$ represent CH, $A^2$ represents C-F, and $A^3$ represents any one substituent indicated in [Table L1] to [Table L3] (hereinafter, referred to as "Compound class SX765").

A compound represented by formula (L-1) wherein $R^{1b}$ represents a methoxy group, $R^{1c}$ represent a chlorine atom, $A^1$, $A^4$, and $A^5$ represent CH, $A^2$ represents C-F, and $A^3$ represents any one substituent indicated in [Table L1] to [Table L3] (hereinafter, referred to as "Compound class SX766").

A compound represented by formula (L-1) wherein $R^{1b}$ represents an ethoxy group, $R^{1c}$ represent a chlorine atom, $A^1$, $A^4$, and $A^5$ represent CH, $A^2$ represents C-F, and $A^3$ represents any one substituent indicated in [Table L1] to [Table L3] (hereinafter, referred to as "Compound class. SX767").

A compound represented by formula (L-1) wherein $R^{1b}$ represents a methoxy group, $R^{1c}$ represent a bromine atom, $A^1$, $A^4$, and $A^5$ represent CH, $A^2$ represents C-F, and $A^3$ represents any one substituent indicated in [Table L1] to [Table L3] (hereinafter, referred to as "Compound class SX768").

A compound represented by formula (L-1) wherein $R^{1b}$ represents an ethoxy group, $R^{1c}$ represent a bromine atom, $A^1$, $A^4$, and $A^5$ represent CH, $A^2$ represents C-F, and $A^3$ represents any one substituent indicated in [Table L1] to [Table L3] (hereinafter, referred to as "Compound class SX769").

A compound represented by formula (L-1) wherein $R^{1b}$ represents a methylsulfanyl group, $R^{1c}$ represent a fluorine atom, $A^1$, $A^4$, and $A^5$ represent CH, $A^2$ represents C-F, and $A^3$ represents any one substituent indicated in [Table L1] to [Table L3] (hereinafter, referred to as "Compound class SX770").

A compound represented by formula (L-1) wherein $R^{1b}$ represents a methylsulfinyl group, $R^{1c}$ represent a fluorine atom, $A^1$, $A^4$, and $A^5$ represent CH, $A^2$ represents C-F, and $A^3$ represents any one substituent indicated in [Table L1] to [Table L3] (hereinafter, referred to as "Compound class SX771").

A compound represented by formula (L-1) wherein $R^{1b}$ represents a methylsulfonyl group, $R^{1c}$ represent a fluorine atom, $A^1$, $A^4$, and $A^5$ represent CH, $A^2$ represents C-F, and $A^3$ represents any one substituent indicated in [Table L1] to [Table L3] (hereinafter, referred to as "Compound class SX772").

A compound represented by formula (L-1) wherein $R^{1b}$ represents a methylsulfanyl group, $R^{1c}$ represent a chlorine atom, $A^1$, $A^4$, and $A^5$ represent CH, $A^2$ represents C-F, and $A^3$ represents any one substituent indicated in [Table L1] to [Table L3] (hereinafter, referred to as "Compound class SX773").

A compound represented by formula (L-1) wherein $R^{1b}$ represents a methylsulfinyl group, $R^{1c}$ represent a chlorine atom, $A^1$, $A^4$, and $A^5$ represent CH, $A^2$ represents C-F, and $A^3$ represents any one substituent indicated in [Table L1] to [Table L3] (hereinafter, referred to as "Compound class SX774").

A compound represented by formula (L-1) wherein $R^{1b}$ represents a methylsulfohyl group, $R^{1c}$ represent a chlorine atom, $A^1$, $A^4$, and $A^5$ represent CH, $A^2$ represents C-F, and $A^3$ represents any one substituent indicated in [Table L1] to [Table L3] (hereinafter, referred to as "Compound class SX775").

A compound represented by formula (L-1) wherein $R^{1b}$ represents a methylsulfanyl group, $R^{1c}$ represent a bromine atom, $A^1$, $A^4$, and $A^5$ represent CH, $A^2$ represents C-F, and $A^3$ represents any one substituent indicated in [Table L1] to [Table L3] (hereinafter, referred to as "Compound class SX776").

A compound represented by formula (L-1) wherein $R^{1b}$ represents a methylsulfinyl group, $R^{1c}$ represents a bromine atom, $A^1$, $A^4$, and $A^5$ represent CH, $A^2$ represents C-F, and $A^3$ represents any one substituent indicated in [Table L1] to [Table L3] (hereinafter, referred to as "Compound class SX777").

A compound represented by formula (L-1) wherein $R^{1b}$ represents a methylsulfonyl group, $R^{1c}$ represents a bromine atom, $A^1$, $A^4$, and $A^5$ represent CH, $A^2$ represents C-F, and $A^3$ represents any one substituent indicated in [Table L1] to [Table L3] (hereinafter, referred to as "Compound class SX778").

A compound represented by formula (L-1) wherein $R^{1b}$ represents a methoxy group, $R^{1c}$ represents a hydrogen atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX779").

A compound represented by formula (L-1) wherein $R^{1b}$ represents an ethoxy group, $R^{1c}$ represents a hydrogen atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX780").

A compound represented by formula (L-1) wherein $R^{1b}$ represents a methoxy group, $R^{1c}$ represent a fluorine atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX781").

A compound represented by formula (L-1) wherein $R^{1b}$ represents an ethoxy group, $R^{1c}$ represent a fluorine atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX782").

A compound represented by formula (L-1) wherein $R^{1b}$ represents a methoxy group, $R^{1c}$ represents a chlorine atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX783").

A compound represented by formula (L-1) wherein $R^{1b}$ represents an ethoxy group, $R^{1c}$ represents a chlorine atom, $A^1$, $A^4$, and $A^5$ represent CH, $A^2$ represents C-Cl, and $A^3$ represents any one substituent indicated in [Table L1] to [Table L3] (hereinafter, referred to as "Compound class. SX784").

A compound represented by formula (L-1) wherein $R^{1b}$ represents a methoxy group, $R^{1c}$ represents a bromine atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX785").

A compound represented by formula (L-1) wherein $R^{1b}$ represents an ethoxy group, $R^{1c}$ represents a bromine atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX786").

A compound represented by formula (L-1) wherein $R^{1b}$ represents a methylsulfanyl group, $R^{1c}$ represents a fluorine atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX787").

A compound represented by formula (L-1) wherein $R^{1b}$ represents a methylsulfinyl group, $R^{1c}$ represents a fluorine atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX788").

A compound represented by formula (L-1) wherein $R^{1b}$ represents a methylsulfonyl group, $R^{1c}$ represents a fluorine atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX789").

A compound represented by formula (L-1) wherein $R^{1b}$ represents a methylsulfanyl group, $R^{1c}$ represents a chlorine atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX790").

A compound represented by formula (L-1) wherein $R^{1b}$ represents a methylsulfinyl group, $R^{1c}$ represents a chlorine atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX791").

A compound represented by formula (L-1) wherein $R^{1b}$ represents a methylsulfonyl group, $R^{1c}$ represents a chlorine atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX792").

A compound represented by formula (L-1) wherein $R^{1b}$ represents a methylsulfanyl group, $R^{1c}$ represents a

bromine atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX793").

A compound represented by formula (L-1) wherein $R^{1b}$ represents a methylsulfinyl group, $R^{1c}$ represents a bromine atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX794").

A compound represented by formula (L-1) wherein $R^{1b}$ represents a methylsulfonyl group, $R^{1c}$ represents a bromine atom, $A^1$, $A^4$, and $A^5$ represent CH, $A^2$ represents C-Cl, and $A^3$ represents any one substituent indicated in [Table L1] to [Table L3] (hereinafter, referred to as "Compound class SX795").

A compound represented by formula (L-1) wherein $R^{1b}$ represents a methoxy group, $R^{1c}$ represents a hydrogen atom, $A^1$, $A^4$, and $A^5$ represent CH, $A^2$ represents C-Me, and $A^3$ represents any one substituent indicated in [Table L1] to [Table L3] (hereinafter, referred to as "Compound class SX796").

A compound represented by formula (L-1) wherein $R^{1b}$ represents an ethoxy group, $R^{1c}$ represents a hydrogen atom, $A^1$, $A^4$, and $A^5$ represent CH, $A^2$ represents C-Me, and $A^3$ represents any one substituent indicated in [Table L1] to [Table L3] (hereinafter, referred to as "Compound class SX797").

A compound represented by formula (L-1) wherein $R^{1b}$ represents a methoxy group, $R^{1c}$ represents a fluorine atom, $A^1$, $A^4$, and $A^5$ represent CH, $A^2$ represents C-Me, and $A^3$ represents any one substituent indicated in [Table L1] to [Table L3] (hereinafter, referred to as "Compound class SX798").

A compound represented by formula (L-1) wherein $R^{1b}$ represents an ethoxy group, $R^{1c}$ represents a fluorine atom, $A^1$, $A^4$, and $A^5$ represent CH, $A^2$ represents C-Me, and $A^3$ represents any one substituent indicated in [Table L1] to [Table L3] (hereinafter, referred to as "Compound class SX799").

A compound represented by formula (L-1) wherein $R^{1b}$ represents a methoxy group, $R^{1c}$ represents a chlorine atom, $A^1$, $A^4$, and $A^5$ represent CH, $A^2$ represents C-Me, and $A^3$ represents any one substituent indicated in [Table L1] to [Table L3] (hereinafter, referred to as "Compound class SX800").

A compound represented by formula (L-1) wherein $R^{1b}$ represents an ethoxy group, $R^{1c}$ represents a chlorine atom, $A^1$, $A^4$, and $R^5$ represent CH, $A^2$ represents C-Me, and $A^3$ represents any one substituent indicated in [Table L1] to [Table L3] (hereinafter, referred to as "Compound class SX801").

A compound represented by formula (L-1) wherein $R^{1b}$ represents a methoxy group, $R^{1c}$ represents a bromine atom, $A^1$, $A^4$, and $A^5$ represent CH, $A^2$ represents C-Me, and $A^3$ represents any one substituent indicated in [Table L1] to [Table L3] (hereinafter, referred to as "Compound class SX802").

A compound represented by formula (L-1) wherein $R^{1b}$ represents an ethoxy group, $R^{1c}$ represents a bromine atom, $A^1$, $A^4$, and $A^5$ represent CH, $A^2$ represents, C-Me, and $A^3$ represents any one substituent indicated in [Table L1] to [Table L3] (hereinafter, referred to as "Compound class SX803").

A compound represented by formula (L-1) wherein $R^{1b}$ represents a methylsulfanyl group, $R^{1c}$ represents a fluorine atom, $A^1$, $A^4$, and $A^5$ represent CH, $A^2$ represents C-Me, and $A^3$ represents any one substituent indicated in [Table L1] to [Table L3] (hereinafter, referred to as "Compound class SX804").

A compound represented by formula (L-1) wherein $R^{1b}$ represents a methylsulfinyl group, $R^{1c}$ represents a fluorine atom, $A^1$, $A^4$, and $A^5$ represent CH, $A^2$ represents C-Me, and $A^3$ represents any one substituent indicated in [Table L1] to [Table L3] (hereinafter, referred to as "Compound class SX805").

A compound represented by formula (L-1) wherein $R^{1b}$ represents a methylsulfonyl group, $R^{1c}$ represents a fluorine atom, $A^1$, $A^4$, and $A^5$ represent CH, $A^2$ represents C-Me, and $A^3$ represents any one substituent indicated in [Table L1] to [Table L3] (hereinafter, referred to as "Compound class SX806").

A compound represented by formula (L-1) wherein $R^{1b}$ represents a methylsulfanyl group, $R^{1c}$ represents a chlorine atom, $A^1$, $A^4$, and $A^5$ represent CH, $A^2$ represents C-Me, and $A^3$ represents any one substituent indicated in [Table L1] to [Table L3] (hereinafter, referred to as "Compound class SX807").

A compound represented by formula (L-1) wherein $R^{1b}$ represents a methylsulfinyl group, $R^{1c}$ represents a chlorine atom, $A^1$, $A^4$, and $A^5$ represent CH, $A^2$ represents C-Me, and $A^3$ represents any one substituent indicated in [Table L1] to [Table L3] (hereinafter, referred to as "Compound class SX809").

A compound represented by formula (L-1) wherein $R^{1b}$ represents a methylsulfonyl group, $R^{1c}$ represents a chlorine atom, $A^1$, $A^4$, and $A^5$ represent CH, $A^2$ represents. C-Me, and $A^3$ represents any one substituent indicated in [Table L1] to [Table L3] (hereinafter, referred to as "Compound class SX809").

A compound represented by formula (L-1) wherein $R^{1b}$ represents a methylsulfanyl group, $R^{1c}$ represents a bromine atom, $A^1$, $A^4$, and $A^5$ represent CH, $A^2$ represents C-Me, and $A^3$ represents any one substituent indicated in [Table L1] to [Table L3] (hereinafter, referred to as "Compound class SX810").

A compound represented by formula (L-1) wherein $R^{1b}$ represents a methylsulfinyl group, $R^{1c}$ represents a bromine atom, $A^1$, $A^4$, and $A^5$ represent CH, $A^2$ represents. C-Me, and $A^3$ represents any one substituent indicated in [Table L1] to [Table L3] (hereinafter, referred to as "Compound class SX811").

A compound represented by formula (L-1) wherein $R^{1b}$ represents a methylstilfonyl group, $R^{1c}$ represents a bromine atom, $A^1$, $A^4$, and $A^5$ represent CH, $A^2$ represents C-Me, and $A^3$ represents any one substituent indicated in

[Table L1] to [Table L3] (hereinafter, referred to as "Compound class SX812").

A compound represented by formula (L-1) wherein $R^{1b}$ represents a methoxy group, $R^{1c}$ represents a hydrogen atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class. SX813").

A compound represented by formula (L-1) wherein $R^{1b}$ represents an ethoxy group, $R^{1c}$ represents a hydrogen atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX814").

A compound represented by formula (L-1) wherein $R^{1b}$ represents a methoxy group, $R^{1c}$ represents a fluorine atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX815").

A compound represented by formula (L-1) wherein $R^{1b}$ represents an ethoxy group, $R^{1c}$ represents a fluorine atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX816").

A compound represented by formula (L-1) wherein $R^{1b}$ represents a methoxy group, $R^{1c}$ represents a chlorine atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX817").

A compound represented by formula (L-1) wherein $R^{1b}$ represents an ethoxy group, $R^{1c}$ represents a chlorine atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX818").

A compound represented by formula (L-1) wherein $R^{1b}$ represents a methoxy group, $R^{1c}$ represents a bromine atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX819").

A compound represented by formula (L-1) wherein $R^{1b}$ represents an ethoxy group, $R^{1c}$ represents a bromine atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX820").

A compound represented by formula (L-1) wherein $R^{1b}$ represents a methylsulfanyl group, $R^{1c}$ represents a fluorine atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX821").

A compound represented by formula (L-1) wherein $R^{1b}$ represents a methylsulfinyl group, $R^{1c}$ represents a fluorine atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX822").

A compound represented by formula (L-1) wherein $R^{1b}$ represents a methylsulfonyl group, $R^{1c}$ represents a fluorine atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX823").

A compound represented by formula (L-1) wherein $R^{1b}$ represents a methylsulfanyl group, $R^{1c}$ represents a chlorine atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX824").

A compound represented by formula (L-1) wherein $R^{1b}$ represents a methylsulfinyl group, $R^{1c}$ represents a chlorine atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX825").

A compound represented by formula (L-1) wherein $R^{1b}$ represents a methylsulfonyl group, $R^{1c}$ represents a chlorine atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX826").

A compound represented by formula (L-1) wherein $R^{1b}$ represents a methylsulfanyl group, $R^{1c}$ represents a bromine atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX827").

A compound represented by formula (L-1) wherein $R^{1b}$ represents a methylsulfinyl group, $R^{1c}$ represents a bromine atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX828").

A compound represented by formula (L-1). wherein $R^{1b}$ represents a methyl sulfonyl group, $R^{1c}$ represents a bromine atom, $A^1$, $A^4$, and $A^5$ represent CH, $A^2$ represents C-$CF_3$, and $A^3$ represents any one substituent indicated in [Table L1] to [Table L3] (hereinafter, referred to as "Compound class SX829").

A compound represented by formula (L-1) wherein $R^{1b}$ represents a methoxy group, $R^{1c}$ represents a hydrogen atom, $A^1$, $A^4$, and $A^5$ represent CH, $A^2$ represents C-$OCF_3$, and $A^3$ represents any one substituent indicated in [Table L1] to [Table L3] (hereinafter, referred to as "Compound class SX830").

A compound represented by formula (L-1). wherein $R^{1b}$ represents an ethoxy group, $R^{1c}$ represents a hydrogen atom, $A^1$, $A^4$, and $A^5$ represent CH, $A^2$ represents C-$OCF_3$, and $A^3$ represents any one substituent indicated in [Table L1] to [Table L3] (hereinafter, referred to as "Compound class SX831").

A compound represented by formula (L-1) wherein $R^{1b}$ represents a methoxy group, $R^{1c}$ represents a fluorine atom, $A^1$, $A^4$, and $A^5$ represent CH, $A^2$ represents C-$OCF_3$, and $A^3$ represents any one substituent indicated in [Table L1] to [Table L3] (hereinafter, referred to as "Compound class SX832").

A compound represented by formula (L-1) wherein $R^{1b}$ represents an ethoxy group, $R^{1c}$ represents a fluorine atom, $A^1$, $A^4$, and $A^5$ represent CH, $A^2$ represents C-$OCF_3$, and $A^3$ represents any one substituent indicated in [Table L1] to [Table L3] (hereinafter, referred to as "Compound class SX833").

A compound represented by formula (L-1) wherein $R^{1b}$ represents a methoxy group, $R^{1c}$ represents a chlorine atom, $A^1$, $A^4$, and $A^5$ represent CH, $A^2$ represents C-$OCF_3$, and $A^3$ represents any one substituent indicated in [Table L1] to [Table L3] (hereinafter, referred to as "Compound class SX834").

A compound represented by formula (L-1) wherein $R^{1b}$ represents an ethoxy group, $R^{1c}$ represents a chlorine atom, $A^1$, $A^4$, and $A^5$ represent CH, $A^2$ represents C-$OCF_3$, and $A^3$ represents any one substituent indicated in [Table L1] to [Table L3] (hereinafter, referred to as "Compound class SX835").

A compound represented by formula (L-1) wherein $R^{1b}$ represents a methoxy group, $R^{1c}$ represents a bromine atom, $A^1$, $A^4$, and $A^5$ represent CH, $A^2$ represents C-$OCF_3$, and $A^3$ represents any one substituent indicated in [Table L1] to [Table L3) (hereinafter, referred to as "Compound class SX836").

A compound represented by formula (L-1) wherein $R^{1b}$ represents an ethoxy group, $R^{1c}$ represents a bromine atom, $A^1$, $A^4$, and $A^5$ represent CH, $A^2$ represents C-$OCF_3$, and $A^3$ represents any one substituent indicated in [Table L1] to [Table L3] (hereinafter, referred to as "Compound class SX837").

A compound represented by formula (L-1) wherein $R^{1b}$ represents a methylsulfanyl group, $R^{1c}$ represents a fluorine atom, $A^1$, $A^4$, and $A^5$ represent CH, $A^2$ represents C-$OCF_3$, and $A^3$ represents any one substituent indicated in [Table L1] to [Table L3) (hereinafter, referred to as "Compound class. SX838").

A compound represented by formula (L-1) wherein $R^{1b}$ represents a methylsulfinyl group, $R^{1c}$ represents a fluorine atom, $A^1$, $A^4$, and $A^5$ represent CH, $A^2$ represents C-$OCF_3$, and $A^3$ represents any one substituent indicated in [Table L1] to [Table L3] (hereinafter, referred to as "Compound class SX839").

A compound represented by formula (L-1) wherein $R^{1b}$ represents a methylsulfonyl group, $R^{1c}$ represents a fluorine atom, $A^1$, $A^4$, and $A^5$ represent CH, $A^2$ represents C-$OCF_3$, and $A^3$ represents any one substituent indicated in [Table L1] to (Table L3) (hereinafter, referred to as "Compound class, SX840").

A compound represented by formula (L-1) wherein $R^{1b}$ represents a methylsulfanyl group, $R^{1c}$ represents a chlorine atom, $A^1$, $A^4$, and $A^5$ represent CH, $A^2$ represents C-$OCF_3$, and $A^3$ represents any one substituent indicated in [Table L1] to [Table L3] (hereinafter, referred to as "Compound class SX841").

A compound represented by formula (L-1) wherein $R^{1b}$ represents a methylsulfinyl group, $R^{1c}$ represents a chlorine atom, $A^1$, $A^4$, and $A^5$ represent CH, $A^2$ represents C-$OCF_3$, and $A^3$ represents any one substituent indicated in [Table L1] to [Table L3] (hereinafter, referred to as "Compound class SX842").

A compound represented by formula (L-1) wherein $R^{1b}$ represents a methylsulfonyl group, $R^{1c}$ represents a chlorine atom, $A^1$, $A^4$, and $A^5$ represent CH, $A^2$ represents C-$OCF_3$, and $A^3$ represents any one substituent indicated in [Table L1] to [Table L3] (hereinafter, referred to as "Compound class SX843").

A compound represented by formula (L-1) wherein $R^{1b}$ represents a methylsulfanyl group, $R^{1c}$ represents a bromine atom, $A^1$, $A^4$, and $A^5$ represent CH, $A^2$ represents C-$OCF_3$, and $A^3$ represents any one substituent indicated in [Table L1] to [Table L3] (hereinafter, referred to as "Compound class SX844").

A compound represented by formula (L-1) wherein $R^{1b}$ represents a methylsulfinyl group, $R^{1c}$ represents a bromine atom, $A^1$, $A^4$, and $A^5$ represent CH, $A^2$ represents C-$OCP_3$, and $A^3$ represents any one substituent indicated in [Table L1] to [Table L3] (hereinafter, referred to as "Compound class SX845").

A compound represented by formula (L-1) wherein $R^{1b}$ represents a methylsulfonyl group, $R^{1c}$ represents a bromine atom, $A^1$, $A^4$, and $A^5$ represent CH, $A^2$ represents C-$OCF_3$, and $A^3$ represents any one substituent indicated in [Table L1] to [Table L3] (hereinafter, referred to as "Compound class SX846").

A compound represented by formula (L-1) wherein $R^{1b}$ represents a methoxy group, $R^{1c}$ represents a hydrogen atom, $A^1$, $A^3$, and $A^5$ represent CH, $A^2$ represents C-F, and $A^4$ represents any one substituent indicated in [Table L1] to [Table L3] (hereinafter, referred to as "Compound class SX847").

A compound represented by formula (L-1) wherein $R^{1b}$ represents an ethoxy group, $R^{1c}$ represents a hydrogen atom, $A^1$, $A^3$, and $A^5$ represent CH, $A^2$ represents C-F, and $A^4$ represents any one substituent indicated in [Table L1] to [Table L3] (hereinafter, referred to as "Compound class SX848").

A compound represented by formula (L-1) wherein $R^{1b}$ represents a methoxy group, $R^{1c}$ represents a fluorine atom, $A^1$, $A^3$, and $A^5$ represent CH, $A^2$ represents C-F, and $A^4$ represents any one substituent indicated in [Table L1] to [Table L3] (hereinafter, referred to as "Compound class SX849"}.

A compound represented by formula (L-1) wherein $R^{1b}$ represents an ethoxy group, $R^{1c}$ represents a fluorine atom, $A^1$, $A^3$, and $A^5$ represent CH, $A^2$ represents C-F, and $A^4$ represents any one substituent indicated in [Table L1] to [Table L3] (hereinafter, referred to as "Compound class SX850").

A compound represented by formula (L-1) wherein $R^{1b}$ represents a methoxy group, $R^{1c}$ represents a chlorine

atom, $A^1$, $A^3$, and $A^5$ represent CH, $A^2$ represents C-F, and $A^4$ represents any one substituent indicated in [Table L1] to [Table L3] (hereinafter, referred to as "Compound class SX851"}.

A compound represented by formula (L-1) wherein $R^{1b}$ represents an ethoxy group, $R^{1c}$ represents a chlorine atom, $A^1$, $A^3$, and $A^5$ represent CH, $A^2$ represents C-F, and $A^4$ represents any one substituent indicated in [Table L1] to [Table L3] (hereinafter, referred to as "Compound class SX852").

A compound represented by formula (L-1) wherein $R^{1b}$ represents a methoxy group, $R^{1c}$ represents a bromine atom, $A^1$, $A^3$, and $A^5$ represent CH, $A^2$ represents C-F, and $A^4$ represents any one substituent indicated in [Table L1] to [Table L3] (hereinafter, referred to as "Compound class SX853").

A compound represented by formula (L-1) wherein $R^{1b}$ represents an ethoxy group, $R^{1c}$ represents a bromine atom, $A^1$, $A^3$, and $A^5$ represent CH, $A^2$ represents C-F, and $A^4$ represents any one substituent indicated in [Table L1] to [Table L3] (hereinafter, referred to as "Compound class SX854").

A compound represented by formula (L-1) wherein $R^{1b}$ represents a methylsulfanyl group, $R^{1c}$ represents a fluorine atom, $A^1$, $A^3$, and $A^5$ represent CH, $A^2$ represents C-F, and $A^4$ represents any one substituent indicated in [Table L1] to [Table L3] (hereinafter, referred to as "Compound class SX8SS").

A compound represented by formula (L-1) wherein $R^{1b}$ represents a methylsulfinyl group, $R^{1c}$ represents a fluorine atom, $A^1$, $A^3$, and $A^5$ represent CH, $A^2$ represents C-F, and $A^4$ represents any one substituent indicated in [Table L1] to [Table L3] (hereinafter, referred to as "Compound class SX856").

A compound represented by formula (L-1) wherein $R^{1b}$ represents a methylsulfonyl group, $R^{1c}$ represents a fluorine atom, $A^1$, $A^3$, and $A^5$ represent CH, $A^2$ represents C-F, and $A^4$ represents any one substituent indicated in [Table L1] to [Table L3] (hereinafter, referred to as "Compound class SX857").

A compound represented by formula (L-1). wherein $R^{1b}$ represents a methylsulfanyl group, $R^{1c}$ represents a chlorine atom, $A^1$, $A^3$, and $A^5$ represent CH, $A^2$ represents C-F, and $A^4$ represents any one substituent indicated in [Table L1] to [Table L3] (hereinafter, referred to as "Compound class SX858").

A compound represented by formula (L-1) wherein $R^{1b}$ represents a methylsulfinyl group, $R^{1c}$ represents a chlorine atom, $A^1$, $A^3$, and $A^5$ represent CH, $A^2$ represents C-F, and $A^4$ represents any one substituent indicated in [Table L1] to [Table L3] (hereinafter, referred to as "Compound class SX859").

A compound represented by formula (L-1). wherein $R^{1b}$ represents a methylsulfonyl group, $R^{1c}$ represents a chlorine atom, $A^1$, $A^3$, and $A^5$ represent CH, $A^2$ represents C-F, and $A^4$ represents any one substituent indicated in [Table L1] to [Table L3] (hereinafter, referred to as "Compound class SX860").

A compound represented by formula (L-1) wherein $R^{1b}$ represents a methylsulfanyl group, $R^{1c}$ represents a bromine atom, $A^1$, $A^3$, and $A^5$ represent CH, $A^2$ represents C-F, and $A^4$ represents any one substituent indicated in [Table L1] to [Table L3] (hereinafter, referred to as "Compound class SX861").

A compound represented by formula (L-1) wherein $R^{1b}$ represents a methylsulfinyl group, $R^{1c}$ represents a bromine atom, $A^1$, $A^3$, and $A^5$ represent CH, $A^2$ represents C-F, and $A^4$ represents any one substituent indicated in [Table L1] to [Table L3] (hereinafter, referred to as "Compound class SX862").

A compound represented by formula (L-1) wherein $R^{1b}$ represents a methylsulfonyl group, $R^{1c}$ represents a bromine atom, $A^1$, $A^3$, and $A^5$ represent CH, $A^2$ represents C-F, and $A^4$ represents any one substituent indicated in [Table L1] to [Table L3] (hereinafter, referred to as "Compound class SX863").

A compound represented by formula (L-1) wherein $R^{1b}$ represents a methoxy group, $R^{1c}$ represents a hydrogen atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX864").

A compound represented by formula (L-1) wherein $R^{1b}$ represents an ethoxy group, $R^{1c}$ represents a hydrogen atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX865").

A compound represented by formula (L-1) wherein $R^{1b}$ represents a methoxy group, $R^{1c}$ represents a fluorine atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX866").

A compound represented by formula (L-1) wherein $R^{1b}$ represents an ethoxy group, $R^{1c}$ represents a fluorine atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX867").

A compound represented by formula (L-1) wherein $R^{1b}$ represents a methoxy group, $R^{1c}$ represents a chlorine atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX868").

A compound represented by formula (L-1) wherein $R^{1b}$ represents an ethoxy group, $R^{1c}$ represents a chlorine atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX869").

A compound represented by formula (L-1) wherein $R^{1b}$ represents a methoxy group, $R^{1c}$ represents a bromine atom, $A^1$, $A^3$, and $A^5$ represent CH, $A^2$ represents C-Cl, and $A^4$ represents any one substituent indicated in [Table L1]

to [Table L3] (hereinafter, referred to as "Compound class SX870").

A compound represented by formula (L-1) wherein $R^{1b}$ represents an ethoxy group, $R^{1c}$ represents a bromine atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX871").

A compound represented by formula (L-1) wherein $R^{1b}$ represents a methylsulfanyl group, $R^{1c}$ represents a fluorine atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX872"),

A compound represented by formula (L-1) wherein $R^{1b}$ represents a methylsulfinyl group, $R^{1c}$ represents a fluorine atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX873").

A compound represented by formula (L-1) wherein $R^{1b}$ represents a methylsulfonyl group, $R^{1c}$ represents a fluorine atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX874").

A compound represented by formula (L-1) wherein $R^{1b}$ represents a methylsulfanyl group, $R^{1c}$ represents a chlorine atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX875").

A compound represented by formula (L-1) wherein $R^{1b}$ represents a methylsulfinyl group, $R^{1c}$ represents a chlorine atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX876"}.

A compound represented by formula (L-1) wherein $R^{1b}$ represents a methylsulfonyl group, $R^{1c}$ represents a chlorine atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX877").

A compound represented by formula (L-1) wherein $R^{1b}$ represents a methylsulfanyl group, $R^{1c}$ represents a bromine atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX878").

A compound represented by formula (L-1) wherein $R^{1b}$ represents a methylsulfinyl group, $R^{1c}$ represents a bromine atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX879").

A compound represented by formula (L-1) wherein $R^{1b}$ represents a methylsulfonyl group, $R^{1c}$ represents a bromine atom, $A^1$, $A^3$, and $A^5$ represent CH, $A^2$ represents C-Cl, and $A^4$ represents any one substituent indicated in [Table L1] to [Table L3] (hereinafter, referred to as "Compound class SX880").

A compound represented by formula (L-1) wherein $R^{1b}$ represents a methoxy. group, $R^{1c}$ represents a hydrogen atom, $A^1$, $A^3$, and $A^5$ represent CH, $A^2$ represents C-Me, and $A^4$ represents any one substituent indicated in [Table L1] to [Table L3] (hereinafter, referred to as "Compound class SX881").

A compound represented by formula (L-1) wherein $R^{1b}$ represents an ethoxy group, $R^{1c}$ represents a hydrogen atom, $A^1$, $A^3$, and $R^5$ represent CH, $A^2$ represents C-Me, and $A^4$ represents any one substituent indicated in [Table L1] to [Table L3] (hereinafter, referred to as "Compound class SX882").

A compound represented by formula (L-1) wherein $R^{1b}$ represents a methoxy group, $R^{1c}$ represents a fluorine atom, $A^1$, $A^3$, and $A^5$ represent CH, $A^2$ represents C-Me, and $A^4$ represents any one substituent indicated in [Table L1] to [Table L3] (hereinafter, referred to as "Compound class SX883").

A compound represented by formula (L-1) wherein $R^{1b}$ represents an ethoxy group, $R^{1c}$ represents a fluorine atom, $A^1$, $A^3$, and $A^5$ represent CH, $A^2$ represents C-Me, and $A^4$ represents any one substituent indicated in [Table L1] to [Table L3] (hereinafter, referred to as "Compound class SX884").

A compound represented by formula (L-1) wherein $R^{1b}$ represents a methoxy group, $R^{1c}$ represents a chlorine atom, $A^1$, $A^3$, and $A^5$ represent CH, $A^2$ represents C-Me, and $A^4$ represents any one substituent indicated in [Table L1] to [Table L3] (hereinafter, referred to as "Compound class SX885").

A compound represented by formula (L-1) wherein $R^{1b}$ represents an ethoxy group, $R^{1c}$ represents a chlorine atom, $A^1$, $A^3$, and $A^5$ represent CH, $A^2$ represents, C-Me, and $A^4$ represents any one substituent indicated in [Table L1] to [Table L3] (hereinafter, referred to as "Compound class SX886").

A compound represented by formula (L-1) wherein $R^{1b}$ represents a methoxy group, $R^{1c}$ represents a bromine atom, $A^1$, $A^3$, and $A^5$ represent CH, $A^2$ represents C-Me, and $A^4$ represents any one substituent indicated in [Table L1] to [Table L3] (hereinafter, referred to as "Compound class SX887").

A compound represented by formula (L-1) wherein $R^{1b}$ represents an ethoxy group, $R^{1c}$ represents a bromine atom, $A^1$, $A^3$, and $A^5$ represent CH, $A^2$ represents, C-Me, and $A^4$ represents any one substituent indicated in [Table L1] to [Table L3] (hereinafter, referred to as "Compound class SX888").

A compound represented by formula (L-1) wherein $R^{1b}$ represents a methylsulfanyl group, $R^{1c}$ represents a fluorine atom, $A^1$, $A^3$, and $A^5$ represent CH, $A^2$ represents C-Me, and $A^4$ represents any one substituent indicated in [Table L1] to [Table L3] (hereinafter, referred to as "Compound class SX889").

A compound represented by formula (L-1) wherein $R^{1b}$ represents a methylsulfinyl group, $R^{1c}$ represents a fluorine atom, $A^1$, $A^3$, and $A^5$ represent CH, $A^2$ represents C-Me, and $A^4$ represents any one substituent indicated in [Table L1] to [Table L3] (hereinafter, referred to as "Compound class SX890").

A compound represented by formula (L-1) wherein $R^{1b}$ represents a methylsulfonyl group, $R^{1c}$ represents a fluorine atom, $A^1$, $A^3$, and $A^5$ represent CH, $A^2$ represents C-Me, and $A^4$ represents any one substituent indicated in [Table L1] to [Table L3] (hereinafter, referred to as "Compound class SX891").

A compound represented by formula (L-1) wherein $R^{1b}$ represents a methylsulfanyl group, $R^{1c}$ represents a chlorine atom, $A^1$, $A^3$, and $A^5$ represent CH, $A^2$ represents C-Me, and $A^4$ represents any one substituent indicated in [Table L1] to [Table L3] (hereinafter, referred to as "Compound class SX892") .

A compound represented by formula (L-1) wherein $R^{1b}$ represents a methylsulfinyl group, $R^{1c}$ represents a chlorine atom, $A^1$, $A^3$, and $A^5$ represent CH, $A^2$ represents C-Me, and $A^4$ represents any one substituent indicated in [Table L1] to [Table L3] (hereinafter, referred to as "Compound class SX893").

A compound represented by formula (L-1) wherein $R^{1b}$ represents a methylsulfonyl group, $R^{1c}$ represents a chlorine atom, $A^1$, $A^3$, and $A^5$ represent CH, $A^2$ represents C-Me, and $A^4$ represents any one substituent indicated in [Table L1] to (Table L3) (hereinafter, referred to as "Compound class SX894").

A compound represented by formula (L-1) wherein $R^{1b}$ represents a methylsulfanyl group, $R^{1c}$ represents a bromine atom, $A^1$, $A^3$, and $A^5$ represent CH, $A^2$ represents C-Me, and $A^4$ represents any one substituent indicated in [Table L1] to [Table L3] (hereinafter, referred to as "Compound class SX895").

A compound represented by formula (L-1) wherein $R^{1b}$ represents a methylsulfinyl group, $R^{1c}$ represents a bromine atom, $A^1$, $A^3$, and $A^5$ represent CH, $A^2$ represents C-Me, and $A^4$ represents any one substituent indicated in [Table L1] to [Table L3] (hereinafter, referred to as "Compound class. SX896").

A compound represented by formula (L-1) wherein $R^{1b}$ represents a methylsulfonyl group, $R^{1c}$ represents a bromine atom, $A^1$, $A^3$, and $A^5$, represent CH, $A^2$ represents C-Me, and $A^4$ represents any one substituent indicated in [Table L1] to [Table L3] (hereinafter, referred to as "Compound class SX897").

A compound represented by formula (L-1) wherein $R^{1b}$ represents a methoxy group, $R^{1c}$ represents a hydrogen atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX898") .

A compound represented by formula (L-1) wherein $R^{1b}$ represents an ethoxy group, $R^{1c}$ represents a hydrogen atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX899").

A compound represented by formula (L-1) wherein $R^{1b}$ represents a methoxy group, $R^{1c}$ represents a fluorine atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX900").

A compound represented by formula (L-1) wherein $R^{1b}$ represents an ethoxy group, $R^{1c}$ represents a fluorine atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX901").

A compound represented by formula (L-1) wherein $R^{1b}$ represents a methoxy group, $R^{1c}$ represents a chlorine atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX902") .

A compound represented by formula (L-1) wherein $R^{1b}$ represents an ethoxy group, $R^{1c}$ represents a chlorine atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX903").

A compound represented by formula (L-1) wherein $R^{1b}$ represents a methoxy group, $R^{1c}$ represents a bromine atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX904").

A compound represented by formula (L-1) wherein $R^{1b}$ represents an ethoxy group, $R^{1c}$ represents a bromine atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX905").

A compound represented by formula (L-1) wherein $R^{1b}$ represents a methylsulfanyl group, $R^{1c}$ represents a fluorine atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX906").

A compound represented by formula (L-1) wherein $R^{1b}$ represents a methylsulfinyl group, $R^{1c}$ represents a fluorine atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX907") .

A compound represented by formula (L-1) wherein $R^{1b}$ represents a methylsulfonyl group, $R^{1c}$ represents a fluorine atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX908") .

A compound represented by formula (L-1) wherein $R^{1b}$ represents a methylsulfanyl group, $R^{1c}$ represents a

chlorine atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX909").

A compound represented by formula (L-1) wherein $R^{1b}$ represents a methylsulfinyl group, $R^{1c}$ represents a chlorine atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX910") .

A compound represented by formula (L-1) wherein $R^{1b}$ represents a methylsulfonyl group, $R^{1c}$ represents a chlorine atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX911").

A compound represented by formula (L-1). wherein $R^{1b}$ represents a methylsulfanyl group, $R^{1c}$ represents a bromine atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX912").

A compound represented by formula (L-1) wherein $R^{1b}$ represents a methylsulfinyl group, $R^{1c}$ represents a bromine atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX913").

A compound represented by formula (L-1). wherein $R^{1b}$ represents a methylsulfonyl group, $R^{1c}$ represents a bromine atom, $A^1$, $A^3$, and $A^5$ represent CH, $A^2$ represents C-$CF_3$, and $A^4$ represents any one substituent indicated in [Table L1] to [Table L3] (hereinafter, referred to as "Compound class SX914").

A compound represented by formula (L-1) wherein $R^{1b}$ represents a methoxy group, $R^{1c}$ represents a hydrogen atom, $A^1$, $A^3$, and $A^5$ represent CH, $A^2$ represents C-$OCF_3$, and $A^4$ represents any one substituent indicated in [Table L1] to [Table L3] (hereinafter, referred to as "Compound class SX915").

A compound represented by formula (L-1) wherein $R^{1b}$ represents an ethoxy group, $R^{1c}$ represents a hydrogen atom, $A^1$, $A^3$, and $A^5$ represent CH, $A^2$ represents C-$OCF_3$, and $A^4$ represents any one substituent indicated in [Table L1] to [Table L3] (hereinafter, referred to as "Compound class SX916").

A compound represented by formula (L-1) wherein $R^{1b}$ represents a methoxy group, $R^{1c}$ represents a fluorine atom, $A^1$, $A^3$, and $A^5$ represent CH, $A^2$ represents C-$OCF_3$, and $A^4$ represents any one substituent indicated in [Table L1] to [Table L3] (hereinafter, referred to as "Compound class SX917").

A compound represented by formula (L-1) wherein $R^{1b}$ represents an ethoxy group, $R^{1c}$ represents a fluorine atom, $A^1$, $A^3$, and $A^5$ represent CH, $A^2$ represents C-$OCF_3$, and $A^4$ represents any one substituent indicated in [Table L1] to [Table L3] (hereinafter, referred to as "Compound class SX918").

A compound represented by formula (L-1) wherein $R^{1b}$ represents a methoxy group, $R^{1c}$ represents a chlorine atom, $A^1$, $A^3$, and $A^5$ represent CH, $A^2$ represents C-$OCF_3$, and $A^4$ represents any one substituent indicated in [Table L1] to (Table L3) (hereinafter, referred to as "Compound class SX919").

A compound represented by formula (L-1) wherein $R^{1b}$ represents an ethoxy group, $R^{1c}$ represents a chlorine atom, $A^1$, $A^3$, and $A^5$ represent CH, $A^2$ represents C-$OCF_3$, and $A^4$ represents any one substituent indicated in [Table L1] to [Table L3] (hereinafter, referred to as "Compound class SX920").

A compound represented by formula (L-1) wherein $R^{1b}$ represents a methoxy group, $R^{1c}$ represents a bromine atom, $A^1$, $A^3$, and $A^5$ represent CH, $A^2$ represents C-$OCF_3$, and $A^4$ represents any one substituent indicated in [Table L1] to [Table L3] (hereinafter, referred to as "Compound class. SX921").

A compound represented by formula (L-1) wherein $R^{1b}$ represents an ethoxy group, $R^{1c}$ represents a bromine atom, $A^1$, $A^3$, and $A^5$ represent CH, $A^2$ represents C-$OCF_3$, and $A^4$ represents any one substituent indicated in [Table L1] to [Table L3] (hereinafter, referred to as "Compound class SX922").

A compound represented by formula (L-1) wherein $R^{1b}$ represents a methylsulfanyl group, $R^{1c}$ represents a fluorine atom, $A^1$, $A^3$, and $A^5$ represent CH, $A^2$ represents C-$OCF_3$, and $A^4$ represents any one substituent indicated in [Table L1] to [Table L3] (hereinafter, referred to as "Compound class SX923").

A compound represented by formula (L-1) wherein $R^{1b}$ represents a methylsulfinyl group, $R^{1c}$ represents a fluorine atom, $A^1$, $A^3$, and $A^5$ represent CH, $A^2$ represents C-$OCF_3$, and $A^4$ represents any one substituent indicated in [Table L1] to [Table L3] (hereinafter, referred to as "Compound class SX924").

A compound represented by formula (L-1) wherein $R^{1b}$ represents a methylsulfonyl group, $R^{1c}$ represents a fluorine atom, $A^1$, $A^3$, and $A^5$ represent CH, $A^2$ represents C-$OCF_3$, and $A^4$ represents any one substituent indicated in [Table L1] to [Table L3] (hereinafter, referred to as "Compound class SX925").

A compound represented by formula (L-1) wherein $R^{1b}$ represents a methylsulfanyl group, $R^{1c}$ represents a chlorine atom, $A^1$, $A^3$, and $A^5$ represent CH, $A^2$ represents C-$OCF_3$, and $A^4$ represents any one substituent indicated in [Table L1] to [Table L3] (hereinafter, referred to as "Compound class SX926").

A compound represented by formula (L-1) wherein $R^{1b}$ represents a methylsulfinyl group, $R^{1c}$ represents a chlorine atom, $A^1$, $A^3$, and $A^5$ represent CH, $A^2$ represents C-$OCF_3$, and $A^4$ represents any one substituent indicated in [Table L1] to [Table L3] (hereinafter, referred to as "Compound class SX927") .

A compound represented by formula (L-1) wherein $R^{1b}$ represents a methylsulfonyl group, $R^{1c}$ represents a chlorine atom, $A^1$, $A^3$, and $A^5$ represent CH, $A^2$ represents C-$OCF_3$, and $A^4$ represents any one substituent indicated in

[Table L1] to [Table L3] (hereinafter, referred to as "Compound class SX928"}.

A compound represented by formula (L-1) wherein $R^{1b}$ represents a methylsulfanyl group, $R^{1c}$ represents a bromine atom, $A^1$, $A^3$, and $A^5$ represent CH, $A^2$ represents C-$OCF_3$, and $A^4$ represents any one substituent indicated in [Table L1] to [Table L3] (hereinafter, referred to as "Compound class SX929") .

A compound represented by formula (L-1) wherein $R^{1b}$ represents a methylsulfinyl group, $R^{1c}$ represents a bromine atom, $A^1$, $A^3$, and $A^5$ represent CH, $A^2$ represents C-$OCF_3$, and $A^4$ represents any one substituent indicated in [Table L1] to [Table L3] (hereinafter, referred to as "Compound class SX930").

A compound represented by formula (L-1) wherein $R^{1b}$ represents a methylsulfonyl group, $R^{1c}$ represents a bromine atom, $A^1$, $A^3$, and $A^5$ represent CH, $A^2$ represents C-$OCF_3$, and $A^4$ represents any one substituent indicated in [Table L1] to [Table L3] (hereinafter, referred to as "Compound class SX931").

Next, the formulation examples of the compounds of the present invention are shown below. In the formulation examples, the "parts" represents "part by weight" unless otherwise specified. The present compound S represents the compounds described in the Compound Classes SX1 to SX931.

Formulation Example 1

Thirty five (35) parts of a mixture of ammonium polyoxyethylene alkyl ether sulfate and wet silica (weight ratio: 1:1), 10 parts of any one of the present compound S, and 55 parts of water are mixed, and the mixture is then finely-ground by a wet grinding method to obtain a formulation.

Formulation Example 2

Fifty (50) parts of any one of the present compound S, 3 parts of calcium lignin sulfonate, 2 parts of sodium lauryl sulfate, and 45 parts of silica are well mixed-grinding to obtain a formulation.

Formulation Example 3

Five (5) parts of any one of the present compound S, 9 parts of polyoxyethylene styryl phenyl ether, 5 parts of polyoxyethylene decyl ether (Number of ethylene oxide additions: 5), 6 parts of calcium dodecylbenzene sulfonate and 75 parts of xylene are well mixed to obtain a formulation.

Formulation Example 4

Two (2) parts of any one of the present compound S, 1 part of silica, 2 parts of calcium lignin sulfonate, 30 parts of bentonite and 65 parts of kaolin clay are mixed-grinding, and thereto is added an appropriate amount of water, and the mixture is well kneaded and is then granulated with a granulator and dried to obtain a formulation.

Formulation Example 5

Ten (10) parts of any one of the present compound S is mixed with a mixture of 18 parts of benzyl alcohol and 9 parts of DMSO, and thereto are added 6.3 parts of GERONOL (registered trademark) TE250, 2.7 parts of Ethylan (registered trademark) NS-500LQ, and 54 parts of Solvent naphtha, and the mixture is mixed to obtain a formulation.

Formulation Example 6

Zero point one (0.1) parts of any one of the present compound S and 39.9 parts of kerosene are dissolved while mixing, and the mixture is placed in an aerosol container, and 60 parts of liquefied petroleum gas (mixture of propane, butane and isobutane; saturated vapor pressure; 0.47 MPa (25°C)) is filled in the container to obtain a formulation.

Formulation Example 7

Zero point two (0.2) parts of any one of the present compound S, 50 parts of pyrethrum extract dreg powder, 30 parts of Machilus thunbergii powder, and 19.8 parts of wood powder are mixed, and an appropriate amount of water is added thereto, and the mixture is well kneaded, and is extructed with an extruder into a plate-like sheet, and the resulting sheet is made a spiral-like form thereof with a punching machine to obtain a formulation.

Next, an efficacy of the present compound on controlling harmful arthropods is shown by Test examples. The following tests were conducted at 25°C.

Test. Method 1

Test compounds are made to a formulation according to a similar method to that described in the Formulation example 1, and thereto is added water containing 0.03 v/v% of Shindain (registered trademark) to prepare a diluted solution containing a prescribed concentration of the test compound.
Cucumber (*cucumber sativus*) seedling (on the developmental stage of the second true leaf) is planted in a cup, and approximately 30 cotton aphids (*Aphis gossypii)* (all stages of life) are released onto the seedling. After one day, the diluted solutions are sprayed into the seedling at a ratio of 10 mL/seedling. After 5 days, the number of the surviving insects is examined and the controlling value is calculated by the following equation.

$$\text{Controlling value (\%)} = \{1 - (Cb \times Tai)/(Cai \times Tb)\} \times 100$$

wherein the symbols in the equation represent the following descriptions.

Cb: Number of the test insects in untreated group;
Cai: Number of the surviving insects at the time of the examination in untreated group;
Tb: Number of the test insects in treated group;
Tai: Number of the surviving insects at the time of the examination in treated group;
Here the "untreated group" represents a group where a similar treatment procedure to that of treated group except not using the test compound is done.

Test Example 1-1

The test was conducted according to the Test method. 1 by making the prescribed concentration 500 ppm and using the below-mentioned present compounds as a test compound, and, as the result of the test, the below-mentioned present compounds showed 90% or more as the controlling value. Present compound Nos: 1, 2, 3, 4, 7, 8, 9, 10, 11, 12, 14, 15, 17, 19, 23, 25, 28, 29, 30, 31, 32, 34, 35, 36, 38, 39, 40, 42, 45, 46, 49, 53, 54, 56, 60, 63, 64, 65, 67, 71, 72, 74, 77, 78, 79, 80, 81, 82, 83, 86, 87, 90, 91, 92, 94, 96, 100, 101, 108, 118, 123, 125, 129, 130, 135, 138, 139, 140, 143, 145, 146, 147, 148, 149

Test Method 2

Test compounds are made to a formulation according to a similar method to that described in the Formulation example 5, and thereto is added water containing 0.03 v/v% of Shindain (registered trademark) to prepare a diluted solution containing a prescribed concentration of the test compound.
Cucumber (*cucumber sativus*) seedling (on the developmental stage of the second true leaf) is planted in a cup, and approximately 30 cotton aphids (*Aphis gossypii*) (all stages of life) are released onto the seedling. After one day, the diluted solutions are sprayed into the seedling at a ratio of 10 mL/seedling. After 5 days, the number of the surviving insects is examined and the controlling value is calculated by the following equation.

$$\text{Controlling value (\%)} = \{1 - (Cb \times Tai)/(Cai \times Tb)\} \times 100$$

wherein the symbols in the equation represent the following descriptions.

Cb: Number of the test insects in untreated group;
Cai: Number of the surviving insects at the time of the examination in untreated group;
Tb: Number of the test insects in treated group;
Tai: Number of the surviving insects at the time of the examination ih treated groups
Here the "untreated group" represents a group where a similar treatment procedure to that of treated group except not using the test compound is done.

Test Example 2-1

The test was conducted according to the Test method 2 by making the prescribed concentration 200 ppm and using the below-mentioned present compounds as a test compound, and, as the result of the test, the below-mentioned present compounds showed 90% or more as the controlling value.

Present compound Nos: 1, 2, 3, 4, 7, 8, 9, 10, 11, 12, 14, 15, 19, 23, 24, 27, 28, 29, 30, 32, 33, 34, 35, 36, 38, 39, 40, 42, 45, 46, 48, 49, 51, 53, 54, 56, 60, 61, 63, 65, 67, 68, 71, 72, 75, 76, 77, 78, 79, 80, 82, 83, 90, 91, 92, 94, 96, 97, 100, 108, 110, 112, 117, 118, 119, 120, 121, 123, 125, 129, 130, 135, 138, 139, 140, 143, 145, 146, 147, 148, 149

Test Method 3

Test compounds are made to a formulation according to a similar method to that described in the Formulation example 1, and thereto is added water containing 0.03 v/v% of Shindain (registered trademark) to prepare a diluted solution containing a prescribed concentration of the test compound.

Rice (Oryza sativa) seedling (on the developmental stage of the second true. leaf) is planted in a container, and the diluted solutions are sprayed into the seedling in a ratio of 10 mL/seedling. Thereafter, 20 of 3rd instar larvae of brown planthoppers (*Nilaparvata lugens*) are released onto the rice leaves. After 5 days, the number of the surviving insects is examined and the mortality is, calculated by the following equation.

Mortality (%) = {1- the number of the surviving insects/20} × 100

Test Example 3-1

The test was conducted according to the Test method 3 by making the prescribed concentration 500 ppm and using the below-mentioned present compounds as a test compound, and, as the result of the test, the below-mentioned present compounds showed 90% or more as the mortality.
Present compound Nos: 3, 7, 9, 10, 11, 12, 13, 14, 15, 16, 17, 19, 20, 21, 22, 25, 27, 28, 30, 32, 35, 38, 39, 40, 41, 42, 45, 46, 48, 49, 51, 53, 54, 56, 58, 59, 64, 65, 67, 71, 72, 73, 74, 75, 76, 77, 78, 79, 80, 82, 83, 87, 90, 91, 92, 94, 96, 100, 106, 108, 118, 121, 122, 123, 125, 128, 129, 130, 131, 132, 133, 134, 135, 136, 138, 139, 140, 143, 145, 146, 147, 148, 149

Test Method 4

Test compounds are made to a formulation according to a similar method to that described in the Formulation example 5, and thereto is added water containing 0.03 v/v% of Shindain (registered trademark) to prepare a diluted solution containing a prescribed concentration of the test compound.

Rice (Oryza sativa) seedling (on the developmental stage of the second true leaf) is planted in a container, and the diluted solutions are sprayed into the seedling in a ratio of 10 mL/seedling. Thereafter, 20 of 3rd instar larvae of brown planthoppers (*Nilaparvata lugens*) are released onto the rice leaves. After 6 days, the number of the surviving insects is examined and the mortality is calculated by the following equation.

Mortality (%) = {1- the number of the surviving insects/20} × 100

Test Example 4-1

The test was conducted according to the Test method 4 by making the prescribed concentration 200 ppm and using the below-mentioned present compounds as a test compound, and, as the result of the test, the below-mentioned present compounds showed 90% or more as the mortality.
Present compound Nos: 1, 2, 3, 4, 7, 8, 9, 10, 11, 12, 13, 14, 15, 16, 19, 20, 21, 22, 23, 24, 25, 26, 27, 28, 29, 30, 32, 33, 35, 38, 39, 40, 41, 42, 46, 48, 49, 51, 53, 54, 56, 58, 59, 60, 61, 64, 65, 67, 70, 71, 72, 73, 74, 75, 76, 77, 78, 79, 80, 82, 83, 85, 86, 90, 91, 92, 94, 95, 96, 97, 98, 100, 106, 108, 110, 112, 117, 118, 119, 120, 121, 123, 125, 128, 131, 132, 133, 134, 135, 136, 138, 139, 140, 143, 145, 146, 147, 148, 149

Test Example 4-2

The test was conducted according to the Test method 4 by making the prescribed concentration 50 ppm and using the below-mentioned present compounds as a test compound, and, as the result of the test, the below-mentioned

present compounds showed 90% or more as the mortality.
Present compound Nos: 2, 92, 95, 102, 134, 138, 148

Test Method 5

Test compounds are made to a formulation according to a similar method to that described in the Formulation example 5, and thereto is added water containing 0.03 v/v% of Shindain (registered trademark) to prepare a diluted solution containing a prescribed concentration of the test compound.
Silverleaf whiteflies (Bemisia tabaci) are released on tomato (Lycopersicon esculentum) seedling that is planted in a container, and then spawn for about 24 hours. The seedling are stored for 8 days, and the larvae of silverleaf whiteflies are hatched from the laid eggs. The diluted solutions are sprayed into the seedling in a ratio of 10 mL/seedling. After 7 days, the number of the surviving insects is examined, and the controlling value is calculated by the following equation.

$$\text{Controlling value (\%)} = \{1-(Cb\times Tai)/(Cai\times Tb)\}\times 100$$

wherein the symbols in the formula represent the following descriptions.

Cb: Number of the insects shortly before the treatment in untreated group;
Cai: Number of the surviving insects at the time of the investigation in untreated group;
Tb: Number of the insects shortly before the treatment in treated group;
Tai: Number of the surviving insects at the time of the investigation in treated group;
Here the "untreated group" represents a group where a similar treatment procedure to that of treated group except not using the test compound is done.

Test Example 5-1

The test was conducted according to the Test method 5 by making the prescribed concentration 200 ppm and using the below-mentioned present compounds as a test compound, and, as the result of the test, the below-mentioned present compounds showed 90% or more as the controlling value. Present compound Nos: 2, 6, 7, 8, 9, 10, 11, 12, 13, 14, 15, 16, 17, 18, 19, 20, 22, 27, 28, 29, 30, 33, 34, 35, 36, 38, 40, 41, 42, 46, 48, 49, 53, 54, 56, 58, 59, 60, 61, 63, 64, 65, 68, 69, 71, 72, 73, 74, 75, 76, 77, 78, 79, 80, 83, 90, 91, 92, 94, 96, 97, 98, 100, 108, 109, 110, 111, 112, 117, 118, 119, 120, 121, 123, 125, 129, 132, 134, 135, 136, 138, 139, 140, 143, 145, 146, 147, 148, 149

Test Example 5-2

The test was conducted according to the Test method 5 by making the prescribed concentration 50 ppm and using the below-mentioned present compounds as a test compound, and, as the result of the test, the below-mentioned present compounds showed 90% or more as the controlling value. Present compound Nos: 2, 19, 94, 95, 117, 139, 145, 147

Test Method 6

Test compounds are made to a formulation according to a similar method to that described in the Formulation example 1, and thereto is added water to prepare a diluted solution containing a prescribed concentration of the test compound.
Into the diluted solution, 30 common house mosquito (*Culex pipiens pallens*) at the last instar larval stage are released, and after 1 day, the state of the house mosquito larvae is examined, and the mortality of insects is calculated by the following equation.

$$\text{Mortality (\%)} = (\text{the Number of the dead insects/the Number of tested insects}) \times 100$$

Test Example 6-1

The test was conducted according to the Test method 6 by making the prescribed concentration 3.5 ppm and using the below-mentioned present compounds as a test compound, and, as the result of the test, the below-mehtioned present compounds showed 91% or more as the mortality.
Present compound Nos: 4, 7, 12, 15, 16, 17, 18, 19, 21, 28, 30, 36, 38, 39, 42, 46, 53, 56, 58, 60, 77, 78, 79, 82, 88, 91, 92, 107, 108, 121, 145, 146

Test Method 7

Test compounds are made to a formulation according to a similar method to that described in the Formulation example 1, and thereto is added water to prepare a diluted solution containing a prescribed concentration of the test compound.
Into the diluted solution, 30 common house mosquito (*Culex pipiens pollens*) at the last instar larval stage are released, and after 8 days, the state of the house mosquito larvae is examined, and the mortality of insects is calculated by the following equation.

Mortality (%) = (the Number of the dead insects/the Number of tested insects) × 100

Test Example 7-1

The test was conducted according to the Test method 7 by making the prescribed concentration 3.5 ppm and using the below-mentioned present compounds as a test compound, and, as the result of the test, the below-mentioned present compounds showed 91% or more as the mortality.
Present compound Nos: 5, 6, 8, 9, 10, 11, 13, 14, 20, 22, 23, 24, 25, 26, 27, 29, 31, 32, 34, 31, 41, 43, 47, 49, 50, 51, 52, 54, 55, 57, 59, 62, 63, 64, 65, 66, 67, 68, 71, 72, 73, 74, 76, 83, 84, 86, 90, 96, 99, 100, 114, 115, 116, 122, 130, 135, 139, 140, 145, 146

Test Method 8

Test compounds are made to a formulation according to a similar method to that described in the Formulation example 1, and thereto is added water containing 0.03 v/v% of Shindain (registered trademark) to prepare a diluted solution containing a prescribed concentration of the test compound.
Cabbage (*Brassicae oleracea*) seedling (on the developmental stage of the second to third true leaf) is planted in a cup, and the diluted solutions are sprayed into the seedling at a ratio of 20 mL/seedling. Thereafter, the stem and leaf thereof is cut out and then is installed into a cup that is covered with filter paper. Five (5) tobacco cutworm (*Spodoptera litura*) at the second instar larval stage are released into the cup. After 5 days, the number of the surviving insects is counted, and the mortality of insects is calculated by the following equation.

Mortality (%) = (1 - the Number of surviving insects/5) × 100

Test Example 8-1

The test was conducted according to the Test method 8 by making the prescribed concentration 500 ppm and using the below-mentioned present compounds as a test compound, and, as the result of the test, the below-mentioned present compounds showed 80% or more as the mortality.
Present compound Nos: 23, 28, 31, 32, 35, 38, 67, 77, 88, 89, 91, 100, 106, 118, 143

Test Method 9

Test compounds are made to a formulation according to a similar method to that described in the Formulation example 1, and thereto is added water containing 0.03 v/v% of Shindain (registered trademark) to prepare a diluted

solution containing a prescribed concentration of the test compound.

Cabbage (*Brassicae oleracea)* seedling (on the developmental stage of the second to third true leaf) is planted in a cup, and the diluted solutions are sprayed into the seedling at a ratio of 20 mL/seedling. Thereafter, the stem and leaf thereof is cut out and then is installed into a cup that is covered with filter paper. Five (5) diamondback moth (*Plutella xylostella*) at the second instar larval stage are released into the cup. After 5 days, the number of the surviving insects is counted, and the mortality of insects is calculated by the following equation.

$$\text{Mortality (\%)} = (1 - \text{the Number of surviving insects}/5) \times 100$$

Test Example 9-1

The test was conducted according to the Test method 9 by making the prescribed concentration 500 ppm and using the below-mentioned present compounds as a test compound, and, as the result of the test, the below-mentioned present compounds showed 80% or more as the mortality.
present compound Nos: 6, 12, 26, 28, 77, 79, 88, 89, 91, 99, 100, 101, 137

Test Method 10

Test compounds are made to a formulation according to a similar method to that described in the Formulation example 5, and thereto is added water to prepare a diluted solution containing a prescribed concentration of the test compound.

Cucumber (*cucumber sativus*) seedling (on the developmental stage of the second true leaf) is planted in a cup, and the diluted solutions are drenched to the foot of the seedling at a ratio of 5 mL/seedling. After 7 days, approximately 30 cotton aphids (*Aphis gossypii*) (all stages of life) are released onto the leaf of the seedling. After 6 days, the number of the surviving insects is examined and the controlling value is calculated by the following equation.

$$\text{Controlling value (\%)} = \{1 - (Cb \times Tai)/(Cai \times Tb)\} \times 100$$

wherein the symbols in the equation represent the following descriptions.

Cb: Number of the test insects in untreated group;
Cai: Number of the surviving insects at the time of the examination in untreated group;
Tb: Number of the test insects in treated group;
Tai: Number of the surviving insects at the time of the examination in treated group;
Here the "untreated group" represents a group where a similar treatment procedure to that of treated group except not using the test compound is done.

Test Example 10-1

The test was conducted according to the Test method 10 by making the prescribed concentration 200 ppm and using the below-mentioned present compounds as a test compound, and, as the result of the test, the below-mentioned present compounds showed 90% or more as the controlling value. Present compound Nos.: 14, 77

Test Method 11

Test compounds are made to a formulation according to a similar method to that described in the Formulation example 5, and thereto is added water to prepare a diluted solution containing a prescribed concentration of the test compound.

Five (5) mL of the diluted solution described above is added to a container, and therein is installed Rice (Oryza sativa) seedling (on the developmental stage of the second true leaf) that is planted in a container having a hole in the bottom. After 7 days, 20 3rd instar larvae of brown planthoppers (*Nilaparvata* lugens) are released. After 6 days, the number of the surviving insects is examined, and the mortality is calculated by the following equation,

$$\text{Mortality (\%)} = (1 - \text{the Number of surviving insects}/20) \times 100$$

Test Example 11-1

The test was conducted according to the Test method 11 by making the prescribed concentration 200 ppm and using the below-mentioned present compounds as a test compound, and, as the result of the test, the below-mentioned present compounds showed 90% or more as the mortality.
Present compound Nos.: 2, 28, 29, 79, 98, 135

Test Method 12

Test compounds are made to a formulation according to a similar method to that described in the Formulation example 5, and thereto is added water containing 0.03 v/v% of Shindain (registered trademark) to prepare a diluted solution containing a prescribed concentration of the test compound.

Cucumber (*Brassicae oloracea*) seedling (on the developmental stage of the second leaf) is planted in a cup, and the diluted solutions are sprayed into the seedling at a ratio of 10 mL/seedling. Thereafter, the second true leaf thereof is cut out and then is installed into a cup, and about twenty (20) instar larvae of Western flower thrips *(Frankliniella occidentalis*) are released. After 6 days, the number of the surviving insects is examined, and the mortality is calculated by the following equation.

$$\text{Mortality (\%)} = (1 - \text{the Number of surviving insects}/20) \times 100$$

Test Example 12-1

The test was conducted according to the Test method 12 by making the prescribed concentration 200 ppm and using the below-mentioned present compounds as a test compound, and, as the result of the test, the below-mentioned present compounds showed 90% or more as the mortality.
Present compound Nos.: 11, 42, 88, 98

Test. Method 13

An acetone solution which is adjusted to 2,000 ppm of the test compound is poured into a container having 20 mL contents, and the test compound is coated uniformly on inner face of the container such that the concentration of the test compound is made 100 $mg/m^2$, and the container is then allowed to dry.

Five (5) larvae of *Haemaphysalis longicornis* are placed in the container, and the container is then covered with the lid. After the prescribed time has been passed, the state of the *Haemaphysalis longicornis* is examined, and the mortality is obtained. The mortality is calculated by the following equation.

$$\text{Mortality (\%)} = (\text{the Number of dead insects}/\text{the Number of tested insects}) \times 100$$

Test Example 13-1

The test results obtained by the case when the test was conducted according to the Test Method 13 is shown below. The test was conducted by making the prescribed time two(2) days and by using the below-mentioned present compound as a test compound, and as the result of the test, the below-mentioned present compound showed 80 % or more as the mortality,
Present compound Nos.: 12, 15, 23, 35, 47, 79, 83, 91, 94, 118, 138

Test Method 14

An acetone solution which is adjusted to 200 ppm of the test compound is poured into a container having 20 mL contents, and the test compound is coated uniformly on inner face of the container such that the concentration of the test compound is made 10 mg/m$^2$, and the container is then allowed to dry.
Five (5) female adult of common house mosquito (Culex *pipiens pallens*) are placed in the container, and the container is then covered with the lid. After the prescribed time has been passed, the state of the common house mosquito is examined, and the mortality is obtained. The mortality is calculated by the following equation.

Mortality (%) = (the Number of dead insects/ the Number of tested insects) × 100

Test Example 14-1

The test results obtained by the case when the test was conducted according to the Test Method 14 is shown below. The test was conducted by making the prescribed time one (1) hour and by using the below-mentioned present compounds as a test compound, and as the result of the test, any of the below-mentioned present compounds showed 80 % or more as the mortality.
Present compound No: 83

Test Example 14-2

The test results obtained by the case when the test was conducted according to the Test Method 14 is shown below. The test was conducted by making the prescribed time one(1) day and by using the below-mentioned present compounds as a test compound, and as the result of the test, any of the below-mentioned present compounds showed 80 % or more as the mortality.
Present compound Nos: 12, 28, 49, 79, 83

Test. Method 15

An acetone solution which is adjusted to 800 ppm of the test compound is poured into a container having 20 mL contents, and the test compound is coated uniformly on inner face of the container such that the concentration of the test compound is made 40 mg/m$^2$, and the container is then allowed to dry.
Five (5) female adult housefly (*Musca domestica*) are placed in the container, and the container is then covered with the lid. After the prescribed time has been passed, the state of the housefly is examined, and the mortality is obtained. The mortality is calculated by the following equation.

Mortality (%) = (the Number of dead insects/ the Number of tested insects) × 100

Test Example 15-1

The test results obtained by the case when the test was conducted according to the Test Method 15 is shown below. The test was conducted by making the prescribed time one(1). day and by using the below-mentioned present compounds as a test compound, and as the result of the test, any of the below-mentioned present compounds showed 80 % or more as the mortality,

Present compound Nos: 19, 28, 72, 78, 79, 143, 145

Test Method 16

An acetone solution which is adjusted to 800 ppm of the test compound is poured into a container having 50 mL contents, and the test compound is coated uniformly on inner face of the container such that the concentration of the test compound is made 40 mg/m$^2$, and the container is then allowed to dry.

Five (5) male adult German cockroach (*Blattella germanica*) are placed in the container, and the container is then covered with the lid. After the prescribed time has been passed, the state of the housefly is examined, and the mortality is obtained. The mortality is calculated by the following equation.

Mortality (%) = (the Number of dead insects/ the Number of tested insects) × 100

Test Example 16-1

The test results obtained by the case when the test was conducted according to the Test Method 16 is shown below. The test was conducted by making the prescribed time three (3) days and by using the below-mentioned present compounds as a test compound, and as the result of the test, any of the below-mentioned present compounds showed 80 % or more as the mortality.

Present compound No: 56

Test Method 17

Each 1 mg of the present compounds is dissolved into 10 μL of a mixed solution of xylene, DMF, and a surfactant (xylene : DMF : surfactant = 4 : 4 : 1 (v/v ratio)). Thereto is added water containing 0.02% by volume of a spreader to prepare diluted solution A containing a prescribed concentration of the present compound.
Each 1 mg of the present ingredients is dissolved into 10 μL of a mixed solution of xylene, DMF, and a surfactant (xylene : DMF: surfactant = 4 : 4 : 1 (v/v ratio)). Thereto is added water containing 0.02% by volume of a spreader to prepare diluted solution B containing a prescribed concentration of the present ingredient.
The diluted solution A is mixed with the diluted solution B to prepare diluted solution C.
Leaf discs of Cucumber (*cucumber sativus*) cotyledon (length 1.5 cm) are placed in each well of 24-well microplate. Two (2) apterous adults and 8 larvae of cotton aphids (Aphis *gossypii*) per one well are released and the diluted solution C is sprayed at 20 μL per one well. The group is defined as "treated group". A well that is sprayed with 20 μL of water containing 0.02% by volume of a spreader instead of the diluted solution C is defined as "untreated group".
After drying the diluted solution C, the upper microplate is covered with a film sheet. After 5 days, the number of the surviving insects in each well is examined.
The controlling value is calculated by the following equation.

Controlling value (%) = {1 - (Tai)/(Cai)} × 100

wherein the symbols in the equation represent the following descriptions.

Cai: Number of the surviving insects at the time of the examination in untreated group;
Tai: Number of the surviving insects at the time of the examination in treated group.

Specific diluted solutions C, which can confirm their effect according to the Test method 17, are described in the following 1) to 5).
1) The diluted solution C comprises the combination recited in List A wherein a concentration of the present compound is 200 ppm and a concentration of the present ingredient is 2,000 ppm. In List A, Comp X represents any compound selected from the present compounds 1 to 136.
List A:

Comp X + Clothianidin; Comp X + thiamethoxam; Comp X + imidacloprid; Comp X + thiacloprid;

Comp X + flupyradifurone; Comp X + sulfoxaflor; Comp X + triflumezopyrim; Comp. X + dicloromezotiaz; Comp X + beta-cyfluthrin; Comp X + tefluthrin; Comp X + fipronil; Comp X + chlorantraniliprole; Comp X + cyantraniliprole; Comp X + tetraniliprole; Comp X + thiodicarb; Comp X + carbofuran; Comp X + fluxametamide; Comp X + afoxolaner; Comp X + fluralaner; Comp X + broflanilide; Comp X + abamectin; Comp X + fluopyram; Comp

X + fluensulfone; Comp X + fluazaindolizine; Comp X + tioxazafen; Comp X + flupyrimin; Comp X + Mycorrhizal Fungi; Comp X + Bradyrhizobium japonicum TA-11; Comp X + Bacillus firmus; Comp X + Bacillus firmus I-1582; Comp X + Bacillus amyloliquefaciens; Comp X + Bacillus amyloliquefaciens FZB42; Comp X + Pasteuria nishizawae; Comp X + Pasteuria nishizawae Pn3.; Comp X + Pasteuria penetrans; Comp X + tebuconazole; Comp X + prothioconazole; Comp X + metconazole; Comp X + ipconazole; Comp X + triticonazole; Comp X + difenoconazole; Comp X + imazalil; Comp X + triadimenol; Comp X + tetraconazole; Comp X + flutriafol; Comp X + mandestrobin; Comp X + azoxystrobin; Comp X + pyraclostrobin; Comp X + trifloxystrobin; Comp X + fluoxastrobin; Comp X + picoxystrobin; Comp X + fenamidone; Comp X + metalaxyl; Comp X + metalaxyl-M; Comp X + fludioxonil;. Comp X + sedaxane; Comp X + penflufen; Comp X + fluxapyroxad; Comp X + benzovindiflupyr; Comp X + boacalid; Comp X + carboxin; Comp X + penthiopyrad; Comp X + flutolanil; Comp X + captan; Comp X + thiram; Comp X + tolclof os -methyl; Comp X + thiabendazole; Comp X + ethaboxam; Comp X + mancozeb; Comp X + picazbutrazoat; Comp X + oxathiapiprolin; Comp X + silthiofam: Comp X + inpyrfluxam.

2) The diluted solution C comprises the combination recited in List A wherein a concentration of the present compound is 200. ppm, and a concentration of the present ingredient is 200 ppm.
3) The diluted solution C comprises the combination recited in List A wherein a concentration of the present compound is 500 ppm, and a concentration of the present ingredient is 50 ppm.
4) The diluted solution C comprises the combination recited in List A wherein a concentration of the present compound is 500 ppm, and a concentration of the present ingredient is 5 ppm.
5) The diluted solution C comprises the combination recited in List A wherein a concentration of the present compound is 500 ppm, and a concentration of the present ingredient is 0.5 ppm.

Industrial Applicability

The compound of the present invention has excellent control effect on harmful arthropods.

## Claims

**1.** A compound represented by formula (I):

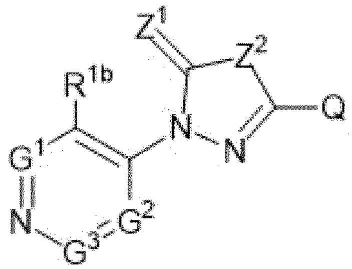

(I)

[wherein

$Z^1$ and $Z^2$ are identical or different from each other and each represents an oxygen atom or a sulfur atom,
a combination of $G^1$, $G^2$, and $G^3$ represents
a combination wherein $G^1$ represents a nitrogen atom, $G^2$ represents $CR^{1C}$, and $G^3$ represents $CR^{1d}$;
a combination wherein $G^1$ represents $CR^{1a}$, $G^2$ represents a nitrogen atom or $CR^{1C}$, $G^3$ represents $CR^{1d}$; or
a combination wherein $G^1$ represents $CR^{1a}$, $G^2$ represents $CR^{1C}$, and $G^3$ represents a nitrogen atom,
$R^{1b}$ represents a C1-C6 chain hydrocarbon group, a C1-C6 alkoxy group {the C1-C6 chain hydrocarbon group and the C1-C6 alkoxy group may be optionally substituted with one or more substituents selected from Group A}, a C3-C7 cycloalkyl group, a C1-C6 alkylsulfanyl group, a C1-C6 alkylsulfinyl group, a C1-C6 alkylsulfonyl group {the C3-C7 cycloalkyl group, the C1-C6 alkylsulfanyl group, the C1-C6 alkylsulfinyl group and the C1-C6 alkylsulfonyl group may be optionally substituted with one or more substituents selected from Group B}, a C3-C7 cycloalkenyl group which may be optionally substituted with one or more substituents selected from Group C, a phenyl group, a three- to seven-membered nonaromatic heterocyclic group {the phenyl group and the three- to seven- membered nonaromatic heterocyclic group may be optionally substituted with one or more substituents selected from Group D, a nitro group, $NR^2R^3$ , or a halogen atom,
$R^{1c}$ represents a C1-C6 chain hydrocarbon group, a C1-C6 alkoxy group {the C1-C6 chain hydrocarbon group

and the C1-C6 alkoxy group may be optionally substituted with one or more substituents selected from Group A}, a C3-C7 cycloalkyl group, a C1-C6 alkylsulfanyl group, a C1-C6 alkylsulfinyl group, a C1-C6 alkylsulfonyl group {the C3-C7 cycloalkyl group, the C1-C6 alkylsulfanyl group, the C1-C6 alkylsulfinyl group and the C1-C6 alkylsulfonyl group may be optionally substituted with one or more substituents selected from Group B}, a C3-C7 cycloalkenyl group which may be optionally substituted with one or more substituents selected from Group C, a phenyl group, a three- to seven-membered nonaromatic heterocyclic group {the phenyl group and the three- to seven- membered nonaromatic heterocyclic group may be optionally substituted with one or more substituents selected from Group D}, a nitro group, $NR^2R^3$, a halogen atom, or a hydrogen atom,

$R^{1a}$ and $R^{1d}$ are identical or different from each other and each represents a C1-C6 chain hydrocarbon group which may be optionally substituted with one or more halogen atoms, a halogen atom, or a hydrogen atom,

Q represents a C6-C10 aryl group which may be optionally substituted with one or more substituents selected from Group H, or a five- to ten- membered aromatic heterocyclic group which may be optionally substituted with one or more substituents selected from Group I,

$R^2$, $R^{2a}$ and $R^5$ are identical or different from each other and each represents a C1-C6 chain hydrocarbon group which may be optionally substituted with one or more halogen atoms, or a hydrogen atom,

$R^3$ and $R^{3a}$ are identical or different from each other and each represents a C1-C6 chain hydrocarbon group which may be optionally substituted with one or more substituents selected from Group E, a C3-C7 cycloalkyl group, a C3-C7 cycloalkenyl group {the C3-C7 cycloalkyl group and the C3-C7 cycloalkenyl group may be optionally substituted with one or more substituents selected from Group C}, a phenyl group, a five- or six-membered aromatic heterocyclic group {the phenyl group and the five- or six- membered aromatic heterocyclic group may be optionally substituted with one or more substituents selected from Group F}, $S(O)_2R^{13}$, or a hydrogen atom,

$R^{13}$ represents a C1-C6 chain hydrocarbon group which may be optionally substituted with one or more halogen atoms, or a phenyl group which may be optionally substituted with one or more substituents selected from Group F.

Group A is a group consisting of a C1-C6 alkoxy group, a C3-C6 alkenyloxy group, a C3-C6 alkynyloxy group, a C1-C6 alkylsulfanyl group, a C1-C6 alkylsulfinyl group, a C1-C6 alkylsulfonyl group, a C3-C6 cycloalkyl group {the C1-C6 alkoxy group, the C3-C6 alkenyloxy group, the C3-C6 alkynyloxy group, the C1-C6 alkylsulfanyl group, the C1-C6 alkylsulfinyl group, the C1-C6 alkylsulfonyl group and the C3-C6 cycloalkyl group may be optionally substituted with one or more halogen atoms}, a hydroxy group, a cyano group and a halogen atom.

Group B is a group consisting of a C1-C6 chain hydrocarbon group, a C1-C6 alkoxy group, a C3-C6 alkenyloxy group, a C3-C6 alkynyloxy group {the C1-C6 chain hydrocarbon group, the C1-C6 alkoxy group, the C3-C6 alkenyloxy group and the C3-C6 alkynyloxy group may be optionally substituted with one or more halogen atoms}, a hydroxy group, a cyano group, a nitro group and a halogen atom.

Group C is a group consisting of a C1-C6 alkyl group which may be optionally substituted with one or more halogen atoms, a cyano group and a halogen atom.

Group D is a group consisting of a C1-C6 chain hydrocarbon group which may be optionally substituted with one or more halogen atoms, a five- or six- membered aromatic heterocyclic group which may be optionally substituted with one or more substituents selected from Group F, $OR^{17}$, $NR^{16}R^{17}$, C (0) $R^{17}$, C (0) $NR^{16}R^{17}$, OC (O) $R^{16a}$, OC (O) $OR^{16a}$, $NR^{17}C$ (O) $R^{16a}$, $NR^{17}C$ (O) $OR^{16a}$, $C(O)OR^{17}$, a cyano group, a nitro group and a halogen atom.

$R^{16a}$ represents a C1-C6 alkyl group, or a C3-C6 cycloalkyl group {the C1-C6 alkyl group and the C3-C6 cycloalkyl group may be optionally substituted with one or more halogen atoms},

$R^{16}$ and $R^{17}$ are identical or different from each other and each represents a C1-C6 alkyl group, a C3-C6 cycloalkyl group {the C1-C6 alkyl group and the C3-C6 cycloalkyl group may be optionally substituted with one or more halogen atoms}, or a hydrogen atom.

Group E is a group consisting of a C1-C6 alkoxy group, a C3-C7 cycloalkyl group {the C1-C6 alkoxy group and the C3-C7 cycloalkyl group may be optionally substituted one or more halogen atoms}, a phenyl group, a five- or six- membered aromatic heterocyclic group {the phenyl group and the five- or six- membered aromatic heterocyclic group may be optionally substituted with one or more substituents selected from Group F}, a three- to seven- membered nonaromatic heterocyclic group which may be optionally substituted with one or more substituents selected from Group G, $NHR^{14}$, $NR^{14}R^{15}$, an amino group, a cyano group and a halogen atom.

$R^{14}$ and $R^{15}$ are identical or different from each other, and each represents a C1-C6 alkyl group which may be optionally substituted with one or more halogen atoms.

Group F is a group consisting of a C1-C6 chain hydrocarbon group, a C1-C6 alkoxy group, a C3-C6 alkenyloxy group, a C3-C6 alkynyloxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C3-C6 alkenyloxy group, the C3-C6 alkynyloxy group, the C1-C6 alkylsulfanyl group, the C1-C6 alkylsulfinyl group and the C1-C6 alkylsulfonyl group

may be optionally substituted with one or more halogen atoms}, $NHR^{14}$, $NR^{14}R^{15}$, $C(O)R^{14}$, $OC(O)R^{14}$, $C(O)OR^{14}$, a hydroxy group, a sulfanyl group, an amino group, a cyano group, a nitro group and a halogen atom. Group G is a group consisting of a C1-C6 chain hydrocarbon group, a C1-C6 alkoxy group, a C3-C6 alkenyloxy group, a C3-C6 alkynyloxy group {the C1-C6 chain hydrocarbon group, the C1-C6 alkoxy group, the C3-C6 alkenyloxy group and the C3-C6 alkynyloxy group may be optionally substituted with one or more halogen atoms}, and a halogen atom.

Group H is a group consisting of a C1-C6 chain hydrocarbon group which may be optionally substituted with one or more substituents selected from Group A, a C3-C7 cycloalkyl group which may be optionally substituted with one or more substituents selected from Group B, a C3-C7 cycloalkenyl group which may be optionally substituted with one or more substituents selected from Group C, a phenyl group which may be optionally substituted with one or more substituents selected from Group D, a five- or six- membered aromatic heterocyclic group which may be optionally substituted with one or more substituents selected from Group D, $OR^{3a}$, $NR^{2a}R^{3a}$, $NR^{2b}R^{3b}$, $NR^5NR^{2a}R^{3a}$, $NR^5OR^{2a}$, $NR^{2a}C(O)R^{6a}$, $NR^5NR^{2a}C(O)R^{6a}$, $NR^5NR^{2a}C(O)OR^7$, $NR^{2a}C(O)NR^8R^9$, $NR^5NR^{2a}C(O)NR^{10}R^{11}$, $N{=}CHNR^{10}R^{11}$, $N{=}S(O)_pR^8R^9$, $C(O)R^{12}$, $C(O)NR^{10}R^{11}$, $C(O)NR^{2a}S(O)_2R^{13}$, $CR^5{=}NOR^{12}$, $NR^{2a}CR^5{=}NOR^{12}$, $S(O)_mR^{13}$, a cyano group, a nitro group and a halogen atom.

p is 0 or 1,

m is 0, 1 or 2,

$R^{2b}$ and $R^{3b}$ combine together with a nitrogen atom to which they are bounded to form a three- to seven-membered nonaromatic heterocyclic group which may be optionally substituted with one or more substituents selected from Group B,

$R^6$ represents a C1-C6 chain hydrocarbon group, a C3-C7 cycloalkyl group, a (C3-C6 cycloalkyl)C1-C3 alkyl group {the C1-C6 chain hydrocarbon group, the C3-C7 cycloalkyl group and the (C3-C6 cycloalkyl) C1-C3 alkyl group may be optionally substituted with one or more halogen atoms}, a phenyl group, a five- or six- membered aromatic heterocyclic group {the phenyl group and the five- or six- membered aromatic heterocyclic group may be optionally substituted with one or more substituents selected from Group F}, or a hydrogen atom.

$R^{6a}$ represents a C1-C6 chain hydrocarbon group, a C3-C7 cycloalkyl group, a (C3-C6 cycloalkyl)C1-C3 alkyl group {the C1-C6 chain hydrocarbon group, the C3-C7 cycloalkyl group and the (C3-C6 cycloalkyl) C1-C3 alkyl group may be optionally substituted with one or more halogen atoms}, a phenyl group, or a five- or six- membered aromatic heterocyclic group {the phenyl group and the five- or six- membered aromatic heterocyclic group may be optionally substituted with one or more substituents selected from Group F},

$R^7$ represents a C1-C6 chain hydrocarbon group, a C3-C7 cycloalkyl group, a (C3-C6 cycloalkyl)C1-C3 alkyl group {the C1-C6 chain hydrocarbon group, the C3-C7 cycloalkyl group and the (C3-C6 cycloalkyl) C1-C3 alkyl group may be optionally substituted with one or more halogen atoms}, or a phenyl C1-C3 alkyl group {the phenyl moiety in the phenyl C1-C3 alkyl group may be optionally substituted with one or more substituents selected from Group F},

$R^8$ and $R^9$ are identical or different from each other and each represents a C1-C6 alkyl group which may be optionally substituted with one or more halogen atoms,

$R^{10}$ represents a C1-C6 alkyl group which may be optionally substituted with one or more halogen atoms, or a hydrogen atom,

$R^{11}$ represents a C1-C6 chain hydrocarbon group which may be optionally substituted with one or more substituents selected from Group B, or a C3-C7 cycloalkyl group which may be optionally substituted with one or more substituents selected from Group G, $S(O)_2R^{6a}$, or a hydrogen atom,

$R^{12}$ represents a C1-C6 chain hydrocarbon group which may be optionally substituted with one or more halogen atoms, a phenyl group which may be optionally substituted with one or more substituents selected from Group F, or a hydrogen atom.

Group I is a group consisting of a C1-C6 chain hydrocarbon group which may be optionally substituted with one or more substituents selected from Group A, a C3-C7 cycloalkyl group which may be optionally substituted with one or more substituents selected from Group B, a C3-C7 cycloalkenyl group which may be optionally substituted with one or more substituents selected from Group C, $OR^{3a}$, $NR^{2a}R^{3a}$, $NR^{2b}R^{3b}$, $NR^5NR^{2a}R^{3a}$, $NR^5OR^{2a}$, $NR^{2a}C(O)R^{6a}$, $NR^5NR^{2a}C(0)R^{6a}$, $NR^5NR^{2a}C(O)OR^7$, $NR^{2a}C(O)NR^8R^9$, $NR^5NR^{2a}C(O)NR^{10}R^{11}$, $N{=}CHNR^{10}R^{11}$, $N{=}S(O)_pR^8R^9$, $C(O)R^{12}$, $C(O)NR^{10}R^{11}$, $C(O)NR^{2a}S(O)_2R^{13}$, $CR^5{=}NOR^{12}$, $NR^{2a}CR^5{=}NOR^{12}$, $S(O)_mR^{13}$, a cyano group, a nitro group and a halogen atom.]

, or its N-oxide or a salt thereof.

**2.** The compound according to claim 1 wherein

$Z^2$ represents an oxygen atom,

Q represents a phenyl group, a naphthyl group {the phenyl group and the naphthyl group may be optionally

substituted with one or more substituents selected from Group J}, a furanyl group, a thienyl group, a pyrazolyl group, an imidazolyl group, an oxazolyl group, a thiazolyl group, an isoxazolyl group, a pyridyl group, a pyridazinyl group, a pyrimidinyl group, a pyrazinyl group, a quinolyl group, an imidazopyridyl group, a benzofuranyl group, or a benzothiophenyl group {the furanyl group, the thienyl group, the pyrazolyl group, the imidazolyl group, the oxazolyl group, the thiazolyl group, the isoxazolyl group, the pyridyl group, the pyridazinyl group, the pyrimidinyl group, the pyrazinyl group, the quinolyl group, the imidazopyridyl group, the benzofuranyl group and the benzothiophenyl group may be optionally substituted with one or more substituents selected from Group K},

Group J is a group consisting of a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, a phenyl group which may be optionally substituted with one or more halogen atoms, a five- or six-membered aromatic heterocyclic group which may be optionally substituted with one or more halogen atoms, $OR^{3a}$, $NR^{2a}R^{3a}$, $NR^{2b}R^{3b}$, $NR^5NR^{2a}R^{3a}$, $NR^5OR^{2a}$, $NR^{2a}C(O)R^{6a}$, $NR^5NR^{2a}C\ (O)\ R^{6a}$, $NR^5NR^{2a}C\ (O)\ OR^7$, $NR^{2a}C\ (O)\ NR^8R^9$, $NR^5NR^{2a}C\ (0)\ NR^{10}R^{11}$, $N{=}CHNR^{10}R^{11}$, $N{=}S\ (O)\ _pR^8R^9$, $C(O)R^{12}$, $C\ (O)\ NR^{10}R^{11}$, $C\ (O)\ NR^{2a}S\ (O)\ _2R^{13}$, $CR^5{=}NOR^{12}$, $NR^{2a}CR^5{=}NOR^{12}$, $S\ (O)_mR^{13}$, a cyano group, a nitro group and a halogen atom.

Group K is a group consisting of a C1-C6 chain hydrocarbon group which may be optionally substituted with one or more halogen atoms, a C3-C7 cycloalkyl group which may be optionally substituted with one or more halogen atoms, a C3-C7 cycloalkenyl group which may be optionally substituted with one or more halogen atoms, $OR^{3a}$, $NR^{2a}R^{3a}$, $NR^{2b}R^{3b}$, $NR^5NR^{2a}R^{3a}$, $NR^5OR^{2a}$, $NR^{2a}C\ (O)\ R^{6a}$, $NR^5NR^{2a}C\ (0)\ R^{6a}$, $NR^5NR^{2a}C\ (O)\ OR^7$, $NR^{2a}C\ (O)\ NR^8R^9$, $NR^5NR^{2a}C(O)NR^{10}R^{11}$, $N{=}CHNR^{10}R^{11}$, $N{=}S(O)_pR^8R^9$, $C(O)R^{12}$, $C(O)NR^{10}R^{11}$, $C\ (O)\ NR^{2a}S\ (O)\ _2R^{13}$, $CR^5{=}NOR^{12}$, $NR^{2a}CR^5{=}NOR^{12}$, $S\ (O)\ _mR^{13}$, a cyano group, a nitro group and a halogen atom,

or its N-oxide or a salt thereof.

**3.** The compound according to claim 1 or claim 2 wherein $G^1$ represents $CR^{1a}$, $G^2$ represents a nitrogen atom or $CR^{1C}$, and $G^3$ represents $CR^{1d}$, or its N-oxide or a salt thereof.

**4.** The compound according to any one of claims 1 to 3 wherein

$R^{1b}$ represents a C1-C6 chain hydrocarbon group which may be optionally substituted with one or more halogen atoms, a C3-C7 cycloalkyl group which may be optionally substituted with one or more halogen atoms, or a halogen atom, and $R^{1c}$ represents a C1-C6 chain hydrocarbon group which may be optionally substituted with one or more halogen atoms, a C3-C7 cycloalkyl group which may be optionally substituted with one or more halogen atoms, a halogen atom, or a hydrogen atom,

or its N-oxide or a salt thereof.

**5.** The compound according to any one of claims 1 to 4 wherein

Q represents a phenyl group, a naphthyl group {the phenyl group and the naphthyl group may be optionally substituted with one or more substituents selected from Group L}, a pyridyl group, or a quinolyl group {the pyridyl group and the quinolyl group may be optionally substituted with one or more substituents selected from Group M},

Group L is a group consisting of a C1-C6 chain hydrocarbon group which may be optionally substituted with one or more halogen atoms, a C3-C7 cycloalkyl group which may be optionally substituted with one or more halogen atoms, a phenyl group which may be optionally substituted with one or more halogen atoms, $OR^{19}$, $S(O)_mR^{19}$, and a halogen atom,

Group M is a group consisting of a C1-C6 chain hydrocarbon group which may be optionally substituted with one or more halogen atoms, a C3-C7 cycloalkyl group which may be optionally substituted with one or more halogen atoms, $OR^{19}$, $S(O)_mR^{19}$, and a halogen atom,

$R^{19}$ represents a C1-C3 chain hydrocarbon group which may be optionally substituted with one or more halogen atoms, or its N-oxide or a salt thereof.

**6.** The compound according to any one of claims 1 to 5 wherein

Q represents a group represented by Q1:

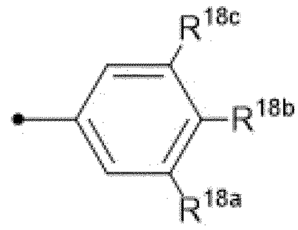

Q1

[wherein,

$R^{18a}$, $R^{18b}$, and $R^{18c}$ are identical or different from each other and each represents a C1-C6 chain hydrocarbon group which may be optionally substituted with one or more halogen atoms, a C3-C7 cycloalkyl group which may be optionally substituted with one or more halogen atoms, a phenyl group which may be optionally substituted with one or more halogen atoms, $OR^{19}$, $S(O)_mR^{19}$, a halogen atom, or a hydrogen atom, and
$R^{19}$ represents a C1-C3 chain hydrocarbon group which may be optionally substituted with one or more halogen atoms] , its N-oxide or a salt thereof.

**7.** The compound according to any one of claims 1 to 6 wherein $Z^1$ and $Z^2$ represent an oxygen atom, or its N-oxide or a salt thereof.

**8.** A composition for controlling harmful arthropod comprising the compound according to any one of claims 1 to 7, or its N-oxide or a salt thereof.

**9.** A composition which comprises one or more ingredients selected from the group consisting of Group (a), Group (b), Group (c) and Group (d), as well as the compound according to any one of claims 1 to 7, or its N-oxide or a salt thereof:

Group (a): a group consisting of an insecticidal ingredient, a miticidal ingredient, and a nematicidal ingredient;
Group (b): a fungicidal ingredient;
Group (c): a plant growth modulating ingredient; and
Group (d): a repellent ingredient.

**10.** A method for controlling a harmful arthropod which comprises applying an effective amount of the compound according to any one of claims 1 to 7, or its N-oxide or a salt thereof, or an effective amount of the composition according to claim 9 to a harmful arthropod or a habitat where a harmful arthropod lives.

**11.** A seed or vegetative reproductive organ carrying an effective amount of the compound according to any one of claims 1 to 7, or its N-oxide or a salt thereof, or an effective amount of the composition according to claim 9.

**12.** A compound represented by formula (II):

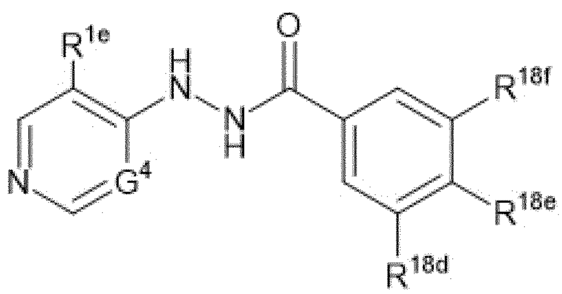

(II)

[wherein,

$G^4$ represents $CR^{1f}$ or a nitrogen atom,

$R^{1e}$ represents a C1-C6 chain hydrocarbon group which may be optionally substituted with one or more halogen atoms, a C3-C7 cycloalkyl group which may be optionally substituted with one or more halogen atoms, or a halogen atom,
$R^{1f}$ represents a C1-C6 chain hydrocarbon group which may be optionally substituted with one or more halogen atoms, a C3-C7 cycloalkyl group which may be optionally substituted with one or more halogen atoms, a halogen atom, or a hydrogen atom,
$R^{18d}$ and $R^{18e}$ are identical or different from each other and each represents a C1-C6 chain hydrocarbon group which may be optionally substituted with one or more halogen atoms, a C3-C7 cycloalkyl group which may be optionally substituted with one or more halogen atoms, a phenyl group which may be optionally substituted with one or more halogen atoms, $OR^{19a}$, $S(O)_nR^{19a}$, a halogen atom, or a hydrogen atom,
$R^{18f}$ represents a C1-C6 chain hydrocarbon group which may be optionally substituted with one or more halogen atoms, a C3-C7 cycloalkyl group which may be optionally substituted with one or more halogen atoms, a phenyl group which may be optionally substituted with one or more halogen atoms, $OR^{19}$, $S(O)_nR^{19a}$, or a halogen atom,
$R^{19a}$ represents a C1-C3 chain hydrocarbon group which may be optionally substituted with one or more halogen atoms, and
n is 0, 1 or 2.

## INTERNATIONAL SEARCH REPORT

International application No.
**PCT/JP2022/025756**

**A. CLASSIFICATION OF SUBJECT MATTER**

***C07D 413/04***(2006.01)i; ***C07D 413/14***(2006.01)i; ***A01P 7/02***(2006.01)i; ***A01P 7/04***(2006.01)i; ***A01N 43/824***(2006.01)i
FI: C07D413/04 CSP; A01P7/02; A01P7/04; A01N43/824 B; C07D413/14

According to International Patent Classification (IPC) or to both national classification and IPC

**B. FIELDS SEARCHED**

Minimum documentation searched (classification system followed by classification symbols)

C07D413/04; C07D413/14; A01P7/02; A01P7/04; A01N43/824

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 97/08158 A1 (BAYER AKTIENGESELLSCHAFT) 06 March 1997 (1997-03-06) entire text | 1-12 |
| A | JP 3-236379 A (SUMITOMO CHEMICAL COMPANY, LIMITED) 22 October 1991 (1991-10-22) entire text | 1-12 |
| A | CN 109232550 A (QINGDAO UNIVERSITY OF SCIENCE & TECHNOLOGY) 18 January 2019 (2019-01-18) entire text | 1-12 |
| A | JP 2016-520522 A (MONSANTO TECHNOLOGY LLC) 14 July 2016 (2016-07-14) entire text | 1-12 |
| A | JP 2011-1347 A (SUMITOMO CHEMICAL COMPANY, LIMITED) 06 January 2011 (2011-01-06) entire text | 1-12 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 September 2022** | **13 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/025756**

| Patent document cited in search report | Publication date (day/month/year) | Patent family member(s) | Publication date (day/month/year) |
| --- | --- | --- | --- |
| WO 97/08158 A1 | 06 March 1997 | JP 11-513026 A<br>US 6013804 A<br>EP 854871 A1<br>KR 10-1999-0044131 A | |
| JP 3-236379 A | 22 October 1991 | US 5124341 A<br>entire text<br>EP 435616 A2<br>KR 10-1991-0011811 A | |
| CN 109232550 A | 18 January 2019 | (Family: none) | |
| JP 2016-520522 A | 14 July 2016 | US 2014/0274690 A1<br>entire text<br>WO 2014/152132 A1<br>EP 2967067 A1<br>CN 105163586 A | |
| JP 2011-1347 A | 06 January 2011 | US 2012/0041009 A1<br>entire text<br>WO 2010/134478 A1<br>EP 2432780 A1<br>CN 102428082 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2021107327 A
- NO 2006044552
- US 20180009778 A1
- WO 2016121970 A1